(19) Europäisches Patentamt
European Patent Office
Office européen des brevets

(11) EP 0 293 567 B1

(12) # EUROPÄISCHE PATENTSCHRIFT

(45) Veröffentlichungstag und Bekanntmachung des Hinweises auf die Patenterteilung:
**31.01.1996 Patentblatt 1996/05**

(21) Anmeldenummer: **88104916.7**

(22) Anmeldetag: **26.03.1988**

(51) Int Cl.$^6$: **C12N 15/12**, C07K 14/47, C12N 1/21, C12N 5/10, A61K 38/17, C12N 5/20, C07K 16/18, G01N 33/577

(54) **Vascular-antikoagulierende Proteine, DNA die diese kodieren, Verfahren zu deren Herstellung sowie deren Verwendung**

Vascular anticoagulating proteins, DNA encoding them, process for their production and their use

Protéines vasculaires anticoagulantes, ADN les codant, leur procédé de production et leur utilisation

(84) Benannte Vertragsstaaten:
**AT BE CH DE ES FR GB GR IT LI LU NL SE**

(30) Priorität: **28.03.1987 DE 3710309**
**28.03.1987 DE 3710430**
**28.03.1987 DE 3710364**
**04.11.1987 DE 3737367**

(43) Veröffentlichungstag der Anmeldung:
**07.12.1988 Patentblatt 1988/49**

(73) Patentinhaber:
**BOEHRINGER INGELHEIM INTERNATIONAL GmbH**
**D-55216 Ingelheim am Rhein (DE)**

(72) Erfinder:
- **Hauptmann, Rudolf, Dr.**
  **A-2483 Ebreichsdorf (AT)**
- **Maurer-Fogy, Ingrid, Dr.**
  **A-1238 Wien (AT)**
- **Bodo, Gerhard, Prof. Dr.**
  **A-1120 Wien (AT)**
- **Swetly, Peter, Prof. Dr.**
  **A-1130 Wien (AT)**
- **Stratowa, Christian, Dr.**
  **A-1010 Wien (AT)**
- **Falkner, Edgar, Dipl.-Ing. Dr.**
  **A-1120 Wien (AT)**
- **Adolf, Günther, Dr.**
  **A-1070 Wien (AT)**
- **Reutelingsperger, Christian Maria Peter, Dr.**
  **NL-6211 JK Maastricht (NL)**

(56) Entgegenhaltungen:
**EP-A- 0 181 465**

- **NATURE, Band 320, Nr. 6057, 6. März 1986, New York, London B.P. Wallner et al.; Seiten 77-81**
- **CELL, Band 46, Nr. 2, 18. Juli 1986, Cabridge, Mass., USA K.-S.HUANGet al.; Seiten 191-199**
- **CELL; Band 46, Nr. 2, 18. Juli 1986, Cabridge, Mass., USA C.J.M.SARIS et al.; Seiten 201-212**
- **ANNUAL REVIEW BIOCHEMISTRY, Band 52, 1983, Standford, USA J.TRAVIS et al.; Seiten 655-709**
- **ANNUAL REVIEW BIOCHEMISTRY, Band 49, 1980, Standford, USA C.M.JACKSON et al.; Seiten 765-811**
- **THE JOURNAL OF CLINICAL INVESTIGATION, Band 74, 1984, New York R.D.ROSENBERG et al.; Seiten 1-6**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Gegenstand der vorliegenden Erfindung sind biologisch aktive Proteine, die für diese kodierenden DNA-Moleküle, Verfahren zu deren Herstellung sowie deren Verwendung.

In den meisten Säugetieren existieren Proteine, die blutgerinnungshemmende Eigenschaften aufweisen. Diese Proteine können in drei Gruppen eingeteilt werden, wobei die Einteilung auf den unterschiedlichen Wirkmechanismen beruht.

1. Proteine, die mit dem Gerinnungsfaktor einen Komplex bilden und dadurch den Gerinnungsfaktor unwirksam machen. Dazu gehören die Proteine:

a) Antithrombin-III (Thromb.Res.5, 439-452 (1974))
b) alpha$_1$-Protease Inhibitor (Ann. Rev. Biochem. 52, 655-709 (1983))
c) alpha$_2$-Makroglobulin (Ann. Rev. Biochem.. 52, 655-709 (1983))
d) $C_1$-Inhibitor (Biochemistry 20, 2738-2743 (1981))
e) Protease Nexin (J. Biol. Chem. 258, 10439-10444, (1983)).

2. Proteine, die einen Gerinnungsfaktor proteolytisch zerschneiden und dadurch den Gerinnungsfaktor desaktivieren. Als einziges Protein dieser Art ist bisher Protein C beschrieben (J. Biol. Chem. 251, 355-363, (1976)).

3. Proteine, die die negativ geladenen Phospholipide abschirmen und/oder hydrolisieren, so daß die Phospholipid-abhängigen Reaktionen des Blutgerinnungsmechanismus gehemmt werden. Bisher sind nur Phospholipasen, die aus verschiedenen Schlangengiftsorten isoliert wurden, beschrieben worden (Eur. J. Biochem. 112, 25-32 (1980)).

Das stufenweise ablaufende Gerinnungssystem ist in den letzten Jahren intensiv untersucht worden. Es wird als ein sich verstärkendes Mehrstufensystem verschiedener miteinander verbundener, proteolytischer Reaktionen verstanden, bei dem ein Enzym ein Zymogen in die aktive Form umsetzt (vgl. Jackson C.M., Nemerson Y., Ann. Rev. Biochem. 49, 765-811, (1980)). Die Geschwindigkeit dieser Reaktion wird in entscheidender Weise durch die Anwesenheit von Phospholipiden und anderer Kofaktoren wie Faktor $V_a$ und Faktor $VIII_a$ vergrößert. In vivo werden die Prokoagulationsreaktionen durch verschiedene Inhibitationsmechanismen geregelt, die einem explosiv thrombotischen Trauma nach einer geringen Aktivierung der Gerinnungskaskade vorbeugen.

Die Antigerinnungsmechanismen können wie folgt eingeteilt werden (Rosenberg, R.D., Rosenberg, J.S., J. Clin. Invest. 74, 1-6 (1984)):

1. Der Serin-Protease-Faktor $X_a$ und Thrombin werden infolge ihrer Bindung an Antithrombin III bzw. an den Antithrombin/Heparin-Komplex inaktiviert. Sowohl die Prothrombin-Aktivierung als auch die Bildung von Fibrin kann auf diese Weise gehemmt werden. Neben Anti-thrombin III gibt es noch verschiedene andere Plasma-Protease-Inhibitoren wie beispielsweise alpha$_2$-Makroglobulin und Antitrypsin, deren Wirkung zeitabhängig ist.

2. Die Entdeckung des Protein C's führte zur Aufdeckung eines weiteren Antigerinnungsmechanismus. Ist das Protein C einmal aktiviert worden, wirkt es durch die selektive Proteolyse der Proteinkofaktoren Faktor $V_a$ und $VIII_a$, durch die die Prothrombinase und das Enzym, das den Faktor X umsetzt, inaktiviert werden, als Antikoagulanz.

3. Plasmin spaltet monomeres Fibrin 1, ein Produkt der Einwirkung von Thrombin auf Fibrinogen, wodurch der Bildung eines unlöslichen Fibrins vorgebeugt wird (Nosssel, H.L., Nature, 291, 165-167 (1981)).

Von den oben genannten, in den Gerinnungsprozeß eingreifenden körpereigenen Proteinen ist z.Zt. nur das Antithrombin III in klinischem Einsatz. Als erheblicher Nachteil hat sich jedoch die bei der Anwendung dieses Proteins auftretende Erhöhung der Blutungsneigung herausgestellt.

Alle bisher als Antikoagulantien eingesetzten Mittel, ob körpereigener oder synthetischer Natur machen in irgendeiner Weise die Gerinnungsfaktoren unwirksam und führen dadurch zu Nebenwirkungen, die sich nachteilig auf den Gerinnungsprozeß auswirken können.

Überraschenderweise konnten nun neben diesen Proteinen weitere körpereigene Stoffe isoliert werden, die zwar die gewünschten blutgerinnungshemmenden Eigenschaften unter besonderen Bedingungen zeigen, hierbei aber die Blutungsgefahr nicht erhöhen. Bei größeren Blutungen verlieren diese Proteine ihre blutgerinnungshemmenden Eigenschaften und stören dadurch bei deren Verwendung nicht die in solchen Fällen überlebensnotwendigen Gerinnungsprozesse. Da sie erstmals isoliert wurden aus stark vaskularisiertem Gewebe, wurden sie "vascular anticoagulating proteins", VAC genannt.

Die aus stark vaskularisierten Geweben wie Nabelschnurgefäßen und Placenta isolierten Proteine weisen Molekulargewichte von ca. $70x10^3$, ca. $60x10^3$, ca. $34x10^3$ und ca. $32x10^3$ auf, von denen die Substanzen mit den Molekulargewichten 34 respektive $32x10^3$ aus einer einzigen Polypeptidkette bestehen. Die genaue biochemische Charakterisierung dieser Proteine, sowie deren Isolierung und Reinigung findet sich in der EP-A-0 181 465 vom 21. Mai 1986.

Proteine mit VAC Aktivitität stellen natürliche Blutgerinnungshemmstoffe dar, die bei der Blutgerinnungskaskade an zwei Stellen eingreifen.

Das erste Mal inhibieren sie bei der durch die Faktoren IXa und VIIIa katalysierten Aktivierung des Faktors X zu Xa, das zweite Mal unterbinden sie die Spaltung von Prothrombin zu Thrombin, die durch die Faktoren Xa und Va vermittel wird. Beiden Aktivierungsschritten ist gemeinsam, daß sie Kalzium-Ionen und Phospholipide benötigen. Offenbar können VAC-Proteine ebenfalls mit Phospholipiden in Wechselwirkung treten, und durch diese Bindung die Aktivierungsschritte der Gerinnnungsfaktoren blockieren.

Ihre Eigenschaften machen die Proteine zu interessanten, pharmakologisch äußerst wertvollen Wirkstoffen. Um sie jedoch in ausreichenden Mengen in hochreiner Form zur Verfügung zu haben ist deren Herstellung auf anderen als proteinreinigendem Weg aus natürlichem Gewebe erforderlich. Hierzu bietet sich die gentechnische Methode an.

Die rekombinante Expression von VAC-alpha ist Gegenstand der, gemäß Artikel 54(3) des europäischen Patentübereinkommens zum Stand der Technik gehörenden, europäischen Patentanmeldung EP-A-279 459.

Aufgabe der vorliegenden Erfindung war daher, Proteine mit VAC-beta-Aktivität gentechnisch herzustellen.

Gelöst wurde diese Aufgabe dadurch, daß die Aminosäuresequenz von Teilen der aus stark vaskularisiertem Gewebe isolierten und hochgereinigten Proteine mit VAC-Aktivität (EPA 0 181 465) aufgeklärt wurde, anhand dieser Sequenzen synthetische DNA-Sonden hergestellt wurden und hiermit geeignete cDNA-Bibliotheken durchsucht wurden. Nach Isolierung von mit den Sonden hybridisierender cDNA, deren Sequenzbestimmung sowie entsprechender Manipulation wurden diese cDNA in geeigneten Wirtssystemen beispielsweise in Bakterien, Hefen oder Säugetierzellen zur Expression gebracht.

Eines von den gereinigten Proteinen wurde enzymatisch mit Trypsin gespalten. Die entstandenen Peptide wurden aufgetrennt und ausgewählte Fragmente sequenziert. Die Sequenz des N-Terminus widersetzte sich jedoch einer direkten Analyse, da die erste Aminosäure offensichtlich blockiert ist.

Die Sequenzinformationen sind in Fig. 1 aufgeführt.

Zur Herstellung einer geeigneten DNA-Sonde sind prinzipiell drei Wege möglich. Ist ein längerer Abschnitt des Proteins, etwa 30 oder mehr Aminosäuren lang, bekannt, besteht die Möglichkeit, unter Beachtung der bevorzugt verwendeten Codons bei Säugern eine

wahrscheinlichste Sequenz für den korrespondierenden mRNA Abschnitt zu erstellen. Eine solche Sonde ist im schlechtesten Fall etwa 66% homolog zur tatsächlichen Sequenz. Dies ist auch der Grund, weshalb die Sonde relativ lang sein müßte, um unter nicht stringenten Bedingungen hybridisieren zu können.

Die zweite Möglichkeit besteht darin, für einen kurzen Peptidabschnitt, ca. sechs bis sieben Aminosäuren lang, alle erdenklichen Variationen an Oligonukleotiden zu synthetisieren. Werden solch komplexe Mischungen beim Durchsuchen von cDNA-Bibliotheken verwendet, können relativ viel "falsch" positiv reagierende Klone isoliert werden. Außerdem kann das Hybridisierungssignal sehr schwach ausfallen, da das perfekt passende Einzeloligonukleotid nur einen geringen Anteil an der Gesamtmischung ausmacht.

Der dritte Weg umgeht zwar nicht die Variabilität einer Oligonukleotidsonde, sorgt aber durch die Wahl eines speziellen Nukleosidtriphosphats (ITP) dafür, daß an die gesuchte (oder auch "falsche") cDNA alle Moleküle der Sonde binden können. So wurden zu dem Tryptischen Peptid [P30/I] passende 23 Basen lange Oligonukleotide unter Verwendung von Inosintriphosphat synthetisiert.

Wie bereits erwähnt, können VAC-Proteine aus stark vaskularisiertem Gewebe isoliert werden. Gewebe der Wahl sind Nabelschnurgefäße oder Placenta. Daher und da bekannt ist, daß in der Placenta im Gegensatz zu anderen Spezial-Geweben fast alle Gene exprimiert werden, wurden die oben erwähnten DNA-Sonden verwendet, um eine placentale cDNA-Bibliothek die aus placentalen Gewebe in an sich bekannter Weise hergestellt worden war nach cDNA-Molekülen, die für VAC-Proteine kodieren, zu durchsuchen.

Zum Durchsuchen der cDNA-Bibliothek nach für VAC-protein kodierender cDNA wurden entsprechend der Sequenzen des tryptischen Peptides P16/II zwei und des Staph A Peptides P20/I/6 ein Oligonukleotid synthetisiert (Fig.2). Diese Oligonukleotide stellen jeweils eine Mischung aller Varianten dar, die jede Kodierungsmöglichkeit der entsprechenden mRNA berücksichtigen. EBI-386 weist bei einer Kettenlänge von 20 Nukleotiden 512 Variationen auf und paßt zu dem Staph-A Peptid P20/I/6. Um die Variation bei dem Oligonukleotid für das tryptische Peptid P16/II niedriger zu halten, wurden zwei Oligonukleotide (20-mere) synthetisiert: EBI-387: 128 Variationen, EBI-388: 64 Variationen.

Weiters wurden zu dem tryptischen VAC-Peptid P30/I passend zwei Oligonukleotide unter Verwendung von Desoxyinosin als Base bei "Wobble" Positionen synthetisiert (Fig.3): EBI-118 und EBI-119. Diese Substitution wurde von E. Ohtsuka et al., J. Biol. Chem. 260/5 (1985), pp.2605-2608, sowie Y. Takahashi et al., Proc. Nat. Acad. Sci. (1985) pp.1931-1935, beschrieben. Inosin basenpaart gut mit Cytosin, stört jedoch kaum die Ausbildung der Doppel-helix, wenn andere Nukleotide als Partner angeboten werden.

Nachdem jedes dieser Oligonukleotide in bekannter Weise radioaktiv markiert worden war, wurden Abzüge von Phagen-Platten hiermit nach bekannten Verfahren hybridisiert.

Aus der Hybridisierung mit EBI-386, -387 und -388 resultierten die Phagen Lambda-P11/3, Lambda-P6/5, Lambda-P6/6. Die Hybridisierung mit EBI-118 und -119 resultierte in den Phagen Lambda-Nr15, Lambda-Nr19 und Lambda-Nr22.

Die aus den Phagen isolierten DNAs wurden mit EcoRI geschnitten und die entstehenden Fragmente isoliert. Die cDNA Inserts der Klone Lambda-P11/3, Lambda-P6/5 und Lambda-P6/6 hatten Größen von etwa 1300 bis 1400 bp. Die Sequenzanalyse zeigte, daß alle drei Klone von ein und derselben mRNA abgeleitet waren. Das 5'Ende der mRNA fehlte jedoch in den cDNAs. Die Inserts der Phagen Lambda-Nr15, Lambda-Nr19 und Lambda-Nr22 hatten Längen von ca. 1600, 1100 und 1000 bp. Die Sequenzanalyse ließ eine annähernd vollständige cDNA vermuten.

Die cDNAs der beiden Phagengruppen Lambda-P11/3, Lambda-P6/5 und Lambda-P6/6 sowie Lambda-Nr15, Lambda-Nr19 und Lambda-Nr22 sind von zwei verschiedenen mRNAs abgeleitet, wie die Sequenzanalyse ergab. Die EcoRI Inserts der drei Klone Lambda-P11/3, Lambda-P6/5 und Lambda-P6/6 wurden isoliert und in den EcoRI-geschnittenen Bluescribe M13$^+$ Vektor ligiert (Vector Cloning Systems, 3770 Tansy Street, San Diego, CA 92121, USA). Die resultierenden Klone wurden pP6/5, pP6/6 und pP11/3 genannt.

Die EcoRI Inserts der drei Klone Lambda-Nr15, Lambda-Nr19 und Lambda-Nr22 wurden isoliert und in den EcoRI-geschnittenen Bluescribe M13$^+$ Vektor ligiert. Die resultierenden Klone wurden pRH201, pRH202 und pRH203 genannt.

Um weitere cDNA Klone zu erhalten, wurde die humane, plazentale Lambda-gt10 Bibliothek nochmals durchsucht, diesmal mit dem radioaktiv markierten EcoRI-Insert des pP11/3 als Sonde.

Insgesamt wurden 69 positiv reagierende Klone erhalten (Lambda-VAC1 bis Lambda-VAC69).

12 dieser Klone wurden im kleinen Maßstab wie oben beschrieben präpariert, die cDNA Inserts mit Eco RI freigesetzt und isoliert. Dabei zeigte sich, daß das Insert des Klons Lambda-VAC10 den gesamten für VAC Protein kodierenden Leserahmen enthält. Zur Charakterisierung der für VAC-alpha und VAC-beta . kodierenden cDNAs wurden ein Northernblot-Experiment, Sequenzanalysen und eine genomische Southernblot-Analyse durchgeführt.

Das Ergebnis ist in Fig.4 abgebildet. Die cDNA des Klons pP11/3 hybridisiert an eine mRNA der Größe von etwa 1700 Basen ("VAC-alpha"), die cDNA des Klons pRH 203 an eine mRNA der Größe von etwa 2200 Basen ("VAC-beta").

Die gentechnische Herstellung von VAC-alpha ist in der Offenlegungsschrift der vorliegenden Anmeldung (EP 0293 567) offenbart.

Da erstens die eingesetzte Radioaktivitätsmenge sowie die aufgetragene Menge mRNA pro Spur etwa gleich war und zweitens die Hybridisierung eines Genom-Blots in derselben Lösung Banden gleicher Intensität mit beiden cDNAs ergab (siehe unten), ist zu schließen, daß die kürzere mRNA ("VAC-alpha") in größeren Mengen als die längere ("VAC-beta") mRNA in Plazenta vertreten ist.

Die Sequenzanalyse der Klone Nr15, Nr19 und Nr22 ergab für die VAC-beta cDNA 1940 bp, die in einen poly-A Abschnitt übergeht (Fig.5). 16 Basen vor dem poly-A Abschnitt findet sich das Polyadenylierungssignal AATAAA. Allerdings kommt diese Konsensussequenz bereits an der Nukleotidposition 1704-1709 vor. Weshalb diese Sequenz nicht als Polyadenylierungssignal verwendet wird, ist unbekannt. Die zusätzlich erforderliche Sequenz an der 3'Seite der AATAAA Sequenz, YGTGTTYY (Gill A. et al., Nature 312 (1984), pp. 473-474) kommt erst relativ weit entfernt vor (TGTGTTAT, Position 1735-1742); möglich, daß dies die Erklärung für ein Nichtakzeptieren dieser ersten Polyadenylierungssequenz darstellt.

Die cDNA enthält einen langen, offenen Leserahmen, der vom Beginn der cDNA bis Position 1087 reicht. Er würde ein Kodierungspotential für 362 Aminosäuren beinhalten. Aufgrund der Tatsache, daß auch ein 34.000 D Protein bei der Reinigung von VAC anfällt (siehe E.P.A. 181.465) wurde das erste Methioninkodon (ATG, Position 107-109) als Translationsstart angenommen. Das resultierende Protein (VAC-beta) ist 327 Aminosäuren lang. Es besitzt 4 Cysteinreste (Aminosäureposition 161, 206, 250 und 293) und eine potentielle N-Glykosylierungsstelle (Asn-Lys-Ser, Aminosäureposition 225-227). Das errechnete Molekulargewicht beträgt 36.837 (Fig.6). In VAC-beta gibt es überdurchschnittlich viele geladene Reste: 97/327 (29,6%), wobei die sauren Aminosäuren (Asp+Glu: 49) über die basischen (Lys+Arg 42) überwiegen; eine Erklärung für das nach SDS-PAGE ermittelte niedrigere Molekulargewicht.

VAC-beta zeigt eine interne Wiederholung einer 67 Aminosäuren langen Sequenz Fig.7). Innerhalb dieser Sequenz sind 7 Aminosäuren (10,4 %) in allen vier Repeats konserviert, 17 Aminosäuren (25,4 %) kommen in drei von vier Repeats vor, an 25 Positionen (37,7 %) enthalten jeweils zwei Repeats dieselbe Aminosäure.

Ein Vergleich mit publizierten Literaturdaten (M.J.Geisow, FEBS Letters 203 (1986), pp. 99-103, M.J.Geisow et al., TIBS 11 (1986), pp. 420-423) ergab überraschenderweise, daß VAC-beta damit zu einer größeren Gruppe $Ca^{++}$ abhängiger Phospholipidbindungsproteine gehört. Es wird eine Konsensussequenz beschrieben (Lys-Gly-fob-Gly-Thr-Asp-Glu-var-var-Leu-Ile-fil-Ile-Leu-Ala-fob-Arg; fob=hydrophob, fil=hydrophil, var=variabel), die an der $Ca^{++}$ Bindung beteiligt sein könnte (M.J.Geisow et al., Nature 320 (1986), pp. 636-638). Diese Sequenz findet sich in jeder der vier wiederholten 67 Aminosäure langen Subsequenzen der erfindungsgemäßen Proteine.

Auffällig ist auch der 6 Aminosäuren lange Abschnitt am Ende jeder Repeat, der fast ausschließlich aus hydrophoben Aminosäuren besteht.

VAC-beta zeigt gute Übereinstimmung mit der 17 Aminosäuren langen Konsensussequenz.

Durch gezielte Mutationen in diesem Bereich könnte versucht werden, die biologische Aktivität der Proteine zu verändern. Erfindungsgemäßen Mutationen sehen beispielsweise den vollständigen oder teilweisen Ersatz der für die 17 Aminosäuren der konservierten Region kodierenden Bereiche vor.

Bei dem Vergleich der Proteine stellt man fest, daß der auffälligste Unterschied zwischen den Proteinen bei den N-terminalen Peptiden vorliegt. Erfindungsgemäße Modifikationen sehen daher den gegenseitigen Austausch dieser N-terminalen Peptide vor. Herstellen lassen sich diese modifizierten Proteine dadurch, daß die für diese N-terminalen Peptide kodierenden DNA-Moleküle, beispielsweise durch Oligonukleotidsynthese in an sich bekannter Weise hergestellt werden und mit der "Rest-DNA", die für die verbleibenden Repeats und die Linker-Abschnitte kodiert, ligiert wird. Mit dieser DNA versehene Expressionsvektoren können in bekannter Weise in geeigneten Wirtsorganismen zur Expression gebracht werden; das exprimierte Protein wird isoliert und gereinigt.

Die Analyse chromosomaler DNA aus humaner Plazenta nach Southern zeigt ein komplexes Bild. Die DNA wurde mit EcoRI, HindIII, BamHI und PstI geschnitten. Die auf Nitrozellulose transferierte DNA wurde sowohl mit einer VAC-alpha DNA (pP11/3) als auch mit einer VAC-beta DNA (pRH203) hybridisiert. Obwohl das Waschen der Filter unter stringenten Bedingungen erfolgte, ergaben sich bei jedem Verdau relativ viele Banden (Fig.8). Der Vergleich der beiden Blots zeigt, daß die Kreuzreaktion von VAC-alpha und -beta DNA unter diesen Bedingungen eher auszuschließen ist. Die Vielzahl der Banden ist entweder durch die Existenz jeweils ähnlicher Gene zu dem VAC-alpha bzw. VAC-beta Gen zu erklären, oder aber es handelt sich um jeweils ein Gen, das durch viele und/oder lange Introns unterbrochen ist.

In Fig.9 ist der Vergleich der Aminosäuresequenzen von VAC-alpha mit VAC-beta wiedergegeben. Die wiederholten Strukturen lassen sich in beiden Proteinen gleich anordnen. Die Verbindungspeptide sind ebenfalls gleich lang mit Ausnahme jener zwischen der 2. und 3. Repeat. In dieses Verbindungspeptid muß bei VAC-alpha eine Lücke eingeführt werden, um eine optimale Anpassung der beiden Sequenzen zu erlauben. Das N-terminale Peptid der beiden Proteine ist unterschiedlich lang, 19 Aminosäuren bei VAC-alpha, 25 Aminosäuren bei VAC-beta. Dieses Peptid weist auch die geringste Homologie auf. Die beiden Proteine sind an 176 von 320 Aminosäurepositionen ident, was einem Homologiegrad von 55,0% entspricht.

An dieser Stelle sei auch der Vergleich der Nukleotidsequenzen der VAC-alpha und -beta cDNAs eingefügt. Werden zwei Gene und deren Produkte miteinander verglichen, sieht man auf der DNA (=RNA) Ebene eine größere Homologie als auf der Aminosäureebene, was durch die Tatsache erklärt ist, daß bei der Nukleinsäure bereits eine Veränderung in einem Basentriplett ausreicht, um eine neue Aminosäure zu kodieren.

In Fig.10 ist der Vergleich der kodierenden Regionen von VAC-alpha und VAC-beta cDNA wiedergegeben. Überraschenderweise zeigen die DNAs nur einen Homologiegrad von 54,2%, also etwas weniger als die beiden Proteine.

In Fig.II sind die Hydrophilizitätsprofile der beiden Proteine abgebildet. Dabei wurde der Algorithmus von Hopp und Wood verwendet (T.P.Hopp et al., Proc.Natl.Acad.Sci. 78 (1981) pp.3824-3828). Die vier Repeatbereiche sind durch die Balken angedeutet, die Verbindungspeptide in der darunter angegebenen Sequenz eingerahmt. Überraschenderweise ist das Verbindungspeptid zwischen der zweiten und dritten Repeat besonders hydrophil. In diesem Peptid befindet sich sowohl bei VAC-alpha als auch bei VAC-beta ein Arginin an identer Position. Es wäre daher möglich, daß dieses Arginin einen bevorzugten Angriffspunkt für eine Protease mit trypsinartiger Spezifität darstellt. Dabei müßte das Molekül in zwei etwa gleich große Hälften zerfallen. Es wäre denkbar, daß bereits ein solches "Halbmolekül" eine biologische, beispielsweise eine antikoagulierende Wirkung entfalten kann. Neben dem gezielten Verdau des Proteins beispielsweise mit einer Protease trypsinartiger Spezifität lassen sich diese Halbmoleküle oder Halbmoleküle mit geringen Modifikationen auf vielfältige Weise darstellen. So ist es möglich, die für diese Halbmoleküle kodierenden DNA-Moleküle, die nach an sich bekannten Verfahren herstellbar sind in geeignete Expressionsvektoren so einzufügen, daß diese DNA von den Expressionskontrollsequenzen reguliert wird und durch Kultivierung eines mit diesen Vektoren transformierten Wirtsorganismus die Halbmoleküle zu exprimieren, zu isolieren und zu reinigen.

Halbmoleküle mit geringen Modifikationen erhält man beispielsweise durch Einfügung von Spaltstellen, die dem vollständigen Protein die für bestimmte Proteasen erforderliche Spezifität verleihen. So läßt sich beispielsweise ein Protein mit einer Spaltstelle mit Faktor Xa-artiger Spezifität herstellen, indem in dem Abschnitt, der für die Linker-Sequenz zwischen der zweiten und dritten Repeat-Struktur kodiert, ein Oligonukleotid eingefügt wird, das für das Erkennungs-Tetrapeptid der Faktor-Xa-Protease kodiert. Hierdurch erhält man zunächst ein vollständiges Protein, das möglicherweise veränderte Eigenschaften, beispielsweise veränderte Stabilität gegenüber proteolytischem Angriff aufweist, andererseits aber eine hochspezifische Spaltstelle für die Faktor Xa-Protease enthält, wodurch gezielt zwei Halbmoleküle hergestellt werden und zum therapeutischem Einsatz gelangen können.

Durch gezielte Mutation der für Proteine mit VAC-Aktivität kodierenden DNA lassen sich außerdem Proteine herstellen, die einem proteolytischen Angriff besser widerstehen. So führt möglicherweise der Austausch des VAC-alpha und VAC-beta gemeinsamen Arginins zwischen der zweiten und dritten Repeat-Struktur durch z.B. Histidin zu besserer Stabilität der Proteine. Auch der gezielte Ersatz der übrigen Arginine und der Lysine durch Histidin, möglich durch Ersatz der entsprechenden Codone führt zu Proteinen, die erschwert durch Trypsin hydrolisiert werden können. Diese gezielten Mutationen lassen die biologischen, beispielsweise antikoagulierenden Eigenschaften der Proteine/Polypeptide im wesentlichen unbeeinflußt, führen andererseits jedoch zur Veränderung, beispielsweise zur Verbesserungen der Stabilität der Proteine, d.h. zu einer Veränderung der Halbwertszeit der Proteine.

In manchen Fällen ist es, beispielsweise für die Expression von Proteinen vorteilhaft, dem reifen, gewünschten Protein/Polypeptid eine Signalsequenz oder auch eine dem Wirtsorganismus eigene Proteinsequenz vorzuschalten, so daß als Expressionsprodukt ein Fusionsprotein entsteht. Die für ein Signalpeptid kodierende Signalsequenz kann microbieller Herkunft sein oder aus Säugetierzellen stammen; vorzugsweise ist das Signalpeptid homolog zu dem Wirtsorganismus. Um diese Produkte in das gewünschte reife Protein überführen zu können, bedarf es einer spezifischen Spaltstelle zwischen dem Signal-/Fusionsanteil und dem reifen Protein. Hierzu kann man beispielsweise, wie oben beschrieben, an dieser Stelle ein Oligonukleotid einfügen, das für das Erkennungs- Tetrapeptid der Faktor-Xa-Protease kodiert. Hierdurch läßt sich das exprimierte Fusionsprotein gezielt mit der Faktor-Xa-Protease spalten. Man erhält das reife Protein.

Ersetzt man gezielt die für Methionin verantwortlichen Codone, außer dem an Aminosäureposition 1, durch Codone, die für Leucin, Isoleucin oder Valin kodieren, so läßt sich ein eventuell erhaltenes unreifes- oder Fusionsprotein durch BrCN-Spaltung in das reife Protein überführen.

Sollte sich erweisen, daß derart modifizierte Proteine gleiche oder gegebenenfalls verbesserte biologische Eigenschaften aufweisen, so wäre dies ein möglicher Weg zur Ausbeutesteigerung und gegebenenfalls ein Verfahren zur Herstellung von Proteinen mit besseren Eigenschaften als die natürlichen VAC-Proteine.

Als weitere gezielte Mutation ist der gezielte Austausch von einem, gegebenenfalls mehreren Cysteinen durch Serin oder Valin zu nennen, indem die entsprechenden Codone ausgetauscht werden. Beeinflußt dieser Austausch die biologische Aktivität nicht nachteilig, so könnte dieser Weg eine Verbesserung der Isolierung und/oder Ausbeute der Proteine bewirken. Nicht auszuschließen ist auch hierbei eine Verbesserung der Eigenschaften der Proteine. Als weitere Mutation ist die Kombination verschiedener vollständiger- oder Teil- Sequenzen der für die erfindungsgemäßen Proteine kodierenden DNAs vorgesehen, wodurch Hybridproteine mit vascular-antikoagulierenden Eigenschaften erhalten werden. Alle durch diese oder ähnliche Mutationen erhältlichen Proteine, die für diese kodierenden DNAs, deren Herstellung und Verwendung sind ebenfalls Gegenstand der vorliegenden Erfindung.

Aus Fig. 11 ist weiterhin leicht zu erkennen, daß weder VAC-alpha noch VAC-beta einen längeren, hydrophoben Bereich aufweisen, der entweder die Sekretion durch, oder das Einlagern der Proteine in eine Membran erlauben würde. Es ist daher anzunehmen, daß es sich bei VAC-alpha und VAC-beta um intrazelluläre Proteine handelt.

Wallner et al (Nature 320 (1986), pp.77-81) berichten die Struktur des humanen Lipocortin I, Saris et al. (Cell 46 (1986), pp.201-212) und Huang et al. (Cell 46 (1986), pp. 191-199) die Struktur des murinen bzw. humanen Calpactin I, das auch Lipocortin II genannt wird. Diese Proteine gehören ebenfalls zu der Klasse der $Ca^{++}$ abhängigen Phospholipidbindungsproteinen. Ihre Struktur läßt sich analog zu VAC-beta beschreiben (Fig.12). Die Homologien zwischen VAC-alpha und VAC-beta sind ausgeprägter als jene zwischen VAC und Lipocortin:

VAC-alpha - VAC-beta : 55,0%
VAC-alpha - Lipocortin I : 41,9%
VAC-alpha - Lipocortin II : 43,8%
VAC-beta - Lipocortin I : 41,7%
VAC-beta - Lipocortin II : 44,6%

Anzunehmen ist daher, daß auch die Lipocortine und VAC-alpha aufgrund ihrer analogen Struktur antikoagulierende Wirkung haben, und demzufolge als Antikoagulanzien einzusetzen sind und weiterhin, daß dies eine generelle Eigenschaft dieser Klasse von $Ca^{++}$ abhängiger Phospholipidbindungsproteine ist. Aufgrund der analogen Strukturen läßt sich auch erklären, daß die erfindungsgemäßen Proteine neben der antikoagulierenden Wirkung auch die Eigenschaften der breits bekannten $Ca^{++}$ abhängigen Phospholipidbindungsproteine aufweisen. Beispielhaft genannt seien hier die antiinflammatorischen Eigenschaften der Lipocortine. Die diesen Proteinen eigene Phospholipase Inhibitor-Wirkung konnte auch für die erfindungsgemäßen Proteine nachgewiesen werden.

Gegenstand der vorliegenden Erfindung ist daher auch die Verwendung der erfindungsgemäßen Proteine als antiinflammatorische Mittel sowie die Verwendung der erfindungsgemäßen Proteine bei der Behandlung sämtlicher für die Lipocortine geltenden Indikationen wie beispielsweise rheumatische Erkrankungen. Auch für diesen Einsatzzweck lassen sich die, den natürlichen Proteinen entsprechenden erfindungsgemäßen Produkte in der bereits erwähnten Weise abändern und deren Eigenschaften gegebenenfalls verbessern.

Bei dem Vergleich der Proteine VAC-alpha, VAC-beta, Lipocortin I und Lipocortin II stellt man fest, daß der auffälligste Unterschied zwischen den Proteinen bei den N-terminalen Peptiden vorliegt, insbesondere zwischen den VAC-Proteinen auf der einen und den Lipocortinen auf der anderen Seite. Eine erfindungsgemäße Modifikation sieht daher den gegenseitigen Austausch dieser N-terminalen Peptide vor. Herstellen lassen sich diese modifizierten Proteine dadurch, daß die für diese N-terminalen Peptiden kodierenden DNA-Moleküle, beispielsweise durch Oligonukleotidsynthese in an sich bekannter Weise hergestellt werden und mit der "Rest-DNA", die für die verbleibenden Repeats und die Linker-Abschnitte kodiert, ligiert wird. Mit dieser DNA versehene Expressionsvektoren können in bekannter Weise in geeigneten Wirtsorganismen zur Expression gebracht werden; das exprimierte Protein wird isoliert und gereinigt.

Viele Mitglieder der $Ca^{++}$ abhängigen Phospholipidbindungsproteine binden an gereinigte sekretorische Vesikel oder andere Bestandteile des Cytoskeletts (siehe R.H.Kretsinger et al., Nature 320 (1986), p.573 für eine Zusammenfassung). Bei der Sekretion kapseln sich die Vesikel vom Golgi-Apparat der Zelle ab, wandern zur Zellmembran und fusionieren mit ihr. Dabei wird der Inhalt der Vesikel von der Zelle freigesetzt. In Analogie zur Kupplung von Erregung mit der Muskelkontraktion wurde vorgeschlagen (W.W.Douglas, Br.J.Pharmac. 34 (1968), 451), daß auch der Stimulus und die Sekretion über $Ca^{++}$ gekoppelt ist. Dabei könnten die $Ca^{++}$ abhängigen Phospholipidbindungsproteine eine wichtige Rolle spielen. In der konservierten, 17 Aminosäuren langen Region jedes Repeats besitzt VAC-beta 4 Hydroxylgruppen-enthaltende Aminosäuren (Asp, Glu, Thr, Ser). Obwohl keines der Proteine die in Calmodulin, Troponin C, S-100 und Parvalbumin beobachtete EF-Hand Struktur aufweist (R.H.Kretsinger et al., CRC Crit. Rev.Biochem. 8 (1980), p119), die in diesen Molekülen für die $Ca^{++}$ Bindung verantwortlich ist, verleitet die Konservierung dieses Unterabschnitts in jeder Repeat zu dem Schluß, daß diese Region für die $Ca^{++}$ Bindung verantwortlich ist.

Durch gezielte Mutationen in diesem Bereich könnte versucht werden, die biologische Aktivität der Proteine zu verändern. Erfindungsgemäßen Mutationen sehen beispielsweise den vollständigen oder teilweisen Ersatz der für die 17 Aminosäuren der konservierten Region kodierenden Bereiche vor:

a. aus den VAC-Proteinen durch Bereiche, die die entsprechenden Aminosäuren der Lipocortine kodieren,
b. aus VAC-alpha durch Bereiche, die die entsprechenden Aminosäuren des VAC-beta kodieren und umgekehrt,
c. aus Lipocortin I durch Bereiche, die die entsprechenden Aminosäuren des Lipocortins II kodieren und umgekehrt,
d. aus den Lipocortinen durch Bereiche, die die entsprechenden Aminosäuren der VAC-Proteine kodieren oder
e. bei den übrigen Polypeptiden/Proteinen durch Bereiche, die die entsprechenden Aminosäuren der jeweils anderen Polypeptiden/Proteine kodieren,
indem die entsprechenden für diese Aminosäuren kodierenden Bereiche ausgetauscht werden.

Die oben erwähnte analoge Struktur läßt außerdem erwarten, daß Proteine, die diese Analogie aufweisen, sowohl antiinflammatorische als auch antikoagulierende Wirkung zeigen und daher entsprechend therapeutisch und/oder prophylaktisch einsetzbar sind.

Gegenstand der vorliegenden Erfindung ist daher insbesondere die Verwendung der Polypeptide als antiinflammatorische Mittel, als antirheumatische Mittel, in den für die Lipocortine geltenden Indikationen, die Verwendung der Polypeptide/Proteine, die Repeat-Bereiche aufweisen, als blutgerinnungs- hemmende Mittel, als Thrombin-inhibierende Mittel, als antiinflammatorische Mittel, als antirheumatische Mittel, Verwendung der Lipocortine als blutgerinnungshemmende Mittel, als Thrombin-inhibierende Mittel und in den für die VAC-Proteine geltenden Indikationen, wobei die bekannten Verwendungen der zum Teil in der vorliegenden Anmeldung beschriebenen Proteine mit solchen Repeat-Bereichen ausdrücklich ausgenommen sind.

Weiterhin sind Mittel zur therapeutischen Behandlung, die neben pharmazeutisch inerten Trägerstoffen eine wirksame Menge eines dieser Polypeptide enthält, Gegenstand der Erfindung.

Gegenstand der vorliegenden Erfindung sind auch DNA-Moleküle, die für ein Polypeptid/Protein mit Repeat-Bereichen kodieren, DNA-Moleküle, die für einen der Repeat-Bereiche kodieren, DNA-Moleküle, dadurch gekennzeichnet, daß die für die vollständigen Repeats kodierenden Bereiche umgeordnet sind, die von diesen kodierten Proteine, deren Herstellung und Verwendung.

Alle durch diese oder ähnliche Mutationen erhältlichen Proteine, die für diese kodierenden DNAs, deren Herstellung und Verwendung sind ebenfalls Gegenstand der vorliegenden Erfindung, wobei die bekannten zum Teil in der vorliegenden Anmeldung beschriebenen Proteine, die für diese kodierenden DNAs, deren bekannte Herstellung und Verwendung ausdrücklich ausgenommen sind.

Gegenstand der vorliegenden Erfindung sind auch DNA-Moleküle nicht-humanen Ursprungs, die von diesen kodierten Proteine, deren Herstellung und Verwendung, die in einer, der vorliegenden Erfindung analogen Weise herstellbar sind.

Gegenstand der Erfindung sind nicht nur Gen-Sequenzen, die spezifisch für die erfindungsgemäßen Proteine codieren, sondern ebenfalls Modifikationen, die leicht und routinemäßig durch Mutation, Abbau, Transposition oder Addition erhalten werden können. Jede Sequenz, die für die erfindungsgemäßen Proteine codiert (d.h. die das biologische Aktivitätsspektrum aufweist, das hier beschrieben ist) und im Vergleich mit den gezeigten degeneriert ist, ist ebenfalls eingeschlossen; Fachleute auf diesem Gebiet sind in der Lage, DNA-Sequenzen der codierenden Regionen zu degenerieren. Ebenso ist jede Sequenz, die für ein Polypeptid mit dem Aktivitätsspektrum der erfindungsgemäßen Proteine codiert, und die mit den gezeigten Sequenzen (oder Teilen davon) unter stringenten Bedingungen hybridisiert (beispielsweise Bedingungen, die für mehr als 85 %, bevorzugt mehr als 90 % Homologie selektieren) beinhaltet.

Die Hybridisierungen werden in 6xSSC/5x Denhardt's Lösung/0,1% SDS bei 65°C durchgeführt. Der Grad der Stringenz wird im Waschschritt festgelegt. So sind für eine Selektionierung auf DNA-Sequenzen mit ca. 85 % oder mehr Homologie die Bedingungen 0,2xSSC/0,01% SDS/65°C und für eine Selektionierung auf DNA-Sequenzen mit ca. 90 % oder mehr Homologie die Bedingungen 0,1x SSC/0,01% SDS 65°C geeignet.

Die Erfindung betrifft weiterhin Expressionsvektoren, die eine für VAC kodierende DNA-Sequenz enthalten, die von einer Expressionskontrollsequenz derart reguliert wird, daß in einer mit diesem Expressionsvektoren transformierten Wirtszelle Polypeptide mit VAC-Aktivität exprimiert werden.

Die Expressionsvektoren der vorliegenden Erfindung werden z.B. hergestellt, indem man in eine Vektor-DNA, welche eine Expressionskontrollsequenz enthält, eine VAC kodierende DNA-Sequenz derart einfügt, daß die Expressionskontroll- sequenz besagte DNA-Sequenz reguliert.

Die Wahl eines geeigneten Vektors ergibt sich aus der zur Transformation vorgesehenen Wirtszelle. Geeignete Wirte sind beispielsweise Mikroorganismen, wie Hefen, z.B. Saccharomyces cerevisiae, und insbesondere Bakterienstämme, die über kein Restriktions- oder Modifikationsenzym verfügen, vor allem Stämme von Escherichia coli, z.B. E.coli X1776, E.coli HB101, E.coli W3110, E.coli HB101/LM1035, E.coli JA221(30) oder E.coli K12 Stamm 294, Bacillus subtilis, Bacillus stearothermophilus, Pseudomonas, Haemophilus, Streptococcus und andere, ferner Zellen höherer Organismen, insbesondere etablierte humane oder tierische Zellinien. Bevorzugte Wirtszellen sind die gesamten Stämme von E.coli, insbesondere E.coli HB101, E.coli JM101 und E.coli W3110.

Grundsätzlich sind alle Vektoren geeignet, welche die erfindungsgemäßen heterologen, für die VAC kodierenden DNA-Sequenzen in dem gewählten Wirt replizieren und exprimieren.

Beispiele für Vektoren, die zur Expression des VAC-Gens in einem E.coli-Stamm geeignet sind, sind Bacteriophagen, z.B. Derivate des Bacteriophagen λ, oder Plasmide, wie insbesondere das Plasmid colEl und seine Derivate, z.B. pMB9, pSF2124, pBR317 oder pBR322. Die bevorzugten Vektoren der vorliegenden Erfindung leiten sich von Plasmid pBR322 ab. Geeignete Vektoren enthalten ein vollständiges Replicon und ein Markierungsgen, welches die Selektion und Identifizierung der mit den Expressionsplasmiden transformierten Mikroorganismen auf Grund eines phänotypischen Merkmals ermöglicht. Geeignete Markierungen verleihen dem Mikroorganismus beispielsweise Resistenz gegenüber Schwermetallen, Antibiotika und dergleichen. Weiterhin enthalten bevorzugte Vektoren der vorliegenden Erfindung außerhalb der Replicon- und Markierungsgen- Regionen Erkennungssequenzen für Restriktionsendonucleasen, so daß an diesen Stellen die für die Aminosäuresequenz von VAC kodierende DNA-Sequenz und gegebenenfalls die Expressions-Kontrollsequenz eingefügt werden können. Ein bevorzugter Vektor, das Plasmid pBR322, enthält ein intaktes Replicon, gegen Tetracyclin und Ampicillin Resistenz verleihende Markierungsgene ($tet^R$ und $amp^R$) und eine Reihe von nur einmal vorkommenden Erkennungssequenzen für Restriktionsendonucleasen, z.B. PstI (schneidet im $amp^R$-Gen, das $tet^R$-Gen bleibt intakt), BamHI, HindIII, SalI (schneiden alle im $tet^R$-Gen, das $amp^R$-Gen bleibt intakt), NruI und EcoRI.

Mehrere Expressionskontrollsequenzen können zur Regulation der VAC-Expression eingesetzt werden. Insbesondere werden Expressionskontrollsequenzen stark exprimierter Gene der zu transformierenden Wirtszelle verwendet. Im Falle von pBR322 als Hybridvektor und E.coli als Wirtsorganismus sind beispielsweise die Expressionskontrollsequenzen (welche unter anderem den Promotor und die ribosomale Bindungsstelle enthalten) des Lactoseoperons, Tryptophanoperons, Arabinoseoperons und dergleichen, des β-Lactamasegens, die entsprechenden Sequenzen des Phage λN-Gens oder des Phage fd-Schichtproteingens und andere geeignet. Während der Promotor des β-Lactamasegens (β-lac-Gen) bereits in Plasmid pBR322 enthalten ist, müssen die übrigen Expressionskontrollsequenzen in das Plasmid eingeführt werden. Bevorzugt als Expressionskontrollsequenz in der vorliegenden Erfindung ist diejenige des Tryptophanoperons (trp po) sowohl von Serratia marcescens als auch aus E.coli und der alkalische Phosphatase-Promotor bzw. deren Hybrid.

Neben diesen besonders gebräuchlichen Promotoren sind auch andere mikrobielle Promotoren entwickelt und benutzt worden. Die Gen-Sequenz für die erfindungsgemäßen Proteine kann beispielsweise unter der Kontrolle des Leftward-Promotors des Bakteriophagen Lambda ($P_L$) eingesetzt werden. Dieser Promotor ist ein besonders effektiver steuerbarer Promotor. Die Steuerung wird möglich durch den Lambda-Repressor, von dem benachbarte Restriktionsschnittstellen bekannt sind. Ein temperaturempfindliches Allel dieses Repressor-Gens kann in einen Vektor, der eine Protein-Gen-Sequenz enthält, eingefügt werden. Wird die Temperatur auf 42°C erhöht, wird der Repressor inaktiviert und der Promotor aktiviert. Durch die Verwendung dieses Systems ist es möglich, eine Clon-Bank zu etablieren, in der eine funktionelle Protein-Gen-Sequenz in Nachbarschaft zu einer Ribosom-Bindungsstelle in variierenden Abständen zu dem Lambda-$P_L$-Promotor plaziert wird. Diese Klone können dann überprüft und der mit der höchsten Ausbeute selektiert werden.

Die Expression und Translation einer Sequenz codierend für die erfindungsgemäßen Proteine kann auch unter Kontrolle anderer Regulationssysteme, die als "homolog" zu dem Organismus in seiner untransformierten Form gelten können, ablaufen. So enthält z.B. chromosomale DNA von einem Lactose-abhängigen E.coli ein Lactose oder Lac-Operon, das durch Ausschüttung des Enzyms Beta-Galactosidase den Lactose-Abbau ermöglicht.

Die Lac-Kontrollelemente können aus dem Bacteriophagen Lambda-plac5, der infektiös für E. coli ist, erhalten werden. Das Lac-Operon des Phagen kann durch Transduktion aus derselben Bakterien-Spezies stammen.

Regulationssysteme, die bei dem erfindungsgemäßen Verfahren Verwendung finden können, können auch aus plasmidischer DNA stammen, die dem Organismus eigen ist. Das Lac-Promotor-Operator-System kann durch IPTG induziert werden.

Andere Promotor-Operator-Systeme oder Teile hiervon können genausogut verwendet werden: beispielsweise Colicine $E_1$-Operator, Galactose-Operator, Xylose-A-Operator, tac- Promotor u.ä..

Zusätzlich zu Prokaryoten können auch eukaryotische Mikroorganismen, wie Hefekulturen verwendet werden. Saccharomyces cerevisiae ist die am meisten verwendete von den eukaryotischen Mikroorganismen, obwohl eine Anzahl anderer Spezies allgemein erhältlich ist.

Zur Replikation und Expression in Hefe geeignete Vektoren enthalten einen Hefe-Replikationsstart und einen selektiven genetischen Marker für Hefe. Hybridvektoren, die einen Hefe-Replikationsstart enthalten, z.B. das chromosomale autonom replizierende Segment (ars), bleiben nach der Transformation innerhalb der Hefezelle extra-chromosomal erhalten und werden bei der Mitose autonom repliziert. Zur Expression in Saccharomyces wird beispielsweise das Plasmid YRp7 (Stinchcomb et al. Natur 282, 39 (1979); Kingsman et al., Gene 7, 141 (1979); Tschumper et al., Gene 10, 157 (1980)) und das Plasmid YEp13 (Bwach et al., Gene 8, 121-133 (1979)) verwendet. Das Plasmid YRp7 enthält das TRP1-Gen, das eine Selektionierungsmarkierung für eine Hefemutante, die unfähig ist, in trypthophanfreiem Medium zu wachsen, bereitstellt; beispielsweise ATCC Nr. 44076.

Das Vorhandensein des TRP1 Schadens als Charakteristikum des Hefe-Wirts Genoms stellt dann ein wirksames Hilfsmittel dar, um Transformation nachzuweisen, wenn ohne Tryptophan kultiviert wird. Ganz ähnlich verhält es sich bei dem Plasmid YEp13, das das Hefe-Gen LEU 2, das zur Ergänzung einer LEU-2-minus-Mutante verwendet werden kann, enthält.

Weitere geeignete Markierungsgene für Hefe sind im Fall von auxotrophen Hefemutanten, im allgemeinen Gene, die Wirtsdefekte komplementieren. Entsprechende Gene sorgen für Prototrophie in einer auxotophen Hefemutante, z.B. das URA3- und HIS3- Gen. Vorzugsweise enthalten Hefe-Hybridvektoren weiterhin einen Replikationsstart und ein Markierungsgen für einen bakteriellen Wirt, insbesondere E.coli, damit die Konstruktion und die Klonierung der Hybridvektoren und ihrer Vorstufen in einem bakteriellen Wirt erfolgen kann. Weitere für die Expression in Hefe geeignete Expressionskontrollsequenzen sind beispielsweise diejenigen des PHO3- oder PHO5-Gens, ferner in glykolytischen Abbau involvierte Promotor, z.B. der PGK- und GAPDH-Promotor.

Andere geeignete Promotor-Sequenzen für Hefe Vektoren beinhalten die 5'-flankierende Region der Gene des ADH I (Ammerer G., Methods of Enzymology 101, 192-201 (1983)), 3-Phosphoglycerate-Kinase (Hitzeman et al., J. Biol. Chem. 255, 2073 (1980)) oder andere glykolytische Enzyme (Kawaski und Fraenkel, BBRC 108, 1107-1112 (1982)) wie Enolase, Glycerinaldehyd-3-phosphat-Dehydrogenase, Hexokinase, Pyruvat-Decarboxylase, Phosphofructokinase, Glucose-6-Phosphat-Isomerase, Phosphoglucose-Isomerase und Glucokinase. Bei der Konstruktion geeigneter Expressionsplasmide können die mit diesen Genen assoziierten Terminationssequenzen ebenfalls in den Expressions-Vektor am 3'-Ende der zu exprimierenden Sequenz eingesetzt werden, um Polyadenylierung und Termination der mRNA zu ermöglichen.

Andere Promotoren, die zudem noch den Vorteil der durch Wachstumsbedingungen kontrollierten Transkription besitzen, sind die Promotor-Regionen der Gene für Alkohol-Dehydrogena- se-2, Isocytochrom C, Saure-Phosphatase, abbauende Enzyme, die mit dem Stickstoff-Metabolismus gekoppelt sind, die oben erwähnte Glycerinaldehyd-3-Phosphat-Dehydrogenase und Enzyme, die für die Verarbeitung von Maltose und Galaktose verantwortlich sind. Promotoren, die durch den Hefe Mating Typ Locus reguliert werden, beispielsweise Promotoren der Gene BARI, MFalpha1, STE2, STE3, STE5 können bei temperaturregulierten Systemen durch die Verwendung von temperaturabhängigen sir Mutationen eingesetzt werden. (Rhine Ph.D. Thesis, University of Oregon, Eugene, Oregon (1979), Herskowitz and Oshima, The Molecular Biology of the Yeast Saccharomyces, part I, 181-209 (1981), Cold Spring Harbor Laboratory). Diese Mutationen beeinflussen die Expression der ruhenden Mating-Typ Kassetten von Hefen und dadurch indirekt die Mating-Typ abhängigen Promotoren. Generell ist jedoch jeder Plasmid Vektor, der einen Hefe-kompatiblen Promotor, originäre Replikations- und Terminationssequenzen enthält, geeignet.

So können auch Hybridvektoren, die der Hefe-2µ-Plasmid-DNA homologe Sequenzen enthalten, verwendet werden. Solche Hybridvektoren werden durch Rekombination innerhalb der Zelle bereits vorhandenen 2µ-Plasmiden einverleibt oder replizieren autonom. 2µ-Sequenzen sind besonders für Plasmide mit großer Transformationshäufigkeit geeignet und gestatten eine hohe Kopienzahl.

Zusätzlich zu Mikroorganismen sind Zellkulturen multizellulärer Organismen ebenfalls geeignete Wirtsorganismen. Im Prinzip ist jede dieser Zellkulturen einsetzbar, ob von Wirbeltier- oder wirbellosen Tierzellkulturen. Größtes Interesse besteht jedoch an Wirbeltierzellen, so daß die Vermehrung von Wirbeltierzellen in Kultur (Gewebe-Kultur) in den letzten Jahren zu einer routinemäßigen Methode wurde (Tissue Culture, Academic Press, Kruse and Patterson, Editors (1973)). Beispiele solch nützlicher Wirtszellinien sind VERO- und HeLa-Zellen, CHO-Zellen und WI38, BHK, COS-7 und MDCK-Zellinien.

Expressionsvektoren für diese Zellen enthalten üblicherweise (wenn nötig) einen Replikationsstartpunkt, einen Promotor, der vor dem zu exprimierenden Gen lokalisiert ist, gemeinsam mit allen notwendigen Ribosomenbindungsstellen, RNA-Splicing-Stelle, Polyadenylierungsstelle und transkriptionelle Terminations-Sequenzen.

Bei der Verwendung in Säugetierzellen stammen die Kontrollfunktionen auf den Expressions-Vektoren oftmals aus viralem Material. Beispielsweise stammen die üblicherweise verwendeten Promotoren aus Polyoma-, Adenovirus 2, und besonders häufig aus Simian Virus 40 (SV 40). Die frühen und späten Promotoren des SV 40 sind besonders nützlich, da beide leicht aus dem Virus als Fragment zu erhalten sind, das auch noch die virale Replikationsstelle des SV 40 enthält. (Fiers et al., Nature 273, 113 (1978)). Auch können kleinere oder größere Fragmente des SV 40 verwendet werden, vorausgesetzt, sie enthalten die annähernd 250 bp lange Sequenz, die von der HindIII Schnittstelle bis zur BglI Schnittstelle in dem viralen Replikationsstartpunkt reicht. Außerdem ist es ebenfalls möglich und oft empfehlenswert, Promotor- oder Kontroll-Sequenzen zu verwenden, die normalerweise mit den gewünschten Gensequenzen verknüpft sind, vorausgesetzt, diese Kontroll-Sequenzen sind kompatibel zu den Wirtszellsystemen.

Ein Replikationsstartpunkt kann entweder durch entsprechende Vektorkonstruktion vorgesehen werden, um einen exogenen Startpunkt einzubauen, beispielsweise aus SV 40 oder anderen viralen Quellen (z.B. Polyoma, Adeno, VSV, PBV, etc.) oder kann durch die chromosomalen Replikationsmechanismen der Wirtszelle vorgesehen werden. Wird der Vektor in das Wirtszellenchromosom integriert, reicht die zuletztgenannte Maßnahme meistens aus.

Insbesondere betrifft die Erfindung zur Replikation und phänotypischen Selektionen befähigte Expressionsvektoren, welche eine Expressionskontrollsequenz und eine für die Aminosäuresequenz von VAC kodierende DNA-Sequenz enthalten, wobei besagte DNA-Sequenz mitsamt Transkriptionsstartsignal und -terminationssignal sowie Translationsstartsignal und -stopsignal in besagtem Expressionsplasmid unter Regulation besagter Expressionskontrollsequenz so angeordnet ist, daß in einer mit besagtem Expressionsplasmid transformierten Wirtszelle VAC exprimiert wird.

Um eine effektive Expression zu erreichen, muß das VAC-Gen richtig (in "Phase") mit der Expressionskontrollsequenz angeordnet sein. Es ist vorteilhaft, die Expressionskontrollsequenz in den Bereich zwischen dem Haupt-mRNA-Start und dem ATG der Genkodiersequenz, welche natürlich mit dem Expressionskontrollsequenz verknüpft ist (z.B. die β-lac-Kodiersequenz bei Verwendung des β-lac-Promotors), mit dem VAC-Gen, welches vorzugsweise sein eigenes Translationsstartsignal (ATG) und Translationsstopsignal (z.B. TAG) mitbringt, zu verknüpfen. Dadurch wird eine effektive Transkription und Translation gewährleistet.

Beispielsweise wird ein Vektor, insbesondere pBR322, mit einer Restriktionsendonuklease geschnitten und, gegebenenfalls nach Modifikation des so gebildeten linearisierten Vektors, eine mit entsprechenden Restriktionsenden versehenen Expressionskontrollsequenz eingeführt. Die Expressionskontrollsequenz enthält am 3'-Ende (in Translationsrichtung) die Erkennungssequenz einer Restriktionsendonuclease, so daß der die Expressionskontrollsequenz bereits enthaltende Vektor mit besagtem Restriktionsenzym verdaut und das mit passenden Enden versehene VAC-Gen eingesetzt werden kann. Dabei entsteht ein Gemisch von zwei Hybridplasmiden, welche das Gen in richtiger bzw. in falscher Orientierung enthalten. Vorteilhaft ist es, den die Expressionskontrollsequenz bereits enthaltenden Vektor noch mit einer zweiten Restriktionsendonuclease innerhalb der Vektor-DNA zu spalten und in das entstandenen Vektor-Fragment das mit richtigen Enden versehene VAC-Gen einzusetzen. Alle Operationen am Vektor erfolgen vorzugsweise in einer Weise, daß die Funktion des Replicons und zumindest eines Markierungsgens nicht beeinträchtigt wird.

In einer bevorzugten Ausführungsform der vorliegenden Erfindung wird ein von pBR322 abgeleiteter Vektor, welcher eine Expressionskontrollsequenz, insbesondere diejenige vom Tryptophan-Operon (trp po), enthält, die am 3'-Ende (zwischen dem Haupt-mRNA-Start und dem ersten ATG) die Erkennungssequenz für eine, vorzugsweise kohäsive Enden bildende, Restriktionsendonuclease, z.B. EcoRI, trägt, mit der genannten Restriktionsendonuclease und im Vektor-DNA-Teil mit einer zweiten Restriktionsendonuclease, welche flache oder bevorzugt kohäsive Enden bildet, z.B. BamHI, verdaut, wonach der so linearisierte Vektor mit der entsprechende Enden aufweisenden VAC-DNA (z.B. mit einem EcoRI-Ende vor dem ATG-Start und einem BamHI-Ende nach dem Translationsstop-Codon) verknüpft wird. Die Verknüpfung erfolgt in bekannter Weise durch Paarung der komplementären (kohäsiven) Enden und Ligierung, z.B. mit $T_4$-DNA-Ligase.

Vorzugsweise können die erfindungsgemäßen DNA-Sequenzen auch in dem Expressionsplasmid pER103 (E. Rastl-Dworkin et al., Gene 21, 237-248 (1983) und EP-A-0.115-613 - hinterlegt bei der DSM unter der Nummer DSM 2773 am 20. Dezember 1983), in dem Plasmid parpER33 (EP-A-0.115-613) oder dem Plasmid pRH100 exprimiert werden, da diese Vektoren alle Regulationselemente enthalten, die zu einer hohen Expressionsrate der klonierten Gene führen.

Erfindungsgemäß wird als Expressionsvektor für das synthetische Protein Gen das Plasmid pRH100 verwendet, das den regulierbaren Tryptophanpromotor aus Serratia marcescens und eine artifizielle Ribosomenbindungsstelle enthält. Zur Herstellung des Expressionsplasmides pRH100 wurde das Plasmid pER103 (Eva Dworkin-Rastl et al., Gene 21 (1983) 237-248, EP-A-O.115-613) mit der Restriktionsendonuklease HindIII linearisiert und die Oligonukleotidsequenz

5' AGCTTAAAGATGAGCTCATCTTTA 3'.
3' ATTTCTACTCGAGTAGAAATTCGA 5'

eingefügt.

Die über den mRNA-Weg, aus genomischer DNA oder synthetisch gewonnene, mit entsprechenden (insbesondere EcoRI- und BamHI-) Enden versehene VAC-DNA kann vor dem Einbringen in ein Expressionsplasmid auch in einen Vektor, z.B. pBR322, kloniert werden, um größere Mengen an VAC-DNA, beispielsweise für die Sequenzanalyse, zu gewinnen. Die Isolierung der Klone, welche das Hybridplasmid enthalten, wird beispielsweise mit einer VAC-DNA spezifischen, radioaktiv-markierten Oligonucleotid-Probe (siehe oben) durchgeführt. Die Charakterisierung der VAC-DNA erfolgt beispielsweise nach dem Verfahren von Maxam und Gilbert (11).

In einer weiteren Ausführungsform der Erfindung werden Teilstücke der VAC-DNA synthetisiert. Die Erfindung betrifft auch ein Verfahren zur Herstellung von transformierten Wirtszellen, dadurch gekennzeichnet, daß man eine Wirtszelle mit einem Expressionsvektor, der eine von einer Expressionskontrollsequenz regulierte, für die Aminosäuresequenz von VAC kodierende DNA-Sequenz enthält, transformiert.

Geeignete Wirtszellen sind beispielsweise die oben genannten Mikroorganismen, wie Stämme von Saccharomyces cerevisiae, Bacillus subtilis und insbesondere Escherichia coli. Die Transformation mit dem erfindungsgemäßen Expressionsplasmiden erfolgt beispielsweise wie in der Literatur beschrieben, so für S. cerevisiae (12), B.subtilis (13) und E.coli (14). Die Isolierung der transformierten Wirtszellen erfolgt vorteilhaft aus einem selektiven Nährmedium, dem das Biocid zugesetzt wird, gegen welches das im Expressionsplasmid enthaltende Markierungs-Gen Resistenz verleiht. Wenn, wie bevorzugt, die Expressionsplasmide das amp$^R$-Gen enthalten, wird dem Nährmedium demgemäß Ampicillin zugesetzt. Zellen, welche das Expressionsplasmid nicht enthalten, werden in einem solchen Medium abgetötet.

Die Erfindung betrifft ebenfalls die auf dem genannten Weg erhältlichen transformierten Wirtszellen.

Die transformierten Wirtszellen können zur Herstellung von Verbindungen mit VAC-Aktivität verwendet werden. Das Verfahren zur Herstellung dieser Verbindung ist dadurch gekennzeichnet, daß die transformierten Wirtszellen kultiviert und das Produkt aus den Wirtszellen freigesetzt und isoliert wird.

Die Erfindung betrifft daher vor allem ein Verfahren zur Herstellung von Verbindungen mit VAC-Aktivität und von Salzen solcher Verbindungen, dadurch gekennzeichnet, daß mit einem Expressionsplasmid, welches eine von einer Expressionskontrollsequenz regulierte, für die Aminosäuresequenz von VAC kodierende DNA-Sequenz enthält, transformierte Wirtszellen in einem flüssigen Nährmedium, welches assimilierbare Kohlenstoff- und Stickstoffquellen enthält, kultiviert werden und das Produkt aus den Wirtszellen freigesetzt und isoliert wird, und, falls erforderlich, ein verfahrensgemäß erhältliches Produkt mit einem zur Aufspaltung der Disulfidbindungen geeigneten Reduktionsmittel versetzt und das erhältliche reduzierte Polypeptid gegebenenfalls mit einem zur Neuknüpfung von Disulfidbindungen geeigneten Oxidationsmittel behandelt wird, und gewünschtenfalls, eine erhältliche VAC-Verbindung in eine andere VAC-Verbindung überführt wird, ein verfahrensgemäß erhältliches Gemisch von Verbindungen mit VAC-Aktivität in die einzelnen Komponenten aufgetrennt wird und/oder, gewünschtenfalls, ein erhaltenes Salz in das Polypeptid und ein erhaltenes Polypeptid in das entsprechende Salz desselben überführt wird.

Die vorliegende Erfindung betrifft insbesondere ein Verfahren zur Herstellung von VAC-Verbindungen.

Die Kultivierung der erfindungsgemäßen transformierten Wirtszellen erfolgt in an sich bekannter Weise. So können für die Kultivierung der erfindungsgemäßen, transformierten Wirtsmikroorganismen verschiedene Kohlenstoffquellen verwendet werden. Beispiele bevorzugter Kohlenstoffquellen sind assimilierbare Kohlenhydrate, wie Glucose, Maltose, Mannit oder Lactose, oder ein Acetat, das entweder allein oder in geeigneten Gemischen verwendet werden kann. Geeignete Stickstoffquellen sind beispielsweise Aminosäuren, wie Casaminosäuren, Peptide und Proteine und ihre Abbauprodukte, wie Trypton, Pepton oder Fleischextrakte; weiterhin Hefeextrakte, Malzextrakt, wie auch Ammoniumsalze, z.B. Ammoniumchlorid, -sulfat oder -nitrat, die entweder allein oder in geeigneten Mischungen verwendet werden können. Anorganische Salze, die ebenfalls verwendet werden können, sind z.B. Sulfate, Chloride, Phosphate und Carbonate von Natrium, Kalium, Magnesium und Calcium.

Weiterhin enthält das Medium z.B. wachstumsfördernde Substanzen, wie Spurenelemente, z.B. Eisen, Zink, Mangan und dergleichen, und vorzugsweise Substanzen, die einen Selektionsdruck ausüben und das Wachstum von Zellen, die das Expressionsplasmid verloren haben, verhindern. So wird dem Medium beispielsweise Ampicillin zugesetzt, wenn das Expressionsplasmid ein amp$^R$-Gen enthält. Ein solcher Zusatz von antibiotisch wirksamen Substanzen bewirkt auch, daß kontaminierende, Antibiotika-empfindliche Mikroorganismen abgetötet werden.

Die Kultivierungsbedingungen, wie Temperatur, pH-Wert des Mediums und Fermentationszeit, werden so ausgewählt, daß maximale VAC-Titer erhalten werden. So wird ein E. coli- oder ein Hefe-Stamm bevorzugt unter aeroben Bedingungen in submerser Kultur unter Schütteln oder Rühren bei einer Temperatur von etwa 20 bis 40°C, vorzugsweise etwa 30°C, und einem pH-Wert von 4 bis 9, vorzugsweise bei pH 7, während etwa 4 bis 20h, vorzugsweise 8 bis 12h, kultiviert. Dabei sammelt sich das Expressionsprodukt intrazellulär an.

Wenn die Zelldichte einen ausreichenden Wert erreicht hat, wird die Kultivierung abgebrochen und das Produkt. falls erforderlich, aus den Zellen des Mikroorganismus freigesetzt. Zu diesem Zeck werden die Zellen zerstört, z.B. durch Behandlung mit einem Detergens, wie SDS oder Triton, oder mit Lysozym oder einem gleichartig wirkenden Enzym lysiert. Alternativ oder zusätzlich kann man mechanische Kräfte, wie Scherkräfte (z.B. X-Presse, French-Presse, Dyno-Mill) oder Schütteln mit Glasperlen oder Aluminiumoxid, oder abwechselndes Einfrieren, z.B. in flüssigem Stickstoff, und Auftauen, z.B. auf 30° bis 40°C, sowie Ultraschall zum Brechen der Zellen anwenden. Die resultierende Mischung, die Proteine, Nucleinsäuren und andere Zellbestandteile enthält, wird nach der Zentrifugation in an sich bekannter Weise an Proteinen angereichert. So wird z.B. der größte Teil der Nicht-Protein-Bestandteile durch Polyäthylenimin-Behandlung abgetrennt und die Proteine einschließlich der VAC-Verbindungen z.B. durch Sättigen der Lösung mit Ammoniumsulfat oder mit anderen Salzen ausgefällt. Weitere Reingungsschritte umfassen beispielsweise chromatographische Verfahren, wie Ionenaustauschchromatographie. HPLC, Reverse-Phase-HPLC und dergleichen. So erfolgt die Auftrennung der Mischbestandteile durch Dialyse, nach Ladung mittels Gel- oder trägerfreier Elektrophorese, nach Molekülgröße mittels einer geeigneten Sephadex-Säule, durch Affinitätschromatographie, z.B. mit Antikörpern, insbesondere monoklonalen Antikörpern, oder mit Thrombin gekuppelt an einen geeigneten Träger zur Affinitätschromatographie, oder durch weitere, insbesondere aus der Literatur bekannte Verfahren.

Beispielsweise umfaßt die Isolierung der exprimierten VAC-Verbindungen die folgenden Stufen.

Abtrennung der Zellen aus der Kulturlösung mittels Zentrifugation; Herstellung eines Rohextraktes durch Zerstören der Zellen, z.B. durch Behandlung mit einem lysierenden Enzym und/oder abwechselndem Einfrieren und Wiederauftauen; Abzentrifugieren der unlöslichen Bestandteile; Ausfällen der DNA durch Zugabe von Polyäthylenimin; Fällung der Proteine durch Ammoniumsulfat; Affinitätschromatographie des gelösten Niederschlags an einer monoklonalen Anti-VAC-Antikörper-Säule; Entsalzung der so gewonnenen Lösung mittels Dialyse oder Chromatographie an Sephadex G25 oder Sephadex G10.

Weitere Reinigungsschritte schließen Gelfiltrationen an Sephadex G50 (oder G75) und Reverse-Phase-HPLC ein. Entsalzung wieder an Sephadex G25.

Zum Nachweis der VAC-Aktivität kann der Test mit Anti-VAC-Antikörpern (z.B. aus Kaninchen/Maus erhältliche, oder aus Hybridomazellen erhältliche monoklonale Antikörper) oder können die in der EPA 0 181 465 beschriebenen Tests herangezogen werden.

Wie bereits erwähnt, ist der alkalische Phosphatasepromotor besonders geeignet für die Expression der erfindungsgemäßen Proteine.

Das Gen für die alkalische Phosphatase (PhoA) aus E.coli unterliegt einer strengen Regulation. In Gegenwart von Phosphat wird das Gen komplett abgeschaltet, bei Abwesenheit von Phosphat im Medium erfolgt Genexpression. H.Shuttleworth et al., Nucleic Acids Res. 14 (1986), p.8689 sowie C.N.Chang et al., Gene 44 (1986), pp.121-125 beschreiben die Nukleotidsequenz dieses Gens. Zur Konstruktion eines geeigneten Expressionsvektors wurde aus mehreren Oligonukleotiden die Promotorregion des PhoA Gens zusammengesetzt und in EcoRI-ClaI geschnittenes pAT153 (Amersham) eingesetzt. Vor der ribosomalen Bindungsstelle wurde eine XhoI Stelle eingeführt. Die originale EcoRI Stelle wird beim Einligieren des synthetischen DNA-Fragmentes zerstört. Nach der ribosomalen Bindungsstelle wurde ein Translationsstart-ATG vorgesehen, dessen G das erste Nukleotid einer SacI (=SstI) Stelle ist. Der Expressionsvektor läßt sich durch Schnitt mit SacI an dieser Stelle linearisieren und der 3'Überhang durch Behandlung mit DNA-Polymerase I - Klenow Fragment in Gegenwart von dGTP in ein gerades Ende überführen. Dadurch kann jedes beliebige Gen an dieser Stelle eingefügt werden, für korrekte Expression muß es mit der ersten Base der kodierenden Region beginnen.

Das HindIII-SalI Fragment des pAT-Anteils wurde entfernt und durch den alkalischen Phosphatase-Transkriptionsterminator ersetzt. Die originale SalI Stelle wurde zerstört. Dafür wurde sie vor dem Terminator zusammen mit der ebenfalls aus pAT153 deletierten BamHI Stelle wieder eingebracht.

Die Sequenz der synthetisch hergestellten DNA ist in Fig.34 abgebildet. Der resultierende Vektor wurde pRH284T genannt.

Zur Expression von VAC-beta wurde vorzugsweise als Expressionsvektor das Plasmid pER103 verwendet (E.Rastl-Dworkin et al., Gene 21 (1983), 237-248). Der Vektor wurde mit HindIII linearisiert. Das 5'überhängende Ende wurde mit dATP und DNA-Polymerase I / Klenowfragment partiell eingefüllt, und der verbleibende Einzelstrangrest mit S1 Nuklease verdaut. Der Vektor wurde mit BamHI nachgeschnitten und das große Fragment isoliert (Fig.13). In den so vorbereiteten Vektor wurde das für VAC-beta kodierende DNA-Molekül ligiert. Hierzu wurde beispielsweise aus dem Klon pRH203 das 440 bp lange MaeIII-BamHI Fragment isoliert, das die Codons 13 bis 157 enthält. Das fehlende 5'Ende wurde durch Oligonukleotide ergänzt:

EBI-307

```
5'  CCATGGCTTGGTGGAAAGCTTGGATCGAACAGGAAGGT        3'
3'  GGTACCGAACCACCTTTCGAACCTAGCTTGTCCTTCCACAGTG  5'  EBI-306
```

Dabei wurden für E.coli optimale Codons verwendet (zB. R.Grantham et al., Nucleic Acids Res. 8 (1980), 1893-1912). Dieser Codonaustausch resultierte in einer neuen HindIII-Stelle bei Codon 5 bis 7. Nach dem Nachschnitt mit BamHI wurde das 5'terminale VAC-Fragment in den vorbereiteten pER103 Vektor ligiert. Der entstehende Vektor wurde pRH211 benannt. Um die für VAC-beta kodierende Region zu ergänzen, wurde aus dem Klon pRH201 das 1230 bp lange BamHI-SphI Fragment isoliert. Der ca. 200 bp lange pBR322 Abschnitt von BamHI bis SphI aus dem Plasmid pRH211 wurde entfernt und durch den entsprechenden VAC-cDNA Teil ersetzt. Es resultierte der Vektor pRH212. Das EcoRI-BamHI Fragment, das den Trp-Promotor (S.marcescens), die ribosomale Bindungsstelle und den synthetisch hergestellten Beginn des VAC-beta Gens enthält, wurde durch Sequenzieren überprüft. Der Nachweis des plasmidkodierten VAC-beta erfolgte im Maxizell-system (A.Sancar et al., J. Bacteriol. 137 (1979), pp.692-693.).

Besonders bevorzugt für die Expression des VAC-beta ist die Konstruktion eines Expressionsvektors ausgehend von pRH284T. Hierzu wurde in den Expressionsvektor in geeigneter Weise das für VAC-beta kodierende Insert ligiert. Als Ausgangsmaterial für dieses Insert kann beispielsweise der Vektor pRH212 dienen:

Der Expressionsvektor pRH284T wurde mit SacI linearisiert und die 3' überhängenden Ende mit DNA-Polymerase I / Klenowfragment und dGTP in gerade Enden übergeführt. Der Vektor wurde mit SalI nachgeschnitten, und das große Fragment isoliert. Das HindIII-SalI Insert des Klons pRH212 wurde isoliert. Das Oligonukleotidpaar

```
5' GCTTGGTGGAA 3'   EBI-684
3' CGAACCACCTTTCGA 5'   EBI-685
```

wurde mit dem VAC-beta Insert und dem vorbereiteten pRH284T ligiert. E.coli HB101 wurde mit der Ligaselösung transformiert. Der resultierende Klon wurde pRH292 benannt (Fig.14).

Da im Expressionsvektor pRH292 (VAC-beta) das Tetracyclinresistenzgen vom Promotor bis zur SalI-Stelle deletiert worden ist, können diese Vekoren keine Tetracyclinresistenz vermitteln.

Tetracylin-resistente Expressionsvektoren erhält man beispielsweise durch die Konstruktion nach dem Schema in Fig. 15. VAC-alpha und VAC-beta cDNA weisen jeweils in der 3' nicht translatierten Region eine SphI Stelle auf. Im beta-Lactamase Gen des Vektors findet sich eine PvuI Stelle. Beide Erkennungssequenzen sind singulär. Daher kann durch Schnitt mit PvuI und SpHI ein Teil des beta-Lactamase-Gens, der phoA-Promotor und die den gesamten kodierenden Teil der VAC-beta plus etwas 3' nicht translatierter cDNA aus den beiden Expressionsvektoren freigesetzt werden. Andererseits kann aus dem Plasmid pAT153 durch Schnitt mit PvuI und EcoRI der Rest des beta-Lactamase Gens, der Replikationsursprung und das gesamte Tetracyclinresistenzgen inklusive des Promotors freigesetzt werden. Werden die SphI- bzw. EcoRI Enden durch enzymatische Behandlung gerade gemacht, erhält man hier kompatible Enden. Werden nun Vektor-Fragment und das VAC-beta cDNA enthaltende Fragment ligiert, entsteht ein Expressionsvektor, der das komplette Tetracyclin enthält: pGN26.

Kompetente Wirtsorganismen, beispielsweise E.coli, besonders bevorzugt E.coli HB101 wurden mit den so hergestellten Expressionsvektoren transformiert und in geeigneten Medien kultiviert.

Ein für die Expression von VAC-beta gut geeignetes Medium sei im folgenden mit seinen Komponenten angeführt.

| | |
|---|---|
| 0,2 -2,0 g/l | $(NH_4)_2HPO_4$ |
| 0,1 - 1,5 g/l | $K_2HPO_4.3H_2O$ |
| 0,1 - 5 g/l | KCl |
| 0,1 - 10 g/l | NaCl |
| 0 - 5 g/l | $NH_4Cl$ |
| 0,1 - 5 g/l | $MgSO_4.7H_2O$ |
| 0,001 - 0,1 g/l | $CaCl_2$ |
| 1 - 50 mg/l | Thiamin.HCl |
| 0,5 - 100 mg/l | $(NH_4)_2Fe(SO_4)_2.6H_2O$ |
| 0,1 - 5 mg/l | $AlCl_3.6H_2O$ |
| 0,1 - 10 mg/l | $CoCl_2.6H_2O$ |
| 0,2 - 5 mg/l | $KCr(SO_4)_2.12H_2O$ |
| 0,1 - 5 mg/l | $CuSO_4.5H_2O$ |
| 0,05 - 1 mg/l | $H_3BO_3$ |
| 0,1 - 5 mg/l | $MnSO_4.H_2O$ |
| 0,1 - 5 mg/l | $NiSO_4.6H_2O$ |
| 0,1 - 5 mg/l | $Na_2MoO_4.2H_2O$ |

Fortsetzung der Tabelle auf der nächsten Seite

(fortgesetzt)

| | |
|---|---|
| 0,1 - 5 mg/l | $ZnSO_4.7H_2O$ |
| 10 - 30 g/l | Caseinhydrolysat (Merck Art.# 2238) |
| 0 - 100 g/l | Caseinhydrolysat (Sigma C9386) |
| 0,10 - 1 mg/l | Cystein |
| 0 - 10 g/l | Hefeextrakt (Difco) |
| 0 - 2 g/l | Citronensäure |
| 0 - 50 g/l | Glucose (Start) |
| 5 - 50 g/l | Glucose (Zufütterung während der Fermentation) |

Besonders geeignet ist das Medium der Zusammensetzung:

Medien: 1) Vorkultur

| | |
|---|---|
| 10 g/l | Trypton |
| 5 g/l | Hefeextrakt |
| 4 g/l | Glucose |
| 9 g/l | $Na_2HPO_4.2H_2O$ |
| 1 g/l | $NH_4Cl$ |
| 1 g/l | KCl |
| 1 ml/l | 1M $MgSO_4.7H_2O$ |
| 100 mg/l | Ampicillin |

Start-pH = 7,2

2) Hauptkultur

| | |
|---|---|
| 0,68 g/l | $(NH_4)_2HPO_4$ |
| 0,62 g/l | $K_2HPO_4.3H_2O$ |
| 2,33 g/l | KCl |
| 0,5 g/l | NaCl |
| 0,53 g/l | $NH_4Cl$ |
| 1,23 g/l | $MgSO_4.7H_2O$ |
| 0,011 g/l | $CaCl_2$ |
| 10 mg/l | Thiamin.HCl |
| 3,92 mg/l | $(NH_4)_2Fe(SO_4)_2.6H_2O$ |
| 0,72 mg/l | $AlCl_3.6H_2O$ |
| 0,71 mg/l | $CoCl_2.6H_2O$ |
| 1,5 mg/l | $KCr(SO_4)_2.12H_2O$ |
| 0,75 mg/l | $CuSO_4.5H_2O$ |
| 0,19 mg/l | $H_3BO_3$ |
| 0,51 mg/l | $MnSO_4.H_2O$ |
| 0,79 mg/l | $NiSO_4.6H_2O$ |
| 0,73 mg/l | $Na_2MoO_4.2H_2O$ |
| 0,86 mg/l | $ZnSO_4.7H_2O$ |
| 21 g/l | Caseinhydrolysat (Merck Art.# 2238) |
| 25 g/l | Caseinhydrolysat (Sigma C9386) |
| 100 mg/l | Cystein |

Fortsetzung der Tabelle auf der nächsten Seite

(fortgesetzt)

| | |
|---|---|
| 2 g/l | Hefeextrakt |
| 1 g/l | Citronensäure |
| 11 g/l | Glucose.$H_2O$ (Start bzw. Feed) |

Zur Fermentation wurde beispielsweise das Vorkulturmedium mit E.coli, transformiert mit dem entsprechenden Expressionsvektor, angeimpft und unter Rühren und Sauerstoffzufuhr inkubiert. Ein Teil dieser Vorkultur wurde dann in einen Fermenter mit dem Hauptkulturmedium überführt und unter Rühren und Belüften kultiviert. Während der Fermentationszeit wurde die Glucosekonzentration und der Sauerstoffpartialdruck beobachtet und entsprechend optimiert. Nach etwa 20 Stunden Fermentationszeit wurde der Ansatz abgekühlt, das Nährmedium von der Biomasse abgetrennt und eingefroren.

Der Nachweis der exprimierten Proteine erfolgte beispielsweise durch Western Blot, der die VAC-beta Bande zeigt. Das Ergebnis ist in Fig. 16 wiedergegeben.

"+Phosphat" ist die Kontrolle ohne Expression, "-Phosphat" zeigt die Expression von VAC-beta (Klon HB101/pRH292) Protein unter der Kontrolle des alkalischen Phosphatase Promotors. Das VAC-beta Protein ist auf dem gefärbten Gel zu erkennen. Die gebildete Menge an VAC-Proteinen beträgt überraschenderweise mindestens 20 mg/1/$OD_{600nm}$ Bakterienkultur.

Der Western Blot zeigt deutlich auch die angefärbte VAC-alpha Bande. Zusätzlich sind einige Proteine niedrigeren Molekulargewichts im Bereich bis 30 kD erkennbar, die möglicherweise durch proteolytische Spaltung am N- und/oder C-Terminus des VAC-alpha Proteins entstanden sind. Auffällig ist außerdem ein durch das Antiserum erkanntes Protein im Bereich kleiner 20 kD, das ein durch Proteolyse entstandenes Halbmolekül des VAC-alpha Proteins darstellen könnte. Überraschenderweise wurde auch VAC-beta durch Anti-VAC-Antiserum erkannt. Da diese Bande wesentlich schwächer als die VAC-alpha Bande gefärbt ist, andererseits aber die VAC-beta Bande im Coomassie Blau gefärbten Gel in ihrer Intensität dem VAC-alpha entspricht, ist daraus zu schließen, daß die Erkennung des VAC-beta Proteins durch das Anti-VAC-Antiserum wesentlich schlechter als jene des VAC-alpha Proteins erfolgt.

Zur Isolierung und Reinigung der exprimierten Proteine wurde die gefrorene Biomasse in einem geeigneten Lyse-Puffer suspendiert. Die Zellen wurden anschließend mechanisch, beispielsweise durch eine Manton-Gaulin Presse zerstört. Nach Zugabe eines Fällungsmittels für Nicht-Protein-Bestandteile, wie Polyethylenimin, wurden die festen Bestandteile beispielsweise durch Zentrifugation entfernt. Nach Ausfällung der Proteine, vorzugsweise durch Ammoniumsulfatfraktionierung, Auflösung des Präzipitates, Entfernung des Fällungsmittels und Klärung der Lösung wurde der so erhaltene Extrakt verschiedenen chromatographischen Reinigungsschritten unterworfen. Anstelle der Ausfällung der Proteine läßt sich der rohe VAC-Extrakt auch durch eine chromatographische Vorreinigung soweit reinigen, daß er anschließend einem Reinigungszyklus unterworfen werden kann. Als geeignetes Säulenmaterial für die Vorreinigung hat sich beispielsweise $SiO_2$ herausgestellt, doch sind auch andere Materialien mit ähnlichen Eigenschaften geeignet. Erfindungsgemäß wurde Silica Catalyst, Carrier, Grade 953 W der Firma Grace verwendet.

Ein für die Reinigung der erfindungsgemäßen Proteine geeigneter chromatographischer Reinigungszyklus bestand beispielsweise aus einer DEAE-Fast-Flow-Sepharose-, einer Sephacryl S-200 High Resolution- und einer Q-Sepharose-Fast-Flow-Chromatographie. Die Reinheit der so erhältlichen erfindungsgemäßen Proteine wurde mittels SDS-PAGE, Western Blot, Gelpermeations HPLC, Reverse HPLC und isoelektrischer Fokussierung bestimmt.

Die für sämtliche Kultivierungs-, Isolierungs- und Reinigungsschritte erforderlichen einzuhaltenden Parameter wie Temperatur, Mengenverhältnisse, Reihenfolge der einzelnen Schritte, pH-Werte, besondere Reagentien etc. sind dem Fachmann bestens bekannt. Die unten angegebenen Beispiele können, falls gewünscht, in geeigneter, dem Fachmann bekannter Weise abgewandelt werden.

Von besonderer Bedeutung ist die Frage, ob ein durch gentechnische Methoden hergestelltes VAC-Protein, im folgenden kurz r-VAC genannt, mit dem aus natürlichem Material erhältlichen VAC-Protein (s. EPA 0 181 465), im folgenden VAC genannt, identisch ist; identisch sowohl in seiner Struktur als auch in seinen biologischen Eigenschaften.

Zur Beantwortung dieser Fragen wurden folgende Methoden verwendet:

1. Gelpermeations-HPLC
2. Reverse Phase HPLC
3. N-terminale Sequenzierung
4. Tryptische Peptid Karte
5. SDS-Gelelektrophorese
6. Western Blot
7. Isoelektrische Fokussierung

Die Gelpermeations-HPLC weist für VAC ein Molekulargewicht von 34.000, für r-VAC von 33.000 auf, was innerhalb der Genauigkeit der Methode als gleichwertig zu betrachten ist. Es ist zu beachten, daß die verwendete Säule streng genommen nicht nach dem Molekulargewicht sondern nach der Molekülgröße differenziert.

Bei der Reverse Phase HPLC eluieren beide Proteine nach einer Retentionszeit von etwa 29 Minuten.

Die N-terminale Sequenzierung des r-VAC bis zur Aminosäure 39 ergab 100%ige Übereinstimmung mit der erwarteten Sequenz. N-terminales Methionin, oft bei gentechnisch hergestellten Proteinen zusätzlich anzutreffen, konnte überraschenderweise nicht nachgewiesen werden. Wie zu erwarten war, liegt der N-Terminus des r-VAC unblockiert vor.

Bei dem Vergleich der tryptischen Fragmentierung ergab sich ein praktisch identisches Peptidmuster.

Auch der Vergleich der beiden Proteine mittels SDS-PAGE zeigte praktisch gleichartiges Verhalten. Beide beinhalten dimere Formen, die offensichtlich über Disulfidbrücken gebunden sind und mit Dithiothreitol reduziert werden können.

Ebenso bestätigte der immunologische Vergleich durch Western Blot die Identität der beiden Proteine.

Der bei der Ermittlung des isoelektrischen Punktes festzustellende Unterschied von +0,1 pH-Einheiten bei r-VAC läßt sich durch den freien N-Terminus erklären.

Zur Überprüfung der biologischen Aktivität des r-VAC wurden verschiedene Koagulationstest durchgeführt und die Ergebnisse mit denen verglichen, die mit VAC aus natürlichem Material erhalten wurden. Alle durchgeführten Tests, der modifizierte Prothrombin Zeit Test, ebenso wie der Thrombin Test, ebenso wie die Faktor $X_a$-Generation in Gewebefaktor-aktiviertem Plasma belegen eindeutig, daß r-VAC biologische Aktivität aufweist, und daß diese nicht zu unterscheiden ist von der des VAC aus natürlichem Material.

Die aus der cDNA abgeleitete Aminosäuresequenz für VAC-beta zeigt eine 54% Homologie zu VAC-alpha. Aufgrund dieses Befundes wurden zwei biologische Aktivitäten von VAC-beta getestet.

1. Der Effekt von VAC-beta auf die Prothrombinase-Aktivität: Fig. 17 zeigt, daß VAC-beta die Prothrombinase-Aktivität inhibiert, ebenso wie VAC-alpha.

2. Phospholipase Inhibitationsaktivität: wie bereits erwähnt, läßt sich VAC-beta aufgrund seiner Primärstruktur in die Familie der Annexine einordnen. (Gusow, M.J. (1987) Febsletters 203, 99-103). Einige andere Mitglieder dieser Familie, z.B. Calpactin I und II, und VAC-alpha haben sich als Phospholipase Inhibitoren herausgestellt. Ihre Aktivität beruht auf einer Calcium-abhängigen Bindung an die Phospholipide wodurch sie den Angriff der Phospholipase auf ihr Substrat blockieren (Davidson et al. (1987) J. Biol. Chem. 262, 1698-1705.

Da nun VAC-beta Calcium-abhängig an Phospholipide bindet, wurde überprüft, ob es ebenso auch mögliche Antiphospholipase- Aktivitäten aufweist. Ein Testverfahren mit $^3$-H-Ölsäure markiertem E.coli wurde hierzu verwendet.

Die Figur 18 zeigt, daß VAC-beta die Phospholipase $A_2$ Aktivität inhibiert. 50% Inhibition wurde bei ca. 65 mM VAC-beta beobachtet. Dies entspricht der für VAC-alpha ermittelten Inhibition. Verminderung der Substrat Konzentration auf die Hälfte resultierte in einer Reduzierung der $ID_{50}$ von VAC auf die Hälfte. Dies ist ein Hinweis darauf, daß VAC auf der Substratebene agiert. Folglich findet VAC wahrscheinlich an die Phospholipidmembranen und inhibiert hierdurch die Phospholipase Aktivität.

Calpactin I bindet an Phospholipidmembranen auf dem mikromolaren $Ca^{++}$ Level (Drust, D.S. & Cremitz, C.E. (1988) Nature 331, 88-91). Daher wurde untersucht, ob VAC in der Lage ist, Phospholipase-Aktivität bei geringer Calcium Konzentration zu inhibtieren. Da die pankreatische Phospholipase $A_2$ bei $Ca^{++}$ Konzentrationen unter 50 mM inaktiv war, was das die niedrigste verwendete Konzentration. Die Figur 19 zeigt die Calcium-Abhängigkeit der VAC-beta induzierten Inhibition der Phospholipase Aktivität. Bei allen $Ca^{++}$ Konzentrationen zeigte VAC-beta Inhibitor-Aktivitäten. Doch scheint ein funktioneller Unterschied zwischen VAC-alpha und VAC-beta zu bestehen. Bei der niedrigsten $Ca^{++}$-Konzentration war VAC-beta signifikant aktiver als VAC-alpha. Dies könnte implizieren, daß VAC-beta weniger $Ca^{++}$ zur Membranbindung benötigt.

Verfahrensgemäß erhältliche VAC-Proteine können in an sich bekannter Weise in andere VAC-Proteine (Peptide) überführt werden.

Untersuchungen haben gezeigt, daß Disulfidbrücken für die gerinnungshemmende Aktivität von natürlichem VAC ohne Bedeutung ist. Je nach Wahl der Wirtszelle kann nicht ausgeschlossen werden, daß eine Verknüpfung der Cystein-Reste im primären Translationsprodukt unter Bildung von Disulfidbrücken in einer Weise erfolgt, die sich vom natürlich ablaufenden Vorgang unterscheidet. Es ist möglich, daß die sich einstellende "falsche" Tertiärstruktur des Produktes eine Verminderung oder sogar den Verlust, evtl. aber auch eine Verbesserung, der wertvollen pharmakologischen Eigenschaften, insbesondere der gerinnungshemmenden Aktivität, zur Folge hat, was sich mit Hilfe der oben genannten VAC-Assays feststellen läßt. In einem solchen Fall ist es gegebenenfalls zweckmäßig, die Disulfidbindungen mit einem geeigneten Reduktionsmittel zu spalten und das reduzierte Polypeptid zur Neuknüpfung der Disulfidbindungen mit einem geeigneten Oxidationsmittel zu behandeln. Anhand der VAC-Aktivität des gebildeten Produktes läßt sich feststellen, ob die gewählten Bedingungen (Reduktions-und/oder Oxidationsmittel) zur gewünschten Steigerung der biologischen Aktivität geführt haben oder ob die Bedingungen in bekannter Weise modifiziert werden müssen.

Zur Aufspaltung von Disulfidbrücken geeignete Reduktionsmittel sind beispielsweise Thiol-Verbindungen, wie Thiophenol, 4-Nitrothiophenol, 1,4-Butandithiol und insbesondere 1,4-Dithiothreit.

Die Reduktion wird vorteilhaft in einem wäßrig-alkalischen Medium, beispielsweise in der verdünnten wässrigen Lösung eines Alkalimetallhydroxids, z.B. Natriumhydroxid, Alkalimetallcarbonats, z.B. Natriumcarbonats, oder einer organischen Base, insbesondere eines Triniederalkylamins, z.B. Triäthylamin, bei Raumtemperatur durchgeführt.

Oxidationsmittel, welche zur Neuknüpfung von Disulfidbindungen in den reduzierten Polypeptiden geeignet sind, sind beispielsweise Luftsauerstoff, welcher durch eine wässrige Lösung des Polypeptids, der gegebenenfalls eine katalytische Menge eines Übergangsmetallsalzes, z.B. Eisen-(III)-sulfat, Eisen-(III)-chlorid oder Kupfer-(II)-sulfat, beigefügt wurde, geleitet wird; Jod, auch in Form des Kaliumjodid-Adduktes $KJ_3$, welches vorzugsweise in alkoholischer, z.B. methanolischer, oder wässrig-alkoholischer, z.B. wässrig-methanolischer Lösung eingesetzt wird; Kaliumhexacyanoferrat-(III) in wässriger Lösung; 1,2-Dijodäthan oder Azodicarbonsäuredimethylester oder -diäthylester, welche in Wasser oder in einer Mischung bestehend aus Wasser und einem mit Wasser mischbaren Alkohol, z.B. Methanol, zur Reaktion gebracht werden. Die Oxidation wird insbesondere bei Raumtemperatur ausgeführt.

Die Abtrennung der Reagentien, insbesondere der Salze und der Oxidations- bzw. der Reduktionsmittel und ihrer Folgeprodukte, von der gewünschten VAC-Verbindung erfolgt nach an sich bekannten Methoden, beispielsweise durch Molekulargewichtsfiltration, z.B. an Sephadex oder Biogel.

Ein verfahrensgemäß erhältliches Gemisch von Verbindungen mit VAC-Aktivität kann in an sich bekannter Weise in die einzelnen Komponenten aufgetrennt werden. Geeignete Trennverfahren sind beispielsweise chromatographische Verfahren, z.B. Adsorptions-Chromatographie, Ionenaustauschchromatographie, HPLC oder Reverse-Phase HPLC, ferner multiplikative Verteilung oder elektrophoretische Methoden, z.B. Elektrophorese an Celluloseacetat oder Gelelektrophorese, insbesondere Polyacrylamid- Gelelektrophorese ("PAGE").

Die erfindungsgemäß herstellbaren Verbindungen können nicht nur in freier Form. sondern auch in Form ihrer Salze, insbesondere ihrer pharmazeutisch annehmbaren Salze, vorliegen. Da sie mehrere Aminosäurereste mit freien Aminogruppen enthalten, können die erfindungsgemäßen Verbindungen z.B. in Form von Säureadditionssalzen vorliegen. Als Säureadditionssalze kommen insbesondere physiologisch verträgliche Salze mit üblichen, therapeutisch anwendbaren Säuren in Betracht; als anorganische Säuren sind die Halogenwasserstoffsäuren, wie die Chlorwasserstoffsäure, aber auch Schwefelsäure und Phosphor- bzw. Pyrophosphorsäure zu nennen; als organische Säuren sind in erster Linie Sulfonsäuren, wie die Benzol- oder p-Toluosulfonsäure oder Niederalkansulfonsäuren, wie Methansulfonsäure, sowie Carbonsäuren, wie Essigsäuren, Milchsäure, Palmitin - und Stearinsäure, Äpfelsäure, Weinsäure, Ascorbinsäure und Citronensäure geeignet. Da die VAC-Verbindungen auch Aminosäurereste mit freien Carboxylgruppen enthalten, können sie auch als Metallsalz, insbesondere als Alkalimetall- oder Erdalkalimetallsalz, z.B. Natrium-, Calcium- oder Magnesiumsalz, oder auch als Ammoniumsalz, abgeleitet von Ammoniak oder einer physiologisch verträglichen, organischen stickstoffhaltigen Base, vorliegen. Da sie aber zugleich freie Carboxylgruppen und freie Aminogruppen enthalten, können sie auch als innere Salz vorliegen.

Je nach Arbeitsweise erhält man die erfindungsgemäßen Verbindungen in freier Form, in Form von Säureadditionssalzen oder Salzen mit Basen. Aus den Säureadditionssalzen und den Salzen mit Basen können in an sich bekannter Weise, beispielsweise durch Einstellen des pH-Wertes auf den isoelektrischen Punkt, die freien Verbindungen gewonnen werden. Von letzteren wiederum lassen sich durch Umsetzen mit Säuren bzw. Basen, z.B. mit solchen, die die oben genannten Salze bilden, und Eindampfen oder Lyophilisieren therapeutisch annehmbare Säureadditionssalze bzw. Salze mit Basen gewinnen.

Die Eigenschaft von Antikörpern, spezifische Antigene zu binden, findet außerhalb des Körpers praktische Anwendung bei der qualitativen und quantitativen Bestimmung (Immuno-Assay) und bei der Reinigung der Antigene (Immunoaffinitätschromatographie). Serum immunisierter Tiere enthält normalerweise eine Vielzahl verschiedener Antikörper, die mit dem gleichen Antigen an verschiedenen Bindungsstellen mit verschiedener Affinität reagieren, dazu aber auch Antikörper gegen andere Antigene, die die früheren Erfahrungen des Individuums widerspiegeln. Die erfolgreiche Anwendung von Antikörpern zur Bestimmung und Reinigung von Antigenen erfordert aber hohe Spezifität und Reproduzierbarkeit.

Homogene Antikörper, die diese Anforderungen erfüllen, sind durch die von Köhler und Milstein (17) beschriebene Hybridoma-Technik zugänglich geworden. Prinzipiell besteht die Technik darin, daß Antikörper ausscheidende B-Lymphozyten, z.B. aus der Milz, immunisierter Tiere mit Tumorzellen verschmolzen ("fusioniert") werden. Die gebildeten Hybridoma-Zellen kombinieren die Fähigkeit zur unbegrenzten Vermehrung durch Teilung mit der Fähigkeit, einen einheitlichen Typ Antikörper zu bilden und auszuscheiden. Durch Kultivierung in einem selektiven Medium, in dem nicht fusionierte Tumorzellen absterben, Hybridoma-Zellen sich aber vermehren, und durch geeignete Manipulationen können Klone, d.h. Zellpopulationen, die sich von einer einzigen Hybridoma-Zelle ableiten und genetisch identisch sind, gewonnen und kultiviert und die durch die Zellen produzierten monoklonalen Antikörper isoliert werden.

Die vorliegende Erfindung betrifft monoklonale Antikörper gegen VAC, Hybridomazellen, die solche Antikörper produzieren und Verfahren zu ihrer Herstellung. Bevorzugt sind Hybridomazellinien und die von diesen ausgeschiedenen monoklonalen Antikörper, die spezifisch mit VAC reagieren. Das Verfahren zur Herstellung von monoklonalen Anti-VAC-beta-Antikörpern ist dadurch gekennzeichnet, daß man Mäuse mit VAC-beta immunisiert, B-Lymphozyten derart immunisierter Tiere mit Myelomazellen fusioniert, die gebildeten Hybridomazellen kloniert, dann in vitro oder durch Injektion in Mäusen kultiviert und aus den Kulturen Antikörper isoliert.

Die Erfindung betrifft ferner Immuno-Affinitätschromatographie-Säulen und Test-Kits für Immunoassays, die diese Antikörper enthalten.

Nach dem erfindungsgemäßen Verfahren werden Mäuse, z.B. Balb/c-Mäuse, auf an sich bekannte Weise immunisiert. In einer bevorzugten Ausführungsform wird VAC etwa wöchentlich oder auch in größeren Abständen während mehreren Wochen, beispielsweise 5 bis 12 Wochen, injiziert, bis sich eine genügende Zahl Antikörper-produzierender B-Lymphozyten gebildet hat.

Zur Steigerung der Immunogenität des eingesetzten VAC wurde es an stark immunogene Träger wie beispielsweise heterologes Albumin oder "keyhole limpet hemocyanin" (KLH) gekoppelt. Bevorzugt wurden verschiedene VAC/KLH Präparationen eingesetzt, wobei nach einem Immunisierungsschema so lange immunisiert wurde, bis sich genügend Antikörper-produzierende Zellen gebildet hatten, beispielsweise bis zu ca. 40 Wochen. B-Lymphozyten enthaltende Organe,z. B. Milzzellen, der immunisierten Mäuse werden entnommen und mit solchen Myelomazellen fusioniert, die aufgrund einer Mutation in einem selektiven Kulturmedium nicht wachsen. Solche Myelomazellen sind bekannt und sind beispielsweise jene mit der Bezeichnung X63-Ag8, X63-Ag8.6.5.3, MPC-11, NS1-Ag4/1, MOPC-21 NS/1 oder SP 2/0. In einer bevorzugten Ausführungsform werden Milzzellen immunisierter Mäuse mit Myelomazellen der Zell-Linie X63-Ag8.6.5.3 fusioniert.

Die Fusion wird nach an sich bekannten Verfahren durch Mischen der B-Lymphozyten und der Myelomazellen unter Zugabe eines Zellfusionsagens, wie Polyethylenglykol, Sendai-Virus, Calciumchlorid oder Lysolecithin durchgeführt. Vorzugsweise wird in Gegenwart von Polyethylenglykol, beispielsweise mit einem Molekulargewicht zwischen 1000 und 4000, fusioniert.

Nach der Fusion werden die entstandenen Hybride nach einem an sich bekannten Verfahren in einem selektiven Kulturmedium, das mit Hypoxanthin, Aminopterin und Thymidin (HAT-Medium) komplementiert ist, kultiviert. Nicht fusionierte Myelomazellen können in diesem Medium nicht wachsen und sterben ebenso wie normale Lymphozyten.

Die Überstände der Hybridoma-Kulturen können mit an sich bekannten Verfahren auf ihren Gehalt an spezifischen Antikörpern geprüft werden, beispielsweise mit Radio-Immunoassay, ELISA oder Agglutinierung. Dabei wird überraschenderweise festgestellt, daß mit dem beschriebenen Verfahren Hybridoma-Zellen gewonnen werden können, die Antikörper spezifisch gegen VAC ausscheiden. Die Hybridomazellen, die Antikörper der gewünschten Spezifität produzieren, werden aus dem aus der Fusionierung hervorgegangenen Gemisch verschiedenster Hybridomazellen durch Klonieren herausselektioniert. Dazu werden nach einem an sich bekannten Verfahren, das "limiting dilution" genannt wird, Kulturen ausgehend von einer einzigen wachsenden Zelle angesetzt.

Zur Massenproduktion werden die Hybridoma-Zellklone, die Antikörper der gewünschten Spezifität produzieren, entweder in an sich bekannten Medien in vitro kultiviert oder zur Vermehrung in Mäuse injiziert. In einer bevorzugten Ausführungsform werden Hybridomazellen in mit Pristan vorbehandelte Mäuse injiziert, Aszites-Flüssigkeit entnommen und daraus durch Fällung mit Ammoniumsulfat-Lösung Antikörper isoliert.

Die mit Hilfe dieser Hybridomazellen gewonnenen VAC spezifischen Antikörper können auf an sich bekannte Weise für die Herstellung von Immuno-Affinitätschromatographie- Säulen verwendet werden. In einer bevorzugten Ausführungsform der Erfindung wird ein geeignetes Trägermaterial (suspendiert in einer Pufferlösung) mit einer Antikörper-Lösung versetzt, ungebundene Anteile werden anschließend ausgewaschen und unbesetzte Stellen des Trägermaterials blockiert.

Die mit Hilfe der Hybridomazellen gewonnenen VAC spezifischen Antikörper können auf an sich bekannte Weise für die Herstellung von Test-Kits verwendet werden. Diese Test-Kits können auf verschiedenen Methoden beruhen, beispielsweise auf Radio-Immuno-Assay, Latex-Agglutinierung, Tüpfel-Tests, Kompetitive oder Sandwich-Radio-Immunoassay, Enzym-Immunoassay, Immuno-Fluoreszenz oder immunochemischen Enzym-Tests. Solche Kits können neben gewöhnlichen Antikörpern verschiedener Herkunft Antikörper-Konjugate mit Enzymen oder Fluoreszenzträgern enthalten, dazu VAC markiert mit radioaktiven Isotopen wie $J^{125}$, oder konjugiert mit Enzymen, beispielsweise mit Meerrettich-Peroxidase oder alkalischer Phosphatase, ferner Enzymsubstrate, geeignete Puffer, Gele, Latex, Polystyrol oder andere Füllmaterialien und Träger.

Die gemäß der vorliegenden Erfindung erhältlichen bekannten Proteine (Peptide) weisen wertvolle pharmakologische Eigenschaften auf und können prophylaktisch oder insbesondere therapeutisch angewendet werden.

Die erfindungsgemäßen neuen VAC Verbindungen können daher in Analogie zu natürlichem VAC zur Therapie und Prophylaxe von Thrombosen und Thromboembolien, einschließlich zur Prophylaxe von postoperativen Thrombosen, zur akuten Schock-Therapie (z. B. bei septischem oder polytraumatischem Schock), zur Therapie von Verbrauchskoagulopathien, bei Hämodialysen, Hämoseparationen, Blutkonserven und im extrakorporalen Kreislauf verwendet werden.

Die Erfindung betrifft ebenfalls pharmazeutische Zusammensetzungen, welche wenigstens eine der erfindungsgemäßen Verbindungen oder deren pharmazeutisch annehmbaren Salze, gegebenenfalls zusammen mit einem pharmazeutisch annehmbaren Träger und/oder Hilfsstoffen, enthalten. Diese Zusammensetzungen können insbesondere bei den oben angegebenen Indikationen Verwendung finden, wenn sie z. B. parenteral, wie intravenös, intracutan, subcutan oder intramuskulär, oder topisch verabreicht werden.

Die Erfindung betrifft ebenfalls die Verwendung der erfindungsgemäßen neuen Verbindungen und diese enthaltende pharmazeutische Zusammensetzungen für die prophylaktische und therapeutische Behandlung des menschlichen und tierischen Körpers, insbesondere bei den oben angegebenen Krankheitsbildern, in erster Linie zur Hemmung der Gerinnung des Blutes innerhalb und außerhalb des menschlichen und tierischen Körpers.

Die Dosierung hängt in erster Linie von der spezifischen Verabreichungsform und vom Zweck der Therapie bzw. Prophylaxe ab. Die Größe der Einzeldosen sowie das Verabreichungsschema kann am besten anhand einer individuellen Beurteilung des jeweiligen Krankheitsfalles bestimmt werden: die dazu erforderlichen Methoden zur Bestimmung von relevanten Blutfaktoren sind dem Fachmann geläufig. Im Normalfall liegt bei einer Injektion die therapeutisch wirksame Menge der erfindungsgemäßen Verbindungen im Dosisbereich von etwa 0,005 bis etwa 0,1 mg/kg Körpergewicht. Bevorzugt wird der Bereich von etwa 0,01 bis etwa 0,05 mg/kg Körpergewicht. Die Verabreichung erfolgt durch intravenöse, intramuskuläre oder subcutane Injektion. Dementsprechend enthalten pharmazeutische Präparate zur parenteralen Verabreichung in Einzeldosis-Form in Abhängigkeit von der Applikationsart pro Dosis etwa 0,4 bis etwa 7,5 mg der erfindungsgemäßen Verbindung. Neben dem Wirkstoff enthalten diese pharmazeutischen Zusammensetzungen üblicherweise noch einen Puffer, z. B. einen Phosphatpuffer, der den pH-Wert zwischen etwa 3,5 und 7 halten soll, und ferner Natriumchlorid, Mannit oder Sorbit zur Einstellung der Isotonie. Sie können in gefriergetrockneter oder gelöster Form vorliegen, wobei Lösungen ein antibakteriell wirkendes Konservierungsmittel, z. B. 0,2 bis 0,3 % 4-Hydroxybenzoesäuremethylester oder -ethylester, enthalten können. Ein Präparat für die topische Anwendung kann als wässrige Lösung, Lotion oder Gelee, ölige Lösung oder Suspension, oder fetthaltige oder insbesondere Emulsions-Salbe vorliegen. Ein Präparat in Form einer wässrigen Lösung erhält man beispielsweise dadurch, daß man die erfindungsgemäßen Wirkstoffe oder ein therapeutisch annehmbares Salz davon in einer wässrigen Pufferlösung von pH 4 bis 6,5 löst und gewünschtenfalls einen weiteren Wirkstoff, z. B. ein Antiinflammatorikum, und/oder ein polymeres Haftmittel, z. B. Polyvinylpyrrolidon, und/oder ein Konservierungsmittel zufügt. Die Konzentration des Wirkstoffs beträgt etwa 0,1 bis etwa 1,5 mg, vorzugsweise 0,25 bis 1,0 mg, in 10 ml einer Lösung bzw. 10 g eines Geles.

Eine ölige Applikationsform für die topische Verabreichung erhält man beispielsweise durch Suspendieren der erfindungsgemäßen Wirkstoffe oder eines therapeutisch annehmbaren Salzes davon in einem Oel, gegebenfalls unter Zusatz von Quellmitteln, wie Aluminiumstearat, und/oder grenzflächenaktiven Mitteln (Tensiden), deren HLB-Wert ("hydrophilic-lipophilicbalance") unter 10 liegt, wie Fettsäuremonoester mehrwertiger Alkohole, z. B. Glycerinmonostearat, Sorbitanmonolaurat, Sorbitanmonostearat oder Sorbitanmonooleat. Eine fetthaltige Salbe erhält man z. B. durch Suspendieren der erfindungsgemäßen Wirkstoffe oder deren Salze in einer streichbaren Fettgrundlage, gegebenenfalls unter Zusatz eines Tensids vom HLB-Wert unter 10. Eine Emulsionssalbe erhält man durch Verreiben einer wässerigen Lösung der erfindungsgemäßen Wirkstoffe oder deren Salze in einer weichen, streichbaren Fettunterlage unter Zusatz eines Tensids, dessen HLB-Wert unter 10 liegt. Alle diese topischen Applikationsformen können auch Konservierungsmittel enthalten. Die Konzentration des Wirkstoffes beträgt 0,1 bis 1,5 mg, vorzugsweise 0,25 bis 1,0 mg, in etwa 10 g der Grundmasse.

Neben den oben beschriebenen und ihren analogen pharmazeutischen Zusammetzungen, welche für einen direkten medizinischen Einsatz am Körper des Menschen oder eines Säugetieres bestimmt sind, betrifft die vorliegende Erfindung auch pharmazeutische Zusammensetzungen und Präparate zur medizinischen Anwendung außerhalb des lebenden Körpers des Menschen oder der Säugetiere. Solche Zusammensetzungen und Präparate verwendet man in erster Linie als gerinnungshemmenden Zusatz zu Blut, welches außerhalb des Körpers einer Zirkulation oder Behandlung (z.B. extrakorporaler Kreislauf oder Dialyse in künstlichen Nieren), Konservierung oder Modifizierung (z.B. Hämoseparation) unterzogen wird. In ihrer Zusammensetzung sind derartige Präparate, wie Vorratslösungen oder auch Zubereitungen in Einzeldosis-Form, den oben beschriebenen Injektionspräparaten ähnlich; zweckmäßigerweise wird aber die Wirkstoffmenge bzw. -konzentration auf das Volumen des zu behandelnden Blutes bezogen. Je nach dem spezifischen Zweck beträgt die geeignete Dosis etwa 0,01 bis etwa 1,0 mg Wirkstoff/1 Blut, wobei die obere Grenze ohne Gefahr noch überschritten werden darf.

Insbesondere betrifft die Erfindung die in den Belegbeispielen beschriebenen, für die für VAC-Proteine kodierenden DNA-Moleküle, solche DNA-Moleküle enthaltende Expressionsplasmide, mit solchen Expressionsplasmiden transformierte Mikroorgansimen, monoklonale Antikörper gegen VAC, Hybridoma-Zellen, die solche Antikörper produzieren, und Test-Kits für Immunoassays, die solche Antikörper enthalten, die in den Beispielen beschriebenen Verfahren zu ihrer Herstellung und das in den Beispielen beschriebene Verfahren zur Herstellung von Proteinen/Polypeptiden mit VAC-Aktivität mit Hilfe der transformierten Mikroorganismen, sowie die in den Beispielen beschriebenen neuen VAC-Verbindungen.

Wird in der vorliegenden Anmeldung von vascular-antikoagulierenden Proteinen oder in der Kurzform VAC-Protein gesprochen, so bedeutet das wenn nicht anders angegeben, daß hiermit Polypeptide/ Proteine gemeint sind, die im wesentlichen die Eigenschaften aufweisen, die den Proteinen eigen sind, die in der EPA 181 465 erstmals beschrieben wurden. Diese Eigenschaften lassen sich durch die dort angegebenen Test- und Charakterisierungsverfahren feststellen und überprüfen (VAC-Aktivität).

Weiterhin fallen unter diese Definition auch die Polypeptide/Proteine, die Aggregationen wie beispielsweise Dimere, Trimere oder Tetramere darstellen, auch wenn diese in der aggregierten Form per se nicht oder nur eingeschränkte biologische Aktivitäten aufweisen, unter der Voraussetzung, daß diese in vivo oder in vitro in zumindest eine aktive Komponente zu überführen sind.

Werden im Rahmen der vorliegenden Erfindung Polypeptide/ Proteine erhalten, die per se nicht oder nur eingeschränkte biologische Aktivitäten aufweisen, beispielsweise Fusionsproteine oder sogenannte "pro-drugs", Polypeptide/Proteine, die für den Fachmann in an sich bekannter Weise in die aktiven Komponenten überführbar sind, beispielsweise durch in vitro oder in vivo Prozessierung oder Polypeptide/Proteine, die erst in vivo ihre Aktivität entfalten, so sollen auch diese unter die Definition vascular-antikoagulierende-, kurz VAC-Proteine gefaßt sein und damit zum Gegenstand der vorliegenden Erfindung gehören.

Unter "Verbindungen mit VAC-Aktivität" werden auch solche, von den genannten transformierten Wirtszellen exprimierte Polypeptide verstanden, welche eine VAC-Wirkung und eine positive Reaktion mit anti-VAC-Antikörpern zeigen und welche die Primärstruktur von VAC oder eine davon abgeleitete Struktur aufweisen. Unter VAC-Verbindungen mit einer von der Primärstruktur von VAC abgeleiteten Struktur werden modifizierte VAC-Verbindungen verstanden, wobei die Modifizierung in einer Verkürzung der Primärstruktur des VAC, in einer Umordnung der Repeat-Struktur oder in einer Modifizierung , die zu einer Veränderung der Stabilität bzw. Aktivität führt.

VAC-DNA/Gen steht in der vorliegenden Erfindung für die DNA-Moleküle, die für die oben definierten VAC-Proteine kodieren.

Die folgenden Beispiele und Zeichnungen dienen zur Illustration der Erfindung und sollen sie in keiner Weise einschränken.

Um die nachfolgenden Beispiele zu vereinfachen, werden oft wiederkehrende Methoden kurz beschrieben.

Plasmide werden mit einem kleinen "p" bezeichnet, gefolgt von Großbuchstaben und Ziffern. Ausgangsplasmide sind käuflich oder ohne Einschränkung öffentlich erhältlich. Sie können auch aus solchen Plasmiden mittels publizierter Methoden konstruiert werden.

"Schneiden" oder "Verdauen" von DNA bezieht sich auf die katalytische Spaltung der DNA mittels Restriktionsendonukleasen (Restriktionsenzyme) an für diese spezifischen Stellen, Restriktionsstellen genannt. Restriktionsendonukleasen sind käuflich erhältlich und werden unter den von den Herstellern empfohlenen Bedingungen (Puffer, Rinderserumalbumin (BSA) als Trägerprotein, Dithiothreit (DTT) als Oxidationsschutz) eingesetzt.

Restriktionsendonukleasen werden mit einem Großbuchstaben, meist gefolgt von Kleinbuchstaben und normalerweise einer römischen Ziffer bezeichnet. Die Buchstaben hängen von dem Mikroorganismus ab, aus dem die betreffende Restriktionsendonuklease isoliert wurde (z.B.: Sma I: Serratia marcescens). Üblicherweise wird etwa 1µg DNA mit einer oder mehreren Einheiten des Enzyms in etwa 20 µl Pufferlösung geschnitten. Normalerweise wird eine Inkubationsdauer von 1 Stunde bei 37°C verwendet, kann aber laut den Verwendungsvorschriften des Herstellers variiert werden. Nach dem Schneiden wird manchmal die 5'Phosphatgruppe durch Inkubation mit alkalischer Phosphatase aus Kalbsdarm (CIP) entfernt. Dies dient zur Verhinderung einer ungewünschten Reaktion der spezifischen Stelle in einer nachfolgenden Ligasereaktion (z.B. Zirkularisierung eines linearisierten Plasmids ohne Insertierung eines zweiten DNA-Fragmentes). Wenn nicht anders angegeben, werden DNA-Fragmente nach dem Schneiden mit Restriktionsendonukleasen normalerweise nicht dephosphoryliert. Reaktionsbedingungen für die Inkubation mit alkalischer Phospatase sind z.B. dem M13 Cloning und Sequencing Handbuch (Cloning and Sequencing handbook, Fa Amersham, PI/129/83/12) zu entnehmen.

Nach der Inkubation wird Protein durch Extraktion mit Phenol und Chloroform entfernt, und die DNA aus der wäßrigen Phase durch Zusatz von Äthanol präzipitiert.

"Isolierung" eines bestimmten DNA Fragments bedeutet die Auftrennung der geschnittenen DNA auf einem z.B. 1% Agarosegel. Nach der Elektrophorese und dem Sichtbarmachen der DNA im UV-Licht durch Anfärben mit Äthidiumbromid (EtBr) wird das gewünschte Fragment anhand mitaufgetragener Molekulargewichtsmarker lokalisiert und durch weitere Elektrophorese an DE 81 Papier (Schleicher und Schuell) gebunden. Die DNA wird durch Spülen mit Niedrigsalzpuffer (200 mM NaCl, 20 mM Tris pH=7,5, 1 mM EDTA) gewaschen und anschließend mit einem Hochsalzpuffer (1 M NaCl, 20 mM Tris pH=7,5, 1 mM EDTA) eluiert. Die DNA wird durch Zusatz von Äthanol präzipitiert.

"Southern Analyse" ist jene Methode, durch die die Gegenwart eines bestimmten DNA-Fragments in einem DNA-Gemisch durch Hybridisierung mit einer bekannten, markierten Oligonukleotidsonde oder einem markierten DNA Fragment nachgewiesen wird. Southern Analyse bedeutet im folgenden, so nicht anders spezifiziert, die Auftrennung des DNA Gemischs auf einem 1% Agarosegel, Denaturierung und Transfer auf Nitrozellulosefilter (Schleicher und Schuell, BA 85) mittels der Methode von E. Southern, J. Mol. Biol. 98 (1978), pp.503-517, und Hybridisierung wie beschrieben in R. Hauptmann et al., Nucleic Acids Res. 13 (1985), pp.4739-4749.

"Transformation" bedeutet das Einbringen von DNA in einen Organismus, so daß die DNA dort replizierbar ist, entweder extra-chromosomal oder als chromosomale Integrante. Transformation von E.coli folgt der im M13 Cloning and Sequencing Handbuch (Cloning and Sequencing Handbook, Fa Amersham, PI/129/83/12) angegebenen Methode.

"Sequenzieren" einer DNA bedeutet die Analyse der Nukleotidsequenz in einer DNA. Dazu wird die DNA mit verschiedenen Restriktionsenzymen geschnitten, und die Fragmente in entsprechend geschnittene M13 mp8, mp9, mp18 oder mp19 Doppelstrang DNA eingebracht, oder die DNA wird mittels Ultraschall, nachfolgender Reparatur der Enden und Größenselektion in Sma I geschnittene, dephosphorylierte M13 mp8 DNA (Shotgun Methode) eingebracht. Nach der Transformation von E.coli JM 101 wird Einzelstrang DNA aus rekombinanten M13 Phagen entsprechend dem M13 Cloning and Sequencing manual (Cloning and Sequencing Handbook, Fa Amersham, PI/129/83/12) isoliert und nach der Sanger'schen Dideoxymethode (F. Sanger et al., Proc. Natl. Acad. Sci. 74 (1977), pp.5463-5467) sequenziert. Die Auswertung der Sequenzen erfolgt mittels der ursprünglich von R. Staden entwickelten (R. Staden, Nucleic Acids Res. 10 (1982), pp.4731-4751) und von Ch. Pieler modifizierten (C. Pieler 1987, Dissertation, Universität Wien) Computerprogramme.

"Ligieren" bezieht sich auf den Prozeß der Bildung von Phosphodiesterbindungen zwischen zwei Enden von Doppelstrang-DNA Fragmenten. Üblicherweise werden zwischen 0,02 und 0,2 µg DNA-Fragmente in 10 µl mit etwa 5 units T4-DNA Ligase ("Ligase") in einer geeigneten Pufferlösung ligiert (T.Maniatis et al., Molecular cloning, 1982, p.474).

"Präparation" von DNA aus Transformanten bedeutet die Isolierung der Plasmid DNA aus Bakterien mittels der alkalischen SDS Methode modifiziert nach Birnboim und Doly (T.Maniatis et al., Molecular cloning, 1982, pp.368-369) unter Weglassen des Lysozyms. Dabei werden die Bakterien aus 1,5 bis 50 ml Kultur verwendet.

"Oligonukleotide" sind kurze Polydesoxynukleotide, die chemisch synthetisiert werden. Dazu wurde der Applied Biosystems Synthesizer Modell 381A verwendet. Die Oligonukleotide werden entsprechend dem Modell 381A User Manual (Applied Biosystems) aufgearbeitet und durch Polyacrylamidgelelektrophorese (PAGE) gereinigt.

"Phosphorylieren" bedeutet die enzymatische Uebertragung des gamma-Phosphatrestes aus ATP auf eine freie 5'OH-gruppe einer Nukleinsäure, meist ein Oligonukleotid. In 10 µl Lösung werden bis zu 100 pMol des Oligonukleotids mit 10 Einheiten T 4 -Poly-nukleotidkinase in Gegenwart von 100 pMol ATP in geeigneter Pufferlösung (70 mM Tris, pH=7,6, 10 mM $MgCl_2$, 5 mM DTT) 30 Minuten bei 37'C phosphoryliert. Die Reaktion wird meist durch 10 Minuten Erhitzen auf 100'C gestoppt.

Einige verwendete Abkürzungen sollen kurz erklärt werden:

| | |
|---|---|
| bp: | Basenpaare |
| BSA: | Rinderserumalbumin |
| DTT: | Dithiothreit |
| EDTA: | Aethylendinitrilotetraessigsäure, diNatriumsalz |
| SDS: | Natriumdodecylsulfat |
| Tris: | Tris(hydroxymethyl)-aminomethan |
| Denhardt: | 0,02% Ficoll, 0,02% Polyvinylpyrrolidon, 0,02% BSA |
| LB: | 10 g/l Trypton, 5 g/l Hefeextrakt, 5 g/l NaCl |
| lx SSC: | 150 mM NaCl, 15 mM tri-Natriumcitrat, pH=7 |
| TE: | 10 mM Tris pH=8,0, 1 mM EDTA |

Verzeichnis der Abbildungen:

1. Tryptische Fragmente aus Placenta-VAC
2. Screening-Oligonukleotide EBI-386, -387 und -388
3. Screening-Oligonukleotide EBI-118 und EBI-119
4. Northern Blot Analyse mit VAC-alpha und VAC-beta cDNA
5. VAC-beta cDNA Sequenz
6. Aminosäurezusammensetzung von VAC-beta
7. Vierfach wiederholte Subsequenz im VAC-beta Protein
8. Genomische Southern Blot Analyse mit VAC-alpha und VAC-beta cDNA
9. Aminosäurevergleich von VAC-alpha mit VAC-beta

10. Nukleotidvergleich zwischen VAC-alpha und -beta cDNA

11. Hydrophilizitätsplot von VAC-alpha und VAC-beta

12. Vergleich von VAC-alpha, VAC-beta, Lipocortin I und Lipocortin II

13. Konstruktion von pRH212

14. Konstruktion von pRH292

15. Konstruktion von pGN25 und pGN26

16. SDS-Gelelektrophorese der exprimierten Proteine

a) Coomassie Blau gefärbtes Proteingel
b) Western Blot

| | |
|---|---|
| Legende: M = | Molekulargewichtsmarke |
| +Phosphat = | Inhibition der VAC Expression |
| -Phosphat = | VAC Expression (pho Promotor induziert) |

17. Effekt von VAC-beta auf die Prothrombinase Aktivität. Prothrombin Aktivierung wurde in Anwesenheit variierender Mengen VAC-beta wie beschrieben gemessen.

18. Effekt von VAC-beta auf die Phospholipase $A_2$ Aktivität. Die Bestimmung der % Inhibition erfolgte wie beschrieben. Inhibition wurde entweder bei 13,2 µM Phospholipid o (offene Kreise) oder bei 6,6 µM Phospholipid gemessen (ausgefüllte Kreise).

19. Auswirkung von $Ca^{++}$ auf die VAC-beta induzierte Inhibition der Phospholipase Aktivität. Die Inhibition wurde bei 6,6 µM Phospholipid und 1 mM (offener Kreis), 0,5 mM (ausgefüllter Kreis), 0,1 mM (offenes Quadrat) oder 0,05 mM $Ca^{++}$ (ausgefülltes Quadrat) bestimmt.

20. Modifizierter Prothrombin Zeit Test mit natürlichem und rekombinantem VAC.

21. Thrombin Zeit Test mit natürlichem und rekombinantem VAC.

22. Bindung von VAC an Phospholipid-Doppelschichten

23. Gelpermeation - HPLC von rekombinantem VAC-beta.

24. Reverse Phase HPLC von rekombinantem VAC-beta.

25. Reverse Phase HPLC von rekombinantem VAC-beta nach Inkubation.

26. Aminosäureanalyse von rekombinantem VAC-beta.

27. N-terminale Sequenzierung von rekombinantem VAC-beta.

28. SDS-Gel von rekombinantem VAC-beta in An- oder Abwesenheit von DTT.

29. Isoelektrische Fokussierung von rekombinantem VAC-beta.

30. Reinigung von rekombinantem VAC-beta; Coomassie Blau gefärbtes SDS-Gelelektrophorese-Gel.

31. Reinigung von rekombinantem VAC-beta; in process Proben.

32. Anordnung der Peptidsequenzen in der VAC-alpha cDNA abgeleiteten Aminosäuresequenz

33. Vierfach wiederholte Subsequenz im VAC-alpha Protein

34. Sequenz des Promotor- und Terminator Teils in pRH284

35. Konstruktion von pRH291

36. Reinigung von VAC-alpha; Coomassie Blau gefärbtes SDS-Gelelektrophorese-Gel
Banden:

1: Roher Extrakt
2: Ammoniumsulfatpellet (gelöst und dialysiert) 12: 5 µg DEAE-FF-Sepharose Fraktionen 1-11
3: VAC-Pool nach DEAE-FF-Sepharose Chromatographie
4: VAC-Pool nach Sephacryl-S 200 HR Chromatographie
5: gereinigtes VAC nach Q-Sepharose-FF-Chromatographie
6: gereinigtes, natürliches VAC aus Human-Placenta
7: Molekulargewichtsmarker (Pharmacia; 94 kD, 67 kD, 43 kD, 30 kD, 20 kD und 14 kD)

37. Sephacryl S-200 HR Chromatographie von vorgereinigtem r-VAC-alpha

38. Q-Sepharose-FF-Chromatographie von vorgereinigtem r-VAC-alpha

39. Gelpermeations-HPLC von natürlichem VAC

40. Gelpermeations-HPLC von rekombinantem VAC-alpha

41. Reverse Phase HPLC von natürlichem VAC

42. Reverse Phase HPLC von rekombinantem VAC-alpha

43. HPLC der tryptischen Fragmente aus natürlichem VAC

44. HPLC der tryptischen Fragmente aus rekombinantem VAC-alpha

45. SDS-Gel von einem Vergleich zwischen natürlichem und rekombinantem VAC-alpha in An- oder Abwesenheit von DTT

46. SDS-Gel von rekombinantem VAC-alpha in An- oder Abwesenheit von DTT

47. Western Blot Analyse von natürlichem VAC und rekombinantem VAC-alpha

48. Isoelektrische Fokussierung von natürlichem VAC und rekombinantem VAC-alpha

49. Faktor Xa Bildung im Plasma durch natürliches und rekombinantes VAC

50. Effekt von VAC-alpha auf die Phospholipase $A_2$ Aktivität. Phospholipase $A_2$ Aktivität wurde wie beschrieben bestimmt. VAC-alpha induzierte Inhibition wurde entweder bei 13,2 µM Phospholipid o (offene Kreise) oder bei 6,6 µM Phospholipid gemessen (ausgefüllte Kreise).

51. Auswirkung von $Ca^{++}$ auf die VAC-alpha induzierte Inhibition der Phospholipase Aktivität. Die Inhibition wurde bei 6,6 µM Phospholipid und 1 mM (offener Kreis), 0,5 mM (ausgefüllter Kreis), 0,1 mM (offenes Quadrat) oder 0,05 mM $Ca^{++}$ (ausgefülltes Quadrat) bestimmt.

52. VAC-alpha cDNA Sequenz

53. HPLC der tryptischen Peptide aus Placenta-VAC

54. HPLC der tryptischen Peptide aus Nabelschnur-VAC

55. Gelpermeations-HPLC von Placenta- und Nabelschnur-VAC

56. Reverse-Phase-HPLC von Placenta-VAC

57. SDS-Gelelektrophorese von Placenta-VAC

Beispiel 1

Das aus Nabelschnurgefäßen und/oder Placenta isolierte und gereinigte Material wurde mit Hilfe einer Reverse-Phase HPLC nachgereinigt.

| | |
|---|---|
| Stationäre Phase: | Bakerbond WP-RP 18, 4,6x250 mm, 5µm Teilchen, 300 A Poren |
| Mobile Phase A: | 0,1% Trifluoressigsäure in Wasser, pH 2,2 |
| Mobile Phase B: | 0,1% Trifluoressigsäure in Acetonitril |
| Gradient: | 20-68% B in 24 min |
| Fluß: | 1 ml/min |
| Detektion: | UV, 214 nm |

Anschließend an diese Reinigungsstufe wurden beide Materialien, jeweils die Substanz, die das Molekulargewicht 32.000 aufwies, mit Trypsin verdaut.

Reaktionsbedingungen:

30 µg VAC aus Placenta in 135 µl 0,15 M
$NH_4HCO_3$, pH 8,0
+ 2% w/w Trypsin (Worthington)
6 Stunden bei 37°C
+ 2% w/w Trypsin (Worthington),
über Nacht bei 37°C
30 µg VAC aus Nabelschnur in 100 µl 1%
$NH_4HCO_3$, pH 8,0
+ 2% w/w Trypsin (Worthington)
6 Stunden bei 37°C
+ 2% w/w Trypsin (Worthington),
über Nacht bei 37°C

Die erhaltenen Bruchstücke wurden mit Hilfe von HPLC aufgetrennt und einer Sequenzierung mit einem Gasphasensequenator Typ 470A von Applied Biosystems, Programm 02 RPTH zugeführt.

HPLC-Trennbedingungen:

| | |
|---|---|
| Stationäre Phase: | µBondapak C18, 3,8x300 mm, 10 µ Teilchen |
| Mobile Phase A: | 0,1% Trifluoressigsäure in Wasser, pH 2,2 |
| Mobile Phase B: | 0,1% Trifluoressigsäure in Acetonitril |
| Gradient: | 0-55% B in 55 min |
| Fluß: | 1 ml/min |
| Detektion: | UV, 214 nm (obere Spur) 280 nm (untere Spur) |

Neben dem tryptischen Verdau wurde das über Reverse-Phase-HPLC gereinigte Material auch noch einer BrCN-Spaltung unterzogen. Auch diese Spaltpeptide wurden sequenziert und mit den Daten der Peptide aus dem tryptischen Verdau verglichen.

BrCN-Spaltung:

111 µg über RP-HPLC gereinigtes VAC wurden in 111 µl 70% Ameisensäure gelöst. Diese enthielt bereits den 250-fachen molaren Überschuß an BrCN (90 µg). Die Inkubation erfolgte im Dunkeln, 17 Stunden bei Raumtemperatur. 100 µl wurden für die HPLC-Auftrennung verwendet.

| | |
|---|---|
| HPLC-Säule: | μBondapak C 18 |
| Mobile Phase A: | 0,1% Trifluoressigsäure in Wasser |
| Mobile Phase B: | 0,1% Trifluoressigsäure in Acetonitril |
| Gradient: | 0 - 70% B in 70 min |
| Fluß: | 1 ml/min |
| Detektion: | UV, 214 und 280 nm |

Ein Vergleich der hiermit erzielten Ergebnisse sowie die Analyse mittels Gelpermeations-HPLC und SDS-Gelelektrophorese belegt die Identität von VAC aus Placenta und VAC aus Nabelschnur

Gelpermeations HPLC:

| | |
|---|---|
| Stationäre Phase: | Waters I-125, 7,8x600 mm, 10 μm Teilchen |
| Mobile Phase: | 0,5M $Na_2SO_4$, 0,02 M $Na_2PO_4$, pH 7,0, 25% Propylenglykol, 0,04% Tween 20 |
| Fluß: | 0,5 ml/min |
| Detektion: | UV, 214 nm |

SDS-Gelelektrophorese

| | |
|---|---|
| SDS-Gel: | 15% |
| Gelstärke: | 0,7 mm |
| Elektrophoresebedingungen: | 20 mA/Platte, 2-3 Stunden Laufzeit |
| Färbung: | Coomassie Blue |
| Proben: | \8 μg VAC aus Nabelschnur bzw.<br>\7 μg VAC aus Placenta |

Beispiel 2

Herstellung einer humanen plazentalen cDNA-Bibliothek

a) Gesamt-RNA Isolierung aus Plazenta

GT: 5 M Guanidinium-Thiocyanat, 50 mM Tris pH=7,4, 25 mM EDTA. Vor Gebrauch 8%(v/v) beta-Mercaptoäthanol zusetzen. 20 ml GT werden 5 bis 10 Minuten vor Gebrauch auf Eis gekühlt, GT soll dabei nicht präzipitieren.

GH: 6 M Guanidium-Hydrochlorid, 25 mM EDTA, 10 mM beta-Mercaptoäthanol, pH= 7,0. Eiskühlen.

Ig tiefgefrorene und mechanisch pulverisierte Plazenta werden in 20 ml GT (0°C) 20 Sekunden mit maximaler Geschwindigkeit mit einem Polytron (Brinkmann) gemixt. Das Volumen des Homogenats wird bestimmt, in 0,3 vol Äthanol (-20°C) gegossen, gemischt und sofort bei 12000 rpm 5'bei -10°C (Beckman JA 21 Zentrifuge, JS13.1 Rotor) zentrifugiert. Ein eventueller Proteinfilm sowie der Überstand werden entfernt. 10 ml eiskaltes GH werden dem Pellet zugesetzt und 10 Sekunden mit dem Polytron homogenisiert. Die Suspension wird 5 Minuten bei -10°C und 12000 rpm zentrifugiert. Der Überstand wird in ein steriles Corexröhrchen transferiert, das Pellet verworfen. Zum Überstand werden 0,025 vol 1 M Essigsäure und 0,75 vol kalter Äthanol (-20°C) zugesetzt und gut durchmischt. Nach ca.2 Stunden Inkubation bei -20°C wird 10 Minuten bei 6000 rpm bei -20°C (JA20 Rotor) zentrifugiert. Der Proteinfilm und der Überstand werden sorgfältig entfernt. 2 ml GH (0°C) werden zum Pellet zugegeben, das Pellet resuspendiert, und die Suspension in ein 15ml Corexröhrchen transferiert. Mit weiteren 8ml GH wird das alte Corexröhrchen nachgespült, die Lösung mit den 2ml vereint. Es ist wichtig, daß das gesamte Pellet suspendiert ist, eventuell ist durch mildes Sonikieren nachzubehandeln. 0,025 vol 1 M Essigsäure und 0,5 vol kalter Äthanol (-70°C) werden zugesetzt und ca. 2 Stunden bei -20°C inkubiert. Zentrifugation (6000 rpm, 10 min, JA20 Rotor), Lösen und Präzipitieren werden zweimal wiederholt, wobei die totale GH-Menge auf 5 ml halbiert wird. Nach der letzten Zentrifugation soll kein Proteinfilm mehr über der Lösung sichtbar sein, sonst ist dieser Reinigungsschritt zu wiederholen. Das Pellet wird mit 5 ml Diäthylpyrocarbonat behandeltem Wasser (0°C) 2 Minuten gevortext. Die klare Lösung wird dekantiert, zu dem eventuell zurückbleibenden Pelletresten wird nochmals Wasser gegeben und gevortext, bis die Lösung klar ist. Durch Zusatz von 0,1 vol 3 M Na-Azetat (pH=5,8), 2,5 vol Äthanol und 1 Stunde Inkubation bei -70°C wird die RNA 10 Minuten bei 6000 rpm bei 4°C abzentrifugiert. Lösen und präzipitieren wird einmal wiederholt. Letztendlich wird die RNA in Aethanol bei -20°C aufbewahrt.

b)poly-A $^{+}$-RNA Isolierung

0,5 mg oligo dT-Zellulose (Collaborative Research, Typ 3, Bindungskapazität: 5 mg poly-A$^{+}$-RNA/g) werden im Bindungspuffer suspendiert (200 mM NaCl, 0,2 % SDS, 10 mM Tris, pH=7,4, 1 mM EDTA). 1 ml dieser Suspension wird in eine Säule pepackt und nacheinander mit 10 ml Wasser, 10 ml 0,1 N NaOH, 10 ml Wasser und letztlich mit 10 ml Bindungspuffer gewaschen. Die Gesamt-RNA wird aus der alkalischen Lösung durch Zentrifugieren (10 Min., 6000 rpm, JA20 Rotor pelletiert. Ca. 10 mg RNA werden in 4,5 ml Wasser gelöst. Nach Zusatz von 50 µl 2% SDS wird die Lösung 3 Min. auf 70°C erhitzt und unmittelbar auf Eis gekühlt. Nach dem Zusatz von 50 µl 1M Tris (pH=7,4), 10 µl 0,5 M EDTA und 200 µl 5 M NaCl wird die Lösung sofort auf die Säule aufgetragen. Die aus der Säule tropfende Lösung wird wieder auf die Säule aufgetragen. Diese Prozedur wird insgesamt dreimal wiederholt. Anschließend wird die Säule mit 30 ml Bindungspuffer gewaschen. Die gebundene RNA wird mit 5 ml 0,2% SDS eluiert. Die Säule wird mit 10 ml Wasser, 10 ml 0,1 N NaOH und 10 ml $H_2O$ gewaschen und mit 10 ml Bindungspuffer äquilibriert. Die RNA Lösung wird nochmals 3 Minuten auf 70°C erhitzt, rasch auf Eis abgekühlt, 50 µl 1 M Tris pH=7,4, 10 µl 0,5 M EDTA und 200 µl 5 M NaCl zugesetzt und auf die Säule aufgetragen. Die durchlaufende Lösung wird insgesamt dreimal auf die oligo-dT Säule aufgetragen. Nach dem Waschen wird die RNA mit 30 ml 0,2% SDS eluiert.

Durch Zugabe von 0,1 vol 3M Na-Azetat pH=5,6 und 2,5 vol Aethanol sowie Inkubation bei -20°C (16 Stunden) wird die RNA ausgefällt. Die RNA wird abzentrifugiert (10 000 rpm, 15 Min., JA-20 Rotor) und in Wasser mit einer Konzentration von 1 µg/µl gelöst.

c) Konstruktion der Lambda-gt10 cDNA Bibliothek

Die Synthese der cDNA, das Methylieren der internen EcoRI-Stellen, das Anbringen von EcoRI Linkern, das Nachschneiden mit EcoRI, die Abtrennung der kurzen DNA-Fragmente, das Ligieren mit den Lambda-gt10 Armen und das in vitro Verpacken der ligierten DNA erfolgte mit dem cDNA Synthese System von Amersham (RPN 1256) sowie dem cDNA Kloniersystem Lambda-gt10 (Amersham, RPN 1257). Die beigefügten Arbeitsvorschriften wurden genau eingehalten. Ausgehend von etwa 3 µg wurden mRNA letztendlich ca. $0{,}5 \times 10^6$ rekombinante Lambda-gt10 Phagen erhalten.

Beispiel 3

cDNA Isolierung

a) Durchsuchen der Lambda-gt10 Bibliothek mit Oligonukleotiden

Zum Durchsuchen der cDNA-Bibliothek nach für VAC-protein kodierender cDNA wurden entsprechend der Sequenzen des tryptischen Peptides P16/II zwei und des Staph A Peptides P20/I/6 ein Oligonukleotid synthetisiert (Fig.2). Diese Oligonukleotide stellen jeweils eine Mischung aller Varianten dar, die jede Kodierungsmöglichkeit der entsprechenden mRNA berücksichtigen. EBI-386 weist bei einer Kettenlänge von 20 Nukleotiden 512 Variationen auf und paßt zu dem Staph-A Peptid P20/I/6. Um die Variation bei dem Oligonukleotid für das tryptische Peptid P16/II niedriger zu halten, wurden zwei Oligonukleotide (20-mere) synthetisiert: EBI-387: 128 Variationen, EBI-388: 64 Variationen.

Weiters wurden zu dem tryptischen VAC-Peptid P30/I passend zwei Oligonukleotide unter Verwendung von Desoxyinosin als Base bei "Wobble" Positionen synthetisiert (Fig.3): EBI-118 und EBI-119. Diese Substitution wurde von E. Ohtsuka et al., J. Biol. Chem. 260/5 (1985), pp.2605-2608, sowie Y. Takahashi et al., Proc. Nat. Acad. Sci. (1985) pp.1931-1935, beschrieben. Inosin basenpaart gut mit Cytosin, stört jedoch kaum die Ausbildung der Doppelhelix, wenn andere Nukleotide als Partner angeboten werden.

Jeweils 60 pMol jedes Oligonukleotids wurden mit 60 pMol gamma-$^{32}$P-ATP (Amersham, PB 218, 5000 Ci/mMol) in 30 µl Lösung unter Verwendung von 20 Einheiten $T_4$ Polynukleotidkinase phosphoryliert. Die Lösung enthielt 70 mM Tris pH 7,5, 10 mM $MgCl_2$ und 5 mM DTT, die Inkubationsdauer betrug 30 Minuten. Durch Zugabe von 30 mM EDTA und 5 Minuten Erhitzen auf 70°C wurde die Reaktion gestoppt. Das markierte Oligonukleotid wurde durch Säulenchromatographie über Biogel P6DG (Biorad) von nicht eingebauter Radioaktivität getrennt. Pro Oligonukleotid wurden zwischen 70 und $110 \times 10^6$ cpm Phosphor-32 eingebaut.

Je 15 pMol EBI-118 und EBI-119 wurden in 90 µl unter Einsatz von 45 pMol gamma-$^{32}$P-ATP (Amersham, PB 218, >5000 Ci/mMol) mit 10 Einheiten T4-Polynukleotidkinase phosphoryliert und anschließend über Biogel P6DG gereinigt. Insgesamt wurden $70 \times 10^6$ cpm Phosphor-32 eingebaut.

Ungefähr $1{,}2 \times 10^6$ pfu (Plaque forming units) rekombinante Phagen einer humanen, plazentalen cDNA Bibliothek im Phagen Lambda-gt10 wurden verwendet, um E.coli C600 zu infizieren. Etwa 50.000 Phagen wurden pro 13,5 cm Petrischale plattiert (LB, 10 mM $MgSO_4$, 15 g/l Bacto-Agar). Nachdem die Plaques fast konfluent waren, wurden von jeder Platte 2 Abzüge auf Nitrozellulosefilter hergestellt (Schleicher und Schuell, BA 85) (T. Maniatis et al., Molecular Cloning, 1982, pp 320-321).

Die Filter wurden 3x20 Minuten in TE bei 100°C behandelt und anschließend über Nacht bei 65°C gewaschen:

Waschlösung: 50 mM Tris pH=8
1 M NaCl
1 mM EDTA
0,1 % SDS

Danach wurden die Filter 4 Stunden bei 49°C prähybridisiert:
Prähybridisierlösung:
6 x SSC

5 x Denhardt
0,1 % SDS
1 mM Na-Pyrophosphat
50 mM $NaH_2PO_4$ pH=6,5
100 µM ATP
80 µg/ml denaturierte Hering Sperm DNA

Die Hybridisierung erfolgte in der gleichen Lösung unter Einsatz der gesamten Menge markierter Oligonukleotide. Die doppelten Abzüge der Platten 1 bis 6 wurden mit EBI-386, jene der Platten 7-12 mit EBI-387 und EBI-388 16 Stunden bei 49°C hybridisiert. Die Abzüge der Platten 13-24 wurden 16 Stunden mit EBI-118 und EBI-119 bei 37°C hybridisiert. Nach erfolgter Hybridisierung wurden die Filter zweimal mit 6x SSC/0,01% SDS gespült, 2 x 30 Minuten bei Raumtemperatur mit 6x SSC / 0,01% SDS und 3 x 20 Minuten in der gleichen Lösung bei 49°C bzw. 37°C gewaschen. Nach dem Lufttrocknen wurden die Filter an Kodak X-Omat S Film bei -70°C unter Verwendung einer Verstärkerfolie exponiert.

Lambda-Phagen, die auf beiden Filtern Hybridisierung mit dem Oligonukleotid zeigten, wurden unter Verwendung desselben Hybridisierungsprotokolls zur Homogenität gereinigt.

Aus der Hybridisierung mit EBI-386, -387 und -388 resultierten die Phagen Lambda-P11/3, Lambda-P6/5, Lambda-P6/6. Die Hybridisierung mit EBI-118 und -119 resultierte in den Phagen Lambda-Nr15, Lambda-Nr19 und Lambda-Nr22.

b) Isolierung der cDNA Inserts

E.coli C 600 wurde mit 2,5 x $10^6$ pfu (Plague forming units) Phagen infiziert und mit 6 ml Topagarose (0,7 % Agarose, 10 mM $MgSO_4$, LB) auf 13,5 cm Agarplatten (LB, 10 mM $MgSO_4$, 1,5% Agarose, 0,2% Glukose) plattiert. Nach 5 1/2 Stunden Inkubation bei 37°C waren die Plagues konfluent. Die Platten wurden 30 Minuten bei 4°C gekühlt und die Agarose mit 10 ml Lambda-Diluent (10 mM Tris, pH=8, 10 mM $MgSO_4$, 0,1 mM EDTA) überschichtet. Die Phagen wurden über Nacht bei 4°C unter leichten Schütteln eluiert. Die überstehende Phagensuspension (ca. 8 ml) wurde in Corexröhrchen transferiert und 10 Minuten bei 15.000 rpm (4°C, JA 21 Zentrifuge) zentrifugiert. Der Überstand wurde in Polycarbonatröhrchen dekantiert und bei 50.000 rpm (20°C, L70 Beckman-Zentrifuge, 20°C, 50 Ti Rotor) bis omega$^2$t = 3x$10^{10}$ (ca 23 Minuten) zentrifugiert. Die pelletierten Phagen wurden nach Entfernen des Überstandes in 0,5 ml Lambda-Diluent resuspendiert und in Eppendorf Röhrchen transferiert. Die Suspension wurde durch kurzes Zentrifugieren von nichtsuspendierten Teilchen befreit, der Überstand in ein neues Röhrchen gefüllt. Nach Zusatz von 10 µg/ml RNaseA und 1 µg/ml DNase I wurde 30 Minuten bei 37°C inkubiert. Nach Zugabe von 25 mM EDTA, 25 mM Tris, pH=8, 0,2% SDS wurde 30 Minuten bei 70°C inkubiert. Als nächstes wurde 1 vol Phenol/Chloroform (1:1) zugegeben, und Proteine durch Kippen des Röhrchens extrahiert. Nach 2 Minuten Zentrifugation in der Eppendorfzentrifuge wurde die wäßrige Phase mit 1 vol Chloroform extrahiert (nur Kippen, nicht Vortexen). Nach der Phasentrennung durch Zentrifugation wurden dem Ueberstand 1/20 vol 3 M Na-Azetat und 1 ml Äthanol zugesetzt. Dabei fiel die Phagen DNA fadenförmig aus. Nach 5 Minuten Inkubation bei 0°C wurde die DNA abzentrifugiert (10 Minuten). Das Pellet wurde einmal mit 70 % Äthanol gewaschen, getrocknet und über Nacht in 50 µl TE gelöst (4°C).

Die DNAs wurden mit EcoRI geschnitten und die entstehenden Fragmente auf einem 1% Agarosegel getrennt. Die cDNA Inserts der Klone Lambda-P11/3, Lambda-P6/5 und Lambda-P6/6 hatten Größen von etwa 1300 bis 1400 bp. Die Sequenzanalyse zeigte, daß alle drei Klone von ein und derselben mRNA abgeleitet waren. Das 5'Ende der mRNA fehlte jedoch in den cDNAs. Die Inserts der Phagen Lambda-Nr15, Lambda-Nr19 und Lambda-Nr22 hatten Längen von ca. 1600, 1100 und 1000 bp. Die Sequenzanalyse ließ eine annähernd vollständige cDNA vermuten. Die cDNAs der beiden Phagengruppen Lambda-P11/3, Lambda-P6/5 und Lambda-P6/6 sowie Lambda-Nr15, Lambda-Nr19 und Lambda-Nr22 sind von zwei verschiedenen mRNAs abgeleitet, wie die nachfolgende Sequenzanalyse ergab.

Die EcoRI Inserts der drei Klone Lambda-P11/3, Lambda-P6/5 und Lambda-P6/6 wurden isoliert und in den EcoRI-geschnittenen Bluescribe M13+ Vektor ligiert (Vector Cloning Systems, 3770 Tansy Street, San Diego, CA 92121, USA). Die resultierenden Klone wurden pP6/5, pP6/6 und pP11/3 genannt.

Die EcoRI Inserts der drei Klone Lambda-NrI5, Lambda-Nr19 und Lambda-Nr22 wurden isoliert und in den EcoRI-geschnittenen Bluescribe M13+ Vektor ligiert. Die resultierenden Klone wurden pRH201, pRH202 und pRH203 genannt.

c)Weitere VAC cDNA Klone

Um weitere cDNA Klone zu erhalten, wurde die humane, plazentale Lambda-gt10 Bibliothek nochmals durchsucht, diesmal mit dem EcoRI-Insert des pP11/3 als Sonde.

Dieses DNA-Fragment wurde durch Nicktranslation radioaktiv markiert (T.Maniatis, Molecular Cloning, 1982, pp.109-112, keine DNase I verwendet). Insgesamt wurden $4x10^5$ Phagen auf 8 Platten durchsucht. Die Behandlung der Nitrozellulosefilter erfolgte wie in T.Maniatis, Molecular Cloning, 1982, pp.320-321 beschrieben. Die Hybridisierungslösung enthielt 6xSSC, 5x Denhardt's, 0,1% SDS sowie $20x10^6$ cpm pP11/3 Insert. Die Hybridisierungsdauer betrug 16 h bei 65°C. Die Filter wurden danach 3x10 Minuten bei Raumtemperatur mit 6xSSC/0,01% SDS und 3x45 Minuten bei 65°C und mit 0,2xSSC gewaschen. Insgesamt wurden 69 positiv reagierende Klone erhalten (Lambda-VAC1 bis Lambda-VAC69).

12 dieser Klone wurden im kleinen Maßstab wie oben beschrieben präpariert, die cDNA Inserts mit Eco RI freigesetzt und auf einem 1% Agarosegel aufgetrennt. Dabei zeigte sich, daß das Insert des Klons Lambda-VAC10 den gesamten für VAC Protein kodierenden Leserahmen enthält.

Beispiel 4

Charakterisierung der cDNAs kodierend für VAC-alpha und VAC-beta

a)Northern Blot Experiment

Zu 2 µg poly-A+ RNA wurden 5 µl Wasser, 16 µl Formamid, 6 µl Formaldehyd und 3 µl 0,1 M NaOH zugesetzt, die Lösung 10 Minuten bei 68°C inkubiert und anschließend auf Eis gekühlt. Nach Zusatz von 5 µl Farbstofflösung (je 0,4% Bromphenolblau und Xylencyanol in 50% Glyzerin, 1 mM EDTA) wurde die RNA auf einem Formamid-Agarosegel aufgetrennt (1,5% Agarose, 10 mM Na-Phosphat pH=7,6, 1 mM EDTA, 5 mM Na-Azetat, 6% Formaldehyd, Elektrophorese 100 V, 3 Stunden, Laufpuffer wie Gelpuffer, ohne Formaldehyd). Als Referenz wurde Gesamt-RNA aufgetragen. Diese Spur wurde nach der Elektrophorese abgetrennt und mit EtBr angefärbt, um die Lage der 28 und 18 S rRNA zu bestimmen. Der Rest des Gels wurde zweimal 10 Minuten in 10xSSC gewaschen, und die RNA mit 20xSSC auf Nitro-zellulosefilter übertragen. Das Filter wurde mit 2xSSC gewaschen, getrocknet und 2 Stunden im Vakuum bei 80°C gebacken. Jeweils 1 µg pP11/3 und pRH203 wurden mit dem Multiprime DNA labelling System (Amersham, RPN 1601) radioaktiv markiert. Das Nitro-zellulosefilter wurde 2 h bei 65°C in 6xSSC/5x Denhardt's/0,1% SDS prähybridisiert. Jeweils eine Spur wurde mit $180x10^6$ cpm pP11/3 bzw. pRH203 hybridisiert (16h 65°C). Die Filter wurden zweimal 30 Minuten bei Raumtemperatur mit 6xSSC/0,01% SDS und zweimal 30 Minuten bei 65°C mit 0,2xSSC/0,01% SDS gewaschen, getrocknet und an Kodak X-Omat S Film mit Verstärkerfolie exponiert.

Das Ergebnis ist in Fig.4 abgebildet. Die cDNA des Klons pP11/3 hybridisiert an eine mRNA der Größe von etwa 1700 Basen ("VAC-alpha"), die cDNA des Klons pRH 203 an eine mRNA der Größe von etwa 2200 Basen ("VAC-beta").

Da erstens die eingesetzte Radioaktivitätsmenge sowie die aufgetragene Menge mRNA pro Spur etwa gleich war und zweitens die Hybridisierung eines Genom-Blots in derselben Lösung Banden gleicher Intensität mit beiden cDNAs ergab (siehe unten), ist zu schließen, daß die kürzere mRNA ("VAC-alpha") in größeren Mengen als die längere ("VAC-beta") mRNA in Plazenta vertreten ist.

b) Sequenzanalyse der VAC-beta cDNA

Die VAC-beta cDNA Sequenz der Klone NrI5, Nr19 und Nr22 ergab 1940 bp und geht in einen poly-A Abschnitt über (Fig.5). 16 Basen vor dem poly-A Abschnitt findet sich das Polyadenylierungssignal AATAAA. Allerdings kommt diese Konsensussequenz bereits an der Nukleotidposition 1704-1709 vor. Weshalb diese Sequenz nicht als Polyadenylierungssignal verwendet wird, ist unbekannt. Die zusätzlich erforderliche Sequenz an der 3'Seite der AATAAA Sequenz, YGTGTTYY (Gill A. et al., Nature 312 (1984), pp. 473-474) kommt erst relativ weit entfernt vor (TGTGTTAT, Position 1735-1742); möglich, daß dies die Erklärung für ein Nichtakzeptieren dieser ersten Polyadenylierungssequenz darstellt.

Die cDNA enthält einen langen, offenen Leserahmen, der vom Beginn der cDNA bis Position 1087 reicht. Er würde ein Kodierungspotential für 362 Aminosäuren beinhalten. Aus Analogiegründen zu VAC-alpha und aufgrund der Tatsache, daß auch ein 34.000 D Protein bei der Reinigung von VAC anfällt (siehe E.P.A. 181.465) wurde das erste Methioninkodon (ATG, Position 107-109) als Translationsstart angenommen. Die Kozakregel ist hier nicht so gut erfüllt wie bei VAC-alpha (AAGAGATGG auf Position 102-110).

Das resultierende Protein (VAC-beta) wäre 327 Aminosäuren lang. Es besitzt 4 Cysteinreste (Aminosäureposition 161, 206, 250 und 293) und eine potentielle N-Glykosylierungsstelle (Asn-Lys-Ser, Aminosäureposition 225-227). Das errechnete Molekulargewicht beträgt 36.837 (Fig.6). In VAC-beta gibt es überdurchschnittlich viele geladene Reste: 97/327 (29,6%), wobei die sauren Aminosäuren (Asp+Glu: 49) über die basischen (Lys+Arg 42) überwiegen; eine Erklärung für das nach SDS-PAGE ermittelte niedrigere Molekulargewicht.

VAC-beta zeigt eine interne Wiederholung einer 67 Aminosäuren langen Sequenz Fig.7). Innerhalb dieser Sequenz sind 7 Aminosäuren (10,4 %) in allen vier Repeats konserviert, 17 Aminosäuren (25,4 %) kommen in drei von vier Repeats vor, an 25 Positionen (37,7 %) enthalten jeweils zwei Repeats dieselbe Aminosäure. Auch VAC-beta zeigt gute Übereinstimmung mit der 17 Aminosäuren langen Konsensussequenz.

c) Genomische Southernblot Analyse

Die Analyse chromosomaler DNA aus humaner Plazenta nach Southern zeigt ein komplexes Bild. Die DNA wurde mit EcoRI, HindIII, BamHI und PstI geschnitten. Die auf Nitrozellulose transferierte DNA wurde sowohl mit einer VAC-alpha DNA (pP11/3) als auch mit einer VAC-beta DNA (pRH203) hybridisiert. Das Waschen der Filter geschah unter stringenten Bedingungen (siehe Punkt a)), d.h. mit 0,2xSSC und 65°C. Trotzdem ergaben sich bei jedem Verdau relativ viele Banden (Fig.8). Der Vergleich der beiden Blots zeigt, daß die Kreuzreaktion von VAC-alpha und -beta DNA unter diesen Bedingungen eher auszuschließen ist. Die Vielzahl der Banden ist entweder durch die Existenz jeweils ähnlicher Gene zu dem VAC-alpha bzw. VAC-beta Gen zu erklären, oder aber es handelt sich um jeweils ein Gen, das durch viele und/oder lange Introns unterbrochen ist.

Beispiel 5

Protein-Analyse

In Fig.9 ist der Vergleich der Aminosäuresequenzen von VAC-alpha mit VAC-beta wiedergegeben. Die wiederholten Strukturen lassen sich in beiden Proteinen gleich anordnen. Die Verbindungspeptide sind ebenfalls gleich lang mit Ausnahme jener zwischen der 2. und 3. Repeat. In dieses Verbindungspeptid muß bei VAC-alpha eine Lücke eingeführt werden, um eine optimale Anpassung der beiden Sequenzen zu erlauben. Das N-terminale Peptid der beiden Proteine ist unterschiedlich lang, 19 Aminosäuren bei VAC-alpha, 25 Aminosäuren bei VAC-beta. Dieses Peptid weist auch die geringste Homologie auf. Die beiden Proteine sind an 176 von 320 Aminosäurepositionen ident, was einem Homologiegrad von 55,0% entspricht.

An dieser Stelle sei auch der Vergleich der Nukleotidsequenzen der VAC-alpha und -beta cDNAs eingefügt. Werden zwei Gene und deren Produkte miteinander verglichen, sieht man auf der DNA (=RNA) Ebene eine größere Homologie als auf der Aminosäureebene, was durch die Tatsache erklärt ist, daß bei der Nukleinsäure bereits eine Veränderung in einem Basentriplett ausreicht, um eine neue Aminosäure zu kodieren.

In Fig.10 ist der Vergleich der kodierenden Regionen von VAC-alpha und VAC-beta cDNA wiedergegeben. Überraschenderweise zeigen die DNAs nur einen Homologiegrad von 54,2%, also etwas weniger als die beiden Proteine.

In Fig.ll sind die Hydrophilizitätsprofile der beiden Proteine abgebildet. Dabei wurde der Algorithmus von Hopp und Wood verwendet (T.P.Hopp et al., Proc.Natl.Acad.Sci. 78 (1981) pp.3824-3828). Die vier Repeatbereiche sind durch die Balken angedeutet, die Verbindungspeptide in der darunter angegebenen Sequenz eingerahmt. Dabei fällt auf, daß das Verbindungspeptid zwischen der zweiten und dritten Repeat besonders hydrophil ist. In diesem Peptid befindet sich sowohl bei VAC-alpha als auch bei VAC-beta ein Arginin an identer Position. Es wäre daher möglich, daß dieses Arginin einen bevorzugten Angriffspunkt für eine Protease mit trypsinartiger Spezifität darstellt. Dabei müßte das Molekül in zwei etwa gleich große Hälften zerfallen. Es wäre denkbar, daß bereits ein solches "Halbmolekül" eine biologische, beispielsweise eine antikoagulierende Wirkung entfalten kann. Auf der anderen Seite könnte vielleicht der Austausch dieses Arginins durch z.B. Histidin den VAC-proteinen eine größere Stabilität vermitteln.

Aus Fig. 11 ist weiterhin leicht zu erkennen, daß weder VAC-alpha noch VAC-beta einen längeren, hydrophoben Bereich aufweisen, der entweder die Sekretion durch, oder das Einlagern der Proteine in eine Membran erlauben würden. Es ist daher anzunehmen, daß es sich bei VAC-alpha und VAC-beta um intrazelluläre Proteine handelt.

Wallner et al (Nature 320 (1986), pp.77-81) berichten die Struktur des humanen Lipocortin I, Saris et al. (Cell 46 (1986), pp.201-212) und Huang et al. (Cell 46 (1986), pp. 191-199) die Struktur des murinen bzw. humanen Calpactin I, das auch Lipocortin II genannt wird. Diese Proteine gehören ebenfalls zu der Klasse der $Ca^{++}$ abhängigen Phospholipidbindungsproteinen. Ihre Struktur läßt sich analog zu VAC-alpha und -beta anschreiben (Fig.12). Die Homologien zwischen VAC-alpha und VAC-beta sind jedoch ausgeprägter als jene zwischen VAC und Lipocortin:

VAC-alpha - VAC-beta : 55,0%
VAC-alpha - Lipocortin I : 41,9%
VAC-alpha - Lipocortin II : 43,8%
VAC-beta - Lipocortin I : 41,7%
VAC-beta - Lipocortin II : 44,6%

Anzunehmen ist daher, daß auch die Lipocortine aufgrund ihrer analogen Struktur antikoagulierende Wirkung haben, und demzufolge als Antikoagulanzien einzusetzen sind und weiterhin, daß dies eine generelle Eigenschaft dieser Klasse von $Ca^{++}$ abhängiger Phospholipidbindungsproteine ist.

Viele Mitglieder der $Ca^{++}$ abhängigen Phospholipidbindungsproteine binden an gereinigte sekretorische Vesikel oder andere Bestandteile des Cytoskeletts (siehe R.H.Kretsinger et al., Nature 320 (1986), p.573 für eine Zusammenfassung). Bei der Sekretion kapseln sich die Vesikel vom Golgi-Apparat der Zelle ab, wandern zur Zellmembran und fusionieren mit ihr. Dabei wird der Inhalt der Vesikel von der Zelle freigesetzt. In Analogie zur Kupplung von Erregung mit der Muskelkontraktion wurde vorgeschlagen (W.W.Douglas, Br.J.Pharmac. 34 (1968), 451), daß auch der Stimulus und die Sekretion über $Ca^{++}$ gekoppelt ist. Dabei könnten die $Ca^{++}$ abhängigen Phospholipidbindungsproteine eine wichtige Rolle spielen. In der konservierten, 17 Aminosäuren langen Region jeder Repeat besitzt VAC-alpha 5 bis 6 Hydroxylgruppen-enthaltende Aminosäuren (Asp, Glu, Thr, Ser), jeweils drei davon an identischer Position. Bei VAC-beta finden sich in jeder Repeat vier dieser Aminosäuren. Obwohl keines der Proteine die in Calmodulin, Troponin C, S-100 und Parvalbumin beobachtete EF-Hand Struktur aufweist (R.H.Kretsinger et al., CRC Crit. Rev.Biochem. 8 (1980), p119), die in diesen Molekülen für die $Ca^{++}$ Bindung verantwortlich ist, verleitet die Konservierung dieses Unterabschnitts in jeder Repeat zu dem Schluß, daß diese Region für die $Ca^{++}$ Bindung verantwortlich ist.

Durch gezielte Mutationen in diesem Bereich könnte versucht werden, die biologische Aktivität von VAC-Proteinen zu verändern.

## Beispiel 6

### Vac-beta Expression in E.coli

a) Expressionsklon pRH212

Als Expressionsvektor wurde das Plasmid pER103 verwendet (E.Rastl-Dworkin et al., Gene 21 (1983), 237-248). Der Vektor wurde mit HindIII linearisiert. Das 5'überhängende Ende wurde mit dATP und DNA-Polymerase I / Klenowfragment partiell eingefüllt, und der verbleibende Einzelstrangrest mit S1 Nuklease verdaut. Der Vektor wurde mit BamHI nachgeschnitten und das große Fragment isoliert (Fig.13). Aus dem Klon pRH203 wurde das 440 bp lange MaeIII-BamHI Fragment isoliert, das die Codons 13 bis 157 enthält. Das fehlende 5'Ende wurde durch Oligonukleotide ergänzt:

```
EBI-307
5'   CCATGGCTTGGTGGAAAGCTTGGATCGAACAGGAAGGT         3'
3'   GGTACCGAACCACCTTTCGAACCTAGCTTGTCCTTCCACAGTG  5'
EBI-306
```

Dabei wurden für E.coli optimale Codons verwendet (zB. R.Grantham et al., Nucleic Acids Res. 8 (1980), 1893-1912). Dieser Codonaustausch resultiert in einer neuen HindIII-Stelle bei Codon 5 bis 7. Das Oligonukleotid EBI-306 wurde phosphoryliert, mit EBI-307 hybridisiert und mit dem VAC-beta Fragment ligiert. Nach dem Nachschnitt mit BamHI wurde das 5'terminale VAC-Fragment in den vorbereiteten pER103 Vektor ligiert. Der entstehende Klon wurde pRH211 benannt. Um die für VAC-beta kodierende Region zu ergänzen, wurde aus dem Klon pRH201 das 1230 bp lange BamHI-SphI Fragment isoliert. Der ca. 200 bp lange pBR322 Abschnitt von BamHI bis SphI aus dem Plasmid pRH211 wurde entfernt und durch den entsprechenden VAC-cDNA Teil ersetzt. Es resultierte der Klon pRH212. Das EcoRI-BamHI Fragment, das den Trp-Promotor (S.marcescens), die ribosomale Bindungsstelle und den synthetisch hergestellten Beginn des VAC-beta Gens enthält, wurde durch Sequenzieren überprüft.

b) Expression von VAC-beta (pRH212)

Der Nachweis des plasmidkodierten VAC-beta erfolgte im Maxizell-system (A.Sancar et al., J. Bacteriol. 137 (1979), pp.692-693.). Ecoli CSR603 ($F^-$, thr-1, leuB6, proA2, phr-1, recAl, argE3, thi-1, uvrA6, ara-14, lacY1, galK2, xyl-5, mtl-1, gyrA98 (nalA98), rpsL31, tsx-33, $Lambda^-$, supE44) wurde mit pRH212 transformiert und bis zu einer $OD_{600}$ bei 37°C

gezüchtet. 20 ml Kultur wurden in einer offenen Petrischale mit einer UV-Germicidlampe (15W) aus 50 cm Entfernung 5 Sekunden lang bestrahlt und anschließend 1 Stunde weiter inkubiert. Der Kultur wurden 100 µg D-Cycloserin zugesetzt. Nach 16 Stunden wurden die Bakterien abzentrifugiert, zweimal mit Hershey-Salzlösung gewaschen (5,4g/l NaCl, 3,0 g/l KCl, 1,1 g/l $NH_4Cl$, 15 mg/l $CaCl_2x2H_2O$, 0,2 g/l $MgCl_2x6H_2O$, 0,2 mg/l $FeCl_3x6H_2O$, 87 mg/l $KH_2PO_4$, 12,1 g/l Tris pH=7,4), in 5 ml Hersheymedium resuspendiert (pro 100 ml Hersheysalze: 2,0 ml 20% Glukose, 0,5 ml 2% Threonin, 1,0 ml 1% Leucin, 1,0 ml 2% Prolin, 1,0 ml 2% Arginin, 0,1 ml 0,1% Thiamin-HCl) und mit 20 µg/ml Indolacrylsäure 2 Stunden inkubiert. Nach Zusatz von 5 µCi/ml $^{35}S$-Methionin und weiterer Inkubation bei 37°C (1 Stunde) wurden die plasmidkodierten Proteine radioaktiv markiert. Die Bakterien wurden abzentrifugiert und in 200 µl SDS-Probenpuffer aufgenommen (Lämmli-Gel System, z.B. L.G.Davies et al., Methods in Molecular Biology (1986) pp.306-310). Die markierten Produkte wurden auf einem 15% Acrylamidgel aufgetrennt. Das Gel wurde getrocknet und an Kodak X-Omat S Film exponiert. Als Referenz wurde gereinigtes VAC-alpha Protein mitlaufen gelassen und durch Anfärben sichtbar gemacht. VAC-alpha läuft etwas schneller als das pRH212 kodierte VAC-beta, wie es auch dem Molekulargewicht nach zu erwarten war. Die Expression von VAC-beta ist außerdem durch Zugabe des Induktors Indolacrylsäure stimulierbar.

c) Expressionsklon pRH292

Der Expressionsvektor pRH284T wurde mit SacI linearisiert und die 3'überhängenden Ende mit DNA-Polymerase I / Klenowfragment und dGTP in gerade Enden übergeführt. Der Vektor wurde mit SalI nachgeschnitten, und das große Fragment isoliert.Das HindIII-SalI Insert des Klons pRH212 wurde isoliert. 0,2 pMol des Oligonukleotidpaares

```
5' GCTTGGTGGAA 3'   EBI-684
3' CGAACCACCTTTCGA 5'   EBI-685
```

wurde mit 0,2 pMol VAC-beta Insert und 0,015 pMol vorbereiteten pRH284T ligiert. E.coli HB101 wurde mit der Ligaselösung transformiert. Der resultierende Klon wurde pRH292 benannt (Fig.14).

Beispiel 7

Nachweis der Expression von Vac-beta in E.coli

a) Anzucht des Klons HB101/pRH292

Medien: 1) Vorkultur

| | |
|---|---|
| 10 g/l | Trypton |
| 5 g/l | Hefeextrakt |
| 4 g/l | Glucose |
| 9 g/l | $Na_2HPO_4.2H_2O$ |
| 1 g/l | $NH_4Cl$ |
| 1 g/l | KCl |
| 1 ml/l | 1M $MgSO_4.7H_2O$ |
| 100 mg/l | Ampicillin |

Start-pH = 7,2

2) Hauptkultur

| | | |
|---|---|---|
| 0,68 | g/l | $(NH_4)_2HPO_4$ |
| 0,62 | g/l | $K_2HPO_4 \cdot 3H_2O$ |
| 2,33 | g/l | KCl |
| 0,5 | g/l | NaCl |
| 0,53 | g/l | $NH_4Cl$ |
| 1,23 | g/l | $MgSO_4 \cdot 7H_2O$ |
| 0,011 | g/l | $CaCl_2$ |
| 10 | mg/l | Thiamin.HCl |
| 3,92 | mg/l | $(NH_4)_2Fe(SO_4)_2 \cdot 6H_2O$ |
| 0,72 | mg/l | $AlCl_3 \cdot 6H_2O$ |
| 0,71 | mg/l | $CoCl_2 \cdot 6H_2O$ |
| 1,5 | mg/l | $KCr(SO_4)_2 \cdot 12H_2O$ |
| 0,75 | mg/l | $CuSO_4 \cdot 5H_2O$ |
| 0,19 | mg/l | $H_3BO_3$ |
| 0,51 | mg/l | $MnSO_4 \cdot H_2O$ |
| 0,79 | mg/l | $NiSO_4 \cdot 6H_2O$ |
| 0,73 | mg/l | $Na_2MoO_4 \cdot 2H_2O$ |
| 0,86 | mg/l | $ZnSO_4 \cdot 7H_2O$ |
| 21 | g/l | Caseinhydrolysat (Merck Art.# 2238) |
| 25 | g/l | Caseinhydrolysat (Sigma C9386) |
| 100 | mg/l | Cystein |
| 2 | g/l | Hefeextrakt |
| 1 | g/l | Citronensäure |
| 11 | g/l | Glucose.$H_2O$ (Start bzw. Feed) |

700 ml Vorkulturmedium wurden mit dem Expressionsklon angeimpft und bei 37°C unter Rühren (1000 rpm (=Umdrehungen pro Minute), Magnetstab) und Sauerstoffzufuhr (5 vvm (=vol/vol/min), Belüftungsfritte) 12 bis 15 Stunden inkubiert. 600 ml dieser Vorkultur wurden in einen Fermenter mit 12 l Hauptkulturmedium überführt. Die Hauptkultur wurde unter folgenden Bedingungen fermentiert:

| | |
|---|---|
| Rührsystem: | Blattrührer bei 1000 rpm |
| Belüftung: | 1,0 vvm |
| Temperatur: | 28°C |
| pH: | 6,5 (25% $NH_3$ zur Korrektur) |

Während der Fermentation wurde die Glucosekonzentration gemessen. Bei Absinken auf 5 g/l wurde 10 g/l Glucose nachgefüttert. Alle weiteren Nachfütterungen zu je 10 g/l erfolgten bei einem Absinken der Glucosekonzentration im Fermenter auf 10 g/l. Fiel der Sauerstoffpartialdruck auf etwa 0,05 atm ab, wurde der Druck im Fermenter auf 0,3 bar erhöht. Nach 20 Stunden wurde auf 15°C abgekühlt, die Biomasse über eine CEPA-Zentrifuge vom Nährmedium abgetrennt und bei -70°C eingefroren.

b) Nachweis des rekombinanten Vac-beta Proteins

Lösungen:

Probenpuffer:

| | |
|---|---|
| 125 mM | Tris pH=6,8 |
| 4 % | SDS |
| 10 % | Mercaptoäthanol |
| 10 % | Glyzerin |
| 0,02 % | Bromphenolblau |

Acrylamidgel:

| Sammelgel: | | Trenngel: | |
|---|---|---|---|
| 3 % | Acrylamid | 13,5 % | Acrylamid |
| 0,1 % | Bisacrylamid | 0,45 % | Bisacrylamid |
| 125 mM | Tris pH=6,8 | 375 mM | Tris pH=8,8 |
| 0,1 % | SDS | 0,1 % | SDS |

Laufpuffer:

| | |
|---|---|
| 14,4 g/l | Glycin |
| 3,025 g/l | Tris |
| 5 ml | 20 % SDS |

| Proteingel-Färbelösung: | | Entfärbelösung: | |
|---|---|---|---|
| 0,1 % | Coomassieblau | 5 % | Methanol |
| 50 % | Methanol | 10 % | Essigsäure |
| 10 % | Essigsäure | | |

Glycerinlösung:

| | |
|---|---|
| 7 % | Essigsäure |
| 2 % | Glyzerin |

Transferpuffer:

| 10x Transferpuffer: | | 1x Transferpuffer: | |
|---|---|---|---|
| 24,2 g/l | Tris | 100 ml/l | 10x Transferpuffer |
| 112,6 g/l | Glycin | 200 ml/l | Methanol |
| | | 1 ml/l | Empigen |

| Blocklösung: | | PBS: | |
|---|---|---|---|
| 1 % | Tween 20 | 8 g/l | NaCl |
| 1 % | BSA (Rinderserumalbumin) | 0,2 g/l | KCl |
| 10 % | fötales Kälberserum (GIBCO) in PBS | 1,15 g/l | $Na_2PO_4$ |
| | | 0,2 g/l | $KH_2PO_4$ |
| | | 0,1 g/l | $MgCl_2$ |
| | | 0,7 g/l | $CaCl_2$ |

alkalische-Phosphatase-Puffer:

| | |
|---|---|
| 100 mM | Tris pH=9,5 |
| 100 mM | NaCl |
| 5 mM | $MgCl_2$ |

Am Ende der Fermentation wurde die optische Dichte der Brühe bestimmt und ein Aliquot entnommen. Die Bakterien wurden in einer Eppendorfzentrifuge pelletiert, mit einer Konzentration von 20 $OD_{600nm}$ (optische Dichte bei 600 nm) in Probenpuffer resuspendiert und fünf Minuten bei 100°C denaturiert. Unlösliche Anteile wurden durch Zentrifugieren in der Eppendorfzentrifuge pelletiert. 5 µl Aliquots wurden auf das Acrylamidgel geladen. Als Referenz dienten Expressionsklone, die ständig bei hoher Phosphatkonzentration gezüchtet worden waren (0,08 mM Phosphat). Unter diesen Bedingungen wurde der alkalische Phosphatasepromotor nicht aktiviert.

Die Proteine wurden bei 6,5 V/cm (Sammelgel) bzw. bei 13 V/cm (Trenngel) entsprechend dem Molekulargewicht aufgetrennt, bis der Bromphenolblaufarbstoff das Ende des Gels erreicht hatte. Eine Hälfte des Gels wurde mit Coomassieblau gefärbt. Dazu wurde das Gel 30 Minuten in der Färbelösung bewegt und anschließend eine Stunde in der Entfärbelösung behandelt. Zur besseren Entfernung des überschüssigen Farbstoff wurde ein mit Aktivkohle gefüllter Dialysenschlauch in die Entfärbelösung getaucht. Das Gel wurde abschließend 30 Minuten in der Glyzerinlösung behandelt und mittels eines Geltrockners getrocknet.

Die Proteine der zweiten Gelhälfte wurden auf ein Nitrozellulosefilter übertragen. Dazu wurde der Sandwich Whatman 3MM Papier-Gel-Nitrozellulose (Schleicher-Schuell, BA85)-Whatman 3MM Papier in einer Biorad-Transferapparatur in lx Transferpuffer zwei Stunden einer Spannung von 150 V (Stromstärke 1000 mA) unter Kühlung ausgesetzt. Das Nitrozellulosefilter wurde über Nacht bei Raumtemperatur in 50 ml Blocklösung behandelt. Das Filter wurde drei Stunden in 5 ml Blocklösung / 1:1000 verdünntes Rabbit-Anti-Vac-Antiserum inkubiert und anschließend 30 Minuten in Fließwasser,sowie dreimal 15 Minuten in PBS / 1% Tween 20 (Merck-Schuchardt # 822184) gewaschen. Das Filter wurde in 20 ml Blocklösung mit einer 1:2000 Verdünnung des Anti-Rabbit IgG (Fc) alkaline phosphatase conjugate (Promega-ProtoBlot Immunoscreening System) drei Stunden bei Raumtemperatur inkubiert. Es wurde wie oben in Fließwasser und PBS / 1% Tween 20 gewaschen. Der Nachweis des gebundenen Antikörper-alkalische Phosphatase-Konjugates erfolgte durch eine Farbreaktion (Promega-ProtoBlot Immunoscreening System). 66 µl NBT (50 mg/ml Nitro-blue-tetrazolium in 70% Dimethylformamid) und 33 µl BCIP (50 mg/ml 5-Bromo-4-chloro-3-indolyl-phosphat in Dimethylformamid) wurden in 10 ml alkalische-Phosphatase-Puffer gelöst. Das Nitrozellulosefilter wurde bis zur Farbentwicklung in dieser Lösung inkubiert und anschließend 30 Minuten mit Fließwasser gewaschen. Das Filter wurde getrocknet und in Plastikfolie eingeschweißt.

In Figur 16 ist das Resultat wiedergegeben. "+Phosphat" ist die Kontrolle ohne Expression, "-Phosphat" zeigt die Expression von Vac-alpha (Klon HB101/pRH291) bzw. Vac-beta (Klon HB101/pRH292) Protein unter der Kontrolle des alkalischen Phosphatase Promotors. Sowohl Vac-alpha als auch Vac-beta Protein sind bereits auf dem gefärbten Gel zu erkennen. Die gebildete Menge an Vac-Proteinen beträgt überraschenderweise mindestens 20 mg/l/OD600nm Bakterienkultur.

Der Westernblot zeigt deutlich die angefärbte Vac-alpha Bande. Zusätzlich sind einige Proteine niedrigeren Molekulargewichts im Bereich bis 30 kD erkennbar, die möglicherweise durch proteolytische Spaltung am N-und/oder C-Terminus des Vac-alpha Proteins entstanden sind. Auffällig ist außerdem ein durch das Antiserum erkanntes Protein im Bereich kleiner 20 kD, das eventuell ein durch Proteolyse entstandenes Halbmolekül des Vac-alpha Proteins darstellen könnte. Überraschenderweise wurde auch Vac-beta durch das Anti-Vac-Antiserum erkannt. Da diese Bande wesentlich schwächer als die Vac-alpha Bande gefärbt ist, andererseits aber die Vac-beta Bande im Coomassie Blau gefärbten Gel in ihrer Intensität dem Vac-alpha entspricht, ist daraus zu schließen, daß die Erkennung des Vac-beta Proteins durch das Anti-Vac-Antiserum wesentlich schlechter als jene des Vac-alpha Proteins erfolgt.

Beispiel 8

Biologische Aktivität von rekombinantem VAC

A. Modifizierter Prothrombin Zeit Test (s.EPA 0 181 465)
Zitriertes, Plättchen freies Plasma (PFP) wurde in An- oder Abwesenheit von rekombinantem VAC (r-VAC) oder natürlichem VAC zwei Minuten bei 37°C gerührt. Nach der Inkubation wurde eine Lösung aus brain tissue factor und $Ca^{++}$ zugegeben. Die Fibrinbildung wurde beobachtet, die Gerinnungszeit optisch registriert. Die Ergebnisse sind in Fig. 20 dargestellt und belegen, daß r-VAC und VAC die Fibrinbildung dosisabhängig inhibieren. Die spezifischen Aktivitäten des r-VAC und des VAC bewegen sich in derselben Größenordnung.

B. Thrombin Zeit Test (s. EPA 01 181 465)
Zitriertes PFP wurde in An- oder Abwesenheit von r-VAC oder VAC zwei Minuten bei 37°C gerührt. Nach der Inkubation wurde Thrombin und $Ca^{++}$ zugegeben. Die Fibrinbildung wurde optisch beobachtet. Sowohl r-VAC als auch VAC inhibieren nicht die Thrombin-induzierte Fibrinbildung wie aus Fig. 21 zu entnehmen ist. Folglich inhibieren sowohl r-VAC als auch VAC die Thrombinbildung, nicht jedoch die Thrombinwirkung.

C. Faktor Xa-Bildung in Gewebefaktor-aktiviertem Plasma (s. EPA 0 181 465)
Zitriertes PFP wurde in An- oder Abwesenheit von r-VAC oder VAC zwei Minuten bei 37°C gerührt. Anschließend wurde eine Lösung von "brain tissue factor" und $Ca^{++}$ zum PFP gegeben, um so die Koagulation in Gang zu setzen. Zu bestimmten Zeiten wurden Proben entnommen und in einem Puffer 1000fach verdünnt. 10 µl dieser Lösung wurden zu 40 µl einer Mischung, die die nachfolgenden Komponenten des Prothrombinase-Komplexes enthielten, zugegeben.

0,6 nM Faktor Va
50 µM Phospholipide (80% Dioleoyl-phospatidylcholin/20% Dioleoyl-phosphatidylserin).
1,0 µM Prothrombin

Nach einer Reaktionszeit von zwei Minuten wurde der Anteil an aktiviertem Prothrombin durch die amidolytische Aktivität unter Verwendung des chromogenen Substrats S 2238 beurteilt. Die Menge an amidolytischer Aktivität, die so gemessen wurde, ist der Menge an aktivem Faktor $X_a$ in dem PFP direkt proportional.

Nach 1000-facher Verdünnung bei Anwesenheit von VAC in dem PFP konnte keine Auswirkung auf die Prothrombin-Aktivierung beobachtet werden.

Eine typische Faktor $X_a$-Bildungskurve und die Auswirkungen von r-VAC und VAC sind in Fig. 49 dargestellt. Durch diese Ergebnisse kann festgestellt werden, daß r-VAC die Gewebefaktorinduzierte Faktor X-Aktivierung im Plasma, in dosisabhängiger Weise wie VAC inhibiert.

Die Ergebnisse zeigen weiterhin indirekt, daß sowohl r-VAC als auch VAC den Faktor $X_a$ direkt nicht in einer zeitabhängigen Weise inaktivieren. Diese Aussage kann aus der Form der Kurve hergeleitet werden. Nachdem ein Maximum erreicht ist, beginnt die Menge an aktivem Faktor $X_a$ in dem PFP, einer Kinetik Pseudo 1. Ordnung folgend, abzunehmen. Diese Abnahme resultiert vermutlich aus der Inaktivierung des Faktors $X_a$ durch Antithrombin III. Die Geschwindigkeitskonstante Pseudo 1. Ordnung, die aus diesen Daten berechnet wurde, beträgt ca. 0,25 $min^{-1}$ und ändert sich nicht bei Abwesenheit von r-VAC und VAC.

Diese Daten belegen eindeutig, daß r-VAC VAC-Aktivität zeigt. Obwohl die spezifische Aktivität von r-VAC in diesem Koagulationstest identisch ist mit der von VAC und obwohl r-VAC Faktor $X_a$ und Thrombin direkt nicht inaktiviert, wurden zusätzlich noch Untersuchungen zur r-VAC-Bindung an Phospholipide durchgeführt, um zweifelsfrei die Zugehörigkeit der VAC-Aktivität zum r-VAC zu demonstrieren.

Die Bindung von r-VAC und VAC an eine Phospholipid-Doppelschicht, die aus Dioleoylphosphatidylcholin und Dioleoylphosphatidylserin (80%/20%) besteht, wurde durch Verwendung eines automatischen Ellipsometers bestimmt. Dies Verfahren wurde von Cuypers et al. in J. Biol. Chem. 258 (1983), 2426-2431 beschrieben.

Ein typisches Ergebnis dieser Studie gibt Fig. 22 wieder. Sowohl r-VAC als auch VAC zeigen identische Bindungskinetik an die Doppelschicht, wenn sie in einer Konzentration von 1 µg VAC/ ml dieser Doppelschicht zugesetzt wurden. Die Bindung erfolgt in Anwesenheit von $Ca^{2+}$ und ist, wie durch die Zugabe von EDTA gezeigt werden konnte, reversibel.

Zusammenfassend kann festgestellt werden, daß r-VAC biologisch aktiv ist und von natürlichem VAC nicht zu unterscheiden ist.

Beispiel 9

Konstruktion Tetracyclin-resistenter VAC-beta Expressionsvektoren (pGN26)

1 µg pAT153 wurde in 50 µl Lösung mit EcoRI geschnitten. Die Enzymaktivität wurde durch Erhitzen auf 70°C zerstört, und die überhängenden Enden durch Zusatz von DNA-Polymerase I/Klenow Fragment (PolIK)(5 units) und den vier Desoxyribonukleosidtriphosphaten (je 20 µM Endkonzentration) in einer fill in Reaktion begradigt. Nach Erhitzen auf 70°C zur Zerstörung der PolIK Aktivität wurde die DNA mit Äthanol ausgefällt. Die linearisierte DNA wurde in 50 µl mit PvuI nachgeschnitten.

pRH292 wurde zunächst mit SphI linearisiert und das 3' überhängende Ende durch die 3' exonukleolytische Aktivität der PolIK in Gegenwart von dGTP abgebaut. Nach Fällen der DNA wurde diese mit PvuI nachgeschnitten. Die Fragmente wurden auf einem Agarosegel getrennt und die entsprechenden Fragmente eluiert (pAT153: 3032 bp, pRH292: 2607 bp). 50 ng pAT153 Fragment wurden mit 50 ng pRH292 Fragment versetzt und in 10 µl ligiert. 100 µl kompetenter E.coli HB101 wurden mit 5 µl der Ligaselösung versetzt und transformiert. Die Selektion erfolgte auf LB-Agar, der 100 µg/ml Ampicillin enthielt. Von den entstandenen Klonen wurden einige auf LB-Agar mit 12,5 µg/ml Tetracyclin ausgestrichen. Die darauf wachsenden Klone wurden zur Herstellung von Plasmid DNA verwendet. Die in einer Minipräparation hergestellte Plasmid-DNA wurde mit verschiedenen Restriktionsenzymen geschnitten und so die Richtigkeit der Konstruktion überprüft. Ein Klon wurde ausgewählt und mit pGN26 (VAC-beta) bezeichnet.

E.coli HB101/pGN26 wurden, wie in Beispiel 7 beschrieben, einer Fermentation unterzogen, wobei diesmal anstelle des Amicillins 5 mg/l Tetracyclin in der Vorkultur verwendet wurden. Während der Hauptfermentation wurden Aliquots entnommen und auf einem 15% Acrylamid/0,1% SDS Gel nach Lämmli untersucht. Es wurde kein Unterschied zu der Fermentation von E.coli HB101/ pRH292 festgestellt. Aus der Biomasse der Fermentation wurde VAC-beta gereinigt. Die Proteinanalyse ergab ebenfalls keinen Unterschied zu dem entsprechenden rekombinanten Protein aus der Fermentation des Klons HB101/pRH292.

Beispiel 10

Reinigung von rekombinantem VAC-beta

Ausgangsmaterial: E.coli HB101/pRH 292.
Die Zellen waren zentrifugiert und
bei -70°C eingefroren worden.

a) Zell Homogenisierung und Extraktion
100 g gefrorener Zellkuchen wurde in 500 ml eiskaltem Lyse-Puffer (100 mM TRIS/HCl, 50 mM EDTA und 200 mM NaCl, pH 7,5) suspendiert und mit einem Ultra-Turrax (5 kurze Impulse) zur Zerstörung von Klumpen behandelt. Die Suspension wurde danach 3-mal durch eine Manton-Gaulin Presse bei 6.000 psi geführt. Zuletzt wurde die Presse mit 300 ml Lyse-Puffer gespült. Zur homogenisierten Suspension wurde langsam eine 5%ige Lösung PEI (Polyethylenimin, Molekulargewicht 30.000-40.000), das mit 5 N HCl auf pH8 eingestellt worden war, bis zu einer Endkonzentration von 0,5% PEI, hinzugefügt. Nach 30-minütigem Rühren in einem Eisbad wurde die Lösung bei 9.000 g, 60 min. und 4°C unter Verwendung einer Beckmann J2-21 Zentrifuge geklärt (roher Extrakt).

b) Chromatographie an Silica
Silica Catalyst Carrier, Grade 953W (Grace GmbH, Worms, BRD) wurde in destilliertem Wasser geklärt und in eine Chromatographiesäule von 5 cm Durchmesser und 20 cm Höhe gefüllt. Nach Äquilibrierung mit Lys-Puffer wurde der rohe Extrakt auf die Säule aufgetragen und mit 3 l Lys-Puffer gewaschen. VAC-beta wurde mit einem Lineargradienten von Tetraethylammoniumchlorid in Lys-Puffer (0-1 M in 200 ml Puffer) eluiert. Fraktionen des Eluats wurden durch SDS-PAGE auf VAC-beta unter Verwendung von Referenz-Material kontrolliert. VAC-beta enthaltende Fraktionen wurden gesammelt.

c) Chromatographie an DEAE-SEPHAROSE FAST FLOW
Die gesammelten Silica-Fraktionen wurden gegen 20 mM TRIS/HCl pH 8,4 dialysiert und auf eine 175 ml DEAE-FF-Sepharose-Kolonne (26 x 330 mm, Pharmacia), die mit 20 mM TRIS/HCl pH 8,4 equilibriert worden war, aufgetragen. Die Kolonne wurde mit 500 ml Pufferlösung gewaschen und VAC-beta mit einem Gradienten von 0-500 mM NaCl in 875 ml Pufferlösung (5 Kolonnen Volumen) eluiert. Das Eluat wurde bei 280 nm kontrolliert und durch SDS-PAGE, unter Verwendung eines Referenz-Materials analysiert. VAC-beta enthaltende Fraktionen wurden gesammelt und unter Verwendung einer AMICON Ultrafiltrationszelle und eines PM 10-Typ Ultrafilters konzentriert.

d) Chromatographie an Sephacryl S-200 "High Resolution"
Eine Pharmacia K 26/100 Kolonne (500 ml) wurde mit Sephacryl S-200 HR (Pharmacia) beladen und mit 20 mM Bis-TRIS/HCl + 100 mM NaCl pH 8,4 bei einer Durchflußrate von 120 ml/Stunde equilibriert. 4 ml Aliquots des konzentrierten DEAE-FF-Sepharose-Pools wurden auf die Säule aufgetragen und die Durchflußrate auf 60-80 ml/Stunde reduziert. Das Eluat wurde bei 280 nm überwacht. Der VAC-beta repräsentierende Peak war der einzige Peak des UV-Profils. Er wurde gesammelt, wobei hochmolekulare Verunreinigungen, die ebenfalls im UV-Profil detektierbar waren verworfen wurden. Das gereinigte VAC-beta wurde durch SDS-PAGE analysiert und bei -20°C aufbewahrt.

Tabelle: Reinigung von rekombinantem VAC-beta
Ausgangsmaterial: 100 g gefrorener Zellkuchen

| | Volumen(ml) | mg Protein/ml x) | mg total |
|---|---|---|---|
| ROHEXTRAKT | 810 | 16,1 | 13.040 |
| SILICA DURCHFLUSS | 2270 | 0,35 | 795 |
| VAC-beta SILICA POOL | 380 | 5,0 | 1.900 |
| VAC-beta DEAE-FF-POOL | 120 | 2,6 | 312 |
| DEAE-FF-POOL KONZENTRAT | 4 | 58 | 232 |
| VAC-beta SEPHACRYL-POOL | 33,5 | 2,2 | 74 |

x) Protein bestimmt durch BIO-RAD Protein Assay (Standard: bovine serum albumin)

Beispiel 11

Analytik und Charakterisierung von VAC-beta

Als In Process Kontrolle zwischen den einzelnen Reinigungsschritten wurde die SDS-Gelelektrophorese verwendet. Die genaue Durchführung dieser Analyse erfolgte gleich wie bei den Endkontrollen und ist dort beschrieben. Eine Serie der während einer Reinigung erhaltenen SDS-Gele ist im Beispiel 10 (Reinigung von VAC-beta) enthalten.

Als Endkontrollen und zur Charakterisierung des gereinigten Proteins wurden die folgenden Methoden verwendet:

a) Gelpermeations-HPLC
b) Reverse Phase HPLC
c) Aminosäureanalyse
d) N-terminale Sequenzierung
e) SDS-Gelelektrophorese
f) Isoelektrische Fokussierung

a) Gelpermeations-HPLC

Säule: Waters, Protein Pak I 125, 2 x (7,8 x 300 mm), 10 µm Teilchendurchmesser, Raumtemperatur

Eluens: 0,5 M $Na_2SO_4$, 0,02 M $NaH_2$ $PO_4$, pH 7,0, 0,04% Tween 20, 25% Propylenglykol

Flußrate: 0,5 ml/min

Detektion: UV-Absorption, 214 nm, 0,5 AUFS

Das Chromatogramm (Fig. 23) von gereinigtem VAC-beta zeigt den Hauptpeak des VAC-beta Monomeren bei einem Molekulargewicht von 29.000, daneben ist eine geringe Menge dimeres VAC-beta (M etwa 50.000) nachweisbar. Diese Werte für das Molekulargewicht liegen relativ weit unterhalb des erwarteten Wertes (M 37.000), was wahrscheinlich darauf zurückzuführen ist, daß die hier verwendete Gelpermeations-Säule nach der Molekülgröße bzw. -gestalt trennt und nicht nach dem Molekulargewicht

b) Reverse Phase HPLC

| | |
|---|---|
| Säule: | Bakerbond WP $C_{18}$, 4,6 x 250 mm, 5 µm Teilchendurchmesser, 30 nm Porendurchmesser, Raumtemperatur |
| Eluens A: | 0,1% Trifluoressigsäure in Wasser |
| Eluens B: | 0,1% Trifluoressigsäure in Acetonitril |
| Gradient: | 20% B für 2 min, 20 - 68% B in 24 min, 68% B für 10 min, 68 - 20% B in 1 min |
| Flußrate: | 1 ml/min |
| Detektion: | UV-Absorption, 214 nm, 0,5 AUFS |

Das Chromatogramm (Fig. 24) stammt direkt aus dem Eluat der letzten Reinigungsstufe und zeigt hauptsächlich den Peak des oxidierten VAC-beta (2 Disulfidbrücken, $t_R$= 26,6 min). Daneben sind etwa 9% reduziertes VAC beta ($t_R$=27,4 min) sowie einige kleinere Peaks von restlichen Verunreinigungen nachweisbar.

Das Chromatogramm (Fig. 25) zeigt dieselbe VAC-beta Probe nach 2 Stunden Inkubation in 3 M Harnstoff, 0,05 M Dithiotreitol. Das Protein liegt hauptsächlich in der reduzierten Form ($r_R$= 27,4 min) vor.

c) Aminosäureanalyse

Gereinigtes VAC-beta wurde über Reverse Phase HPLC (Methode siehe unter b) entsalzt. Der Hauptpeak von VAC-beta wurde in einem Hydrolyseröhrchen gesammelt und getrocknet.

Die Hydrolyse erfolgte mit 6 N Salzsäure (mit 1% Phenol) in der Gasphase (110°C, 22 Stunden).

Die Bestimmung der Aminosäuren erfolgte mit einem Aminosäureanalysator ((Typ 6300, Fa. Beckman) über eine Nachsäulenderivatisierung mit Ninhydrin.

Die Aminosäureanalyse (Fig. 26a und b) einer VAC-beta Probe weist eine Gesamtabweichung gegenüber der theoretischen Zusammensetzung von 6.56% auf.

d) N-terminale Sequenzierung

Gereinigtes VAC-beta wurde über Reverse Phase HPLC (Methode siehe unter b) entsalzt. Der Hauptpeak von VAC-beta wurde gesammelt und getrocknet.

Der Rückstand wurde in 75 µl 0,1% Trifluoressigsäure gelöst und direkt für die Sequenzierung verwendet.

Die Sequenzierung erfolgte mit einem Gasphasensequenator der Fa. Applied Biosystems (Modell 470 A9) mit dem Programm 39apth.

Mit einer Ausgangsmenge von etwa 1 nM konnte bis zur 39. Aminosäure sequenziert werden. Es wurde 100%ige Übereinstimmung mit der erwarteten Sequenz gefunden. N-terminales Methionin wurde offensichtlich zu 100% abgespalten. (Fig. 27)

e) SDS-Gelelektrophorese

Die SDS-Gelelektrophoresen wurden weitgehend nach der Originalvorschrift von U.K. Lämmli durchgeführt. Für in Process Kontrollen wurden VAC-beta-Proben vor dem Auftragen mit Dithiothreitol versetzt und aufgekocht. Endkontrollen erfolgten sowohl unter reduzierenden als auch unter nicht reduzierenden Bedingungen.

Die Färbung der Gele erfolgte mit Coomassie Blue.

Fig. 28 zeigt ein SDS-Gel von VAC-beta Proben.
Spur 1 und 10: Molekulargewichtsmarker
Spur 2 und 3: In Process Probe von VAC-beta mit und ohne DTT (Dithiothreitol)
Spur 4 und 6: 10 µg VAC-beta nicht reduziert
Spur 5 und 7: 10 µg VAC-beta mit DTT reduziert
Spur 8 und 9: 1 µg VAC-beta nicht reduziert

VAC-beta ohne Reduktionsmittel ist als Doppelbande nachweisbar. Die intensivere untere Bande bei M etwa 36.000 ist die oxidierte Form, die obere Bande bei M etwa 38.000 die reduzierte Form von VAC-beta. Die mengenmäßige Verteilung der beiden Formen ist besser aus den Spuren 8 und 9 ersichtlich.

Nach Zugabe von DTT als Reduktionsmittel ist daher nur mehr die obere Bande sichtbar (siehe Spur 3, 5 und 7). Da die Spuren 4 bis 7 Stark überladen sind, sind neben den VAC-beta Banden auch einige Banden von restlichen Verunreinigungen sichtbar.

f) Isoelektrische Fokussierung

Die Färbung erfolgte mit Coomassie Blue.

Die isoelektrische Fokussierung Fig. 29a und b (3.3.1988/51) von weitgehend gereinigtem VAC-beta zeigt, daß VAC-beta direkt aus der letzten Reinigungsstufe (Spur 2 und 3) zwei Hauptbanden bei pH 5,35 bzw. 5,45 ergibt. Der isoelektrische Punkt (pI) von VAC-beta ist damit deutlich basischer als der von VAC-alpha (pI=4,9), was denErwartungen entspricht, da VAC-beta weniger saure Aminosäuren enthält. Nach der Reduktion mit Dithiothreitol (Spur 4 und 5) ist die Hauptbande bei pH 5,45 stark angereichert. Diese stellt offensichtlich die reduzierte Form von VAC-beta dar.

<u>Beispiel 12</u>

<u>Biologische Aktivitäten von VAC-beta</u>

1. Effekt von VAC-beta auf die Prothrombinase Aktivität.

Der Prothrombinase Komplex wurde aus gereinigten bovinen Koagulationsfaktoren und Phospholipiden rekonstituiert. Er bestand aus 0,3 nM Faktor Xa, 0,6 nM Faktor Va, 1 µM Prothrombin, 2 µM Phospholipid Vesikeln (25% Dioleoyl-phosphatidylserin, 75 % Dioleoyl-phosphatidylcholin) und 3 mM $CaCl_2$.

Prothrombin Aktivierung wurde folgendermaßen gemessen: Faktor Xa, Va, Phospholipide, $Ca^{++}$ und variierende Mengen VAC-beta wurden zwei Minuten bei 37°C inkubiert. Die Aktivierung des Prothrombin wurde durch dessen Zugabe gestartet. Zu verschiedenen Zeiten wurden Proben der Mischung in folgendem Puffer aufgenommen:
50 mM TRIS/HCl, 175 mM NaCl, 0,5 mg/ml BSA, 20 mM EDTA und 0,23 mM S2238. Amidolytische Aktivität wurde bei 405 nm verfolgt. Aus einer Standardkurve, die mit bekannten Mengen Thrombin erstellt worden war, wurde anschließend die Menge an aktiviertem Prothrombin bestimmt. Fig. 17 gibt das Ergebnis wieder.

2. Phospholipase Inhibitor Aktivitäten von VAC

E.coli wurde metabolisch mit $^3$H-Ölsäure markiert und weiterbehandelt wie beschrieben (Davidson, F.F. et al. (1987) J. Biol. Chem. 262, 1690-1705). Die E.coli Membran Präparation enthielt 198 µM Phospholipide mit einer spezifischen Aktivität von 1,2 x $10^4$ cpm/nMol Phospholipid. Diese Membran Präparation diente als Phospholipasesubstrate in dem Assay, der wie folgt durchgeführt wurde: 103 µl 0,1 M TRIS/HCl, pH 8,0, unterschiedliche Mengen an VAC und $Ca^{++}$ enthaltend und 10 µl E.coli Membranen wurden bei Raumtemperatur inkubiert. Zum Zeitpunkt 0 wurden 10 µl 0,1 M TRIS/HCl, pH 8,0, 2 ng bovine Pankreas Phospholipan $A_2$ enthaltend zugegeben. Nach zwei Minuten wurde die Reaktion durch Zugabe von 50 µl 2N HCl und 50 µl 0,1 M TRIS/HCl pH 8,0, das 100 ng/ml BSA enthielt, gestoppt. Die Mischung wurde bei 14.000 rpm fünf Minuten zentrifugiert. Die Menge an freigesetzter $^3$H-Ölsäure wurde durch Szintillationszählung des Überstandes bestimmt. Prozent Inhibition der Phospholipase $A_2$ Aktivität wurde folgendermaßen berechnet :

$$100\%-[(cpm_{pla+vac}-cpm_{blank})/(cpm_{pla}-cpm_{blanc})]*100\%$$

Hierbei bedeutet $cpm_{pla+VAC}$, $cpm_{pla}$ und $cpm_{blank}$ die cpm, die in den Überständen der Mischungen, entweder Phospholipase $A_2$ und VAC-beta, Phospholipase $A_2$ oder VAC-beta enthaltend, gemessen wurden. Die Ergebnisse finden sich in den Figuren 18 und 19.

<u>Beispiel 13</u>

Herstellung der Expressionsvektoren pRH281/n und pRH28l/nT (n=5,6,7,8,9)

Der in der EPA Nr. 186 098 beschriebene Expressionsvektor pRH100 hat verschiedene Nachteile:

a) konstanter und nicht optimaler Abstand zwischen ribosomaler Bindungsstelle (RBS) und Translationsstart-ATG
b) Zwischen RBS und ATG gibt es einige C's und G's
c) die -35 Region des Serratia marcescens trp Promotors ist verglichen mit der E.coli Sequenz nicht optimal (TTGACT statt TTGACA)

d) die EcoRI-Stelle vor dem Promotor ist überflüssig.

Es wurde ein Satz von Oligonukleotiden mit folgender Sequenz synthetisiert:

```
:Trp-1->   ::Trp-3->
AATTGACGCTGATGGCTAAAACATTGTGCAAAAAGAGGGTTGACATTGCC
    CTGCGACTACCGATTTTGTAACACGTTTTTCTCCCAACTGTAACGG
           <-Trp-2::
                         :-->Transkriptionsstart
                         ::Trp-5->
TTCGCGAACCAGTTAACTAGTACACAAGTTCACGGCTCGAGACGGTAAGG
AAGCGCTTGGTCAATTGATCATGTGTTCAAGTGCCGAGCTCTGCCATTCC
                          <-Trp-4::            ----
                                                RBS

            SstI       ClaI
          ------EcoRI ----
                ------ :
AGGTTTAATATGAGCTCGAATTCAT
TCCAAATTATACTCGAGCTTAAGTAGC
----     ---      <-Trp-6:
RBS      Translationsstart-ATG
   : n=5 :
```

Jeweils 100 pMol der Oligonukleotide Trp-2, Trp-3, Trp-4 und Trp- 5 wurden in 10 µl phosphoryliert. Nach der Reaktion wurden äquimolare Mengen (je 100 pMol) Trp-1 und Trp-2, Trp-3 und Trp-4 sowie Trp-5 und Trp-6 vereint, auf 100°C erhitzt und durch langsames Abkühlen hybridisiert. Die Oligonukleotidpaare wurden vereint und in 55 µl ligiert. pAT153 wurde mit EcoRI und ClaI doppelt geschnitten und das große Vektorfragment isoliert. 50 ng pAT153 Fragment wurden mit 20 pMol synthetischer Promotor DNA in 20 µl ligiert. Kompetente E.coli HB101 wurden mit dem entstandenen Plasmid transformiert. Von den entstandenen Kolonien wurden einige ausgesucht, die Plasmid DNA isoliert und der Bereich der eingefügten DNA durch Sequenzieren überprüft. Ein Plasmid wurde ausgewählt und mit pRH281/5 bezeichnet, wobei 5 die Zahl der Nukleotide zwischen der RBS und dem Translationsstart- ATG symbolisiert.

Ausgehend von diesem Expressionsvektor wurde das XhoI-SstI Insert durch folgende Oligonukleotidpaare wie oben beschrieben ersetzt:

```
a)  TCGAGACGGTAAGGAGGTTTAAATATGAGCT      Trp-9
        CTGCCATTCCTCCAAATTTATAC          Trp-10

b)  TCGAGACGGTAAGGAGGTTTAAATAATGAGCT     Trp-11
        CTGCCATTCCTCCAAATTTATTAC         Trp-12

c)  TCGAGACGGTAAGGAGGTTTAAAATAATGAGCT    Trp-13
        CTGCCATTCCTCCAAATTTTATTAC        Trp-14
```

```
d)  TCGAGACGGTAAGGAGGTTTAAAAATAATGAGCT     Trp-15
        CTGCCATTCCTCCAAATTTTTATTAC          Trp-16
```

Die resultierenden Expressionvektoren wurden pRH281/6, pRH281/7, pRH281/8 und pRH281/9 benannt.

Diese neuen Expressionsvektoren weisen folgende Eigenschaften auf:

a) die originale EcoRI-Stelle von pAT153 wurde zerstört.
b) die -35 Region des Serratia marcescens Promotors ist jener des trp-Promotors aus E.coli angepaßt.
c) vor der artifiziellen ribosomalen Bindungsstelle befindet sich eine singuläre XhoI-Stelle, die das Einbringen einer anderen RBS erlaubt.
d) Das G des Tanslationsstart-ATG ist die erste Base einer SstI (=SacI) Stelle. Durch Schnitt nit SstI und anschließendem Abbau des 3' Überhanges entsteht ein gerades Ende, an das eine beliebige cDNA oder ein Gen ligiert werden kann. Beginnt diese DNA mit der ersten Base des zu translatierenden Bereichs, erfolgt eine korrekte Transkription und Translation in E.coli.
e) 3'seitig dieser SstI Stelle befinden sich eine singuläre EcoRI, ClaI und HindIII Stelle, die für ein gerichtetes Klonieren einer zu exprimierenden DNA verwendet werden kann. Außerdem können auch die singulären Schnittstellen im Tetracyclin Resistenzgen zu diesem Zweck gebraucht werden.
f) Die Variation /5 bis /9 erlaubt, den optimalen Abstand zwischen RBS und ATG für das jeweilige Gen zu bestimmen.

Manchmal ist es für eine optimale Expression und für die Stabilität des Plasmides nötig, hinter das exprimierte Gen einen Transkriptionsterminator zu setzen (R.Gentz et al., Proc.Natl.Acad.Sci.USA 78 (1981), 4936-4940). Das HindIII-SalI Fragment des pRH281/5 wurde durch Doppelverdau herausgeschnitten. Das fehlende Stück wurde durch ein den phoA Transkriptionsterminator (H.Shuttleworth et al., Nucl.Acids Res. 14 (1986), 8689; C.N.Chang et al., Gene 44 (1986), 121-125) enthaltendes Oligonukleotidpaar ersetzt:

```
:EBI-456->
AGCTTGGATCCGTCGACCGCGCCCGGCAGTGAATTTTCGCTGCCGGGTGG
    ACCTAGGCAGCTGGCGCGGGCCGTCACTTAAAAGCGACGGCCCACC
    :------
-----BamHI ------
HindIII      SalI

                      :
                TTTTTTTGCTGC
                AAAAAAACGACGAGCT
                      <-EBI-459:
```

10 pMol des hybridisierten Oligonukleotidpaares wurden mit 100 ng HindIII-SalI doppelt geschnittenen pRH281/5 Vektorteil in 20 µl ligiert. Nach Transformation von E.coli HB101, Isolierung der Plasmid-DNA einiger Kolonien und deren Sequenzüberprüfung wurde ein Plasmid ausgewählt und mit pRH281/5T bezeichnet.

In analoger Weise wie oben beschrieben wurde aus diesem Plasmid die Reihe pRH281/6T, pRH281/7T, pRH281/8T und pRH281/9T unter Verwendung der Oligonukleotide Trp-9 bis Trp-16 hergestellt.

<u>Analogbeispiel 1</u>

<u>Sequenzanalyse der VAC-alpha cDNA</u>

Die cDNA Klone pP6/5, pP6/6 und pP11/3 wurden total, sowie die der Klone Lambda-VAC1 bis -12 partiell sequenziert. Das Resultat ist in Fig.52 abgebildet. Es wurden insgesamt 1465 Basen sequenziert. Die cDNA weist einen langen, offenen Leserahmen auf, der für 320 Aminosäuren kodieren kann. Wird die DNA Sequenz in eine Aminosäuresequenz übersetzt, so können alle sequenzierten Peptide in dieser Sequenz untergebracht werden (Fig.32). Es handelt sich daher bei dieser cDNA um jene, deren korrespondierende mRNA für VAC-protein kodiert. Da die Sequenzen der zweiten isolierten cDNA (siehe unten) für ein ähnliches, aber von VAC verschiedenes Protein kodiert, wird hier der Name VAC-alpha eingeführt.

Dem ersten ATG-Codon (Basen 35-37) geht im selben Leserahmen ein Stop-Codon voran. Die Basen 30 bis 38 erfüllen ziemlich gut die Kozakregel (M. Kozak, Nature 308 (1984), pp. 241-246), die die Konsensussequenz in der Nähe des Translationsstartcodons mit CC(A/G)CCAUGG angibt, die entsprechende Sequenz hier lautet TCGCTATGG. Die 3' nichttranslatierte Region ist 471 Basen lang. 15 Basen vor Beginn des poly-A Abschnitts befindet sich die Polyadenylierungssequenz AATAAA (N.J.Proudfoot et al., Nature 263 (1976), pp. 211-214). Wird eine Kettenlänge von 150 bis 200 Basen für den Poly-A Abschnitt der mRNA gerechnet, beträgt die Gesamtlänge der mRNA auf Grund der cDNA Sequenz 1600-1650 Basen. Da im Northern Blot Experiment ein höherer Wert bestimmt wurde, ist die 5'nichttranslatierte Region in keiner cDNA komplett enthalten.

Die cDNA des Klons pP6/5 weist im Gegensatz zu allen anderen cDNA Klonen an Position 100 C statt A auf. Dadurch würde sich das Triplett 98-100 (22. Codon) von GAA nach GAC ändern und für Asp anstelle von Glu kodieren. Diese Abweichung kann mehrere Ursachen haben: a) die Reverse Transkriptase baute ein falsches Nukleotid ein, b) es handelt sich um die Transkripte zweier alleler, an dieser Stelle verschiedener Gene oder c) es gibt zwei nicht allele Gene, die sich an dieser Position unterscheiden.

Der lange, offene Leserahmen kodiert für ein Protein mit 320 Aminosäuren, von dem das Met-1 wahrscheinlich abgespalten, und das folgende Alanin an der Aminogruppe, möglicherweise durch Acylierung, blockiert wird. Das errechnete Molekulargewicht beträgt 35.896 D und ist höher als der Wert nach SDS-PAGE. Allerdings ist der Anteil geladener Aminosäuren (Asp, Glu, Lys, Arg, His) mit 30,6 % (98/320) überdurchschnittlich hoch verglichen mit dem durchschnittlichen Wert von 25,1 %. Dies würde das veränderte Wanderungsverhalten des Proteins bei der SDS-PAGE erklären. Innerhalb der stark geladenen Aminosäuren überwiegen die sauren Aminosäuren (Asp und Glu) mit 54 gegenüber den basischen (Lys und Arg) mit 41. Dies erklärt den sauren, isolektrischen Punkt des VAC-alpha Proteins (pI=4,4 bis 4,8) VAC-alpha enthält nur ein für Cystein codierendes Triplett (Aminosäure-Position 316); eine typische N-Glykosilierungsstelle (Asn-XXX-Ser/Thr) ist nicht vorhanden. Eine Strukturanalyse der Aminosäuresequenz (modifiziert nach Pustell, J et al., Nucleic Acids Res. 10 (1982) pp 4765-4782) ergibt eine vierfache Wiederholung einer 67 Aminosäure langen Sequenz (Fig.33), im folgenden "Repeats" genannt. Innerhalb dieser Sequenz sind 7 Aminosäuren (10,4 %) in allen vier Repeats konserviert, 15 Aminosäuren (22,4 %) kommen in drei von vier Repeats vor, an 28 Positionen (41,8 %) enthalten jeweils zwei Repeats dieselbe Aminosäure.

Ein Vergleich mit publizierten Literaturdaten (M.J.Geisow, FEBS Letters 203 (1986), pp. 99-103, M.J.Geisow et al., TIBS 11 (1986), pp. 420-423) ergab überraschenderweise, daß VAC-alpha damit zu einer größeren Gruppe $Ca^{++}$ abhängiger Phospholipidbindungsproteine gehört. Es wird eine Konsensussequenz beschrieben (Lys-Gly-fob-Gly-Thr-Asp-Glu-var-var-Leu-Ile-fil-Ile-Leu-Ala-fob-Arg; fob=hydrophob, fil=hydro-phil, var=variabel), die an der $Ca^{++}$ Bindung beteiligt sein könnte (M.J.Geisow et al., Nature 320 (1986), pp. 636-638). Diese Sequenz findet sich in jeder der vier wiederholten 67 Aminosäure langen Subsequenzen der erfindungsgemäßen Proteine (Fig.33). Auffällig ist auch der 6 Aminosäuren lange Abschnitt am Ende jeder Repeat, der fast außchließlich aus hydrophoben Aminosäuren besteht ("oooooo" in Fig.33).

Analogbeispiel 2

Expression von VAC-alpha in E.coli

a) pRH284T: Expressionsvektor mit dem alkalischen Phosphatasepromotor und Terminator

Das Gen für die alkalische Phosphatase (PhoA) aus E.coli unterliegt einer strengen Regulation. In Gegenwart von Phosphat wird das Gen komplett abgeschaltet, bei Abwesenheit von Phosphat im Medium erfolgt Genexpression. H.Shuttleworth et al., Nucleic Acids Res. 14 (1986), p.8689 sowie C.N.Chang et al., Gene 44 (1986), pp.121-125 beschreiben die Nukleotidsequenz dieses Gens.

Aus mehreren Oligonukleotiden wurde die Promotorregion des PhoA Gens zusammengesetzt und in EcoRI-ClaI geschnittenes pAT153 eingesetzt. Vor der ribosomalen Bindungsstelle wurde eine XhoI Stelle eingeführt. Die originale EcoRI Stelle wird beim Einligieren des synthetischen DNA-Fragmentes zerstört. Nach der ribosomalen Bindungsstelle wurde ein Translationsstart-ATG vorgesehen, dessen G das erste Nukleotid einer SacI (=SstI) Stelle ist. Der Expressionsvektor läßt sich durch Schnitt mit SacI an dieser Stelle linearisieren und der 3'Überhang durch Behandlung mit DNA-Polymerase I - Klenow Fragment in Gegenwart von dGTP in ein gerades Ende überführen. Dadurch kann jedes beliebige Gen an dieser Stelle eingefügt werden, für korrekte Expression muß es mit der ersten Base der kodierenden Region beginnen.

Das HindIII-SalI Fragment des pAT-Anteils wurde entfernt und durch den alkalischen Phosphatase-Transkriptionsterminator ersetzt. Die originale SalI Stelle wurde zerstört. Dafür wurde sie vor dem Terminator zusammen mit der ebenfalls aus pAT153 deletierten BamHI Stelle wieder eingebracht. Die Sequenz der synthetisch hergestellten DNA ist in Fig.34 abgebildet.

b) Expressionsklon pRH291

Der cDNA Klon pP6/5 wurde mit BglII und PstI geschnitten und das 980 bp lange Fragment isoliert, das den Hauptteil der kodierenden und ca. 200 bp 3'nichttranslatierte Region enthält. Mittels Oligonukleotiden wurde das fehlende 5'Ende der kodierenden Region ersetzt. Dabei wurde durch zwei Mutationen (GGC → GGT, Gly-7 und ACT → ACC, Thr-8) gleichzeitig eine KpnI-Schnittstelle in die VAC-cDNA eingeführt.

Die Oligonukleotide hatten folgendes Aussehen:

```
       |EBI-678                                            ||EBI-677
5'     GCACAGGTTCTCAGAGGTACCGTGACTGACTTCCCTGGATTTGATGAGCGGGCT   3'
       CGTGTCCAAGAGTCTCCATGGCACTGACTGAAGGGACCTAAACTACTCGCCCGA
       |                                      EBI-680||

   GATGCAGAAACTCTTCGGAAGGCTATGAAAGGCTTGGGCACAGATGAGGAGAGC
   CTACGTCTTTGAGAAGCCTTCCGATACTTTCCGAACCCGTGTCTACTCCTCTCG
                                                     EBI-

           ||EBI-682                                      |
   ATCCTGACTCTGTTGACATCCCGAAGTAATGCTCAGCGCCAGGAAATCTCTGCA  3'
   TAGGACTGAGACAACTGTAGGGCTTCATTACGAGTCGCGGTCCTTTAGAG      5'
   679||                                       EBI-681|
```

Die Oligonukleotide EBI-680, -677, -678 und -682 wurden am 5'Ende phosphoryliert. EBI-678 und -680, EBI-677 und-679 sowie EBI-682 und -681 wurden paarweise auf 100°C erhitzt und langsam abgekühlt (je 100 pMol in 20 µl). Die Doppelstrang-Oligonukleotide wurden vereint und ligiert. pRH284T wurde mit SacI linearisiert, das 3'überhängende Ende durch Behandlung mit DNA-Polymerase I / Klenow Fragment in Gegenwart von dGTP zu einem geraden Ende reduziert, und mit BamHI nachgeschnitten.Ca. 30 ng Vektor, 200 ng cDNA und 250 nMol Oligonukleotide wurden in 15 µl Lösung ligiert. Kompetente E.coli HB101 wurden mit der Ligaselösung transformiert. Der resultierende Klon wurde pRH291 benannt (Fig.35).

Analogbeispiel 3

Reinigung von rekombinantem VAC-alpha

Ausgangsmaterial: E.coli HB101/pRH 291.
Die Zellen waren zentrifugiert und bei -70°C eingefroren worden.

a) Zell Homogenisierung und Extraktion

103,5 g gefrorener Zellkuchen wurde in 500 ml eiskaltem Lyse-Puffer (100 mM TRIS/HCl, 50 mM EDTA und 200 mM NaCl, pH 7,5) suspendiert und mit einem Ultra-Turrax (5 kurze Impulse) zur Zerstörung von Klumpen behandelt. Die Suspension wurde danach 3-mal durch eine Manton-Gaulin Presse bei 6.000 psi geführt. Zuletzt wurde die Presse mit 300 ml Lyse-Puffer gespült. Zur homogenisierten Suspension wurde langsam eine 5%ige Lösung PEI (Polyethylenimin, Molekulargewicht 30.000-40.000), das mit 5 N HCl auf pH8 eingestellt worden war, bis zu einer Endkonzentration von 0,5% PEI, hinzugefügt. Nach 30-minütigem Rühren in einem Eisbad wurde die Lösung bei 9.000 g, 60 min. und 4°C unter Verwendung einer Beckmann J2-21 Zentrifuge geklärt (roher Extrakt).

b) Ammoniumsulfat Fraktionierung:

Festes Ammoniumsulfat wurde langsam zum gerührten rohen Extrakt bis zu einer Sättigung von 30% (176 g/l) gegeben. Der Niederschlag wurde nach einer Nacht im Kühlraum entfernt. Der klare Überstand wurde langsam bis zu einer Sättigung von 65% mit festem Ammoniumsulfat versetzt (235 g/l Überstand) und zwei Stunden gerührt. Das Präzipitat wurde dann durch Zentrifugation bei 10.000 g 30 min. bei 4°C gesammelt. Es wurde in 500 ml 20 mM TRIS/HCl + 50 mM NaCl, pH 7,4 gelöst und gegen denselben Puffer so lange dialysiert, bis alles Ammoniumsulfat entfernt worden war (bestimmt durch das Ausbleiben einer $BaSO_4$-Bildung nach Zugabe von $BaCl_2$ zum Dialysat).

c) Chromatographie an DEAE-SEPHAROSE FAST FLOW

Das Dialysat wurde durch Zentrifugation geklärt und auf eine 160 ml DEAE-FF-Sepharose-Kolonne (Pharmacia), die mit 20 mM TRIS/HCl + 50 mM NaCl pH 7,4 equilibriert worden war, aufgetragen. Sobald der $OD_{280nm}$ des Eluates den Puffer-Level erreichte, wurde ein Gradient von 50 - 500 mM NaCl in 20 mM TRIS/HCl pH 7,4 gefahren (insgesamt 10 Kolonnen-Volumina des linearen Gradienten).

Das Eluat wurde bei $OD_{280nm}$ kontrolliert und durch SDS-PAGE und Western Blot, unter Verwendung eines Kaninchen-Antiserums gegen Placenta-VAC hergestellt, wie für das Rinder-VAC in der EPA 0 181 465 beschrieben, und eines Schweine Anti-Kaninchen IgG gekuppelt an alkalischer Phosphatase, analysiert. VAC-enthaltende Fraktionen wurden gesammelt, wobei einige Fraktionen am Ende des Hauptpeaks verworfen wurden. Der VAC-Pool wurde unter Verwendung einer AMICON Ultrafiltrationszelle und eines PM 10-Typ Ultrafilters konzentriert.

d) Chromatographie an Sephacryl S-200 "High Resolution"

Eine Pharmacia K 26/100 Kolonne (500 ml) wurde mit Sephacryl S-200 HR (Pharmacia) beladen und mit 20 mM Bis-TRIS/HCl + 100 mM NaCl pH 6,3 bei einer Durchflußrate von 15-20 ml/Stunde equilibriert. 6-15 ml Aliquots des konzentrierten DEAE-FF-Sepharose-Pools (total 87,4 ml) und anschließend der Bis-TRIS-Puffer (s.o.) wurden auf die Säule aufgetragen. Das Eluat wurde bei OD 280 nm überwacht. Der VAC repräsentierende Peak war der letzte auffällige Peak des UV-Profils wie durch SDS-PAGE und Western Blots von Aliquots gezeigt werden konnte. Er wurde gesammelt, die Pools aller Versuche vereinigt (insgesamt 7) und gegen 20 mM Bis-TRIS/HCl pH 6,3 dialysiert.

e) Chromatographie an einer Q-SEPHAROSE-FAST-FLOW

Eine 40 ml Kolonne (K 16/20) einer Q-Sepharose-Fast-Flow (Pharmacia) wurde an das FPLC-System (Pharmacia) angeschlossen und mit 20 mM Bis-TRIS/HCl pH 6,3 equilibriert. Der dialysierte VAC-Pool wurde auf die Säule aufgetragen und so lange mit 20 mM Bis-TRIS gewaschen, bis der $OD_{280nm}$ des Eluates wieder den Puffer-Level erreicht hatte. Ein NaCl-Gradient in 20 mM Bis-TRIS/HCl pH 6,3 wurde zur Elution verwendet:

0-105 mM NaCl in 1 Kolonnen-Volumen ( 40 ml)
105-245 mM NaCl in 20 Kolonnen-Volumen (800 ml)
245-350 mM NaCl in 2 Kolonnen-Volumen ( 80 ml)

VAC konnte im letzten prominenten Peak des UV-Profils identifiziert werden, der bei ungefähr 0,14 M NaCl eluierte. Die Reinheit wurde mittels SDS-PAGE, Western Blot, Reverse HPLC und isoelektrischer Fokussierung bestimmt. Der VAC-Pool wurde bei -20°C aufbewahrt.

Analogbeispiel 4

Vergleich Rekombinantes/natürliches VAC-alpha

Verwendete Methoden:

a) Gelpermeations-HPLC
b) Reverse Phase HPLC
c) N-terminale Sequenzierung
d) Tryptic Peptid Map
e) SDS-Gelelektrophorese
f) Western Blot
g) Isoelektrische Fokussierung

Für den Vergleich wurde einerseits natürliches VAC, andererseits rekombinantes VAC-alpha verwendet. Im folgenden werden die Versuchsbedingungen für die einzelnen Analysenmethoden beschrieben.

a) Gelpermeations-HPLC

| | |
|---|---|
| Säule: | Waters Protein Pak I 125, 2 x (7,8 x 300 mm), 10 µm Teilchendurchmesser |
| Eluens: | 0,5 M $Na_2SO_4$, 0,02 M $NaH_2PO_4$, pH 7,0, 0,04% Tween 20, 25% Propylenglykol |
| Fluß: | 0,5 ml/min |

Detektion: UV-Absorption, 214 nm

Die Chromatogramme von natürlichem und rekombinantem VAC-alpha zeigen den Hauptpeak des VAC-Monomeren bei einem Molekulargewicht von 34.000 bzw. 33.000. Daneben gibt es jeweils einen geringen Anteil von früher eluierendem Dimeren des VAC. Die Eichung der Molekulargewichtsskala erfolgte über 4 Standardproteine. Die Säule trennt streng genommen nach Molekülgröße und nicht nach Molekulargewicht.

b) Reverse Phase HPLC

| | |
|---|---|
| Säule: | Bakerbond WP $C_{18}$, 4,6 x 250, 5 µm Teilchendurchmesser, 30 nm Porendurchmesser |
| Eluens A: | 0,1% Trifluoressigsäure in Wasser |
| Eluens B: | 0,1% Trifluoressigsäure in Acetonitril |
| Gradient: | 20% B für 2 min, 20 - 68% B in 24 min, 68% B für 10 min, 68 - 20% B in 1 min |
| Fluß: | 1,0 ml/min |
| Detektion: | UV-Absorption, 214 nm und 280 nm |

Die Chromatogramme von natürlichem und rekombinantem VAC zeigen für VAC eine Retentionszeit von etwa 29 min. Die daneben vorliegenden sehr kleinen Peaks sind nur zum Teil Verunreinigungen der VAC-Probe. Alle mit BW gekennzeichneten Peaks stammen aus dem Blindwert der Säule.

c) N-terminale Sequenzierung

Sequenziert wurde ein über Reverse Phase HPLC entsalzter Peak von rekombinantem VAC-alpha. Die Sequenzierung erfolgte mit einem Gasphasensequenator der Firma Applied Biosystems (Modell 470A) mit dem Programm 40APTH. Die Probe wurde in 50 µl 70% HCOOH gelöst. Am Sequenator wurden 2 x 25 µl aufgetragen. Mit einer Ausgangsmenge von 2,3 nMol konnte bis zur Aminosäure 39 sequenziert werden. Es wurde 100%ige Übereinstimmung mit der erwarteten Sequenz (aus natürlichem Protein und cDNA) gefunden. Zusätzliches N-terminales Met konnte nicht nachgewiesen werden (≤ 1%). Der N-Terminus liegt bei rekombinantem VAC-alpha frei und nicht blockiert vor wie bei natürlichem VAC.

d) Tryptic Peptid Map

Verglichen wurden natürliches VAC und rekombinantes VAC-alpha. Aus beiden Proben wurde VAC über Reverse Phase HPLC entsalzt, getrocknet und in 0,1% $NH_4HCO_3$ gelöst. Für die Spaltung wurden 4 Gewichts% Trypsin (Worthington, TPCK behandelt, gelöst zu je 1 mg/ml in Wasser) zugesetzt, nach 6 Stunden Inkubation bei 37°C nochmals 4 Gewichts% Trypsin. Nach weiterer Inkubation über Nacht wurden die entstandenen Peptide über Reverse Phase HPLC getrennt. Die beiliegenden Chromatogramme (214 nm und 280 nm) zeigen ein praktisch identisches Peptidmuster.

| | |
|---|---|
| Säule: | Waters µBondapak $C_{18}$, 3,8 x 300 mm, 10 µm Teilchendurchmesser 10 nm Porendurchmesser |
| Eluens A: | 0,1% Trifluoressigsäure in Wasser |
| Eluens B: | 0,1% Trifluoressigsäure in Acetonitrill |

Gradient: 0 - 55% B in 55 min, 55% B für 15 min, 55 - 0% B in 1 min

Fluß: 1,0 ml/min

Detektion: UV-Absorption, 214 nm und 280 nm

e) SDS-Gelelektrophorese

SDS-Gelelektrophorese wurde weitgehend nach der Originalvorschrift von U.K. Laemmli durchgeführt (Nature 227, 680-685 (1979)). Die Silberfärbung erfolgte nach der Methode von Oakley (Anal. Biochem. 105, 361-363 (1980)). Das erste Gel zeigt einen Vergleich zwischen natürlichem und rekombinantem VAC-alpha. Der Gehalt an dimerem VAC wurde durch Scannen mit einem Laserdensitometer quantifiziert und betrug 2% beim natürlichen und 4% beim rekombinantenm VAC. Das zweite Gel zeigt nur rekombinantes VAC-alpha, aufgetragen mit und ohne DTT (Dithiothreitol, Reduktionsmittel). Daraus ist ersichtlich, daß das SDS-stabile Dimere offensichtlich über Disulfidbrücken gebunden ist, die mit DTT reduziert werden können.

Stammlösungen der Reagenzien

15% Ammonium-persulfat-Lösung (APS)

150 mg Ammonium-persulfat werden in 1 ml dest. Wasser gelöst.

30% Acrylamid + 0,8% Bis Acrylamid

| Acrylamid | Bis-acrylamid | Wasser auf |
|---|---|---|
| 15 g | 0,4 g | 50 ml |
| 30 g | 0,8 g | 100 ml |
| 45 g | 1,2 g | 150 ml |

Das Acrylamid wird in dem entsprechenden Wasservolumen gelöst und vor der Verwendung filtriert.

Trenngel-Puffer

1,5 M TRIS-HCL, 0,4% SDS (Sodium-dodecylsulfat), pH 8,8 18,16 g TRIS + 0,4 g SDS mit konz. HCl auf pH 8,8 einstellen und mit dest. Wasser auf 100 ml auffüllen.

Stackinggel-Puffer

0,5 M TRIS-HCl, 0,4% SDS, pH 6.8
6,04 g TRIS + 0,4 g SDS mit konz. HCl auf pH 6,8 einstellen und mit dest. Wasser auf 100 ml auffüllen.

Laufpuffer

25 mM TRIS, 192 mM Glycin, 0,1% SDS, 0,005% Na-azid pH 8,5 12 g TRIS + 57,6 g Glycin + 4 g SDS + 0,2 g Natriumazid werden in ca. 3,5 1 dest. Wasser gelöst, auf pH 8,5 eingestellt und auf 4,0 1 aufgefüllt. Es empfiehlt sich die Leitfähigkeit des neuen Laufpuffers mit den vorhergehenden Chargen zu vergleichen.

0,05% Coomassie-Blue-Färbelösung

0,55 g Coomassie Brillant Blue R 850 werden in 500 ml Methanol gelöst, 30 min gerührt und filtriert. Dazu gibt man 100 ml Eisessig und 500 ml dest. Wasser.

Entfärbelösung

a) Manuelles Entfärben
Die Entfärbelösung entspricht der Färbelösung ohne Farbstoff: 500 ml Methanol + 500 ml dest. Wasser + 100 ml Eisessig
b) Elektrophoretisches Entfärben in 7,5% Essigsäure

4 x SDS Auftragepuffer (ca. 1 Monat haltbar)

| | | |
|---|---|---|
| Farbstoffkonzentrat: | Bromphenolblau<br>Glycerin<br>Wasser (dest.) auf | 50 mg<br>0,5 ml<br>10 ml |

Die Lösung ist ca. 3 Monate haltbar.

| | | |
|---|---|---|
| Auftragepuffer: | Farbstoffkonzentrat<br>SDS<br>Glycerin<br>Wasser (dest.) auf | 0,4 ml<br>0,8 g<br>4 g<br>10 ml |

1 x SDS Auftragepuffer

Verdünnung 1:4 des 4 x SDS Auftragepuffers mit dest. Wasser.

Silberfärbung nach Oakley

Reagenzien

| | | |
|---|---|---|
| Destainer: | Ethanol<br>Essigsäure (conc.)<br>Dest. Wasser auf | 200 ml<br>62,5 ml<br>1000 ml |

10% Glutardialdehyd-Lösung:

| | | |
|---|---|---|
| 25 % Glutardialdehyd-Lösung | 20 ml | bzw. 40 ml |
| Dest. Wasser auf | 50 ml | bzw. 100 ml |

Die Lösung muß im Kühlschrank aufbewahrt werden.

0,1 N ammoniakalische Silberlösung:

Erst direkt vor Gebrauch herstellen!

| | | |
|---|---|---|
| 0,1 N Silbernitrat (16,9 g/l) | 23 ml | 46 ml |
| 25 % Ammoniaklösung | 0,95 ml | 1,9 ml |
| 0,36 % Natriumhydroxid (1,8 g/0,5 l) | 10,5 ml | 21 ml |
| dest. Wasser auf | 50 ml | 100 ml |

Entwickler-Lösung:

Unmittelbar vor Gebrauch herstellen!

| | |
|---|---|
| 0,5 % Citronensäure (1,25 g/250 ml) | 5 ml |
| dest. Wasser | 1 l |
| 37 % Formaldehyd | 1 ml |

Entfärbe-Lösung (Photo-Fixierer):

Kodak-Fixierer (Photolabor) Verdünnung 1:4 mit dest. Wasser. Die Verdünnung ist mehrfach verwendbar, jedoch nimmt die Dauer des Entfärbens nach häufigerer Verwendung zu.

2% Glycerin-Lösung:

23 g Glycerin 87% in 1 l dest. Wasser

Durchführung der Silberfärbung nach Oakley

Nach beendeter Elektrophorese wurde das Gel an einer Ecke markiert und durchlief danach folgende Färbeschritte im Schüttler:

- 30 min im Destainer-Bad
- 30 min in 20% Glutardialdehyd-Lösung (50 ml)
- 30 min in fließendem Wasser oder über Nacht in ca. 2 1 Wasser
- 10 min in ammoniakalischer Silberlösung (50 ml)
- 3 min in fließendem Wasser
- ca. 5 min in Entwickler-Lösung

Der Entwicklungsvorgang wurde nicht nach einer exakten Zeitdauer beendet, sondern dann, wenn die Banden ausreichend gefärbt waren oder spätestens wenn sich der Hintergrund zu färben begann. Gestoppt wurde die Entwicklung durch

- 30 min Wässern der Gele (in einer Wanne oder unter Fließwasser)

Die Entwicklung dauerte auch noch an, wenn das Gel bereits im Wasser war! (Diffusionszeit). Das Entfärben des Gels war nur dann erforderlich, wenn der Hintergrund zu stark gefärbt war!

- ca. 5-10 min in Kodak-Fixierer, bis ein optimaler Kontrast zwischen den Banden und dem Hintergrund erreicht war. Achtung! Das Entfärben erfolgte auch noch nach unmittelbarem Abstoppen in fließendem Wasser (Diffusionszeit).
- 30 min in 2% Glycerin-Lösung

Danach konnte das Gel auf Filterpapier bei 80°C 1 Std. getrocknet werden.

f) Western Blot

Der immunologische Nachweis erfolgte für VAC mit rabbit anti VAC Serum (1:1000 verdünnt) und mit swine anti rabbit IgG (1:500 verdünnt), das mit Alkalischer Phosphatase konjugiert ist. Zum Nachweis der Enzymaktivität der Alkalischen Phosphatase wurden die Substrate BCIP (5-Bromo-4-chloro-3-indolyl-phosphat) und NBT (Nitro Blue Tetrazolium) eingesetzt. Die vergleichende Proteinfärbung auf der Nitrocellulose erfolgte mit Amidoschwarz.

Western-blotting im semi-dry Verfahren

1. Materialien:

Semy-dry Electroblotter (Fa. Sartorius) SM 175 56; Filterpapier in der Größe des zu blottenden Gels; Nitrocellulose-Membran (Porengröße 0,45 µm) in Gelgröße;

2. Reagenzien

Anodenpuffer 1: pH 10,4

| | |
|---|---|
| 0,3 M TRIS | 3,63 g/80 ml |
| 20 % Methanol | 20 ml |

Anodenpuffer 2: pH 10,4

| | |
|---|---|
| 25 mM TRIS | 0,3 g/80 ml |
| 20 % Methanol | 20 ml |

Kathodenpuffer: pH 9,4

| | | |
|---|---|---|
| 25 mM TRIS | 0,3 g/80 ml | |
| 40 mM ε-Aminocapronsäure | 0,52 g/80 ml | (MW 131,3) |
| 20 % Methanol | 20 ml | |

Amidoschwarz-Protein-Färbemedium

| | |
|---|---|
| 0,5% (w/v) Amidoschwarz | 0,5 g in 40 ml |
| 50% Methanol | 50 ml |
| 10% Essigsäure | 10 ml |

Entfärber

| | | |
|---|---|---|
| Methanol | - Wasser - | Essigssäure |
| 5 | - 5 - | 1 |

| PBS (Phosphate buffered saline) 10x Konzentrat | pH 7.2 |
|---|---|
| 136 mM Natriumchlorid | 80 g |
| 26 mM Kaliumchlorid | 2 g |
| 80 mM di-Natriumhydrogenphosphat | 11,3 g |
| (80 mM di-Natriumhydrogenphosphat .12$H_2O$ | 28,7 g) |
| 14 mM Kalium-dihydrogenphosphat | 2 g |
| mit dest. Wasser auffüllen auf | 1 l |
| vor Gebrauch 1/10 verdünnen !!! | |

Blocking Puffer:

1% BSA
0,1% Tween 20
in lx PBS

Waschpuffer:

1 x PBS
1 x PBS + 0,1% Tween 20

1 x PBS

Färbepuffer für Alkalische Phosphatase-Färbung:

| | |
|---|---|
| pH 9,9 | |
| 100 mM TRIS | 1,22 g |
| 100 mM Natriumchlorid | 0,58 g |
| 5 mM Magnesiumchlorid. $6H_2O$ | 0,10 g |
| mit dest. Wasser auffüllen auf | 100 ml |

NBT (Nitro-Blue Tetrazolium) - Lösung

50 mg NBT (Fa. Sigma N-6876)/ml 70% Dimethylformamid

BCIP (5-Bromo-4-chloro-3-indolyl-phosphat) - Lösung

50 mg BCIP (Fa. Sigma B-8503)/ml in 100% Dimethylformamid

Färbemedium für Alkalische Phosphatase

| | |
|---|---|
| Färbungspuffer | 10 ml |
| BCIP | 0,033 ml |
| NBT | 0,065 ml |

Antikörperreaoenzien:

Rabbit anti VAC-Serum mit alkalischer Phosphatase konjugiert.

3. Durchführung des Blots

Das Blotten erfolgte bei 0,8 $mA/cm^2$ Gelfläche über 60 min

4. Nachweis der Proteine auf der Nitrocellulose-Membran

4.1. Proteinfärbung mit Amidoschwarz

Der für die Proteinfärbung vorgesehene Anteil der Nitrocellulose-Membran wurde abgeschnitten und 5 Minuten in Amidoschwarz-Färbelösung gelegt. Danach wurde die mehrfach verwendbare Färbelösung abgegossen und überschüssige Färbelösung von der Nitrocellulose-Membran mit Wasser abgespült.

4.2 Immunologischer Nachweis

4.2.1 Blockierung der Nitrocellulose-Membran

Vor dem immunologischen Nachweis wurde die Nitrocellulose-Membran mindestens 60 min (oder auch über Nacht) mit 1% BSA in lxPBS mit 0,1% Tween 20 blockiert.

4.2.2. Nachweis mit Enzym-markierten Antikörpern

Nach dem Blockieren wurde die Nitrocellulose-Membran mit rabbit anti VAC Serum (in geeigneter Verdünnung in Blockierungsmedium) 60 min inkubiert.

Danach wurde 3x gewaschen: lx PBS, PBS mit 0,1% Tween, 20,lx PBS. Darauf erfolgte die Inkubation der Membran mit swine anti rabbit IgG, das mit Alkalischer Phosphatase konjugiert war (ebenso in einer geeigneten Verdünnung in Blockierungsmedium über 60 min). Danach wurde erneut 3x mit PBS gewaschen wie oben angegeben.

4.2.3 Enzymatische Färbung des Blots

Die Nitrocellulose-Membran wurde in Färbemedium gelegt (für ein Minigel reichten 10 ml aus) und im Schüttler langsam bewegt, bis sich eine ausreichende Anfärbung der Banden zeigte. Die Färbung wurde durch Auswaschen des Färbemediums mit Wasser beendet, danach die Nitrocellulose-Membran an der Luft getrocknet.

5. Bewertung des Western-Blots

Die immunologisch/enzymatisch nachgewiesenen Banden des Blots wurden mit den Banden der Proteinfärbung des Blots und auch mit dem entsprechenden Bandenmuster der SDS-Elektrophorese verglichen und einander zugeordnet.

7. Isoelektrische Fokussierung

Der isoelektrische Punkt (pI) für rekombinantes VAC-alpha liegt mit 4,9 um 0,1 pH-Einheiten höher als für natürliches VAC (4,8).

Polyacrylamidgel-Isoelektrische Fokussierung (PAGIF oder IEF)

Material und Reagenzien

Polyacrylamidgelplatten für Isoelektrische Fokussierung auf Folie aufpolymerisiert. (LKB -"PAGplate", SERVA - "Servalyt Precotes")

Elektrodenlösungen für die jeweiligen pH-Bereiche:

pH 3,5-9,5 (LKB-PAGplate)

| | |
|---|---|
| Anodenlösung: | 1 M Phosphorsäure |
| Kathodenlösung: | 1 M Natronlauge |

pH 5,5-8,5 (LKB-PAGplate)

| | |
|---|---|
| Anodenlösung: | 0,4 M HEPES (mit Na-azid) |
| Kathodenlösung: | 0,1 M NaOH |

pH 4,0-6,5 (LKB-PAGplate)

| | |
|---|---|
| Anodenlösung: | 0,5 M Phosphorsäure + 0,1 M Glutaminsäure |
| Kathodenlösung: | 0,1 M Beta-Alanin (mit Na-azid) |

pH 3,5-9,5 (SERVALYT-Precotes)

| | |
|---|---|
| Anodenlösung: | 25 mM Asparaginsäure + 25 mM Glutaminsäure |
| Kathodenlösung: | 2 M Ethylendiamin + 25 mM Arginin Base + 37,5 mM Lysin Base |
| Kühlvermittler: | Kerosin (Fa. SERVA) |
| Fixierlösung: | 10% Trichloressigsäure (TCA) mit 5% Sulfosalicylsäure 0,05% Coomassie Blue Färbelösung |
| Entfärber: | 500 ml Methanol + 500 ml Wasser + 100 ml Eisessig |

Proben:
Die Proben sollen salzarm oder besser noch salzfrei sein. Höhere Salzkonzentration müssen vor der IEF gegen ca. 5-10 mM Puffer oder Wasser dialysiert werden. NaCl kann jedoch bis zu 100 mM enthalten sein, ohne daß die Lauffronten gestört werden.

Fixierung des IEF-Gels vor Proteinfärbung

Das Gel wurde ca. 20 min in die Fixierlösung gelegt, jedoch ohne dabei zu schütteln! (Es löste sich dabei leicht von der Folie ab) Danach wurde 5 min gewässert.

Proteinfärbung der IEF-Gele mit Coomassie Blue

Die fixierten Gele wurden ca. 2 Std. in Färbelösung gelegt und dabei nur sehr leicht bewegt.

Entfärben der IEF-Gele

Nach der Färbung wurde die überschüssige Färbelösung mit Wasser abgespült und durch häufiges Wechseln des Entfärbers das Gel entfärbt. Nach ausreichendem Entfärben wurde das Gel wieder gewässert, bis der Entfärber ausgewaschen war.

Trocknen der IEF-Gele

Die Gele wurden auf eine nasse, wasserdurchlässige Klarsichtfolie oder Dialysemembran gelegt, mit der wasserundurchlässigen Seite nach oben und 1 Std. bei 80°C getrocknet.

Analogbeispiel 5

Herstellung von monoklonalen Antikörpern gegen VAC-alpha

1. Kopplung von VAC-alpha an "keyhole limpet" - Hämocyanin (KLH)

VAC-alpha wurde folgendermaßen kovalent an "keyhole limpet" - Hämocyanin (KLH; SIGMA chemical company, St. Louis, USA, Katalog-Nummer H-2133) gekoppelt.

1.1 VAC-alpha/KLH Präparation 1:

0,51 mg VAC-alpha wurden in 1,5 ml isotoner phosphat-gepufferter Natriumchlorid-Lösung pH 7,2 (im folgenden "PGS" abgekürzt) gelöst und mit 0,12 ml einer KLH-Lösung (8 mg/ml in PGS) gemischt. 0,017 ml einer 25%igen Glutardialdehydlösung wurden hinzugefügt und das Gemisch 45 Minuten lang bei Raumtemperatur inkubiert. Danach wurde über Nacht gegen 1 1 PGS dialysiert. Portionen dieser Lösung wurden bei -20°C gelagert.

1.2 VAC-alpha/KLH Präparation 2:

2,54 mg VAC-alpha wurden in 2 ml 0,1 M Kaliumphosphat-Puffer pH 7,0 gelöst und mit 0,3 ml KLH-Lösung (siehe 1.1) gemischt. 0,023 ml einer 25%igen Glutardialdehyd-Lösung wurden hinzugefügt und das Gemisch 45 Minuten lang bei Raumtemperatur inkubiert. Danach wurde über Nacht gegen 1 l PGS (siehe 1.1) dialysiert. Portionen dieser Lösungen wurden bei -20°C gelagert.

2. Immunisierung

Eine 10 Wochen alte weibliche Balb/c-Maus wurde folgendermaßen immunisiert:

| Immunisierung | Tag | VAC-alpha/KLH Präparation | Adjuvans |
|---|---|---|---|
| 1 | 1 | 1 (0.15 ml) | komplett |
| 2 | 24 | 1 (0.15 ml) | inkomplett |
| 3 | 49 | 1 (0.15 ml) | inkomplett |
| 4 | 188 | 2 (0.1 ml) | inkomplett |
| 5 | 225 | 2 (0.1 ml) | inkomplett |
| 6 | 258 | 2 (0.1 ml) | inkomplett |
| 7 | 265 | 2 (0.1 ml) | inkomplett |
| 8 | 271 | 2 (0.1 ml) | inkomplett |
| 9 | 272 | 2 (0.1 ml) | inkomplett |
| 10 | 273 | 2 (0.1 ml) | kein |

Alle Immunsierungen, ausgenommen die letzte, erfolgten durch intraperitoneale Injektion einer Emulsion gleicher Volumina von Antigen-Lösung und Freund'schem Adjuvans.

3. Zellfusion und Hybridom-Selektion

Peritoneale Zellen wurden aus Balb/c Mäusen durch Spülen mit einer sterilen Saccharose-Lösung (110 g/l in deionisiertem Wasser) gewonnen, durch Zentrifugation gesammelt und in Roswell Park Memorial Institute 1640 Medium mit Natrium-Penicillin G (100 Einheiten/ml) und Streptomycin (50 Einheiten/ml) (im folgenden "Kulturmedium genannt) mit 10% fötalem Kalbsserum in einer Zelldichte von etwa 3,5 x $10^4$ Zellen/ml suspendiert. 0,1 ml - Aliquots wurden in die Vertiefungen von Gewebekulturplatten (96 Vertiefungen pro Platte) pipettiert; die Platten wurden über Nacht inkubiert.

Einen Tag nach der letzten Immunisierung wurde die Maus in Äther anästhesiert und getötet. Die Milz wurde aseptisch entnommen und die Milzzellen wurden durch vorsichtiges Schaben auf einem Stahlgitter gewonnen. Die Zellen wurden in 10 ml Kulturmedium suspendiert und durch Zentrifugation gesammelt.

P3x63Ag8,653 Maus-Myelomzellen (Kerney et al., J. Immunol. 123, 1548, 1979) wurden in stationärer Suspensionskultur in Kulturmedium mit 10% fötalem Kalbsserum gezüchtet. Etwa $10^8$ Zellen wurden durch Zentrifugation gesammelt. Die Myelomzellen wurden zu den Milzzellen zugegeben; die gemischte Zellpopulation wurde neuerlich durch Zentrifugation gesammelt und in 3 ml einer sterilen Lösung von 40% Polyäthylenglycol 4000 (Merck, Darmstadt BRD, Katalognummer 9727) in Kulturmedium suspendiert. Die Suspension wurde 1,5 Minuten bei Raumtemperatur vorsichtig geschüttelt und anschließend 1 Minute stehen gelassen. 3 ml Kulturmedium wurden unter ständigem Schütteln über einen Zeitraum von 1,5 Minuten zugetropft, die Suspension dann eine Minute stehengelassen. Anschließend wurden weitere 6 ml Kulturmedium unter Schütteln über 1,5 Minuten zugetropft und 1 Minute stehen gelassen. Die Suspension wurde in der Folge unter Schütteln mit 12 ml Kulturmedium mit 10% fötalem Kalbsserum tropfenweise während drei Minuten verdünnt, dann 15 Minuten bei Raumtemperatur stehen gelassen. Die Zellen wurden durch Zentrifugation gesammelt und in 150 ml Kulturmedium mit 20% fötalem Kalbsserum, Thymidin (1,6 x $10^{-5}$ M), Hypoxanthin ($10^{-4}$ M) und Aminopterin (4 x $10^{-7}$) (in der Folge "HAT-Medium" genannt) suspendiert. Je 0,1 ml dieser Suspension wurden in die Vertiefungen einer Zellkultur-Platte pipettiert, in die am Vortag peritoneale Zellen eingesetzt worden waren (siehe oben); die Platten wurden drei Tage inkubiert. 0,075 ml HAT-Medium wurden zugegeben, anschließend wurden die Kulturen weitere 8 Tage inkubiert. Alle Inkubationen der Zellkulturen wurden bei 37°C in einer wasserdampf-gesättigten Atmosphäre von 5% $CO_2$ in Luft ausgeführt.

3. Screening nach Hybridomen, die Antikörper gegen VAC-alpha produzieren

Das Screening wurde mit Hilfe eines Enzym-Immuntest durchgeführt. Die Vertiefungen einer für Enzym-Immuntests geeigneten Mikrotiter-Platte wurden mit VAC-alpha beschichtet (0,005 mg/ml in 0,1 M Natriumkarbonat-Puffer pH 9,6; über Nacht bei 2-8°C oder eine Stunde bei 37°C). Die Lösung wurde entfernt und ie Platten wurden einmal mit deionisiertem Wasser gewaschen. Freie Protein-Bindungsstellen wurden mit einer Lösung von Rinderserum-Albumin (5µ/ml in isotoner phosphat-gepufferter Kochsalzlösung pH 7,2 [PGS]) eine Stunde bei Raumtemperatur blockiert, anschließend einmal mit Wasser gewaschen. In jede Vertiefung wurden 0,1 ml Hybridom-Überstand zugegeben und 2-3 Stunden bei Raumtemperatur inkubiert. Anschließend wurden die Überstände entfernt, die Vertiefungen wurden dreimal mit Wasser gewaschen.

0,05 ml einer Lösung von mit Meerrettich-Peroxidase konjugierten Kaninchen-Antikörpern gegen Maus-Immunglobuline (Dakopatts, Kopenhagen, Dänemark, Katalognummer P161; 1:2000 in PGS mit 5 mg/ml Rinderserumalbumin wurden zugegeben und drei Stunden bei Raumtemperatur inkubiert, anschließend dreimal mit Wasser gewaschen. 0,1 ml einer frisch hergestellten Mischung gleicher Volumina von Lösungen von ortho-Phenylendiamin (8,65 mg/ml in 0,1 M Kaliumzitrat pH 5), Natrium Perborat (3,75 mg/ml in 0,1 M Kaliumzitrat pH 5) und Wasser wurden zugegeben. Nach 30 Minuten Inkubation bei Raumtemperatur wurden 0,1 ml 4 N $H_2SO_4$ zugegeben. Die optische Dichte der Lösungen wurde in einem Mehrkanal-Photometer für Mikrotiter-Platten bei 492 nm bestimmt.

Zellkultur-Medium mit 20% fötalem Kalbsserum wurde als negative Kontrolle, Serum der immunisierten Maus (in PGS mit 5 mg/ml Rinderserumalbumin verdünnt) als positive Kontrolle verwendet. Kulturüberstände aller Hybridom-Kulturen wurden in zwei unabhängigen Tests 11 bzw. 13 Tage nach der Zellfusion getestet. Kulturen, die in beiden Tests ein positives Signal gaben, wurden in weiteren Screening-Test untersucht, in denen sowohl unbeschichtete als auch mit VAC-alpha beschichtete Testplatten eingesetzt wurden. Zwei der Kulturen gaben wiederholt positive Ergebnisse mit beschichteten, nicht aber mit unbeschichteten Platten. Die Ergebnisse sind in der folgenden Tabelle zusammengefaßt:

| Probe | Optische Dichte bei 492 nm | | | |
|---|---|---|---|---|
| | Experiment 1 | | Experiment 2 | |
| | beschichtet | | beschichtet | |
| | | unbeschichtet | | unbeschichtet |
| Mausserum 1: 100 | 0,291 | 0,152 | 1,735 | 0,475 |
| Hybridom VAA-8 | 1,061 | 0,102 | 1,169 | 0,108 |
| Hybridom VAA-9 | 0,552 | 0,187 | 0,387 | 0,109 |
| Negative Kontrolle | 0,032 | 0,031 | 0,071 | 0,055 |

Analogbeispiel 6

Mutanten des VAC-alpha

Folgende Mutationen wurden an der VAC-alpha DNA durchgeführt (die Aminosäurenummerierung und die Basennummerierungen beziehen sich auf die Figur 52):

a) Verkleinerung des VAC-Moleküls: VAC-alpha besitzt eine vierfach repetierte 67/68 Aminosäuren lange Sequenz mit einer singulären N-terminalen Extension (Fig.33). Drei verkürzte Formen wurden hergestellt:

i) Deletion der Aminosäuren 2 bis 18: Eliminierung der singulären N-terminalen Extension, Met-1 sitzt vor Ala-19.
ii) Deletion der dritten und vierten Repeat: das Arg-161 Codon wurde zu einem Stopcodon mutiert.
iii) Deletion der N-terminalen singulären Extension, sowie der ersten und zweiten Repeat. Met-1 sitzt vor Ala-165.

b) Ein möglicher Angriffspunkt beim proteolytischen Abbau des VAC-alpha stellt vielleicht das Arg-161 dar. Diese Aminosäure wurde daher mutiert:

i) Arg-161 zu Glu-161
ii) Arg-161 zu Gln-161.

c) Durch Einbau einer Faktor Xa-Schnittstelle zwischen der zweiten und dritten Repeat kann vielleicht ein der Inhibition der Koagulationskaskade entkommender Faktor Xa an VAC gebunden werden und/oder dieses sogar in zwei funktionelle Hälften zerlegen, und so die Wirksamkeit von VAC-alpha erhöhen. Es wurde daher folgende Mutation durchgeführt: Asp-168 zu Glu-168, Glu-169 zu Gly-169 und Ala-170 zu Arg-170. Daraus ergibt sich die Faktor Xa Erkennungssequenz Ile-Glu-Gly-Arg.

d) Um die Frage der Wichtigkeit des Cysteins-316 für die biologische Wirksamkeit zu beantworten, und zugleich seine Beteiligung an der Dimerenbildung zu überprüfen, wurde

i) Cys-316 zu Ser-316 und
ii) Cys-316 zu Val-316 mutiert.

e) Um den Einfluß der N-terminalen Extension zu studieren, wurde diese Extension (Ala-2 bis Ala-19) ersetzt durch die jeweiligen N-Termini aus Lipocortin I, Calpactin I und VAC-beta.

Als erster Schritt wurde aus dem Expressionsvektor pRH291 der Expressionsvektor pGN10 hergestellt. Dieser Expressionsvektor besitzt die Phagen Lambda cII RBS an Stelle der in pRH291 verwendeten STII-RBS. pRH291 wurde mit XhoI und KpnI doppelt geschnitten. Das große Fragment wurde isoliert. 40 pMol des Oligonukleotidpaars EBI-725/EBI-727 mit folgender Sequenz:

```
EBI-725 TCGAGTTATCTAAGGAAATACTTACATATGGCACAGGTTCTCAGAGGTAC
EBI-727     CAATAGATTCCTTTATGAATGTATACCGTGTCCAAGAGTCTC
          XhoI          RBS          ATG               KpnI
```

wurden in 6 µl hybridisiert und anschließend mit ca. 200 ng pRH291/großes Fragment in 20 µl Lösung ligiert. Kompetente E.coli HB101 wurden transformiert. Von den entstandenen Ampicillin- resistenten Kolonien wurden die Plasmide einiger Klone isoliert und die Sequenz des neuen Stücks DNA überprüft. Ein Klon wurde ausgewählt und mit pGN10 bezeichnet.

Als Vorbereitung für die Mutagenesereaktionen wurde aus pGN10 das PvuII-SphI Fragment isoliert. PvuII schneidet im phoA Promotor Teil, SphI in der nicht translatierten Region der VAC-alpha cDNA. Das die VAC-alpha cDNA enthaltende Fragment wurde gereinigt und in die SmaI/SphI doppelt geschnittene Doppelstrangform des M13mp18 kloniert. Von den entstandenen Plaques nach Transformation von E.coli JM101 wurde eine ausgewählt, und die M13/VAC-alpha Einzelstrang DNA in größerem Maßstab präpariert.

Die Mutationsreaktionen wurden mit Hilfe des "Oligonucleotide- directed in vitro mutagenesis system" (Amersham, code RPN.2322) unter genauer Einhaltung des beigefügten Protokolls durchgeführt.

Nach der Sequenzüberprüfung der enstandenen Mutanten wurde die entsprechende Doppelstrangform der rekombinanten M13mp8/VAC-alpha Phagen-DNA in Form einer Plasmid Minipräparation isoliert. Mit Ausnahme der Mutation mit EBI-977 (ClaI-Stelle auf Nt.105-110) wurden diese DNAs mit XhoI und SphI doppelt geschnitten, und das die VAC-cDNA enthaltende Fragment isoliert. Ca. 50 ng dieses Fragments wurden mit 50 ng XhoI-SphI doppelt geschnittenem pRH281/5 Vektorteil in 10 µl Lösung ligiert und das entstandene Plasmid in kompetente E.coli HB101 transformiert. Die Plasmide einiger resultierender Kolonien wurden isoliert und durch Restriktionsenzymverdau auf die Richtigkeit der Konstruktion überprüft. Außerdem wurde die letztendlich ausgewählte Kolonie im Labormaßstab fermentiert, und die Proteine des Bakteriums einer Westernblotanalyse unter Verwendung des Rabbit-anti-VAC-Antiserums unterzogen.

Die nachfolgende Tabelle gibt einen Überblick über die hergestellten Mutanten:

| Mutations-Oligo | resultierender Expressionsvektor | Eigenschaften des neuen Klons |
|---|---|---|
| EBI-1051 | pGN42 | Deletion von Ala-2 bis Arg-18 |
| EBI-964 | pGN29 | Translationsstop auf 161 (Deletion von Arg-161 bis Asp-320) |
| EBI-1105 | pGN43 | Deletion von Ala-2 bis Asp-164 |
| EBI-1103 | pGN44 | Arg-161 zu Gln-161 |
| EBI-1104 | pGN45 | Arg-161 zu Glu-161 |
| EBI-960 | pGN30 | Faktor Xa-Schnittstelle auf 167 bis 170 |
| EBI-961 | pGN41 | Cys-316 zu Ser-316 |
| EBI-959 | pGN46 | Cys-316 zu Val-316 |
| EBI-977 | pGN31 | ClaI-Stelle auf Nt.105 bis 110 |

Die Sequenzen der Mutationsoligos sind (5'-->3'):

| | |
|---|---|
| EBI-1051: | CCGAAGAGTTTCTGCATCAGCCATATGTAAGTATTTCCTTAG |
| EBI-964: | AATTCCAGCATCAGGGTCTTAGTTAGCCTGAAGGAGAAC |
| EBI-1105: | AGCTTCATCAATTCCAGCCATATGTAAGTATTTCC |
| EBI-1103: | AATTCCAGCATCAGGGTCCTGGTTAGCCTGAAGGAGAAC |
| EBI-1104: | AATTCCAGCATCAGGGTCTTCGTTAGCCTGAAGGAGAAC |
| EBI-960: | GCCTGAGCATCTTGTTCAACTTGACGACCTTCAATTCCAGCA TCAGGGTCTCTG |
| EBI-961: | CGTTAGTCATCTTCTCCTGAGAGCAGCAGAAGAGC |
| EBI-959: | CGTTAGTCATCTTCTCCCACGAGCAGCAGAAGAGC |
| EBI-977: | CAACAGAGTCAGGATCGATTCCTCATCTGTGCCC |

Die angegebenen Sequenzen sind komplemetär zu der DNA-Sequenz in Figur 52, was durch die Klonierung der VAC-alpha cDNA in den M13mp18 bedingt ist.

Die mutierte DNA mit der ClaI-Stelle auf Nt.105 bis 110 wurde mittels EcoRI-HindIII Doppelverdau aus der Doppelstrangform des rekombinanten M13mp18 freigesetzt, isoliert und in EcoRI-HindIII doppelt geschnittenen Bluescribe M13+ (Stratagene, La Jolla, Kalifornien, USA) kloniert. Das entstandene Plasmid wurde pGN31 genannt. pGN31 wurde mit ClaI und XhoI doppelt geschnitten, und das große Fragment isoliert. Durch Einsatz verschiedener Oligonukleotidpaare wurde der für Lipocortin I (K.-S. Huang et al. Cell 46 (1986), 191-199), Calpactin I (=Lipocortin II, K.-S. Huang et al. Cell 46 (1986), 191-199) und VAC-beta spezifische N- Terminus eingefügt:

a) Lipocortin I-spezifischer N-Terminus

```
:EBI-972->              :Start Lipocortin
TCGAGAGGTTGAGGTGATTTTATGGCAATGGTATCAGAATTCCTCAAGCA
    CTCCAACTCCACTAAAATACCGTTACCATAGTCTTAAGGAGTTCGT
    :

GGCCTGGTTTATTGAAAATGAAGAGCAGGAATATGTTCAAACTGTGAAGT
CCGGACCAAATAACTTTTACTTCTCGTCCTTATACAAGTTTGACACTTCA

      ::EBI-977->
CATCCAAAGGTGGTCCCGGATCAGCGGTGAGCCCCTATCCTACCTTCAAT
GTAGGTTTCCACCAGGGCCTAGTCGCCACTCGGGGATAGGATGGAAGTTA
     <-EBI-988::
         :Fortsetzung VAC-alpha
CCATCCTCGGATGCAGAAACTCTTCGGAAGGCTATGAAAGGCTTGGGCAC
GGTAGGAGCCTACGTCTTTGAGAAGCCTTCCGATACTTTCCGAACCCGTG


AGATGAGGAAT
TCTACTCCTTAGC
   <-EBI-982:
```

Je 1 pMol EBI-978 und EBI-988 wurden gemeinsam in 6 µl phosphoryliert. Die Reaktion wurde durch Erhitzen auf 100°C gestoppt, jeweils 1 pMol EBI-972 und EBI-982 zugesetzt, die Lösung nochmals auf 100°C erhitzt und langsam abgekühlt. Die beiden Oligonukleotidpaare wurden durch Zugabe von Ligase miteinander ligiert. Danach wurde ca. 1 µg pGN31 ClaI-XhoI großes Fragment zugesetzt, das Volumen auf 40 µl erweitert und ligiert. Nach der Transformation von kompetenten E.coli JM101 wurden einige Kolonien ausgesucht und deren Plasmide gemäß dem Bluescribe-Protokoll von Stratagene mit Hilfe eines Helperphagen in Einzelstrang-DNA überführt und sequenziert. Eines der richtigen Plasmide wurde ausgewählt und mit pGN32 bezeichnet. Mit XhoI und HindIII (befindet sich hinter der SphI-Stelle in der Multiklonierstelle des Bluescribe M13+) wurde das Insert aus dem Vektor geschnitten und gereinigt. pRH281/5 wurde ebenfalls mit XhoI und HindIII doppelt verdaut und das Vektorfragment isoliert. 200 ng pGN32 Insert und etwa 50 ng pRH281/5-Vektorteil wurden in 20 µl ligiert. Die DNA wurde in E.coli HB101 transformiert, und die Plasmid-DNA einiger Ampicillin-resistenter Klone durch Restriktionsenzymanalyse auf die Richtigkeit der Konstruktion überprüft. Außerdem wurde die letztendlich ausgewählte Kolonie im Labormaßstab fermentiert, und die Proteine des Bakteriums einer Westernblotanalyse unter Verwendung des Rabbit-anti-VAC- Antiserums unterzogen. Das resultierende Expressionsplasmid für das Lipocortin I/VAC-alpha-Hybrid wurde pGN35 genannt.

b) Calpactin I spezifischer N-Terminus:

```
:EBI-990->                Start Calpactin
TCGAGAGGTTGAGGTGATTTTATGTCTACTGTTCACGAAATCCTGTGCAA
    CTCCAACTCCACTAAAATACAGATGACAAGTGCTTTAGGACACGTT
    :

GCTCAGCTTGGAGGGTGATCACTCTACACCCCCAAGTGCATATGGGTCTG
CGAGTCGAACCTCCCACTAGTGAGATGTGGGGGTTCACGTATACCCAGAC
                                                <-

::EBI-1001->                      :Fortsetzung VAC-alpha
TCAAAGCCTATACTAACTTTGATGCTGAGCGGGATGCAGAAACTCTTCGG
AGTTTCGGATATGATTGAAACTACGACTCGCCCTACGTCTTTGAGAAGCC
EBI-985::

AAGGCTATGAAAGGCTTGGGCACAGATGAGGAAT
TTCCGATACTTTCCGAACCCGTGTCTACTCCTTAGC
                             <-EBI-994:
```

Die Herstellung des rekombinanten Bluescribe M13+ Plasmids pGN33, seine Sequenzüberprüfung und das Umklonieren in den Expressionsvektor pRH281/5 erfolgte analog zu der für das Lipocortin I - Hybrid beschriebenen Prozedur. Das resultierende Expressionsplasmid (Calpactin I/VAC-alpha-Hybrid) wurde pGN36 genannt.

c) VAC-beta spezifischer N-Terminus:

```
:EBI-987->               :VAC-beta Start
TCGAGAGGTTGAGGTGATTTTATGGCCTGGTGGAAATCCTGGATTGAACA
   CTCCAACTCCACTAAAATACCGGACCACCTTTAGGACCTAACTTGT
   :
                                               :VAC-
GGAGGGTGTCACAGTGAAGAGCAGCTCCCACTTCAACCCAGACCCTGATG
CCTCCCACAGTGTCACTTCTCGTCGAGGGTGAAGTTGGGTCTGGGACTAC

alpha Fortsetzung
CAGAAACTCTTCGGAAGGCTATGAAAGGCTTGGGCACAGATGAGGAAT
GTCTTTGAGAAGCCTTCCGATACTTTCCGAACCCGTGTCTACTCCTTAGC
                                       <-EBI-989:
```

Zur Herstellung des rekombinanten BluescribeM13+ Plasmids pGN34 wurde hier nur das Oligonukleotidpaar (je 1 pMol in 6 µl) durch Erhitzen und langsames Abkühlen hybridisiert und anschließend wie oben in den XhoI-ClaI doppelt geschnittenen pGN31 Vektor ligiert. Die Herstellung des Expressionsvektors pGN37 (VAC-beta/VAC-alpha-Hybrid in pRH281/5) erfolgte wie oben beschrieben.

## Patentansprüche

**1.** DNA, kodierend für das Vascular-Antikoagulierende Protein-beta (VAC-beta) mit der Sequenz

ATG GCC TGG TGG AAA GCC TGG ATT GAA CAG GAG GGT GTC ACA GTG
AAG AGC AGC TCC CAC TTC AAC CCA GAC CCT GAT GCA GAG ACC CTC
TAC AAA GCC ATG AAG GGG ATC GGG ACC AAC GAG CAG GCT ATC ATC
GAT GTG CTC ACC AAG AGA AGC AAC ACG CAG CGG CAG CAG ATC GCC
AAG TCC TTC AAG GCT CAG TTC GGC AAG GAC CTC ACT GAG ACC TTG
AAG TCT GAG CTC AGT GGC AAG TTT GAG AGG CTC ATT GTG GCC CTT
ATG TAT CCG CCA TAC AGA TAC GAA GCC AAG GAG CTG CAT GAC GCC
ATG AAG GGC TTA GGA ACC AAG GAG GGT GTC ATC ATT GAG ATC CTG
GCC TCT CGG ACC AAG AAC CAG CTG CGG GAG ATA ATG AAG GCG TAT
GAG GAA GAC TAT GGG TCC AGC CTG GAG GAG GAC ATC CAA GCA GAC
ACA AGT GGC TAC CTG GAG AGG ATC CTG GTG TGC CTC CTG CAG GGC
AGC AGG GAT GAT GTG AGC AGC TTT GTG GAC CCG GCA CTG GCC CTC
CAA GAC GCA CAG GAT CTG TAT GCG GCA GGC GAG AAG ATT CGT GGG
ACT GAT GAG ATG AAA TTC ATC ACC ATC CTG TGC ACG CGC AGT GCC
ACT CAC CTG CTG AGA GTG TTT GAA GAG TAT GAG AAA ATT GCC AAC
AAG AGC ATT GAG GAC AGC ATC AAG AGT GAG ACC CAT GGC TCA CTG
GAG GAG GCC ATG CTC ACT GTG GTG AAA TGC ACC CAA AAC CTC CAC
AGC TAC TTT GCA GAG AGA CTC TAC TAT GCC ATG AAG GGA GCA GGG
ACG CGT GAT GGG ACC CTG ATA AGA AAC ATC GTT TCA AGG AGC GAG
ATT GAC TTA AAT CTT ATC AAA TGT CAC TTC AAG AAG ATG TAC GGC
AAG ACC CTC AGC AGC ATG ATC ATG GAA GAC ACC AGC GGC GAC TAC
AAG AAC GCC CTG CTG AGC CTG GTG GGC AGC GAC CCC TGA,

wobei degenerierte und modifizierte Formen dieser Sequenz eingeschlossen sind, die für ein Protein mit der biologischen Aktivität von VAC-beta kodieren.

**2.** DNA nach Anspruch 1, dadurch gekennzeichnet, daß sie der Formel

ATG GCC TGG TGG AAA GCC TGG ATT GAA CAG GAG GGT GTC ACA GTG
AAG AGC AGC TCC CAC TTC AAC CCA GAC CCT GAT GCA GAG ACC CTC
TAC AAA GCC ATG AAG GGG ATC GGG ACC AAC GAG CAG GCT ATC ATC
GAT GTG CTC ACC AAG AGA AGC AAC ACG CAG CGG CAG CAG ATC GCC
AAG TCC TTC AAG GCT CAG TTC GGC AAG GAC CTC ACT GAG ACC TTG
AAG TCT GAG CTC AGT GGC AAG TTT GAG AGG CTC ATT GTG GCC CTT
ATG TAT CCG CCA TAC AGA TAC GAA GCC AAG GAG CTG CAT GAC GCC
ATG AAG GGC TTA GGA ACC AAG GAG GGT GTC ATC ATT GAG ATC CTG
GCC TCT CGG ACC AAG AAC CAG CTG CGG GAG ATA ATG AAG GCG TAT
GAG GAA GAC TAT GGG TCC AGC CTG GAG GAG GAC ATC CAA GCA GAC
ACA AGT GGC TAC CTG GAG AGG ATC CTG GTG TGC CTC CTG CAG GGC
AGC AGG

entspricht, wobei gegebenenfalls nach dem letzten AGG ein Stopcodon steht sowie degenerierte Formen dieser DNA.

3. DNA nach Anspruch 1, dadurch gekennzeichnet, daß sie der Formel

GAT GAT GTG AGC AGC TTT GTG GAC CCG GCA CTG GCC CTC
CAA GAC GCA CAG GAT CTG TAT GCG GCA GGC GAG AAG ATT CGT GGG
ACT GAT GAG ATG AAA TTC ATC ACC ATC CTG TGC ACG CGC AGT GCC
ACT CAC CTG CTG AGA GTG TTT GAA GAG TAT GAG AAA ATT GCC AAC
AAG AGC ATT GAG GAC AGC ATC AAG AGT GAG ACC CAT GGC TCA CTG
GAG GAG GCC ATG CTC ACT GTG GTG AAA TGC ACC CAA AAC CTC CAC
AGC TAC TTT GCA GAG AGA CTC TAC TAT GCC ATG AAG GGA GCA GGG
ACG CGT GAT GGG ACC CTG ATA AGA AAC ATC GTT TCA AGG AGC GAG
ATT GAC TTA AAT CTT ATC AAA TGT CAC TTC AAG AAG ATG TAC GGC
AAG ACC CTC AGC AGC ATG ATC ATG GAA GAC ACC AGC GGC GAC TAC
AAG AAC GCC CTG CTG AGC CTG GTG GGC AGC GAC CCC TGA

entspricht, wobei gegebenfalls vor dem ersten GAT ein Initiationscodon steht und degenerierte Formen dieser DNA.

4. DNA nach Anspruch 1, dadurch gekennzeichnet, daß in den Abschnitt, der für die Linker-Sequenz zwischen der zweiten und dritten Repeat-Struktur von Position +165 bis +181 gemäß Fig. 7 kodiert, ein Oligonukleotid eingefügt wird, das für eine Erkennungssequenz einer spezifischen Protease kodiert.

5. DNA nach Anspruch 1, dadurch gekennzeichnet, daß das für Arginin an Position +167 gemäß Fig. 7 kodierende Triplett ersetzt wird durch ein Codon, das für eine Aminosäure kodiert, die nicht von Trypsin als bevorzugte Spaltstelle erkannt wird, beispielsweise für Histidin.

6. DNA nach einem der Ansprüche 1 bis 4, dadurch gekennzeichnet, daß die für Lysin und/oder Arginin kodierenden Tripletts gezielt ersetzt werden durch Tripletts, die für Histidin kodieren.

7. DNA nach einem der Ansprüche 1 bis 6, dadurch gekennzeichnet, daß das oder gegebenenfalls die für Cystein kodierenden Tripletts ersetzt wird/werden durch für Serin oder Valin kodierende Tripletts.

8. DNA nach Anspruch 1, dadurch gekennzeichnet, daß sie für ein Polypeptid/Protein umfassend einen Polypeptidbereich der Position +26 bis +92 oder Position +98 bis +164 oder Position +182 bis +249 oder Position +258 bis +324 gemäß Fig. 7 kodiert.

**9.** DNA nach einem der Ansprüche 1 bis 8, dadurch gekennzeichnet, daß die für die vollständigen Repeats an Position +26 bis +92, Position +98 bis +164, Position +182 bis +249 und Position +258 bis +324 gemäß Fig. 7 kodierenden Bereiche umgeordnet sind.

**10.** DNA nach einem der Ansprüche 1-9, dadurch gekennzeichnet, daß die für die 17 Aminosäuren der konservierten Region kodierenden Bereiche an Position +36 bis +51 und/oder an Position +108 bis +123 und/oder an Position +192 bis +208 und/oder an Position +268 bis +283 gemäß Fig. 7 modifiziert sind.

**11.** DNA nach einem der Ansprüche 1 bis 10, dadurch gekennzeichnet, daß die für die 17 Aminosäuren der konservierten Region kodierenden Bereiche an den Positionen +36 bis +51 und/oder an Position +108 bis +123 und/oder an Position +192 bis +208 und/oder an Position +268 bis +283 gemäß Fig. 7 kodierenden vollständig oder teilweise ersetzt werden durch Bereiche, die die entsprechenden Aminosäuren bei den Lipocortinen kodieren oder durch Bereiche die die entsprechenden Aminosäuren des VAC-alpha kodieren und umgekehrt.

**12.** DNA nach einem der Ansprüche 1 bis 11, dadurch gekennzeichnet, daß der Bereich, der für das N-terminale Peptid kodiert, Position 1 bis 25 gemäß Fig. 7, ganz oder teilweise ausgetauscht wird durch Oligonukleotide, die für das N-terminale Peptid eines der anderen, eine Repeat-Struktur aufweisenden Proteine kodiert.

**13.** DNA nach einem der vorhergehenden Ansprüche, dadurch gekennzeichnet, daß dem N-terminalen 5'-Ende eine für den jeweiligen Wirt homologe Signalsequenz vorangestellt ist.

**14.** DNA nach dem vorhergehenden Anspruch, dadurch gekennzeichnet, daß die Signalsequenz am 3'-Ende eine spezifische Erkennungs-Spaltstelle für eine Protease kodiert.

**15.** DNA nach einem der Ansprüche 1 bis 7, dadurch gekennzeichnet, daß dem N-terminalen 5'-Ende eine für einen Fusionsproteinanteil kodierende Sequenz vorangestellt ist.

**16.** DNA nach dem vorhergehenden Anspruch, dadurch gekennzeichnet, daß die für den Fusionsproteinanteil kodierende DNA am 3'-Ende eine spezifische Erkennungs-Spaltstelle für eine Protease kodiert.

**17.** DNA, dadurch gekennzeichnet, daß sie ein Allel zu einer der in der vorhergehenden Ansprüchen beschriebenen DNA darstellt.

**18.** DNA, dadurch gekennzeichnet, daß sie eine degenerative Form einer der in den vorhergehenden Ansprüchen beschriebenen DNA darstellt oder daß sie aus Kombinationen verschiedener vollständiger - oder Teil-Sequenzen einer der vorhergehenden Ansprüche besteht, kodierend für Hybridproteine den wesentlichen mit vascular-antikoagulierenden Eigenschaften des VAC-beta.

**19.** DNA, dadurch gekennzeichnet, daß sie mit einem der DNA-Moleküle nach einem der Ansprüche 1 bis 18 unter Bedingungen hybridisieren, die eine Homologie größer als 85%, vorzugsweise größer als 90% erkennen lassen, wobei die DNA aus natürlichen, synthetischen oder halbsynthetischen Ursprungs sein kann und mit den DNA-Molekülen nach einem der Ansprüche 1 bis 18 durch Mutation, Nukleotid-Substitutionen, Nukleotid- Deletionen, Nukleotid-Insertionen und Nukleotid- Inversionen verwandt sein kann und für ein Polypeptid/ Protein mit im wesentlichen den biologischen Eigenschaften der vascular-antigoagulierenden Proteine kodiert.

**20.** DNA nach einem der vorhergehenden Ansprüche, dadurch gekennzeichnet, daß sie in einen Vektor eingefügt ist.

**21.** DNA nach Anspruch 20, dadurch gekennzeichnet, daß die DNA nach einem der vorhergehenden Ansprüche in einem Vektor in funktioneller Verbindung mit einer Expressionskontrollsequenz verknüpft ist, in Mikroorganismen und Säugetierzellen replizierbar.

**22.** DNA nach einem der Ansprüche 20 oder 22, dadurch gekennzeichnet, daß der Vektor ein Plasmid ist, replizierbar in Prokaryoten.

**23.** DNA nach einem der Ansprüche 20 oder 21, dadurch gekennzeichnet, daß der Vektor ein Plasmid ist, replizierbar in Eukaryoten.

**24.** DNA nach einem der Ansprüche 20 oder 21, dadurch gekennzeichnet, daß sie in Säugetierzellen replizierbar ist.

**25.** Wirtsorganismus, dadurch gekennzeichnet, daß er mit einer DNA nach einem der Ansprüche 20 bis 24 transformiert ist.

**26.** Wirtsorganismus nach Anspruch 25, dadurch gekennzeichnet, daß er ein Prokaryot, vorzugsweise E.coli ist.

**27.** Wirtsorganismus nach Anspruch 25, dadurch gekennzeichnet, daß er ein Eukaryot ist.

**28.** Wirtsorganismus nach Anspruch 25, dadurch gekennzeichnet, daß er eine Säugetierzellinie ist.

**29.** Verfahren zur Herstellung von DNA-Molekülen, die für VAC-beta kodieren, dadurch gekennzeichnet, daß man aus einer genomischen DNA-Bibliothek das für dieses Polypeptid kodierende Gen isoliert oder aus der zu diesem Gen gehörenden mRNA eine komplementäre für dieses Polypeptid kodierende DNA mit Hilfe des Enzyms Reverse Transkriptase herstellt oder daß die für dieses Polypeptid kodierende DNA mit Hilfe chemisch und/oder enzymatischer Verfahren hergestellt wird.

**30.** Verfahren nach dem vorhergehenden Anspruch, dadurch gekennzeichnet, daß die DNA einer der in den Ansprüchen 1 bis 18 beschriebenen entspricht.

**31.** Verfahren zur Herstellung einer DNA nach einem der Ansprüche 20 bis 24, dadurch gekennzeichnet, daß man in eine mit Hilfe von Restriktionsendonukleasen geschnittene Vektor-DNA eine mit entsprechenden Enden versehene DNA einfügt, die für ein Polypetid im wesentlichen mit den Eigenschaften der vaskular- antikoagulierenden Proteine kodiert.

**32.** Verfahren zur Herstellung einer DNA nach einem der Ansprüche 21 bis 24, dadurch gekennzeichnet, daß man in eine mit Restriktionsendonukleasen geschnittene Vektor-DNA, die Expressionskontrollsequenzen enthält, eine mit entsprechenden Enden versehene DNA, die für ein Polypeptid mit im wesentlichen den Eigenschaften von vaskular-antikoagulierenden Proteinen kodiert, so einfügt, daß die Expressionskontrollsequenzen die eingefügte DNA reguliert.

**33.** Verfahren zur Herstellung transformierter Wirtsorganismen nach einem der Ansprüche 25 bis 28, dadurch gekennzeichnet, daß man den Wirtsorganismus mit einem der Vektoren nach einem der Ansprüche 22 bis 24 transformiert.

**34.** Polypeptid, das einem nicht-nativen Derivat von VAC-beta entspricht mit im wesentlichen Eigenschaften der vascular-antikoagulierenden Proteine, abgeleitet aus der VAC-beta Sequenz

```
Met Ala Trp Trp Lys Ala Trp Ile Glu Gln Glu Gly Val Thr Val
Lys Ser Ser Ser His Phe Asn Pro Asp Pro Asp Ala Glu Thr Leu
Tyr Lys Ala Met Lys Gly Ile Gly Thr Asn Glu Gln Ala Ile Ile
Asp Val Leu Thr Lys Arg Ser Asn Thr Gln Arg Gln Gln Ile Ala
Lys Ser Phe Lys Ala Gln Phe Gly Lys Asp Leu Thr Glu Thr Leu
Lys Ser Glu Leu Ser Gly Lys Phe Glu Arg Leu Ile Val Ala Leu
Met Tyr Pro Pro Tyr Arg Tyr Glu Ala Lys Glu Leu His Asp Ala
Met Lys Gly Leu Gly Thr Lys Glu Gly Val Ile Ile Glu Ile Leu
Ala Ser Arg Thr Lys Asn Gln Leu Arg Glu Ile Met Lys Ala Tyr
Glu Glu Asp Tyr Gly Ser Ser Leu Glu Glu Asp Ile Gln Ala Asp
```

Thr Ser Gly Tyr Leu Glu Arg Ile Leu Val Cys Leu Leu Gln Gly
Ser Arg Asp Asp Val Ser Ser Phe Val Asp Pro Ala Leu Ala Leu
Gln Asp Ala Gln Asp Leu Tyr Ala Ala Gly Glu Lys Ile Arg Gly
Thr Asp Glu Met Lys Phe Ile Thr Ile Leu Cys Thr Arg Ser Ala
Thr His Leu Leu Arg Val Phe Glu Glu Tyr Glu Lys Ile Ala Asn
Lys Ser Ile Glu Asp Ser Ile Lys Ser Glu Thr His Gly Ser Leu
Glu Glu Ala Met Leu Thr Val Val Lys Cys Thr Gln Asn Leu His
Ser Tyr Phe Ala Glu Arg Leu Tyr Tyr Ala Met Lys Gly Ala Gly
Thr Arg Asp Gly Thr Leu Ile Arg Asn Ile Val Ser Arg Ser Glu
Ile Asp Leu Asn Leu Ile Lys Cys His Phe Lys Lys Met Tyr Gly
Lys Thr Leu Ser Ser Met Ile Met Glu Asp Thr Ser Gly Asp Tyr
Lys Asn Ala Leu Leu Ser Leu Val Gly Ser Asp Pro,

wobei gegebenenfalls an Position 1 das Methionin abgespalten ist und das Alanin an Position 2 gegebenenfalls blockiert ist und/oder wobei gegebenenfalls intramolekulare Disulfidbrücken zwischen den Cysteinen an den Positionen 161 und/oder 206 und/oder 250 und/oder 293 und/oder Aggregationen durch intermolekulare Disulfidbrücken zwischen den genannten Positionen vorliegen.

**35.** Polypeptid nach Anspruch 34, dadurch gekennzeichnet, daß es von einer DNA gemäß einem der Ansprüche 1 bis 18 kodiert ist.

**36.** Polypeptid nach Anspruch 34, dadurch gekennzeichnet, daß es der Formel

Met Ala Trp Trp Lys Ala Trp Ile Glu Gln Glu Gly Val Thr Val
Lys Ser Ser Ser His Phe Asn Pro Asp Pro Asp Ala Glu Thr Leu
Tyr Lys Ala Met Lys Gly Ile Gly Thr Asn Glu Gln Ala Ile Ile
Asp Val Leu Thr Lys Arg Ser Asn Thr Gln Arg Gln Gln Ile Ala
Lys Ser Phe Lys Ala Gln Phe Gly Lys Asp Leu Thr Glu Thr Leu
Lys Ser Glu Leu Ser Gly Lys Phe Glu Arg Leu Ile Val Ala Leu
Met Tyr Pro Pro Tyr Arg Tyr Glu Ala Lys Glu Leu His Asp Ala
Met Lys Gly Leu Gly Thr Lys Glu Gly Val Ile Ile Glu Ile Leu
Ala Ser Arg Thr Lys Asn Gln Leu Arg Glu Ile Met Lys Ala Tyr

Glu Glu Asp Tyr Gly Ser Ser Leu Glu Glu Asp Ile Gln Ala Asp
Thr Ser Gly Tyr Leu Glu Arg Ile Leu Val Cys Leu Leu Gln Gly
Ser Arg

entspricht, wobei gegebenenfalls an Position 1 das Methionin abgespalten ist und das Alanin an Position 2 gegebenenfalls blockiert ist und/oder wobei gegebenenfalls Aggregationen durch beispielsweise intermolekulare Disulfidbrücken zwischen den Cysteinen an Position 161 vorliegen.

**37.** Polypeptid nach Anspruch 34, dadurch gekennzeichnet, daß es der Formel

X Asp Asp Val Ser Ser Phe Val Asp Pro Ala Leu Ala Leu
Gln Asp Ala Gln Asp Leu Tyr Ala Ala Gly Glu Lys Ile Arg Gly
Thr Asp Glu Met Lys Phe Ile Thr Ile Leu Cys Thr Arg Ser Ala
Thr His Leu Leu Arg Val Phe Glu Glu Tyr Glu Lys Ile Ala Asn
Lys Ser Ile Glu Asp Ser Ile Lys Ser Glu Thr His Gly Ser Leu
Glu Glu Ala Met Leu Thr Val Val Lys Cys Thr Gln Asn Leu His
Ser Tyr Phe Ala Glu Arg Leu Tyr Tyr Ala Met Lys Gly Ala Gly
Thr Arg Asp Gly Thr Leu Ile Arg Asn Ile Val Ser Arg Ser Glu
Ile Asp Leu Asn Leu Ile Lys Cys His Phe Lys Lys Met Tyr Gly
Lys Thr Leu Ser Ser Met Ile Met Glu Asp Thr Ser Gly Asp Tyr
Lys Asn Ala Leu Leu Ser Leu Val Gly Ser Asp Pro

entspricht, wobei X für Methionin oder Wasserstoff steht und/oder wobei gegebenenfalls intramolekulare Disulfidbrücken zwischen den Cysteinen an den Positionen 40 und/oder 84 und/oder 127 und/oder Aggregationen durch intermolekulare Disulfidbrücken zwischen den genannten Positionen vorliegen.

**38.** Polypeptid nach einem der vorhergehenden Ansprüche, völlig frei von homologen Polypeptiden.

**39.** Polypeptid nach einem der vorhergehenden Ansprüche, dadurch gekennzeichnet, daß es ein Leader-Peptid enthält.

**40.** Polypeptid nach einem der vorhergehenden Ansprüche, dadurch gekennzeichnet,

a. daß es einen Fusionsproteinanteil enthält und/oder
b. daß es aus Teilbereichen der Polypeptide nach einem der vorhergehenden Ansprüche besteht (Hybridproteine) und/oder
c. daß es als Dimeres, Trimeres, Tetrameres oder Multimeres vorliegt.

**41.** Verfahren zur Herstellung von Polypeptiden, dadurch gekennzeichnet, daß

a. ein geeigneter Wirtsorganismus mit genetischen Informationen, gemäß einem der Ansprüche 1 bis 24 kodierend für ein VAC-beta-Polypeptid transformiert,
b. die Information zur Produktion des VAC-Proteins im Wirtsorganismus exprimiert und
c. das VAC-Polypeptid isoliert wird.

**42.** Verfahren nach Anspruch 41, dadurch gekennzeichnet, daß der Wirtsorganismus gemäß den Ansprüchen 25 bis 28 definiert ist.

**43.** Verfahren nach Anspruch 41 oder 42, dadurch gekennzeichnet, daß die genetischen Informationen in den DNA-Molekülen gemäß einem der Ansprüche 1 bis 24 enthalten sind.

**44.** Verfahren nach einem der Ansprüche 41 bis 43, dadurch gekennzeichnet, daß das VAC-Protein gemäß einem der Ansprüche 34 bis 40 definiert ist.

**45.** Nicht-natives VAC-beta Polypeptid herstellbar nach einem der Ansprüche 41 bis 44.

**46.** Pharmazeutisch tolerierbare Salze der Polypeptide nach einem der Ansprüche 34 bis 40 oder 45.

**47.** Verwendung der Polypeptide nach einem der Ansprüche 34 bis 40 oder 45 zur Herstellung von Arzneimitteln zur therapeutischen oder prophylaktischen Behandlung des menschlichen oder tierischen Körpers.

**48.** Verwendung der Polypeptide nach einem der Ansprüche 34 bis 40 oder 45 zur Herstellung pharmazeutischer Präparate.

**49.** Verwendung der Polypeptide nach einem der Ansprüche 34 bis 40 oder 45 als blutgerinnungshemmende Mittel.

**50.** Verwendung der Polypeptide nach einem der Ansprüche 34 bis 40 oder 45 als antiinflammatorische Mittel.

**51.** Verwendung der Polypeptide nach einem der Ansprüche 34 bis 40 oder 45 als antirheumatisches Mittel.

**52.** Verwendung der Polypeptide nach einem der Ansprüche 34 bis 40 oder 45 in den für die Lipocortine geltenden Indikationen.

**53.** Verwendung der Polypeptide nach einem der Ansprüche 34 bis 40 oder 45 als Thrombin-inhibierende Mittel.

**54.** Mittel zur therapeutischen Behandlung, dadurch gekennzeichnet, daß es neben pharmazeutisch inerten Trägerstoffen eine wirksame Menge eines Polypeptides nach einem der Ansprüche 34 bis 40 oder 45 enthält.

**55.** Hybrid-Zellinie, dadurch gekennzeichnet daß sie monoklonale Antikörper gegen eines der Polypeptide nach einem der Ansprüche 34 bis 40 oder 45 sezerniert, die nicht an natives VAC-alpha und nativen VAC-beta binden und dieses nicht neutralisieren.

**56.** Monoklonale Antikörper dadurch gekennzeichnet, daß sie spezifisch die Wirkung der Polypeptide nach einem der Ansprüche 34 bis 40 oder 45 ganz oder teilweise neutralisieren oder spezifisch an eines der besagten Polypeptide binden, nicht aber an natives VAC-alpha und VAC-beta binden und diese nicht neutralisieren.

**57.** Verwendung der monoklonalen Antikörper nach Anspruch 56 zur qualitativen und/oder quantitativen Bestimmung eines der Polypeptide nach einem der Ansprüche 34 bis 40 oder 45.

**58.** Verwendung der monoklonalen Antikörper nach Anspruch 56 zur Reinigung eines der Polypeptide nach einem der Ansprüche 34 bis 40 oder 45.

**59.** Test Kit zur Bestimmung von Polypeptiden nach einem der Ansprüche 34 bis 40 oder 45 dadurch gekennzeichnet, daß er monoklonale Antikörper nach Anspruch 56 enthält.

**60.** Verfahren zur Herstellung von monoklonalen Antikörpern nach Anspruch 56 dadurch gekennzeichnet, daß Wirtstiere mit einem der Polypeptide nach einem der Ansprüche 34 bis 40 oder 45 immunisiert, B-Lymphozyten dieser Wirtstiere mit Myelomzellen fusioniert, die monoklonalen Antikörper ausscheidenden Hybrid-Zellinien subkloniert und in vitro oder in vivo kultiviert werden.

**61.** Pharmazeutisch tolerierbare Addukte und kovalente Verbindungen zwischen den Polypeptiden nach einem der Ansprüche 34 bis 40 oder 45 mit einem inerten Träger, zur therapeutischen oder prophylaktischen Behandlung des menschlichen oder tierischen Körpers.

**62.** Pharmazeutisch tolerierbare Addukte oder kovalente Verbindungen zwischen den Polypeptiden nach einem der Ansprüche 34 bis 40 oder 45 mit zum Beispiel Polyethylenglykol, zur therapeutischen oder prophylaktischen Behandlung des menschlichen oder tierischen Körpers.

**63.** Verwendung der Polypeptide nach einem der Ansprüche 34 bis 40 oder 45 als blutkonservierendes Mittel.

## Claims

**1.** DNA coding for the vascular anticoagulant protein-beta (VAC-beta) having the sequence

ATG GCC TGG TGG AAA GCC TGG ATT GAA CAG GAG GGT GTC ACA GTG
AAG AGC AGC TCC CAC TTC AAC CCA GAC CCT GAT GCA GAG ACC CTC
TAC AAA GCC ATG AAG GGG ATC GGG ACC AAC GAG CAG GCT ATC ATC
GAT GTG CTC ACC AAG AGA AGC AAC ACG CAG CGG CAG CAG ATC GCC
AAG TCC TTC AAG GCT CAG TTC GGC AAG GAC CTC ACT GAG ACC TTG
AAG TCT GAG CTC AGT GGC AAG TTT GAG AGG CTC ATT GTG GCC CTT
ATG TAT CCG CCA TAC AGA TAC GAA GCC AAG GAG CTG CAT GAC GCC
ATG AAG GGC TTA GGA ACC AAG GAG GGT GTC ATC ATT GAG ATC CTG
GCC TCT CGG ACC AAG AAC CAG CTG CGG GAG ATA ATG AAG GCG TAT
GAG GAA GAC TAT GGG TCC AGC CTG GAG GAG GAC ATC CAA GCA GAC
ACA AGT GGC TAC CTG GAG AGG ATC CTG GTG TGC CTC CTG CAG GGC
AGC AGG GAT GAT GTG AGC AGC TTT GTG GAC CCG GCA CTG GCC CTC
CAA GAC GCA CAG GAT CTG TAT GCG GCA GGC GAG AAG ATT CGT GGG
ACT GAT GAG ATG AAA TTC ATC ACC ATC CTG TGC ACG CGC AGT GCC
ACT CAC CTG CTG AGA GTG TTT GAA GAG TAT GAG AAA ATT GCC AAC
AAG AGC ATT GAG GAC AGC ATC AAG AGT GAG ACC CAT GGC TCA CTG
GAG GAG GCC ATG CTC ACT GTG GTG AAA TGC ACC CAA AAC CTC CAC
AGC TAC TTT GCA GAG AGA CTC TAC TAT GCC ATG AAG GGA GCA GGG
ACG CGT GAT GGG ACC CTG ATA AGA AAC ATC GTT TCA AGG AGC GAG
ATT GAC TTA AAT CTT ATC AAA TGT CAC TTC AAG AAG ATG TAC GGC
AAG ACC CTC AGC AGC ATG ATC ATG GAA GAC ACC AGC GGC GAC TAC
AAG AAC GCC CTG CTG AGC CTG GTG GGC AGC GAC CCC TGA,

including degenerate and modified forms of this sequence, which code for a protein with the biological activity of VAC-beta.

**2.** DNA according to claim 1, characterised in that it corresponds to the formula

ATG GCC TGG TGG AAA GCC TGG ATT GAA CAG GAG GGT GTC ACA GTG
AAG AGC AGC TCC CAC TTC AAC CCA GAC CCT GAT GCA GAG ACC CTC
TAC AAA GCC ATG AAG GGG ATC GGG ACC AAC GAG CAG GCT ATC ATC
GAT GTG CTC ACC AAG AGA AGC AAC ACG CAG CGG CAG CAG ATC GCC
AAG TCC TTC AAG GCT CAG TTC GGC AAG GAC CTC ACT GAG ACC TTG
AAG TCT GAG CTC AGT GGC AAG TTT GAG AGG CTC ATT GTG GCC CTT
ATG TAT CCG CCA TAC AGA TAC GAA GCC AAG GAG CTG CAT GAC GCC
ATG AAG GGC TTA GGA ACC AAG GAG GGT GTC ATC ATT GAG ATC CTG
GCC TCT CGG ACC AAG AAC CAG CTG CGG GAG ATA ATG AAG GCG TAT
GAG GAA GAC TAT GGG TCC AGC CTG GAG GAG GAC ATC CAA GCA GAC
ACA AGT GGC TAC CTG GAG AGG ATC CTG GTG TGC CTC CTG CAG GGC
AGC AGG

optionally having a stop codon after the last AGG, and degenerate forms of this DNA.

**3.** DNA according to claim 1, characterised in that it corresponds to the formula

GAT GAT GTG AGC AGC TTT GTG GAC CCG GCA CTG GCC CTC
CAA GAC GCA CAG GAT CTG TAT GCG GCA GGC GAG AAG ATT CGT GGG
ACT GAT GAG ATG AAA TTC ATC ACC ATC CTG TGC ACG CGC AGT GCC
ACT CAC CTG CTG AGA GTG TTT GAA GAG TAT GAG AAA ATT GCC AAC
AAG AGC ATT GAG GAC AGC ATC AAG AGT GAG ACC CAT GGC TCA CTG
GAG GAG GCC ATG CTC ACT GTG GTG AAA TGC ACC CAA AAC CTC CAC
AGC TAC TTT GCA GAG AGA CTC TAC TAT GCC ATG AAG GGA GCA GGG
ACG CGT GAT GGG ACC CTG ATA AGA AAC ATC GTT TCA AGG AGC GAG
ATT GAC TTA AAT CTT ATC AAA TGT CAC TTC AAG AAG ATG TAC GGC
AAG ACC CTC AGC AGC ATG ATC ATG GAA GAC ACC AGC GGC GAC TAC
AAG AAC GCC CTG CTG AGC CTG GTG GGC AGC GAC CCC TGA

optionally having an initiation codon before the first GAT, and degenerate forms of this DNA.

**4.** DNA according to claim 1, characterised in that an oligonucleotide which codes for a recognition sequence of a specific protease is inserted in the section which codes for the linker sequence between the second and third repeat structures from positions +165 to +181 according to Fig. 7.

**5.** DNA according to claim 1, characterised in that the triplet coding for arginine at position +167 according to Fig. 7 is replaced by a codon which codes for an amino acid which is not recognised by trypsin as a preferred cleavage site, for example histidine.

**6.** DNA according to one of claims 1 to 4,
characterised in that the triplets coding for lysine and/or arginine are replaced by triplets which code for histidine.

**7.** DNA according to one of claims 1 to 6,
characterised in that the triplet or possibly triplets coding for cysteine is or are replaced by triplets coding for serine or valine.

**8.** DNA according to claim 1, characterised in that it codes for a polypeptide/protein comprising a polypeptide region from position +26 to +92 or position +98 to +164 or position +182 to +249 or position +258 to +324 according to Fig. 7.

**9.** DNA according to one of claims 1 to 8,
characterised in that the areas coding for the complete repeats at position +26 to +92, position +98 to +164, position +182 to +249 and position +258 to +324 according to Fig. 7 are rearranged.

**10.** DNA according to one of claims 1 to 9,
characterised in that the areas coding for the 17 amino acids of the conserved region at position +36 to +51 and/or at position +108 to +123 and/or at position +192 to +208 and/or at position +268 to +283 according to Fig. 7 are modified.

**11.** DNA according to one of claims 1 to 10,
characterised in that the areas coding for the 17 amino acids of the conserved region at positions +36 to +51 and/or at position +108 to +123 and/or at position +192 to +208 and/or at position +268 to +283 according to Fig. 7 are totally or partly replaced by areas which code for the corresponding amino acids in the lipocortins or by areas which code for the corresponding amino acids of VAC-alpha and vice versa.

**12.** DNA according to one of claims 1 to 11,
characterised in that the area which codes for the N-terminal peptide, position 1 to 25 in Fig. 7, is wholly or partly replaced by oligonucleotides which code for the N-terminal peptide of one of the other proteins having a repeat structure.

**13.** DNA according to one of the preceding claims, characterised in that a signal sequence which is homologous for the host in question precedes the N-terminal 5'-end.

**14.** DNA according to the preceding claim, characterised in that the signal sequence at the 3'-end codes for a specific recognition cleavage site for a protease.

**15.** DNA according to one of claims 1 to 7,
characterised in that a sequence coding for a fusion protein component precedes the N-terminal 5'-end.

**16.** DNA according to the preceding claim, characterised in that the DNA coding for the fusion protein component at the 3'-end codes for a specific recognition cleavage site for a protease.

**17.** DNA, characterised in that it is an allele to a DNA described in the preceding claims.

**18.** DNA, characterised in that it is a degenerate form of a DNA described in the preceding claims or consists of combinations of different complete or partial sequences of one of the preceding claims, coding for hybrid proteins essentially having the vascular anticoagulant properties of VAC-beta.

**19.** DNA, characterised in that it hybridises with one of the DNA molecules according to one of claims 1 to 18 which shows a homology of more than 85%, preferably more than 90%, the DNA being of natural, synthetic or semi-synthetic origin and optionally being related to the DNA molecules according to one of claims 1 to 18 by mutation, nucleotide substitution, nucleotide deletion, nucleotide insertion and nucleotide inversion and coding for a polypeptide/protein having essentially the biological properties of the vascular anticoagulant proteins.

**20.** DNA according to one of the preceding claims, characterised in that it is inserted into a vector.

**21.** DNA according to claim 20, characterised in that the DNA according to one of the preceding claims is linked into a vector in functional connection with an expression control sequence and is replicable in microorganisms and mammalian cells.

**22.** DNA according to one of claims 20 or 22,
characterised in that the vector is a plasmid which is replicable in prokaryotes.

**23.** DNA according to one of claims 20 or 21,
characterised in that the vector is a plasmid which is replicable in eukaryotes.

**24.** DNA according to one of claims 20 or 21, characterised in that it is replicable in mammalian cells.

**25.** Host organism, characterised in that it is transformed with a DNA according to one of claims 20 to 24.

**26.** Host organism according to claim 25, characterised in that it is a prokaryote, preferably *E. coli.*

**27.** Host organism according to claim 25, characterised in that it is a eukaryote.

**28.** Host organism according to claim 25, characterised in that it is a mammalian cell line.

**29.** Process for preparing DNA molecules which code for VAC-beta, characterised in that the gene coding for this polypeptide is isolated from a genomic DNA library or a complementary DNA coding for this polypeptide is prepared from the mRNA corresponding to this gene by means of the enzyme Reverse Transcriptase or the DNA coding for this polypeptide is prepared using chemical and/or enzymatic processes.

**30.** Process according to the preceding claim, characterised in that the DNA corresponds to one of those described in claims 1 to 18.

**31.** Process for preparing a DNA according to one of claims 20 to 24, characterised in that a DNA provided with suitable ends, coding for a polypeptide substantially having the properties of the vascular anticoagulant proteins, is inserted in a vector DNA cut with restriction endonucleases.

**32.** Process for preparing a DNA according to one of claims 21 to 24, characterised in that a DNA provided with suitable ends and coding for a polypeptide substantially having the properties of vascular anticoagulant proteins is inserted in a vector DNA cut with restriction endonucleases which contains expression control sequences in such a way that the expression control sequences regulate the DNA inserted.

**33.** Process for preparing transformed host organisms according to one of claims 25 to 28, characterised in that the host organism is transformed with one of the vectors according to one of claims 22 to 24.

**34.** Polypeptide corresponding to a non-native derivative of VAC-beta, having essentially the properties of the vascular anticoagulant proteins, derived from the VAC-beta sequence

Met Ala Trp Trp Lys Ala Trp Ile Glu Gln Glu Gly Val Thr Val
Lys Ser Ser Ser His Phe Asn Pro Asp Pro Asp Ala Glu Thr Leu
Tyr Lys Ala Met Lys Gly Ile Gly Thr Asn Glu Gln Ala Ile Ile
Asp Val Leu Thr Lys Arg Ser Asn Thr Gln Arg Gln Gln Ile Ala
Lys Ser Phe Lys Ala Gln Phe Gly Lys Asp Leu Thr Glu Thr Leu
Lys Ser Glu Leu Ser Gly Lys Phe Glu Arg Leu Ile Val Ala Leu
Met Tyr Pro Pro Tyr Arg Tyr Glu Ala Lys Glu Leu His Asp Ala
Met Lys Gly Leu Gly Thr Lys Glu Gly Val Ile Ile Glu Ile Leu
Ala Ser Arg Thr Lys Asn Gln Leu Arg Glu Ile Met Lys Ala Tyr
Glu Glu Asp Tyr Gly Ser Ser Leu Glu Glu Asp Ile Gln Ala Asp
Thr Ser Gly Tyr Leu Glu Arg Ile Leu Val Cys Leu Leu Gln Gly
Ser Arg Asp Asp Val Ser Ser Phe Val Asp Pro Ala Leu Ala Leu
Gln Asp Ala Gln Asp Leu Tyr Ala Ala Gly Glu Lys Ile Arg Gly
Thr Asp Glu Met Lys Phe Ile Thr Ile Leu Cys Thr Arg Ser Ala

Thr His Leu Leu Arg Val Phe Glu Glu Tyr Glu Lys Ile Ala Asn
Lys Ser Ile Glu Asp Ser Ile Lys Ser Glu Thr His Gly Ser Leu
Glu Glu Ala Met Leu Thr Val Val Lys Cys Thr Gln Asn Leu His
Ser Tyr Phe Ala Glu Arg Leu Tyr Tyr Ala Met Lys Gly Ala Gly
Thr Arg Asp Gly Thr Leu Ile Arg Asn Ile Val Ser Arg Ser Glu
Ile Asp Leu Asn Leu Ile Lys Cys His Phe Lys Lys Met Tyr Gly
Lys Thr Leu Ser Ser Met Ile Met Glu Asp Thr Ser Gly Asp Tyr
Lys Asn Ala Leu Leu Ser Leu Val Gly Ser Asp Pro,

whilst optionally at position 1 the methionine is cleaved and the alanine at position 2 is optionally blocked and/or there are optionally intramolecular disulphide bridges between the cysteines at positions 161 and/or 206 and/or 250 and/or 293 and/or aggregations caused by intermolecular disulphide bridges between the said positions.

**35.** Polypeptide according to claim 34, characterised in that it is coded by a DNA according to one of claims 1 to 18.

**36.** Polypeptide according to claim 34, characterised in that it corresponds to the formula

Met Ala Trp Trp Lys Ala Trp Ile Glu Gln Glu Gly Val Thr Val
Lys Ser Ser Ser His Phe Asn Pro Asp Pro Asp Ala Glu Thr Leu
Tyr Lys Ala Met Lys Gly Ile Gly Thr Asn Glu Gln Ala Ile Ile
Asp Val Leu Thr Lys Arg Ser Asn Thr Gln Arg Gln Gln Ile Ala
Lys Ser Phe Lys Ala Gln Phe Gly Lys Asp Leu Thr Glu Thr Leu
Lys Ser Glu Leu Ser Gly Lys Phe Glu Arg Leu Ile Val Ala Leu
Met Tyr Pro Pro Tyr Arg Tyr Glu Ala Lys Glu Leu His Asp Ala
Met Lys Gly Leu Gly Thr Lys Glu Gly Val Ile Ile Glu Ile Leu
Ala Ser Arg Thr Lys Asn Gln Leu Arg Glu Ile Met Lys Ala Tyr
Glu Glu Asp Tyr Gly Ser Ser Leu Glu Glu Asp Ile Gln Ala Asp
Thr Ser Gly Tyr Leu Glu Arg Ile Leu Val Cys Leu Leu Gln Gly
Ser Arg

whilst optionally at position 1 the methionine is cleaved and the alanine at position 2 is optionally blocked and/or there are optionally aggregations caused by intermolecular disulphide bridges, for example, between the cysteines at position 161.

**37.** Polypeptide according to claim 34, characterised in that it corresponds to the formula

X Asp Asp Val Ser Ser Phe Val Asp Pro Ala Leu Ala Leu
Gln Asp Ala Gln Asp Leu Tyr Ala Ala Gly Glu Lys Ile Arg Gly
Thr Asp Glu Met Lys Phe Ile Thr Ile Leu Cys Thr Arg Ser Ala
Thr His Leu Leu Arg Val Phe Glu Glu Tyr Glu Lys Ile Ala Asn
Lys Ser Ile Glu Asp Ser Ile Lys Ser Glu Thr His Gly Ser Leu
Glu Glu Ala Met Leu Thr Val Val Lys Cys Thr Gln Asn Leu His
Ser Tyr Phe Ala Glu Arg Leu Tyr Tyr Ala Met Lys Gly Ala Gly
Thr Arg Asp Gly Thr Leu Ile Arg Asn Ile Val Ser Arg Ser Glu
Ile Asp Leu Asn Leu Ile Lys Cys His Phe Lys Lys Met Tyr Gly
Lys Thr Leu Ser Ser Met Ile Met Glu Asp Thr Ser Gly Asp Tyr
Lys Asn Ala Leu Leu Ser Leu Val Gly Ser Asp Pro

wherein X represents methionine or hydrogen and/or there are optionally intramolecular disulphide bridges between the cysteines at positions 40 and/or 84 and/or 127 and/or aggregations caused by intermolecular disulphide bridges between the said positions.

**38.** Polypeptide according to one of the preceding claims, totally free from homologous polypeptides.

**39.** Polypeptide according to one of the preceding claims, characterised in that it contains a leader peptide.

**40.** Polypeptide according to one of the preceding claims, characterised in that

a. it contains a fusion protein component and/or
b. it consists of partial areas of the polypeptides according to one of the preceding claims (hybrid proteins) and/or
c. it is present as a dimer, trimer, tetramer or multimer.

**41.** Process for preparing polypeptides, characterised in that

a. a suitable host organism is transformed with genetic information according to one of claims 1 to 24 coding for a VAC-beta polypeptide,
b. the information is expressed in the host organism to produce the VAC-protein and
c. the VAC-polypeptide is isolated.

**42.** Process according to claim 41, characterised in that the host organism is defined according to claims 25 to 28.

**43.** Process according to claim 41 or 42, characterised in that genetic information is contained in the DNA molecules according to one of claims 1 to 24.

**44.** Process according to one of claims 41 to 43, characterised in that the VAC-protein is defined according to one of claims 34 to 40.

**45.** Non-native VAC-beta polypeptide which may be prepared according to one of claims 41 to 44.

**46.** Pharmaceutically acceptable salts of the polypeptides according to one of claims 34 to 40 or 45.

**47.** Use of the polypeptides according to one of claims 34 to 40 or 45 for the preparation of pharmaceutical compositions for the therapeutic or prophylactic treatment of the human or animal body.

**48.** Use of the polypeptides according to one of claims 34 to 40 or 45 for the production of pharmaceutical preparations.

**49.** Use of the polypeptides according to one of claims 34 to 40 or 45 as an anticoagulant.

**50.** Use of the polypeptides according to one of claims 34 to 40 or 45 as an anti-inflammatory agent.

**51.** Use of the polypeptides according to one of claims 34 to 40 or 45 as an anti-rheumatic agent.

**52.** Use of the polypeptides according to one of claims 34 to 40 or 45 in the indications which apply to the lipocortins.

**53.** Use of the polypeptides according to one of claims 34 to 40 or 45 as a thrombin-inhibiting agent.

**54.** Agent for therapeutic treatment, characterised in that it contains, in addition to pharmaceutically inert carriers, an effective amount of a polypeptide according to one of claims 34 to 40 or 45.

**55.** Hybrid cell line, characterised in that it secretes monoclonal antibodies against one of the polypeptides according to one of claims 34 to 40 or 45, which do not bind to native VAC-alpha and native VAC-beta and do not neutralise the latter.

**56.** Monoclonal antibodies, characterised in that they wholly or partly specifically neutralise the activity of the polypeptides according to one of claims 34 to 40 or 45 or specifically bind to one of said polypeptides, but do not bind to native VAC-alpha and VAC-beta and do not neutralise them.

**57.** Use of the monoclonal antibodies according to claim 56 for qualitative and/or quantitative evaluation of one of the polypeptides according to one of claims 34 to 40 or 45.

**58.** Use of the monoclonal antibodies according to claim 56 for purifying one of the polypeptides according to one of claims 34 to 40 or 45.

**59.** Test kit for detecting polypeptides according to one of claims 34 to 40 or 45, characterised in that it contains monoclonal antibodies according to claim 56.

**60.** Process for preparing monoclonal antibodies according to claim 56, characterised in that host animals are immunised with one of the polypeptides according to one of claims 34 to 40 or 45, B-lymphocytes of these host animals are fused with myeloma cells, the hybrid cell lines which secrete monoclonal antibodies are subcloned and cultivated *in vitro or in vivo.*

**61.** Pharmaceutically acceptable adducts and covalent compounds between the polypeptides according to one of claims 34 to 40 or 45 with an inert carrier for the therapeutic or prophylactic treatment of the human or animal body.

**62.** Pharmaceutically acceptable adducts or covalent compounds between the polypeptides according to one of claims 34 to 40 or 45 with polyethyleneglycol, for example, for the therapeutic or prophylactic treatment of the human or animal body.

**63.** Use of the polypeptides according to one of claims 34 to 40 or 45 as a blood-preserving agent.

**Revendications**

**1.** ADN codant la protéine anticoagulante vasculaire bêta (VAC-bêta) de séquence

ATG GCC TGG TGG AAA GCC TGG ATT GAA CAG GAG GGT GTC ACA GTG
AAG AGC AGC TCC CAC TTC AAC CCA GAC CCT GAT GCA GAG ACC CTC
TAC AAA GCC ATG AAG GGG ATC GGG ACC AAC GAG CAG GCT ATC ATC
GAT GTG CTC ACC AAG AGA AGC AAC ACG CAG CGG CAG CAG ATC GCC
AAG TCC TTC AAG GCT CAG TTC GGC AAG GAC CTC ACT GAG ACC TTG
AAG TCT GAG CTC AGT GGC AAG TTT GAG AGG CTC ATT GTG GCC CTT
ATG TAT CCG CCA TAC AGA TAC GAA GCC AAG GAG CTG CAT GAC GCC
ATG AAG GGC TTA GGA ACC AAG GAG GGT GTC ATC ATT GAG ATC CTG
GCC TCT CGG ACC AAG AAC CAG CTG CGG GAG ATA ATG AAG GCG TAT
GAG GAA GAC TAT GGG TCC AGC CTG GAG GAG GAC ATC CAA GCA GAC
ACA AGT GGC TAC CTG GAG AGG ATC CTG GTG TGC CTC CTG CAG GGC
AGC AGG GAT GAT GTG AGC AGC TTT GTG GAC CCG GCA CTG GCC CTC
CAA GAC GCA CAG GAT CTG TAT GCG GCA GGC GAG AAG ATT CGT GGG
ACT GAT GAG ATG AAA TTC ATC ACC ATC CTG TGC ACG CGC AGT GCC
ACT CAC CTG CTG AGA GTG TTT GAA GAG TAT GAG AAA ATT GCC AAC
AAG AGC ATT GAG GAC AGC ATC AAG AGT GAG ACC CAT GGC TCA CTG
GAG GAG GCC ATG CTC ACT GTG GTG AAA TGC ACC CAA AAC CTC CAC
AGC TAC TTT GCA GAG AGA CTC TAC TAT GCC ATG AAG GGA GCA GGG
ACG CGT GAT GGG ACC CTG ATA AGA AAC ATC GTT TCA AGG AGC GAG
ATT GAC TTA AAT CTT ATC AAA TGT CAC TTC AAG AAG ATG TAC GGC
AAG ACC CTC AGC AGC ATG ATC ATG GAA GAC ACC AGC GGC GAC TAC
AAG AAC GCC CTG CTG AGC CTG GTG GGC AGC GAC CCC TGA,

les formes dégénérées et modifiées de cette séquence qui codent une protéine ayant l'activité biologique de VAC-bêta étant incluses.

**2.** ADN selon la revendication 1 caractérisé en ce qu'il correspond à la formule

ATG GCC TGG TGG AAA GCC TGG ATT GAA CAG GAG GGT GTC ACA GTG
AAG AGC AGC TCC CAC TTC AAC CCA GAC CCT GAT GCA GAG ACC CTC
TAC AAA GCC ATG AAG GGG ATC GGG ACC AAC GAG CAG GCT ATC ATC
GAT GTG CTC ACC AAG AGA AGC AAC ACG CAG CGG CAG CAG ATC GCC
AAG TCC TTC AAG GCT CAG TTC GGC AAG GAC CTC ACT GAG ACC TTG
AAG TCT GAG CTC AGT GGC AAG TTT GAG AGG CTC ATT GTG GCC CTT
ATG TAT CCG CCA TAC AGA TAC GAA GCC AAG GAG CTG CAT GAC GCC
ATG AAG GGC TTA GGA ACC AAG GAG GGT GTC ATC ATT GAG ATC CTG
GCC TCT CGG ACC AAG AAC CAG CTG CGG GAG ATA ATG AAG GCG TAT
GAG GAA GAC TAT GGG TCC AGC CTG GAG GAG GAC ATC CAA GCA GAC
ACA AGT GGC TAC CTG GAG AGG ATC CTG GTG TGC CTC CTG CAG GGC
AGC AGG

un codon d'arrêt étant présent éventuellement derrière le dernier AGG, ainsi que les formes dégénérées de cet ADN.

**3.** ADN selon la revendication 1 caractérisé en ce qu'il correspond à la formule

GAT GAT GTG AGC AGC TTT GTG GAC CCG GCA CTG GCC CTC
CAA GAC GCA CAG GAT CTG TAT GCG GCA GGC GAG AAG ATT CGT GGG
ACT GAT GAG ATG AAA TTC ATC ACC ATC CTG TGC ACG CGC AGT GCC
ACT CAC CTG CTG AGA GTG TTT GAA GAG TAT GAG AAA ATT GCC AAC
AAG AGC ATT GAG GAC AGC ATC AAG AGT GAG ACC CAT GGC TCA CTG
GAG GAG GCC ATG CTC ACT GTG GTG AAA TGC ACC CAA AAC CTC CAC
AGC TAC TTT GCA GAG AGA CTC TAC TAT GCC ATG AAG GGA GCA GGG
ACG CGT GAT GGG ACC CTG ATA AGA AAC ATC GTT TCA AGG AGC GAG
ATT GAC TTA AAT CTT ATC AAA TGT CAC TTC AAG AAG ATG TAC GGC
AAG ACC CTC AGC AGC ATG ATC ATG GAA GAC ACC AGC GGC GAC TAC
AAG AAC GCC CTG CTG AGC CTG GTG GGC AGC GAC CCC TGA

un codon d'initiation étant présent éventuellement devant le premier GAT, et les formes dégénérées de cet ADN.

**4.** ADN selon la revendication 1 caractérisé en ce qu'un oligonucléotide qui code une séquence de reconnaissance d'une protéase spécifique est inséré dans le segment qui code la séquence lieur entre la seconde et la troisième structure de répétition de la position +165 à +181 selon la figure 7.

**5.** ADN selon la revendication 1 caractérisé en ce que le triplet codant l'arginine en position +167 selon la figure 7 est remplacé par un codon qui code un acide aminé qui n'est pas reconnu par la trypsine en tant que site de clivage préféré, par exemple l'histidine.

**6.** ADN selon l'une des revendications 1 à 4 caractérisé en ce que le triplet codant la lysine et/ou l'arginine est remplacé de manière ciblée par des triplets qui codent l'histidine.

**7.** ADN selon l'une des revendications 1 à 6 caractérisé en ce que le triplet ou éventuellement les triplets codant la cystéine est/sont remplacé(s) par des triplets codant la sérine ou la valine.

**8.** ADN selon la revendication 1 caractérisé en ce qu'il code un polypeptide/protéine englobant un domaine polypeptidique de la position +26 à +92 ou de la position +98 à +164 ou de la position +182 à +249 ou de la position +258 à +324 selon la figure 7.

**9.** ADN selon l'une des revendications 1 à 8 caractérisé en ce que les domaines codant les répétitions complètes en position +26 à +92, en position +98 à +164, en position +182 à +249 et en position +258 à +324 selon la figure 7 sont réarrangés.

**10.** ADN selon l'une des revendications 1-9 caractérisé en ce que les domaines codant les 17 acides aminés de la région conservée en position +36 à +51 et/ou en position +108 à +123 et/ou en position +192 à +208 et/ou en position +268 à +283 selon la figure 7 sont modifiés.

**11.** ADN selon l'une des revendications 1 à 10 caractérisé en ce que les domaines codant les 17 acides aminés de la région conservée aux positions +36 à +51 et/ou en position +108 à +123 et/ou en position +192 à +208 et/ou en position +268 à +283 selon la figure 7 sont remplacés totalement ou partiellement par des domaines qui codent les acides aminés correspondants dans les lipocortines ou par des domaines qui codent les acides aminés de VAC-alpha correspondants et inversement.

**12.** ADN selon l'une des revendications 1 à 11 caractérisé en ce que le domaine qui code le peptide N-terminal, position 1 à 25 selon la figure 7, est remplacé totalement ou partiellement par des oligonucléotides qui codent le peptide N-terminal de l'une des autres protéines présentant une structure de répétition.

**13.** ADN selon l'une des revendications précédentes caractérisé en ce que l'extrémité 5' N-terminale est précédée par une séquence signal homologue pour l'hôte correspondant.

**14.** ADN selon la revendication précédente caractérisé en ce que la séquence signal code à l'extrémité 3' un site de clivage de reconnaissance spécifique pour une protéase.

**15.** ADN selon l'une des revendications 1 à 7, caractérisé en ce que l'extrémité 5' N-terminale est précédée par une séquence codant une partie de protéine de fusion.

**16.** ADN selon la revendication précédente, caractérisé en ce que l'ADN codant la partie de protéine de fusion code à l'extrémité 3' un site de clivage de reconnaissance spécifique pour une protéase.

**17.** ADN caractérisé en ce qu'il représente un allèle de l'un des ADN décrits dans les revendications précédentes.

**18.** ADN caractérisé en ce qu'il représente une forme dégénérative de l'un des ADN décrits dans les revendications précédentes ou en ce qu'il consiste en combinaisons de différentes séquences complètes ou partielles de l'une des revendications précédentes, codant des protéines hybrides ayant sensiblement les propriétés anticoagulantes vasculaires de VAC-bêta.

**19.** ADN caractérisé en ce qu'il s'hybride avec l'une des molécules d'ADN selon l'une des revendications 1 à 18 dans des conditions qui font apparaître une homologie supérieure à 85%, de préférence supérieure à 90%, l'ADN pouvant être d'origine naturelle, synthétique ou semisynthétique et pouvant être apparenté aux molécules d'ADN selon l'une des revendications 1 à 18 par mutation, substitutions de nucléotides, délétions de nucléotides, insertions de nucléotides et inversions de nucléotides et codant un polypeptide/protéine présentant sensiblement les propriétés biologiques des protéines anticoagulantes vasculaires.

**20.** ADN selon l'une des revendications précédentes caractérisé en ce qu'il est inséré dans un vecteur.

**21.** ADN selon la revendication 20 caractérisé en ce que l'ADN selon l'une des revendications précédentes est lié dans un vecteur en liaison fonctionnelle avec une séquence de contrôle de l'expression, réplicable dans des micro-organismes et des cellules de mammifère.

**22.** ADN selon l'une des revendications 20 ou 21 caractérisé en ce que le vecteur est un plasmide réplicable dans des procaryotes.

**23.** ADN selon l'une des revendications 20 ou 21, caractérisé en ce que le vecteur est un plasmide réplicable dans des eucaryotes.

**24.** ADN selon l'une des revendications 20 ou 21, caractérisé en ce qu'il est réplicable dans des cellules de mammifère.

**25.** Organisme hôte caractérisé en ce qu'il est transformé avec un ADN selon l'une des revendications 20 à 24.

**26.** Organisme hôte selon la revendication 25, caractérisé en ce qu'il s'agit d'un procaryote, de préférence E. coli.

**27.** Organisme hôte selon la revendication 25, caractérisé en ce qu'il s'agit d'un eucaryote.

**28.** Organisme hôte selon la revendication 25, caractérisé en ce qu'il s'agit d'une lignée cellulaire de mammifère.

**29.** Procédé de préparation de molécules d'ADN qui codent VAC-bêta, caractérisé en ce que l'on isole à partir d'une banque d'ADN génomique le gène codant ce polypeptide ou l'on prépare à partir de l'ARNm correspondant à ce gène un ADN complémentaire codant ce polypeptide à l'aide de l'enzyme transcriptase inverse ou en ce que l'ADN codant ce polypeptide est préparé au moyen de procédés chimiques et/ou enzymatiques.

**30.** Procédé selon la revendication précédente, caractérisé en ce que l'ADN correspond à l'un des ADN décrits dans les revendications 1 à 18.

**31.** Procédé de préparation d'un ADN selon l'une des revendications 20 à 24, caractérisé en ce que l'on insère dans un ADN vecteur coupé à l'aide d'endonucléases de restriction un ADN muni d'extrémités correspondantes qui code un polypeptide présentant sensiblement les propriétés des protéines anticoagulantes vasculaires.

**32.** Procédé de préparation d'un ADN selon l'une des revendications 21 à 24, caractérisé en ce que l'on insère dans un ADN vecteur coupé avec des endonucléases de restriction, qui contient des séquences de contrôle de l'expression, un ADN muni d'extrémités correspondantes qui code un polypeptide présentant sensiblement les propriétés des protéines anticoagulantes vasculaires de telle manière que les séquences de contrôle de l'expression régulent l'ADN inséré.

**33.** Procédé de préparation d'organismes hôtes transformés selon l'une des revendications 25 à 28, caractérisé en ce que l'on transforme l'organisme hôte avec l'un des vecteurs selon l'une des revendications 22 à 24.

**34.** Polypeptide qui correspond à un dérivé non natif de VAC-bêta, présentant sensiblement les propriétés dès protéines anticoagulantes vasculaires, dérivé de la séquence de VAC-bêta

Met Ala Trp Trp Lys Ala Trp Ile Glu Gln Glu Gly Val Thr Val
Lys Ser Ser Ser His Phe Asn Pro Asp Pro Asp Ala Glu Thr Leu
Tyr Lys Ala Met Lys Gly Ile Gly Thr Asn Glu Gln Ala Ile Ile
Asp Val Leu Thr Lys Arg Ser Asn Thr Gln Arg Gln Gln Ile Ala
Lys Ser Phe Lys Ala Gln Phe Gly Lys Asp Leu Thr Glu Thr Leu
Lys Ser Glu Leu Ser Gly Lys Phe Glu Arg Leu Ile Val Ala Leu
Met Tyr Pro Pro Tyr Arg Tyr Glu Ala Lys Glu Leu His Asp Ala
Met Lys Gly Leu Gly Thr Lys Glu Gly Val Ile Ile Glu Ile Leu
Ala Ser Arg Thr Lys Asn Gln Leu Arg Glu Ile Met Lys Ala Tyr
Glu Glu Asp Tyr Gly Ser Ser Leu Glu Glu Asp Ile Gln Ala Asp
Thr Ser Gly Tyr Leu Glu Arg Ile Leu Val Cys Leu Leu Gln Gly
Ser Arg Asp Asp Val Ser Ser Phe Val Asp Pro Ala Leu Ala Leu
Gln Asp Ala Gln Asp Leu Tyr Ala Ala Gly Glu Lys Ile Arg Gly
Thr Asp Glu Met Lys Phe Ile Thr Ile Leu Cys Thr Arg Ser Ala
Thr His Leu Leu Arg Val Phe Glu Glu Tyr Glu Lys Ile Ala Asn
Lys Ser Ile Glu Asp Ser Ile Lys Ser Glu Thr His Gly Ser Leu
Glu Glu Ala Met Leu Thr Val Val Lys Cys Thr Gln Asn Leu His
Ser Tyr Phe Ala Glu Arg Leu Tyr Tyr Ala Met Lys Gly Ala Gly
Thr Arg Asp Gly Thr Leu Ile Arg Asn Ile Val Ser Arg Ser Glu
Ile Asp Leu Asn Leu Ile Lys Cys His Phe Lys Lys Met Tyr Gly
Lys Thr Leu Ser Ser Met Ile Met Glu Asp Thr Ser Gly Asp Tyr
Lys Asn Ala Leu Leu Ser Leu Val Gly Ser Asp Pro,

dans laquelle la méthionine en position 1 est éventuellement clivée et l'alanine en position 2 est éventuellement bloquée et/ou dans laquelle il existe éventuellement des ponts disulfures intramoléculaires entre les cystéines aux positions 161 et/ou 206 et/ou 250 et/ou 293 et/ou des agrégations par des ponts disulfures intermoléculaires entre les positions citées.

**35.** Polypeptide selon la revendication 34, caractérisé en ce qu'il est codé par un ADN selon l'une des revendications 1 à 18.

**36.** Polypeptide selon la revendication 34, caractérisé en ce qu'il correspond à la formule

Met Ala Trp Trp Lys Ala Trp Ile Glu Gln Glu Gly Val Thr Val
Lys Ser Ser Ser His Phe Asn Pro Asp Pro Asp Ala Glu Thr Leu
Tyr Lys Ala Met Lys Gly Ile Gly Thr Asn Glu Gln Ala Ile Ile
Asp Val Leu Thr Lys Arg Ser Asn Thr Gln Arg Gln Gln Ile Ala
Lys Ser Phe Lys Ala Gln Phe Gly Lys Asp Leu Thr Glu Thr Leu
Lys Ser Glu Leu Ser Gly Lys Phe Glu Arg Leu Ile Val Ala Leu
Met Tyr Pro Pro Tyr Arg Tyr Glu Ala Lys Glu Leu His Asp Ala
Met Lys Gly Leu Gly Thr Lys Glu Gly Val Ile Ile Glu Ile Leu
Ala Ser Arg Thr Lys Asn Gln Leu Arg Glu Ile Met Lys Ala Tyr
Glu Glu Asp Tyr Gly Ser Ser Leu Glu Glu Asp Ile Gln Ala Asp
Thr Ser Gly Tyr Leu Glu Arg Ile Leu Val Cys Leu Leu Gln Gly
Ser Arg

dans laquelle la méthionine en position 1 est éventuellement clivée et l'alanine en position 2 est éventuellement bloquée et/ou dans laquelle il existe éventuellement des agrégations par exemple par des ponts disulfures intermoléculaires entre les cystéines en position 161.

**37.** Polypeptide selon la revendication 34, caractérisé en ce qu'il correspond à la formule

X Asp Asp Val Ser Ser Phe Val Asp Pro Ala Leu Ala Leu
Gln Asp Ala Gln Asp Leu Tyr Ala Ala Gly Glu Lys Ile Arg Gly
Thr Asp Glu Met Lys Phe Ile Thr Ile Leu Cys Thr Arg Ser Ala
Thr His Leu Leu Arg Val Phe Glu Glu Tyr Glu Lys Ile Ala Asn
Lys Ser Ile Glu Asp Ser Ile Lys Ser Glu Thr His Gly Ser Leu
Glu Glu Ala Met Leu Thr Val Val Lys Cys Thr Gln Asn Leu His
Ser Tyr Phe Ala Glu Arg Leu Tyr Tyr Ala Met Lys Gly Ala Gly
Thr Arg Asp Gly Thr Leu Ile Arg Asn Ile Val Ser Arg Ser Glu
Ile Asp Leu Asn Leu Ile Lys Cys His Phe Lys Lys Met Tyr Gly
Lys Thr Leu Ser Ser Met Ile Met Glu Asp Thr Ser Gly Asp Tyr
Lys Asn Ala Leu Leu Ser Leu Val Gly Ser Asp Pro

dans laquelle X représente la méthionine ou l'hydrogène et/ou dans laquelle il existe éventuellement des ponts disulfures intramoléculaires entre les cystéines aux positions 40 et/ou 84 et/ou 127 et/ou des agrégations par des ponts disulfures intermoléculaires entre les positions citées.

**38.** Polypeptide selon l'une des revendications précédentes, totalement exempt de polypeptides homologues.

**39.** Polypeptide selon l'une des revendications précédentes, caractérisé en ce qu'il contient un peptide signal.

**40.** Polypeptide selon l'une des revendications précédentes, caractérisé

a. en ce qu'il contient une partie de protéine de fusion et/ou
b. en ce qu'il consiste en domaines partiels des polypeptides selon l'une des revendications précédentes (protéines hybrides) et/ou
c. en ce qu'il est sous forme de dimère, de trimère, de tétramère ou de multimère.

**41.** Procédé de préparation de polypeptides, caractérisé en ce que

a. un organisme hôte approprié est transformé avec des informations génétiques selon l'une des revendications 1 à 24 codant un polypeptide VAC-bêta,
b. l'information pour la production de la protéine VAC est exprimée dans l'organisme hôte et
c. le polypeptide VAC est isolé.

**42.** Procédé selon la revendication 41, caractérisé en ce que l'organisme hôte est défini selon les revendications 25 à 28.

**43.** Procédé selon la revendication 41 ou 42, caractérisé en ce que les informations génétiques sont contenues dans les molécules d'ADN selon l'une des revendications 1 à 24.

**44.** Procédé selon l'une des revendications 41 à 43, caractérisé en ce que la protéine VAC est définie selon l'une des revendications 34 à 40.

**45.** Polypeptide VAC-bêta non natif qui peut être préparé selon l'une des revendications 41 à 44.

**46.** Sels pharmaceutiquement tolérables des polypeptides selon l'une des revendications 34 à 40 ou 45.

**47.** Utilisation des polypeptides selon l'une des revendications 34 à 40 ou 45 pour la préparation de médicaments pour le traitement thérapeutique ou prophylactique du corps humain ou animal.

**48.** Utilisation des polypeptides selon l'une des revendications 34 à 40 ou 45 pour la production de préparations pharmaceutiques.

**49.** Utilisation des polypeptides selon l'une des revendications 34 à 40 ou 45 comme agents anticoagulants.

**50.** Utilisation des polypeptides selon l'une des revendications 34 à 40 ou 45 comme agents anti-inflammatoires.

**51.** Utilisation des polypeptides selon l'une des revendications 34 à 40 ou 45 comme agents antirhumatismaux.

**52.** Utilisation des polypeptides selon l'une des revendications 34 à 40 ou 45 dans les indications valant pour les lipocortines.

**53.** Utilisation des polypeptides selon l'une des revendications 34 à 40 ou 45 comme agents inhibant la thrombine.

**54.** Agent de traitement thérapeutique caractérisé en ce qu'il contient, outre des supports pharmaceutiquement inertes, une quantité efficace d'un polypeptide selon l'une des revendications 34 à 40 ou 45.

**55.** Lignée cellulaire hybride caractérisée en ce qu'elle sécrète des anticorps monoclonaux contre l'un des polypeptides selon l'une des revendications 34 à 40 ou 45 qui ne se lient pas à VAC-alpha native et à VAC-bêta native et ne neutralisent pas celle-ci.

**56.** Anticorps monoclonaux caractérisés en ce qu'ils neutralisent spécifiquement, totalement ou en partie, l'effet des polypeptides selon l'une des revendications 34 à 40 ou 45 ou se lient spécifiquement à l'un desdits polypeptides mais ne se lient pas à VAC-alpha native et à VAC-bêta native et ne neutralisent pas celles-ci.

**57.** Utilisation des anticorps monoclonaux selon la revendication 56 pour la détermination qualitative et/ou quantitative de l'un des polypeptides selon l'une des revendications 34 à 40 ou 45.

**58.** Utilisation des anticorps monoclonaux selon la revendication 56 pour la purification de l'un des polypeptides selon l'une des revendications 34 à 40 ou 45.

**59.** Trousse de test pour la détermination de polypeptides selon l'une des revendications 34 à 40 ou 45, caractérisée en ce qu'elle contient des anticorps monoclonaux selon la revendication 56.

**60.** Procédé de préparation d'anticorps monoclonaux selon la revendication 56 caractérisé en ce que des animaux hôtes sont immunisés avec l'un des polypeptides selon l'une des revendications 34 à 40 ou 45, des lymphocytes B de ces animaux hôtes sont fusionnés avec des cellules de myélome, les lignées cellulaires hybrides produisant des anticorps monoclonaux sont sous-clonées et cultivées in vitro ou in vivo.

**61.** Produits d'addition et composés covalents pharmaceutiquement tolérables entre les polypeptides selon l'une des revendications 34 à 40 ou 45 avec un support inerte, pour le traitement thérapeutique ou prophylactique du corps humain ou animal.

**62.** Produits d'addition ou composés covalents pharmaceutiquement tolérables entre les polypeptides selon l'une des revendications 34 à 40 et 45 avec par exemple le polyéthylèneglycol, pour le traitement thérapeutique ou prophylactique du corps humain ou animal.

**63.** Utilisation des polypeptides selon l'une des revendications 34 à 40 ou 45 comme agents de conservation du sang.

Tryptic peptides

| | |
|---|---|
| P5 | H A L K |
| P7 | G E T S G D Y K |
| P11/I | W G T D E E K |
| P11/II | T P E E L R |
| P12 | G A G T D D H T L I R |
| P14 | Q E I S A A F K |
| P15 | T L F G R |
| P16/I | L Y D A Y E L K |
| P16/II | W?L Y D A Y E L K |
| P17 | V L T E I I A S R |
| P18 | G T V T D F P G F D E R |
| P20/I | Q V Y E E E Y G S S L E D D V V G D T S G Y Y Q R |
| P20/II | L I V A L M K |
| P21/I | S E I D L F N I R K |
| P23/I | F I T I F G T R |
| P24 | K N F A T S L Y S M I K |
| P25 | S?G T D E E K F I T I F G T |
| P27 | D L L D D L K S E L T G K F E K |
| P29/I | G L G T D E E S I L T L L T S R |
| P29/II | M L V V L L Q A N R D P D A G I D E A Q V X Q X A Q A L F Q A |
| P30/I | X I P A Y L A E T L Y Y A M K |
| P30/II | E T X G N L E Q L L L A V V K |

BrCN-Peptides

| | |
|---|---|
| BrCN 1 | K G A G T D D H T L I R V |
| BrCN 4 | I K G D T S G D Y K K A |
| BrCN 15 | K P S R L Y D A Y E L K H A L K G A G T N E K V L T E I I |
| BrCN 22 | K G L G T D E E S I L T L L T S X X N A Q |

FIG. 1.

```
Tryptic peptide P16/II

          W? L  Y  D  A  Y  E  L  K
5'  ...UGGCUNUAUGAUGCNUAUGAA...  3'        mRNA
                C  C     C  G
            UUA
              G

3'     ACCGAAATACTACGAATACT      5'
              G  G  G  G  G                 EBI - 387
              C        C
              T        T

3'     ACCAACATACTACGAATACT      5'
              T  G  G  G  G                 EBI - 388
                       C
                       T

Staph-A Peptide P20/I/6
           (Subfragment of the tryptic peptide P20/I)

          D  D  V  V  G  D  T  S  G  Y  Y  R
5'  ...GAUGAUGUNGUNGGNGAUACN...  3'          mRNA
          C  C           C

3'     CTACTACAACAACCACTATG      5'
          G  G  G  G  G  G                  EBI - 386
                C  C  C
                T  T  T
```

FIG. 2.

FIG. 3.

Tryptic peptide P30/I

```
X  I  P  A  Y  L  A  E  T  L  Y  Y  A  M  K
              5'  ...GAAACNCUNUACUACGCNAUGAAA...  3'    mRNA
                       G         U  U       G
                            UUA
                              G

              3'     CTCTGIIAIATIATICGITACTT     5'  EBI - 118
              3'     CTTTGIIAIATIATICGITACTT     5'  EBI - 119
```

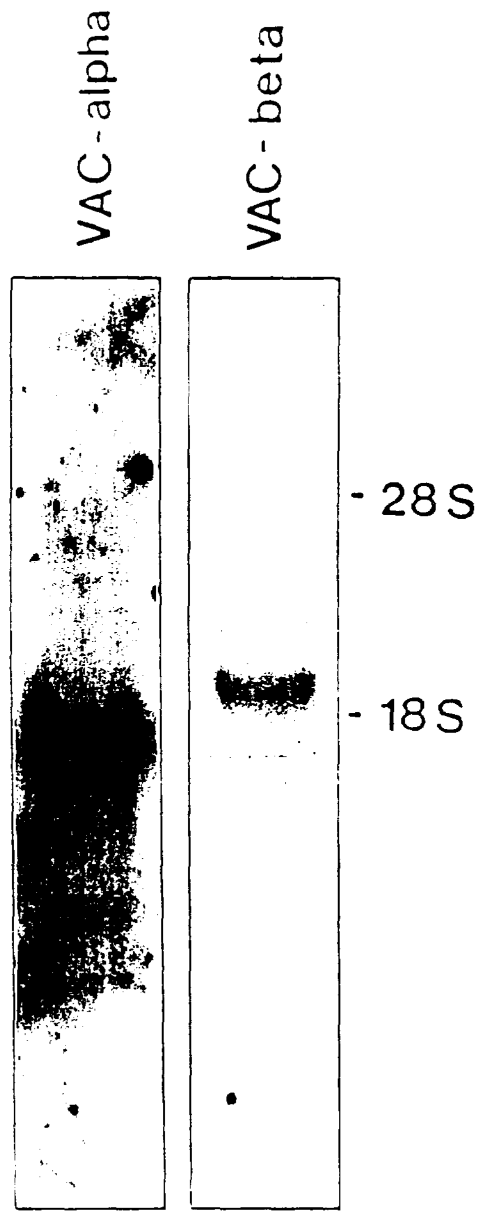


FIG. 4.

VAC-beta cDNA

```
          AGGCCTGCTCACTCCTCAGCTGCAGGAGCCAGACGTGTGGAGTCCCA

GCAGAGGCCAACCTGTGTCTCTTCATCTCCGTGAGAAAGGTGCCCCCGAAGTGAAAGAG

 1               5                  10                 15
Met Ala Trp Trp Lys Ala Trp Ile Glu Gln Glu Gly Val Thr Val
ATG GCC TGG TGG AAA GCC TGG ATT GAA CAG GAG GGT GTC ACA GTG    45

                20                  25                 30
Lys Ser Ser Ser His Phe Asn Pro Asp Pro Asp Ala Glu Thr Leu
AAG AGC AGC TCC CAC TTC AAC CCA GAC CCT GAT GCA GAG ACC CTC    90

                35                  40                 45
Tyr Lys Ala Met Lys Gly Ile Gly Thr Asn Glu Gln Ala Ile Ile
TAC AAA GCC ATG AAG GGG ATC GGG ACC AAC GAG CAG GCT ATC ATC   135

                50                  55                 60
Asp Val Leu Thr Lys Arg Ser Asn Thr Gln Arg Gln Gln Ile Ala
GAT GTG CTC ACC AAG AGA AGC AAC ACG CAG CGG CAG CAG ATC GCC   180

                65                  70                 75
Lys Ser Phe Lys Ala Gln Phe Gly Lys Asp Leu Thr Glu Thr Leu
AAG TCC TTC AAG GCT CAG TTC GGC AAG GAC CTC ACT GAG ACC TTG   225

                80                  85                 90
Lys Ser Glu Leu Ser Gly Lys Phe Glu Arg Leu Ile Val Ala Leu
AAG TCT GAG CTC AGT GGC AAG TTT GAG AGG CTC ATT GTG GCC CTT   270

                95                  100                105
Met Tyr Pro Pro Tyr Arg Tyr Glu Ala Lys Glu Leu His Asp Ala
ATG TAT CCG CCA TAC AGA TAC GAA GCC AAG GAG CTG CAT GAC GCC   315

                110                 115                120
Met Lys Gly Leu Gly Thr Lys Glu Gly Val Ile Ile Glu Ile Leu
ATG AAG GGC TTA GGA ACC AAG GAG GGT GTC ATC ATT GAG ATC CTG   360

                125                 130                135
Ala Ser Arg Thr Lys Asn Gln Leu Arg Glu Ile Met Lys Ala Tyr
GCC TCT CGG ACC AAG AAC CAG CTG CGG GAG ATA ATG AAG GCG TAT   405

                140                 145                150
Glu Glu Asp Tyr Gly Ser Ser Leu Glu Glu Asp Ile Gln Ala Asp
GAG GAA GAC TAT GGG TCC AGC CTG GAG GAG GAC ATC CAA GCA GAC   450
```

F I G. 5/1

```
                    155                      160                      165
Thr Ser Gly Tyr Leu Glu Arg Ile Leu Val Cys Leu Leu Gln Gly
ACA AGT GGC TAC CTG GAG AGG ATC CTG GTG TGC CTC CTG CAG GGC    495

                    170                      175                      180
Ser Arg Asp Asp Val Ser Ser Phe Val Asp Pro Ala Leu Ala Leu
AGC AGG GAT GAT GTG AGC AGC TTT GTG GAC CCG GCA CTG GCC CTC    540

                    185                      190                      195
Gln Asp Ala Gln Asp Leu Tyr Ala Ala Gly Glu Lys Ile Arg Gly
CAA GAC GCA CAG GAT CTG TAT GCG GCA GGC GAG AAG ATT CGT GGG    585

                    200                      205                      210
Thr Asp Glu Met Lys Phe Ile Thr Ile Leu Cys Thr Arg Ser Ala
ACT GAT GAG ATG AAA TTC ATC ACC ATC CTG TGC ACG CGC AGT GCC    630

                    215                      220                      225
Thr His Leu Leu Arg Val Phe Glu Glu Tyr Glu Lys Ile Ala Asn
ACT CAC CTG CTG AGA GTG TTT GAA GAG TAT GAG AAA ATT GCC AAC    675

                    230                      235                      240
Lys Ser Ile Glu Asp Ser Ile Lys Ser Glu Thr His Gly Ser Leu
AAG AGC ATT GAG GAC AGC ATC AAG AGT GAG ACC CAT GGC TCA CTG    720

                    245                      250                      255
Glu Glu Ala Met Leu Thr Val Val Lys Cys Thr Gln Asn Leu His
GAG GAG GCC ATG CTC ACT GTG GTG AAA TGC ACC CAA AAC CTC CAC    765

                    260                      265                      270
Ser Tyr Phe Ala Glu Arg Leu Tyr Tyr Ala Met Lys Gly Ala Gly
AGC TAC TTT GCA GAG AGA CTC TAC TAT GCC ATG AAG GGA GCA GGG    810

                    275                      280                      285
Thr Arg Asp Gly Thr Leu Ile Arg Asn Ile Val Ser Arg Ser Glu
ACG CGT GAT GGG ACC CTG ATA AGA AAC ATC GTT TCA AGG AGC GAG    855

                    290                      295                      300
Ile Asp Leu Asn Leu Ile Lys Cys His Phe Lys Lys Met Tyr Gly
ATT GAC TTA AAT CTT ATC AAA TGT CAC TTC AAG AAG ATG TAC GGC    900

                    305                      310                      315
Lys Thr Leu Ser Ser Met Ile Met Glu Asp Thr Ser Gly Asp Tyr
AAG ACC CTC AGC AGC ATG ATC ATG GAA GAC ACC AGC GGC GAC TAC    945

                    320                      325
Lys Asn Ala Leu Leu Ser Leu Val Gly Ser Asp Pro  *
AAG AAC GCC CTG CTG AGC CTG GTG GGC AGC GAC CCC TGA GGCACAG    991
```

# F I G. 5/2

AAGAACAAGAGCAAAGACCATGAAGCCAGAGTCTCCAGGACTCCTCACTCAACCTCGGC 1050
CATGGACGCAGGTTGGGTGTGAGGGGGGTCCCAGCCTTTCGGTCTTCTATTTCCCTATT 1109
TCCAGTGCTTTCCAGCCGGGTTTCTGACCCAGAGGTGGAACCGGCCTGGACTCCTCTTC 1168
CCAACTTCCTCCAGGTCATTTCCCAGTGTGAGCACAATGCCAACCTTAGTGTTTCTCCA 1227
GCCAGACAGATGCCTCAGCATGAAGGGCTTGGGGACTTGTGGATCATTCCTTCCTCCCT 1286
GCAGGAGCTTCCCAAGCTGGTCACAGAGTCTCCTGGGCACAGGTTATACAGACCCCAGC 1345
CCCATTCCCATCTACTGAAACAGGGTCTCCACAAGAGGGGCCAGGGAATATGGGTTTTT 1404
AACAAGCGTCTTACAAAACACTTCTCTATCATGCAGCCGGAGAGCTGGCTGGGAGCCCT 1463
TTTGTTTTAGAACACACATCCTTCAGCAGCTGAGAAATGAACACGAATCCATCCCAACC 1522
GAGATGCCATTAACATTCATCTAAAAATGTTAGGCTCTAAATGGACGAAAAATTCTCTC 1581
GCCATCTTAATAACAAAATAAACTACAAATTCCTGACCCAAGGACACTGTGTTATAAGA 1640
GGCGTGGGCTCCCCTGGTGGCTGACCAGGTCAGCTGCCCTGGCCTTGCACCCCTCTGCA 1699
TGCAGCACAGAAGGGTGTGACCATGCCCTCAGCACCACTCTTGTCCCCACTGAACGGCA 1758
ACTGAGACTGGGTACCTGGAGATTCTGAAGTGCCTTTGCTGTGGTTTTCAAAATAATAA 1817
AGATTTGTATTCAACTC-polyA 1834

F I G. 5/3

Amino acid compostion of VAC-beta

| | | |
|---|---|---|
| Ala | 25 | ( 7,7 %) |
| Cys | 4 | ( 1,2 %) |
| Asp | 20 | ( 6,1 %) |
| Asn | 9 | ( 2,8 %) |
| Glu | 29 | ( 8,9 %) |
| Gln | 12 | ( 3,7 %) |
| Phe | 9 | ( 2,8 %) |
| Gly | 20 | ( 6,1 %) |
| His | 6 | ( 1,8 %) |
| Ile | 23 | ( 7,0 %) |
| Lys | 27 | ( 8,3 %) |
| Leu | 33 | (10,1 %) |
| Met | 11 | ( 3,4 %) |
| Pro | 6 | ( 1,8 %) |
| Arg | 15 | ( 4,6 %) |
| Ser | 27 | ( 8,3 %) |
| Thr | 21 | ( 6,4 %) |
| Val | 13 | ( 4,0 %) |
| Trp | 3 | ( 0,9 %) |
| Tyr | 14 | ( 4,3 %) |
| | | MW = 36837 |
| charged: | | 97 (29,6 %) |

FIG. 6.

# FIG. 7.

VAC-beta : Quadruplication of a 67 amino acid long sequence

```
                                                  MAWWKAWIEQEGVTVKSSSHFNPDP

DAETLYKAMKG-IGTNEQAIIDVLTKRSNTQRQQIAKSFKAQFGKDLTETLKSELSGKFERLIVALMY    PPYRY

EAKELHDAMKG-LGTKEGVIIEILASRTKNQLREIMKAYEEDYGSSLEEDIQADTSGYLERILVCLLQ    GSRDDVSSFVDPALALQ

DAQDLYAAGEKIRGTDEMKFITILCTRSATHLLRVFEEYEKIANKSIEDSIKSETHGSLEEAMLTVVK    CTQNLHSY

FAERLYYAMKG-AGTRDGTLIRNIVSRSEIDLNLIKCHFKKMYGKTLSSMIMEDTSGDYKNALLSLVG    SDP*

      KG-hGTDExxLIpILApR                                     oooooo

dAe LY AMKG  GT Eg ll lL sRS tqL  I k ykk yGKsLee IkseTSG lEralv Lvk
                                      fe              d         l
```

"Consensus": x : 50% of amino acids identical
X : 75% of amino acids identical
X̲ : 100% of amino acids identical

"oooooo" : Hydrophobic region

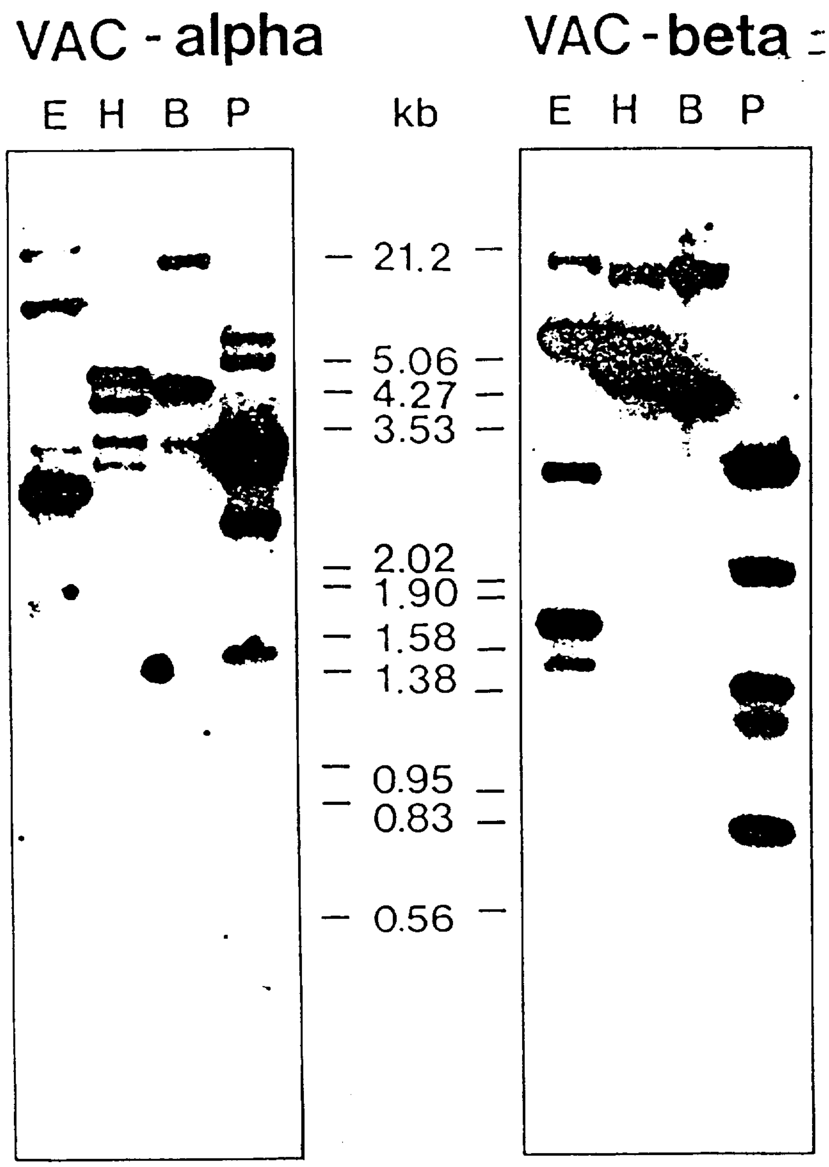
VAC - alpha
VAC - beta
E H B P
kb
E H B P
21.2
5.06
4.27
3.53
2.02
1.90
1.58
1.38
0.95
0.83
0.56

FIG. 8.

# FIG. 9.

Comparison of VAC-alpha and VAC-beta

```
alpha                                                   MAQVLRGTVTDFPGFDERA
 beta                                               MAWWKAWIEQEGV..KSSSH.NPDP

alpha DAETLRKAMKGLGTDEESILTLLTSRSNAQRQEISAAFKTLFGRDLLDDLKSELTGKFEKLIVALMK   PSRLY
 beta .....Y.....I..N.QA.IDV..K...T...Q.AKS..AQ..K..TET.....S....R......Y   .PYR.

alpha DAYELKHALKGAGTNEKVLTEIIASRTPEELRAIKQVYEEEYGSSLEDDVVGDTSGYYQRMLVVLLQ   ANRDPDAG-IDEAQVEQ
 beta E.K..HD.M..L..K.G.II..L....KNQ..E.MKA...D......E.IQA.....LE.I..C...   GS..DVSSFV.P.LAL.

alpha DAQALFQAGELKWGTDEEKFITIFGTRSVSHLRKVFDKYMTISGFQIEETIDRETSGNLEQLLLAVVK   SIRSIPAY
 beta ...D.YA...KIR....M.....LC...AT..LR..EE.EK.ANKS..DS.KS..H.S..EAM.T...   CTQNLHS.

alpha LAETLYYAMKGAGTDDHTLIRVMVSRSEIDLFNIRKEFRKNFATSLYSMIKGDTSGDYKKALLLLCG   EDD*
 beta F..R..........R.G....NI........NL.KCH.K.MYGKT.S...ME.......N...S.V.   S.P*
```

Comparison of VAC-alpha and beta cDNA

```
VA                          ATGGCACAGGTTCTCAGAGGCACTGTGACTGACTTCCCTGGA    42
VB    ATGGCCTGGTGGAAAGCCTG.ATTG.ACAGGAGG.T.T...A....AGAG.AG.T.CCAC    60

VA    TTTGATGAGCGGGCTGATGCAGAAACTCTTCGGAAGGCTATGAAAGGCTTGGGCACAGAT   102
VB    ..CA.CCCAGACC..........G..C..CTAC..A..C.....G..GA.C..G..CA.C   120

VA    GAGGAGAGCATCCTGACTCTGTTGACATCCCGAAGTAATGCTCAGCGCCAGGAAATCTCT   162
VB    ...C..GCT...A.CGA.G..C.C..CAAGA....C..CA.G.....G...C.G...G.C   180

VA    GCAGCTTTTAAGACTCTGTTTGGCAGGGATCTTCTGGATGACCTGAAATCAGAACTAACT   222
VB    AAGT.C..C...G...A...C....A...C..CACT..G.C.T....G..T..G..C.G.   240

VA    GGAAAATTTGAAAAATTAATTGTGGCTCTGATGAAACCCTCTCGGCTTTATGATGCTTAT   282
VB    ..C..G.....G.GGC.C........C..T...T.T..GC.ATACAGA..C..A..CA.G   300

VA    GAACTGAAACATGCCTTGAAGGGAGCTGGAACAAATGAAAAAGTACTGACAGAAATTATT   342
VB    ..G...C.TG.C...A.......CTTA.....C..G..GGGT..CA.C.TT..G..CC.G   360

VA    GCTTCAAGGACACCTGAAGAACTGAGAGCCATCAAACAAGTTTATGAAGAAGAATATGGC   402
VB    ..C..TC....CAAGA.CC.G...C.G.AG..A.TGA.G.CG.....G.....C.....G   420

VA    TCAAGCCTGGAAGATGACGTGGTGGGGGACACTTCAGGGTACTACCAGCGGATGTTGGTG   462
VB    ..C........G..G...A.CCAA.CA.....AAGT..C...CTGG..A....CC.....   480

VA    GTTCTCCTTCAGGCTAACAGAGACCCTGATGCTGGAATTGATGAAGCTCAAGTTGAACAA   522
VB    TGC.....G....GC.G...G..TGA..TGAGCA.CT...TG..CCCGGC.C.G.CC.TC   540

VA    GATGCTCAGGCTTTATTTCAGGCTGGAGAACTTAAATGGGGGACAGATGAAGAAAAGTTT   582
VB    C.A.ACGCACAGGATC.GT.T..G.C..GCGAG..GATTC.TGGGAC...T..G.T.AAA   600

VA    ATCACCATCTTTGGAACACGAAGTGTGTCTCATTTGAG...AAAGGTGTTTGACAAGTAC   639
VB    T...T..C.A.CCTGTGCACGC.CAGTG.CAC.CACCTGCTG.GA........AG....T   660
```

FIG. 10/1

```
VA   ATGACTATATCAGGATTTCAAATTGAGGAAACCATTGACCGCGAGACTTCTGGCAATTTA   699
VB   GA..AA..TG.CAACAAGAGC........C.G...CA.GA.T.....CCA....TCAC.G   720

VA   GAGCAACTACTCCTTGCTGTTGTGAAATCTATTCGAAGTATACCTGCCTACCTTGCAGAG   759
VB   ...G.GGCCA.G..CA....G.......GC.CC.A..ACC.C.ACAG....T........   780

VA   ACCCTCTATTATGCTATGAAGGGAGCTGGGACAGATGATCATACCCTCATCAGAGTCATG   819
VB   .GA.....C....CC...........A.....GCG....GGG.....G..A...AA...C   840

VA   GTTTCCAGGAGTGAGATTGATCTGTTTAACATCAGGAAGGAGTTTAGGAAGAATTTTGCC   879
VB   .....A.....C........CT.AAA.CTT....AATGTC.C..C.A.....TG.AC.G.   900

VA   ACCTCTCTTTATTCCATGATTAAGGGAGATACATCTGGGGACTATAAGAAAGCTCTTCTG   939
VB   .AGA.C..CAGCAG......C.T..A...C..CAGC..C.....C.....C..C..G...   960

VA   CTGCTCTGTGGAGAAGATGAC                                          960
VB   AGC..GGTG..CAGC..CCC.                                          981
```

FIG. 10/2

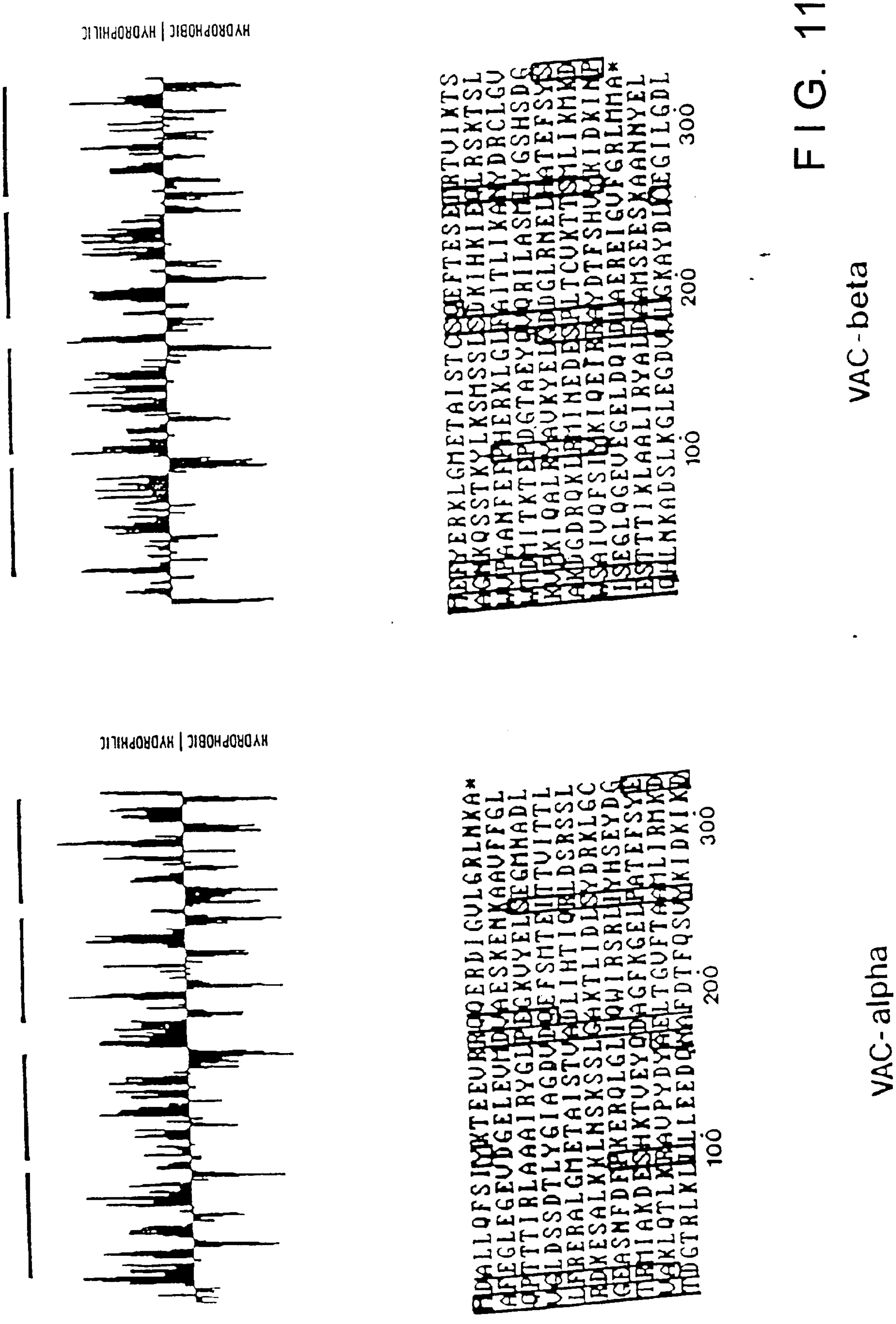
HYDROPHOBIC | HYDROPHILIC
100
200
300
VAC-beta
HYDROPHOBIC | HYDROPHILIC
100
200
300
VAC-alpha

FIG. 11.

Comparison VAC/VAC-beta/Lipocortin I/Lipocortin II

```
VA                             MAQVLRGTVTDFPGFDERA
VB                           MAWWKAWIEQEGVTVKSSSHFNPDP
LCI          MAMVSEFLKQAWFIENEEQEYVQTVKSSKGGPGSAVSPYPTFNPSS
LCII                  MSTVHEILCKLSLEGDHSTPPSAYGSVKPYTNFDAER

VA    DAETLRKAMKGLGTDEESILTLLTSRSNAQRQEISAAFKTLFGRDLLDDLKSELTGKFEKLIVALMK    PSRLY
VB    DAETLYKAMKGIGTNEQAIIDVLTKRSNTQRQQIAKSFKAQFGKDLTETLKSELSGKFERLIVALMY    PPYRY
LCI   DVAALHKAIMVKGVDEATIIDILTKRNNAQRQQIKAAYLQETGKPLDETLKKALTGHLEEVVLALLK    TPAQF
LCII  DALNIETAVKTKGVDEVTIVNILTNRSNVQRQDIAFAYQRRTKKELPSALKSALSGHLETVILGLLK    TPAQY

VA    DAYELKHALKGAGTNEKVLTEIIASRTPEELRAIKQVYEEEYGSSLEDDVVGDTSGYYQRMLVVLLQ    ANRDPDAG-IDEAQVEQ
VB    EAKELHDAMKGLGTKEGVIIEILASRTKNQLREIMKAYEEDYGSSLEEDIQADTSGYLERILVCLLQ    GSRDDVSSFVDPALALQ
LCI   DADELRAAMKGLGTDEDTLIEILASRTNKEIRDINRVYREELKRDLAKDITSDTSGDFRNALLSLAK    GDRSEDFGVNED-LADS
LCII  DASELKASMKGLGTDEDSLIEIICSRTNQELQEINRVYKEMYKTDLEKDIISDTSGDFRKLMVALAK    GRRAEDGSVIDYELIDQ

VA    DAQALFQAGELKWGTDEEKFITIFGTRSVSHLRKVFDKYMTISGFQIEETIDRETSGNLEQLLLAVVK   SIRSIPAY
VB    DAQDLYAAGEKIRGTDEMKFITILCTRSATHLLRVFEEYEKIANKSIEDSIKSETHGSLEEAMLTVVK   CTQNLHSY
LCI   DARALYEAGERRKGTDVNVFNTILTTRSYPQLRRVFQKYTKYSKHDMNKVLDLELKGDIEKCLTAIVK   CATSKPAF
LCII  DARELYDAGVKRKGTDVPKWISIMTERSVCHLQKVFERYKSYSPYDMLESIKKEVKGDLENAFLNLVQ   CIQNKPLY

VA    LAETLYYAMKGAGTDDHTLIRVMVSRSEIDLFNIRKEFRKNFATSLYSMIKGDTSGDYKKALLLLCG    EDD*
VB    FAERLYYAMKGAGTRDGTLIRNIVSRSEIDLNLIKCHFKKMYGKTLSSMIMEDTSGDYKNALLSLVG    SDP*
LCI   FAEKLHQAMKGVGTRHKALIRIMVSRSEIDMNDIKAFYQKMYGISLCQAILDETKGDYEKILVALCG    GN*
LCII  FADRLYDSMKGKGTRDKVLIRIMVSRSEVDMLKIRSEFKRKYGKSLYYYIQQDTKGDYQKALLYLCG    GDD*
```

VA : human VAC-alpha
VB : human VAC-beta
LCI : human Lipocortin I
LCII: human Lipocortin II

FIG. 12.

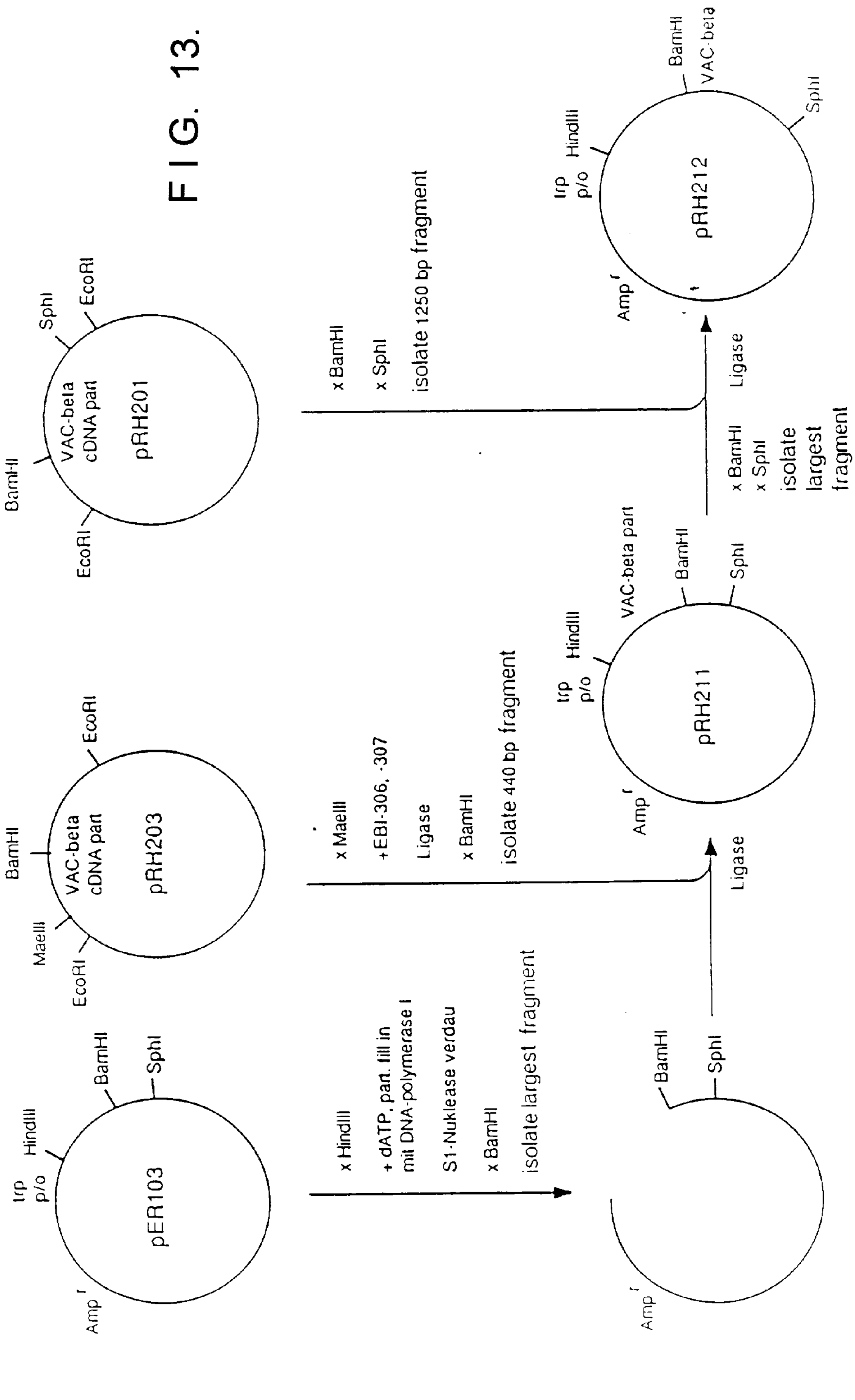
pER103
trp p/o
HindIII
BamHI
SphI
Amp r
x HindIII
+ dATP, part. fill in mit DNA-polymerase I
S1-Nuklease verdau
x BamHI
isolate largest fragment
BamHI
SphI
Amp r
pRH203
VAC-beta cDNA part
BamHI
MaeIII
EcoRI
EcoRI
x MaeIII
+EBI-306, -307
Ligase
x BamHI
isolate 440 bp fragment
Ligase
pRH211
trp p/o
HindIII
VAC-beta part
BamHI
SphI
Amp r
pRH201
VAC-beta cDNA part
BamHI
SphI
EcoRI
EcoRI
x BamHI
x SphI
isolate 1250 bp fragment
x BamHI
x SphI
isolate largest fragment
Ligase
pRH212
trp p/o
HindIII
BamHI
VAC-beta
SphI
Amp r

FIG. 13.

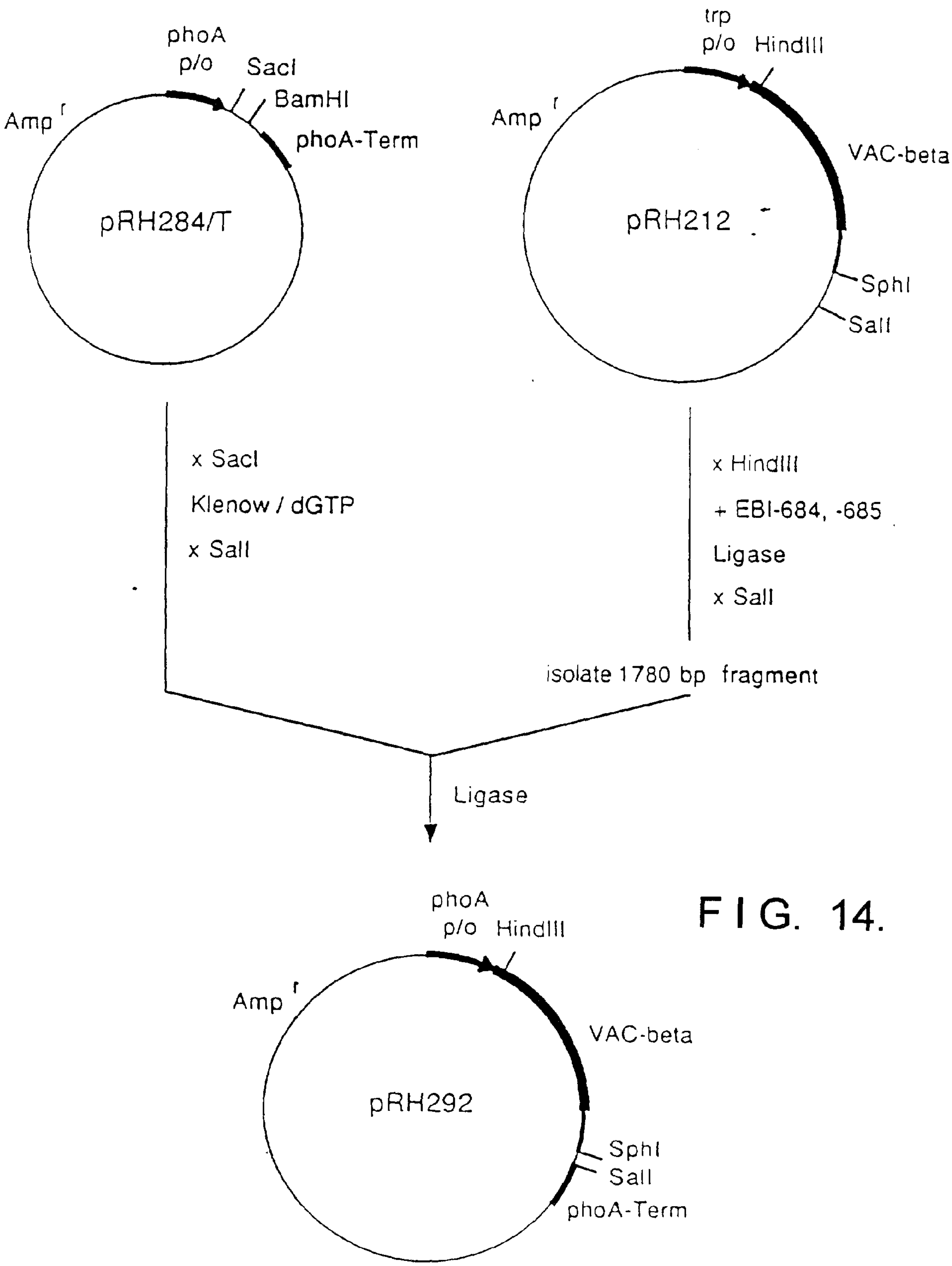
phoA
p/o
SacI
BamHI
Amp r
phoA-Term
pRH284/T
trp
p/o
HindIII
Amp r
VAC-beta
pRH212
SphI
SalI
x SacI
Klenow / dGTP
x SalI
x HindIII
+ EBI-684, -685
Ligase
x SalI
isolate 1780 bp fragment
Ligase
phoA
p/o
HindIII
Amp r
VAC-beta
pRH292
SphI
SalI
phoA-Term

FIG. 14.

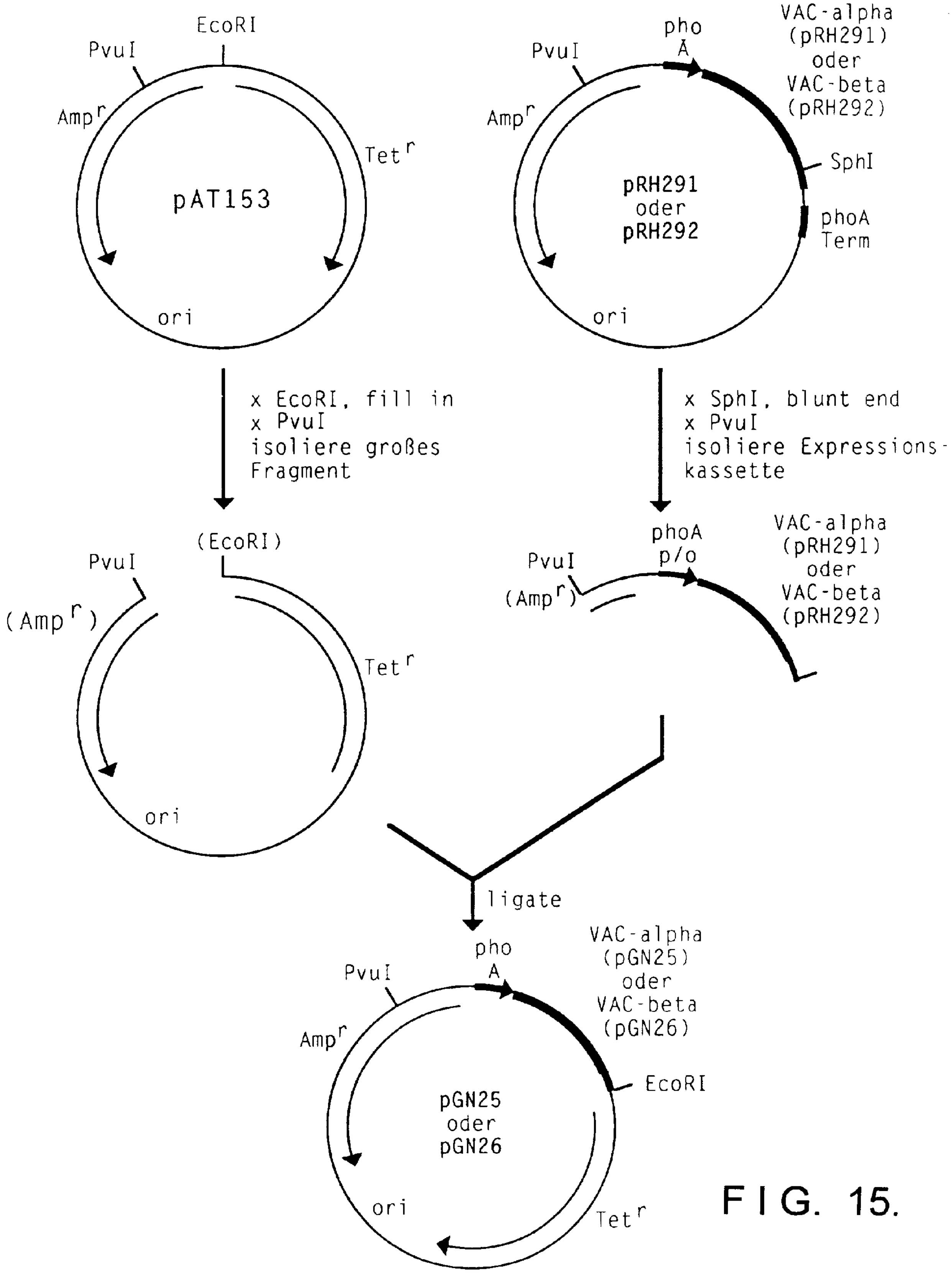
EcoRI
PvuI
Amp^r
Tet^r
pAT153
ori
pho A
VAC-alpha (pRH291) oder VAC-beta (pRH292)
PvuI
Amp^r
SphI
pRH291 oder pRH292
phoA Term
ori
x EcoRI, fill in
x PvuI
isoliere großes Fragment
x SphI, blunt end
x PvuI
isoliere Expressions-kassette
(EcoRI)
PvuI
(Amp^r)
Tet^r
ori
phoA p/o
VAC-alpha (pRH291) oder VAC-beta (pRH292)
PvuI
(Amp^r)
ligate
pho A
VAC-alpha (pGN25) oder VAC-beta (pGN26)
PvuI
Amp^r
EcoRI
pGN25 oder pGN26
ori
Tet^r

FIG. 15.

FIG. 16.

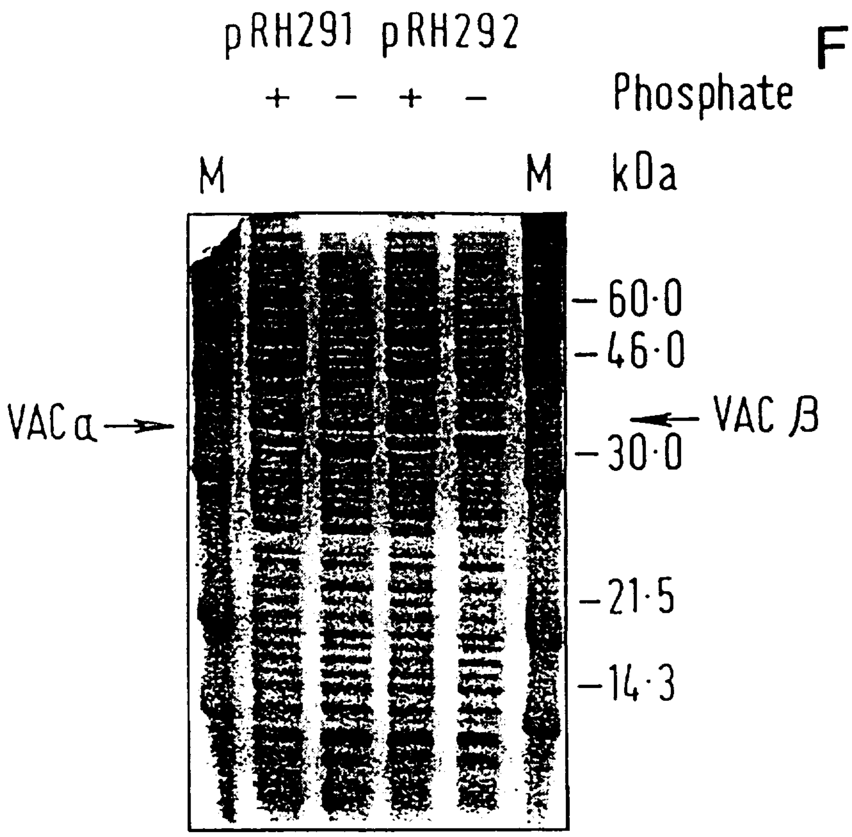
pRH291 pRH292
+ − + −
Phosphate
M M
kDa
60·0
46·0
VAC α
VAC β
30·0
21·5
14·3

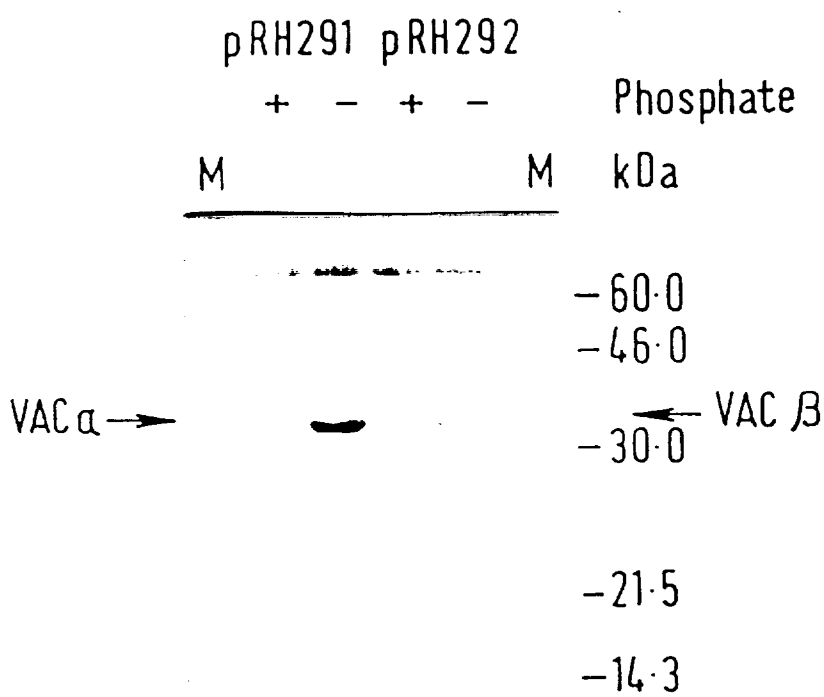
pRH291 pRH292
+ − + −
Phosphate
M M
kDa
60·0
46·0
VAC α
VAC β
30·0
21·5
14·3

F I G. 17.

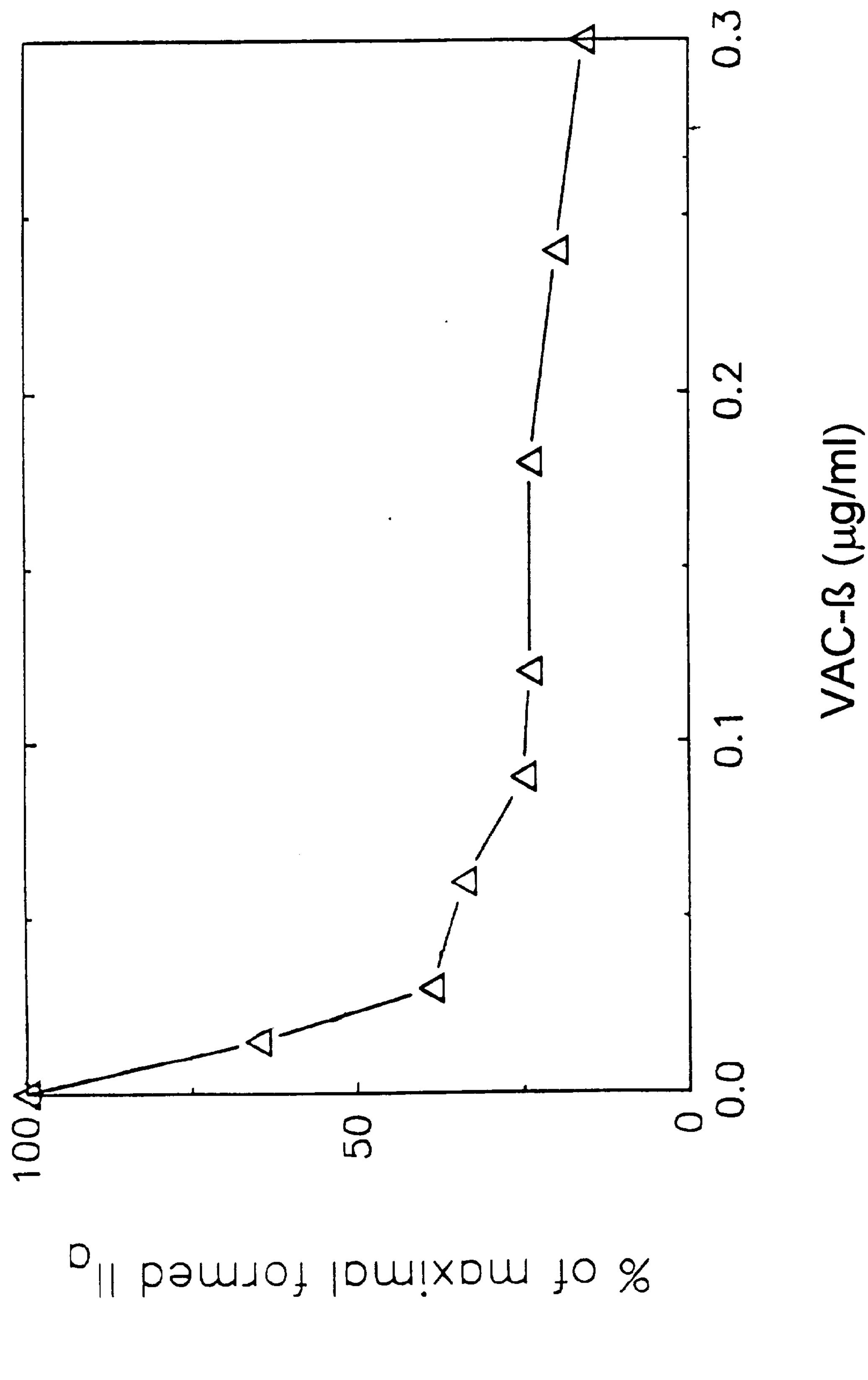

FIG. 18.

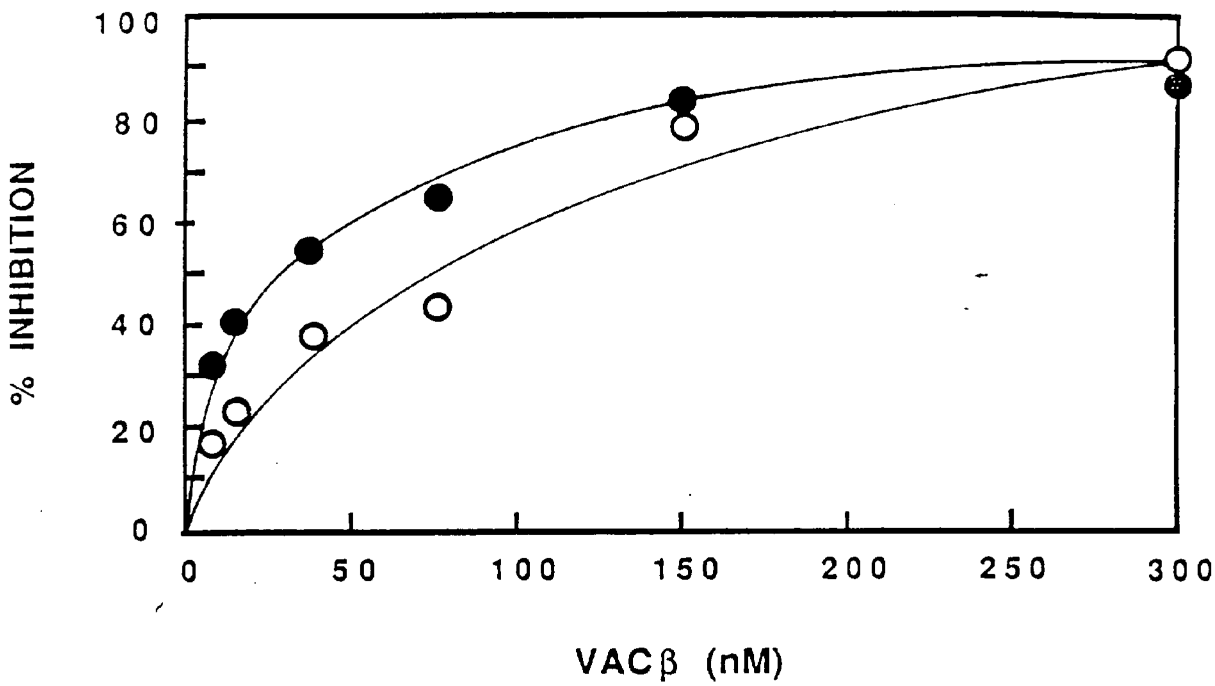

FIG. 19.

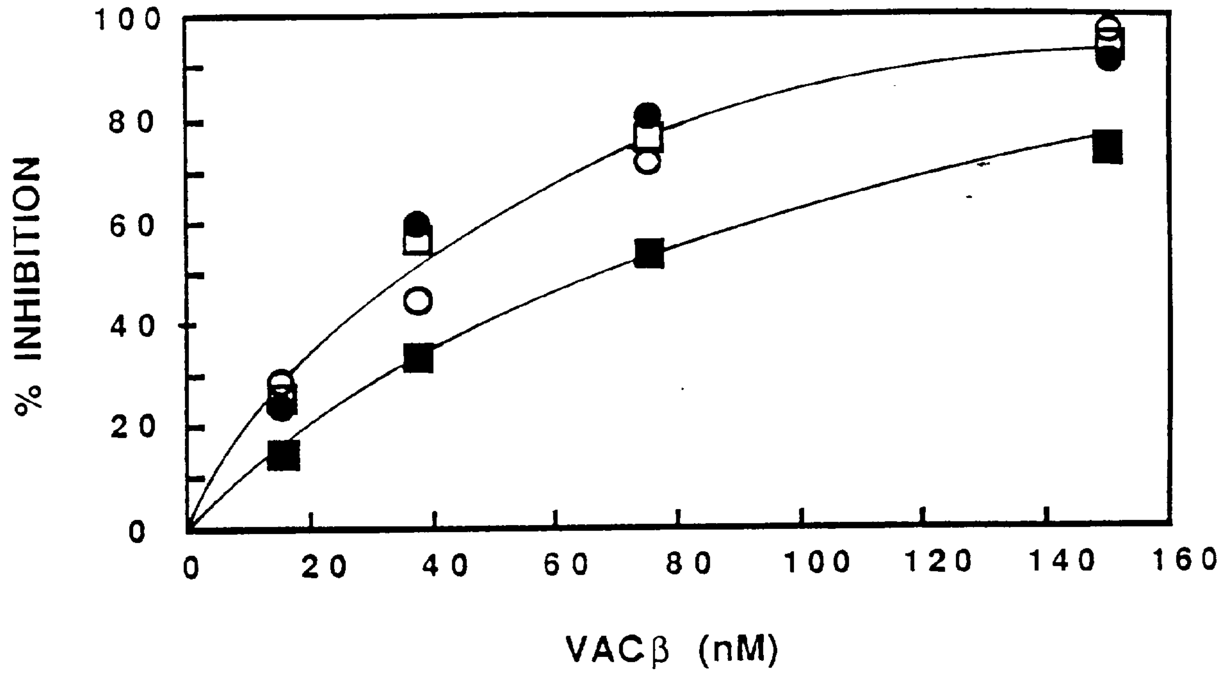

FIG. 20.

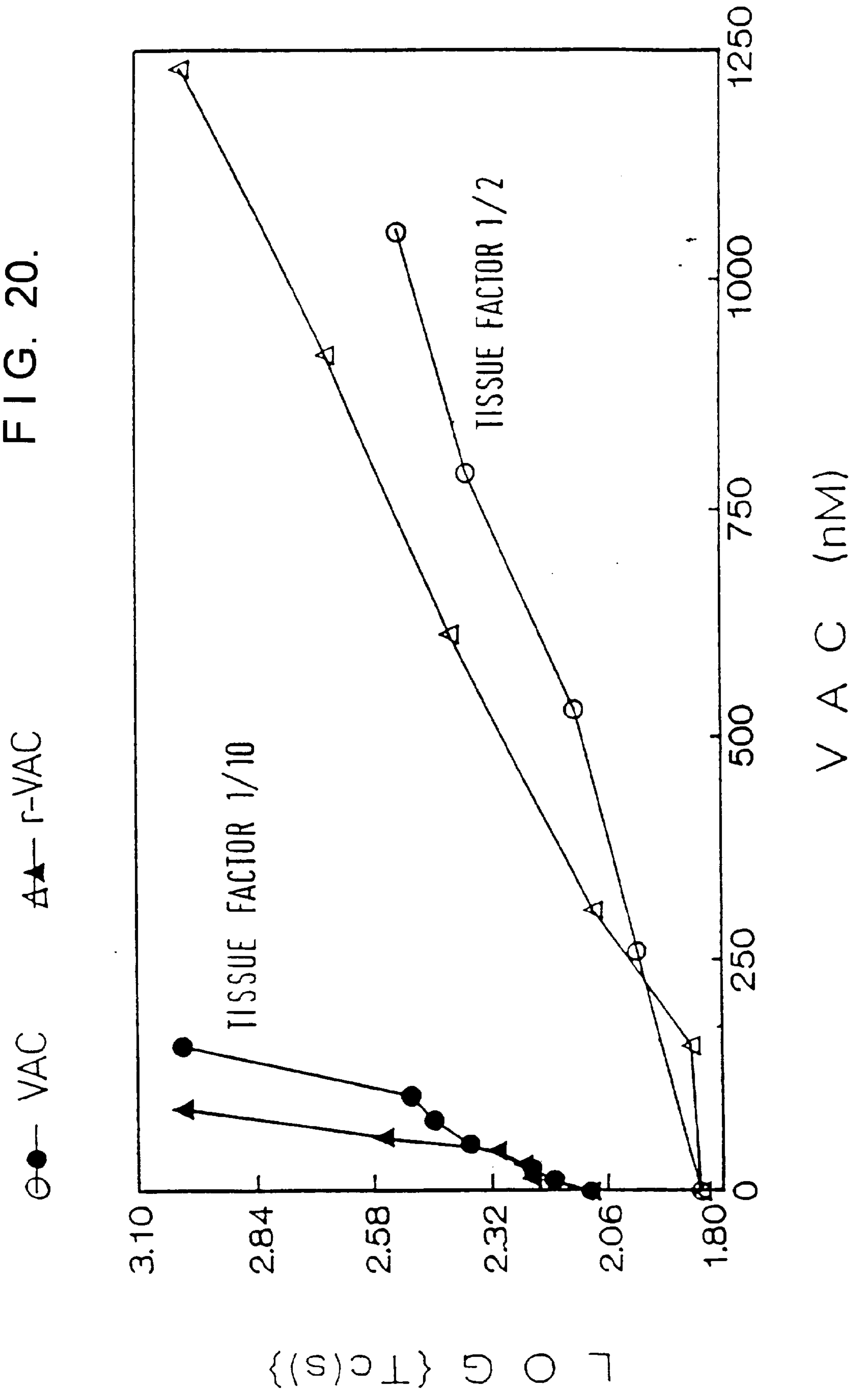
VAC
r-VAC
TISSUE FACTOR 1/10
TISSUE FACTOR 1/2
3.10
2.84
2.58
2.32
2.06
1.80
0
250
500
750
1000
1250
V A C (nM)
L O G {Tc(s)}

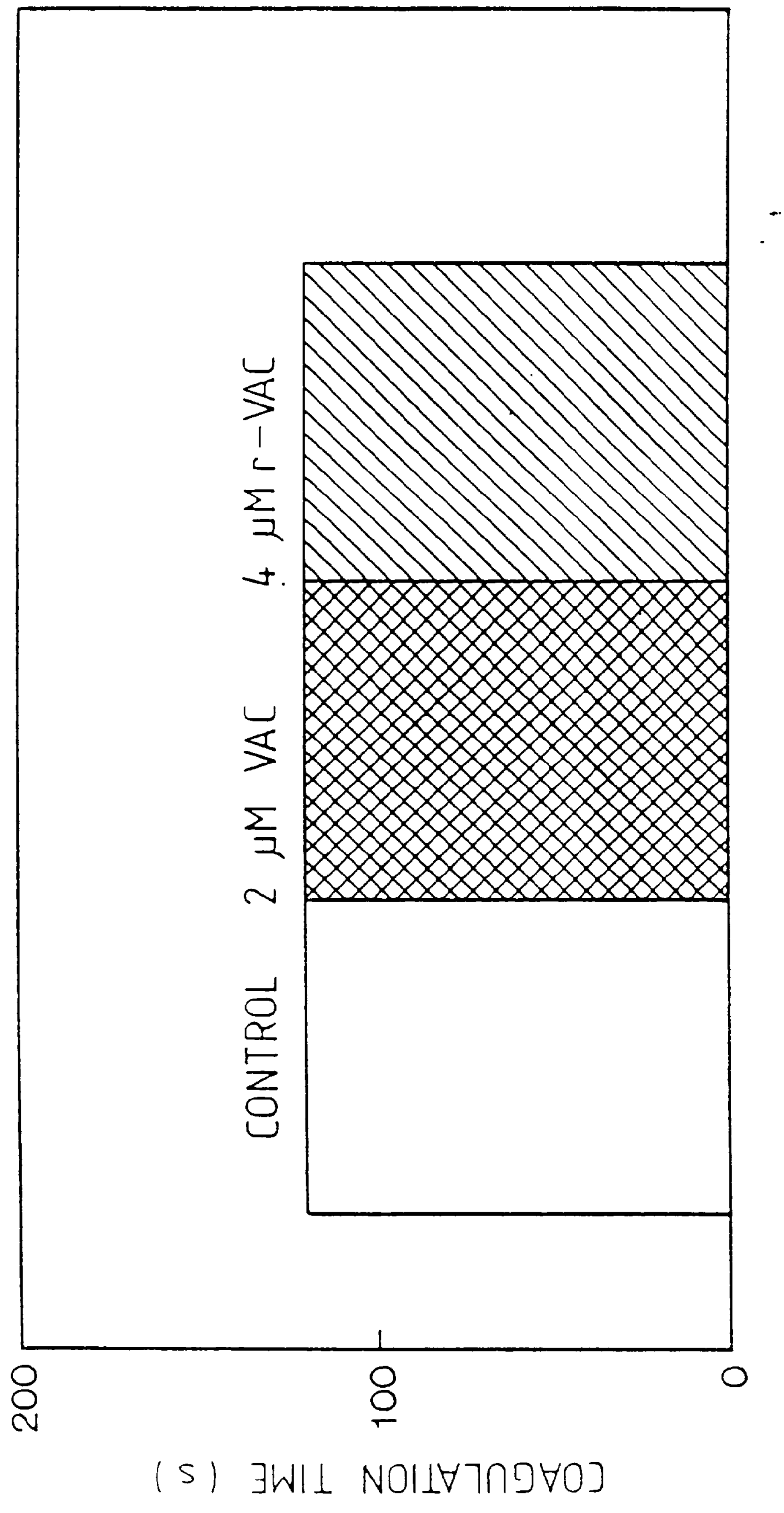


FIG. 21.

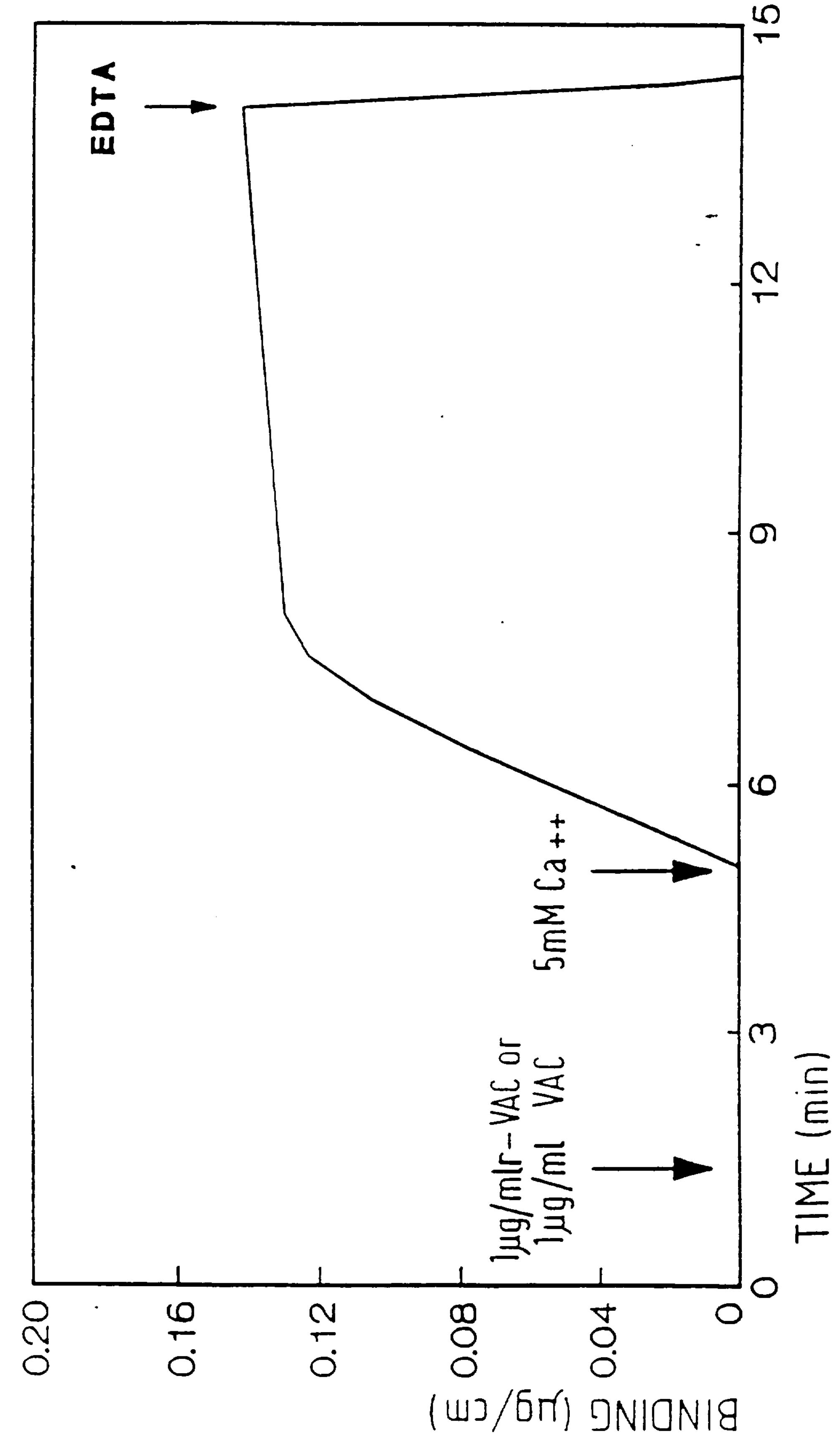
EDTA
5mM Ca ++
1µg/ml r-VAC or
1µg/ml VAC
BINDING (µg/cm)
0.20
0.16
0.12
0.08
0.04
0
TIME (min)
0
3
6
9
12
15

FIG. 22.

FIG. 23.

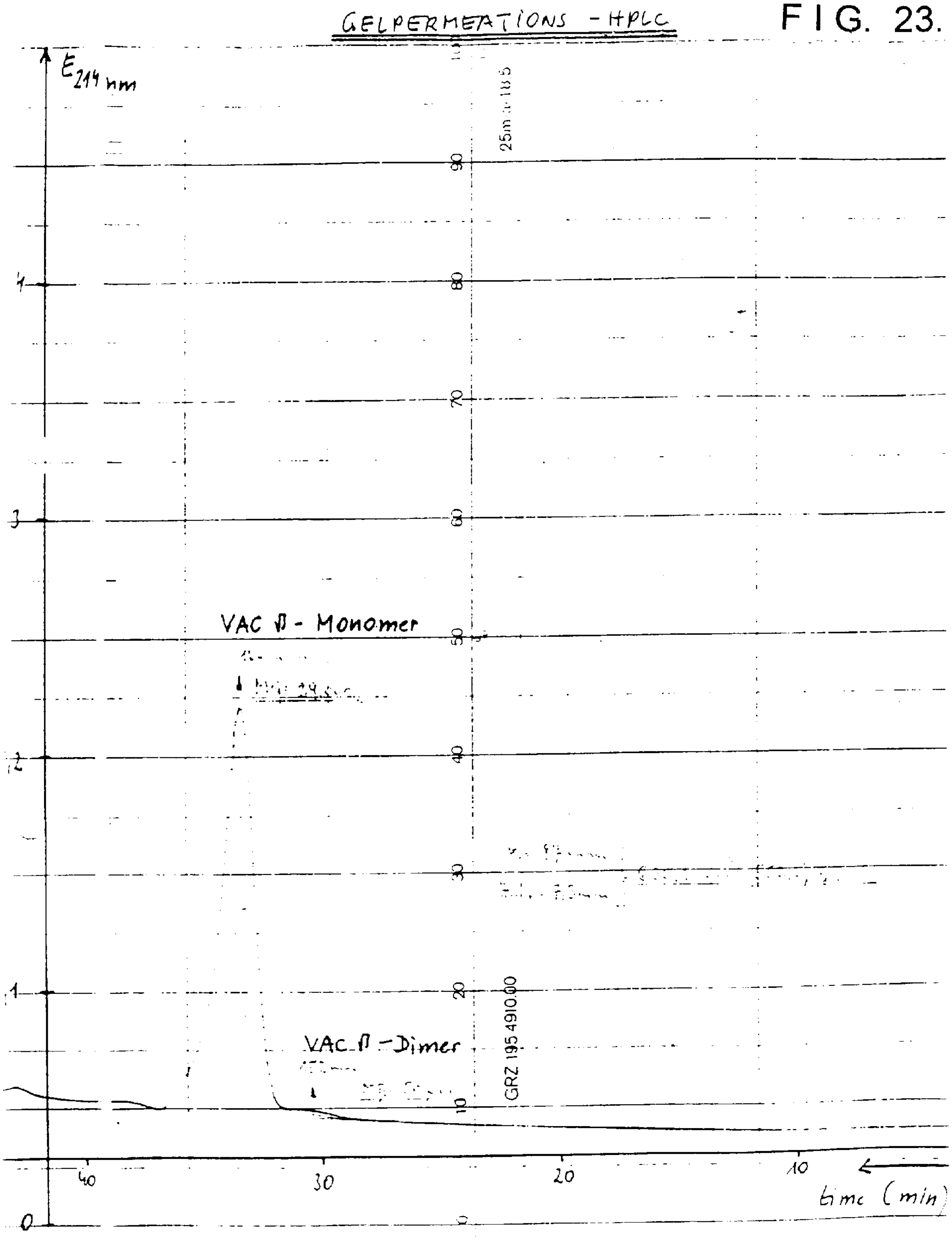
GELPERMEATIONS - HPLC
$E_{214}$ nm
VAC β - Monomer
VAC β - Dimer
time (min)
40
30
20
10
0
1
2
3
4
25mm 185
GRZ 195 4910.00

FIG. 24.

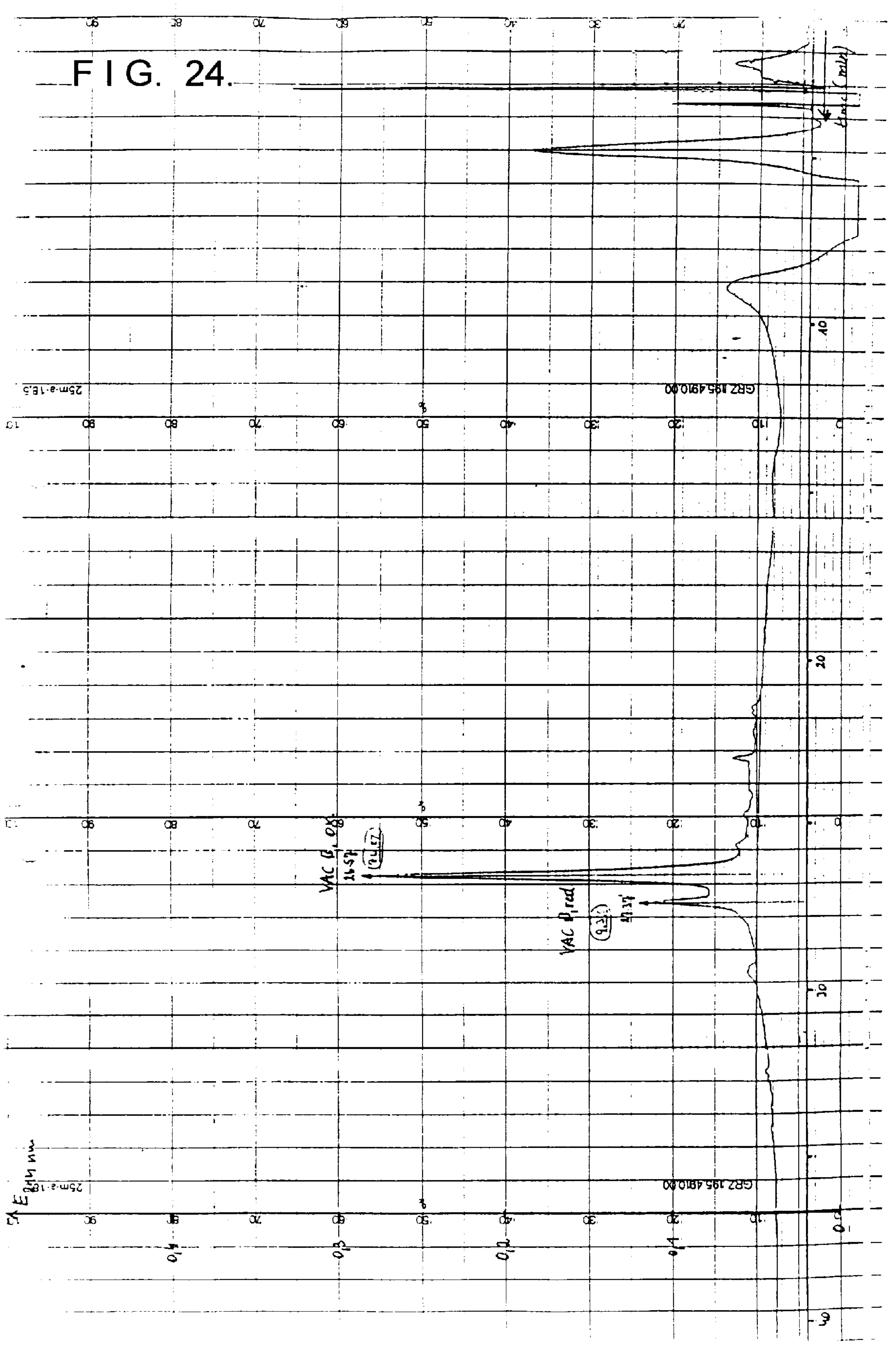

FIG. 25.

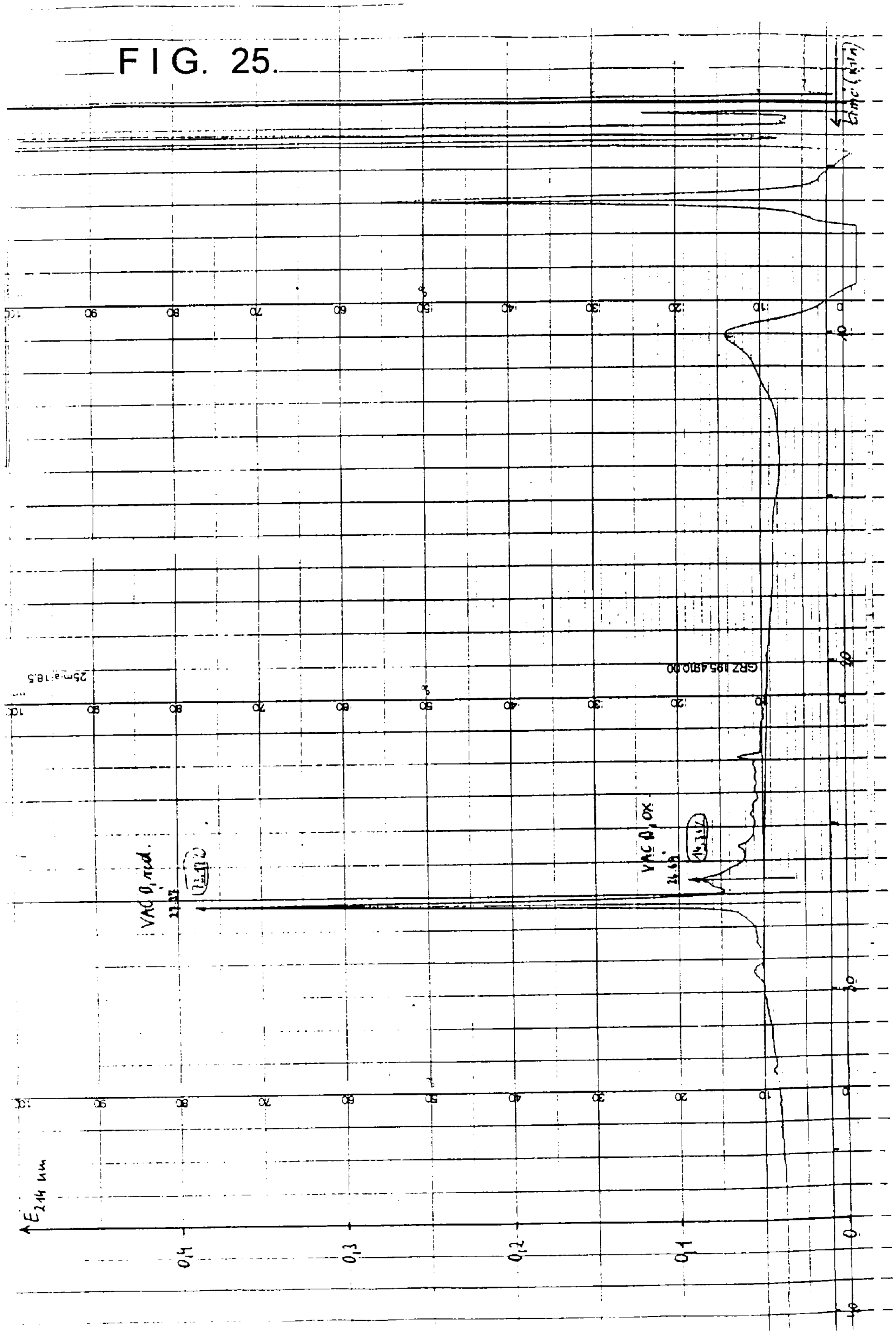

FIG. 26 a

BECKMAN AMINOSAUREANALYSE

01-Feb-89

==============================

PROBE : Svac?

LAUFNUMMER : 22

PROTEIN : VAC "beta"

CHROMATOGRAMM VOM : 21.04.89 3:17:36

INJIZIERTES VOLUMEN : 50 µl

STANDARDWERTE : Durchschnittswerte von Lauf 22

| Name | IST [in nMol] | SOLL | rel IST [ I ] | rel IST [ II ] | rel SOLL |
|---|---|---|---|---|---|
| ASP | 14.07 | 29 | 29.59 | 6.35 | 7.25 |
| THR | 11.15 | 21 | 23.45 | 5.03 | 5.25 |
| SER | 12.14 | 27 | 25.26 | 5.57 | 6.75 |
| GLU | 19.97 | 41 | 41.99 | 9.01 | 10.25 |
| PRO | 5.38 | 6 | 11.31 | 2.43 | 1.50 |
| GLY | 11.94 | 20 | 25.12 | 5.39 | 5.00 |
| ALA | 12.19 | 25 | 25.63 | 5.50 | 6.25 |
| CYS | 2.22 | 4 | 4.66 | 1.00 | 1.00 |
| VAL | 5.36 | 13 | 12.32 | 2.64 | 3.25 |
| MET | 4.61 | 10 | 9.69 | 2.08 | 2.50 |
| ILE | 9.79 | 23 | 20.59 | 4.41 | 5.75 |
| LEU | 15.69 | 33 | 33.00 | 7.08 | 8.25 |
| TYR | 6.55 | 14 | 13.77 | 2.95 | 3.50 |
| PHE | 4.40 | 9 | 9.25 | 1.99 | 2.25 |
| HIS | 2.75 | 6 | 5.79 | 1.24 | 1.50 |
| LYS | 12.56 | 27 | 26.42 | 5.66 | 6.75 |
| NH3 | 18.43 | 0 | 38.77 | 8.31 | 0.00 |
| ARG | 7.23 | 15 | 15.20 | 3.26 | 3.75 |

| Name | SOLL | korr.IST | ger.IST. | ABW | ABW[%] |
|---|---|---|---|---|---|
| ASP | 29 | 29.23 | 29 | + 0.225 | 0.777 |
| THR | 21 | 23.16 | 23 | + 2.159 | 10.283 |
| SER | 27 | 25.64 | 26 | - 1.361 | 5.042 |
| GLU | 41 | 41.46 | 41 | + 0.476 | 1.167 |
| PRO | 6 | 11.17 | 11 | + 5.174 | 86.239 |
| GLY | 20 | 24.81 | 25 | + 4.806 | 24.032 |
| ALA | 25 | 25.31 | 25 | + 0.315 | 1.260 |
| CYS | 4 | 4.61 | 5 | + 0.606 | 15.150 |
| VAL | 13 | 12.17 | 12 | - 0.828 | 6.366 |
| MET | 10 | 9.57 | 10 | - 0.426 | 4.256 |
| ILE | 23 | 20.33 | 20 | - 2.674 | 11.626 |
| LEU | 33 | 32.59 | 33 | - 0.406 | 1.229 |
| TYR | 14 | 13.60 | 14 | - 0.401 | 2.862 |
| PHE | 9 | 9.13 | 9 | + 0.132 | 1.472 |
| HIS | 6 | 5.72 | 6 | - 0.285 | 4.745 |
| LYS | 27 | 26.09 | 26 | - 0.909 | 3.363 |
| NH3 | 0 | 39.29 | 0 | 0.000 | 0.000 |
| ARG | 15 | 15.01 | 15 | + 0.014 | 0.093 |

AS - Sollwert := 323

AS - Istwert := 330

Gesamtabweichung := 21.20 AS / 6.56 %

Durchschnittliche Proteinmenge := 0.510 nanoMol

ALA/LEU - Proteinmenge := 0.482 nanoMol

****************************************************************************

3v acb   1/21/88   9:17:36

FIG. 26 b

| | | | |
|---|---|---|---|
| Acquisition method | ninhec | Quantitation method | [illegible] |
| Units | None | System number | [illegible] |
| Channel | 1 | Vial | [illegible] |
| Injection | -1 | Total injections | [illegible] |
| Run time | 60.00 min | Sample rate | 1 per sec |
| Injection volume | -1 uL | Sample amount | 1.00 |
| Internal standard amt | 1.00 | Scale factor | 1.00 |
| Mode | Analysis | Response factors | Replace |
| Version | REV 4.0 | Channel to calibrate | 1 |
| Description | | | |

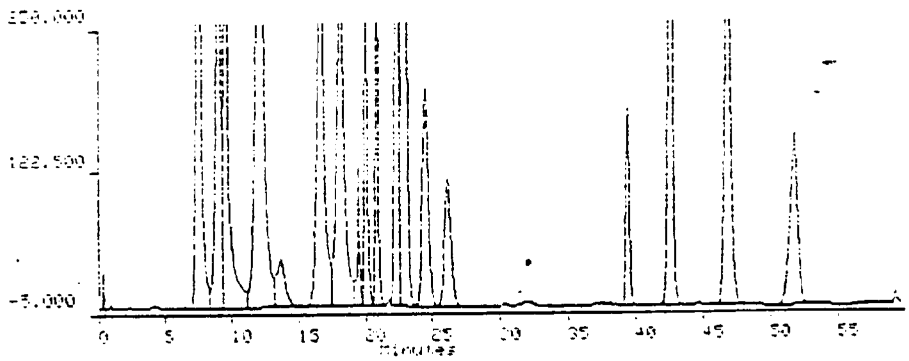


| Peak Name | Ret time | Area | Height | Type | Amount | RF |
|---|---|---|---|---|---|---|
| ASP | 7.67 | 12849108 | 589326 | ** | 10.737 | 1.1967e+06 |
| THR | 8.13 | 9054415 | 353489 | ** | 7.982 | 1.1344e+06 |
| SER | 9.67 | 13162093 | 508590 | ** | 9.327 | 1.4112e+06 |
| GLU | 12.20 | 21594486 | 581113 | ** | 15.972 | 1.3520e+06 |
| PRO | 13.73 | 1915925 | 42433 | ** | 4.469 | 4.2872e+05 |
| GLY | 16.50 | 12516849 | 365263 | ** | 9.017 | 1.3882e+06 |
| ALA | 17.27 | 11631603 | 238150 | ** | 9.421 | 1.2345e+06 |
| CYS | 19.60 | 1605991 | 124559 | ** | 0.864 | 1.8584e+06 |
| VAL | 20.13 | 5026579 | 424565 | ** | 4.445 | 1.1292e+06 |
| MET | 20.93 | 4866735 | 296576 | ** | 3.498 | 1.3911e+06 |
| ILE | 22.47 | 9170982 | 414530 | ** | 7.277 | 1.2603e+06 |
| LEU | 23.07 | 16274567 | 687357 | ** | 11.841 | 1.3744e+06 |
| TYR | 24.60 | 6190710 | 135610 | BB | 4.916 | 1.2593e+06 |
| PHE | 26.33 | 4001320 | 114100 | BB | 3.306 | 1.2105e+06 |
| HIS | 39.53 | 3146100 | 179089 | BB | 2.111 | 1.4904e+06 |
| LYS | 42.73 | 16208683 | 945200 | BB | 9.546 | 1.6980e+06 |
| NH3 | 46.93 | 13233635 | 414991 | BB | 10.856 | 1.2190e+06 |
| ARG | 51.87 | 6455633 | 155583 | BB | 5.202 | 1.2409e+06 |

FIG 27.

PROBE: 3.VAC /S * S200 * FR 54-58.

SEQUENCER PROGRAMM: 38APTH  ABBAUSCHRITTE: 1+38  PTH-AS-HPLC BIS ABBAUSCHRITT NR.: 38  PTH-AS NACHWEISBAR BIS ABBAUSCHRITT NR.: 38

36µg [~ Inmol] PROBE GELÖST IN 75 µL 0.1% TFA ; 3×25 µL AM SEQUENCER AUFGETRAGEN.

SEQUENZ:

| | | 1 | 2 | 3 | 4 | 5 | 6 | 7 | 8 | 9 | 10 |
|---|---|---|---|---|---|---|---|---|---|---|---|
| AS: 1-10 | I. | $X_{ALA}$ 1) | TRP | TRP | LYS | $X_{ALA}$ 1) | TRP | ILE | (GLU) 2) | GLN | (GLU) 2) |
| AS: 11-20 | I. | GLY | VAL | THR | VAL | LYS | SER | SER | SER | HIS 3) | (PHE) |
| AS: 21-30 | I. | ASN | PRO | (ASP) 4) | PRO | (ASP) | $X_{ALA}$ 1) | $X_{GLU}$ | THR | LEU | TYR |
| AS: 31-40 | I. | LYS | $X_{ALA}$ 1) | MET | (LYS) | GLY | ILE | GLY | (THR) | ASN | |

1) PTH-ALA NACHWEIS GESTÖRT DURCH DMPTU (BIS ZU 12 000 000 IU (AS 8) ≙ 10 nmol PTH-AS) [SEQUENCER-DEFEKT!]
2) RETENTIONSZEITEN VERSCHOBEN VON 1.85' AUF 2.45'
3) ——— " ——— 5.50' AUF 4.10'
4) ——— " ——— 1.66 AUF 1.87'

MENGENABSCHÄTZUNG:

| KOMPO-NENTE | AMINOSÄURE VON | BIS | MENGE [nmol] | ANTEIL [%] |
|---|---|---|---|---|
| I | 1 | (38) | ~0.45 | / |
| II | | | | |
| III | | | | |
| IV | | | | |
| V | | | | |
| VI | | | | |
| VII | | | | |
| VIII | | | | |

GESAMTMENGE: ~0.45 nmol
(BERECHNET AUS ILE 7-8, 80%, R.Y.)

VERGLEICHE MIT: 1240

SDS - Gelelektrophorese

VACβ - Standard vergleich

FIG. 28.

12,5% SDS - PAGE

Coomassiefärbung

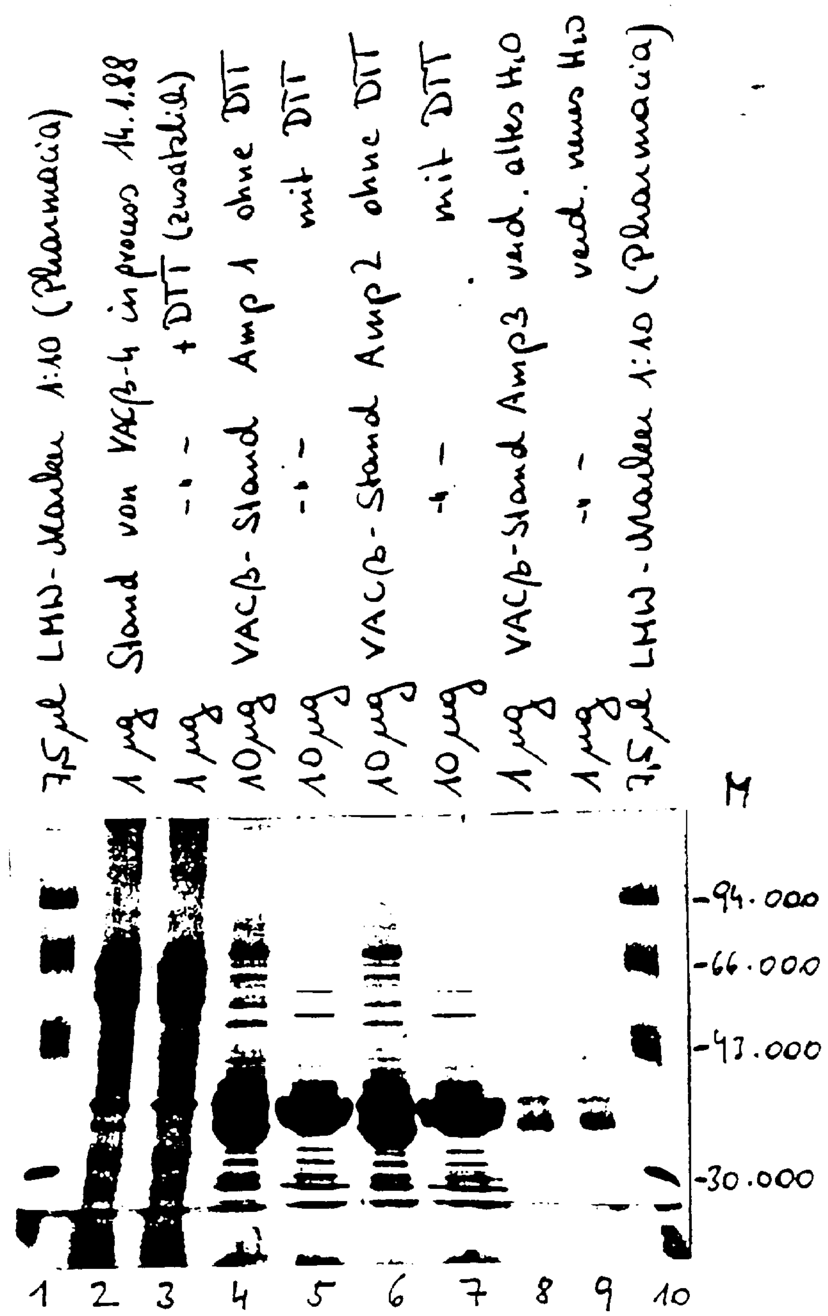

FIG. 29a

ISOELEKTRISCHE FOKUSSIERUNG (PAGIF)

pH - Bereich: 3.5-9.5
Platten-Fabrikat : LKB-PAGplate

Elektrodenlösungen: Anode 1 M Phosphorsäure
Kathode 1 M Natronlauge

Laufzeit: Präfokussierung 500 Vh
Fokussierung 3000 Vh

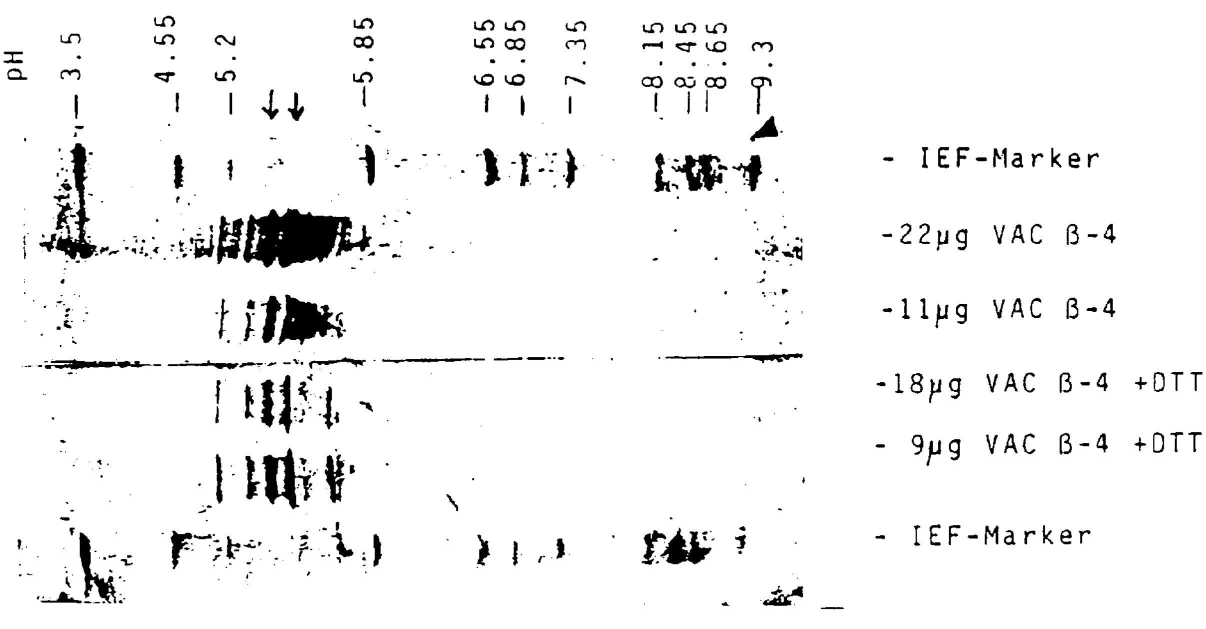


VAC ß-4 : 1. Hauptbande pI 5.35
2. Hauptbande pI 5.45 + DTT

FIG. 29 b

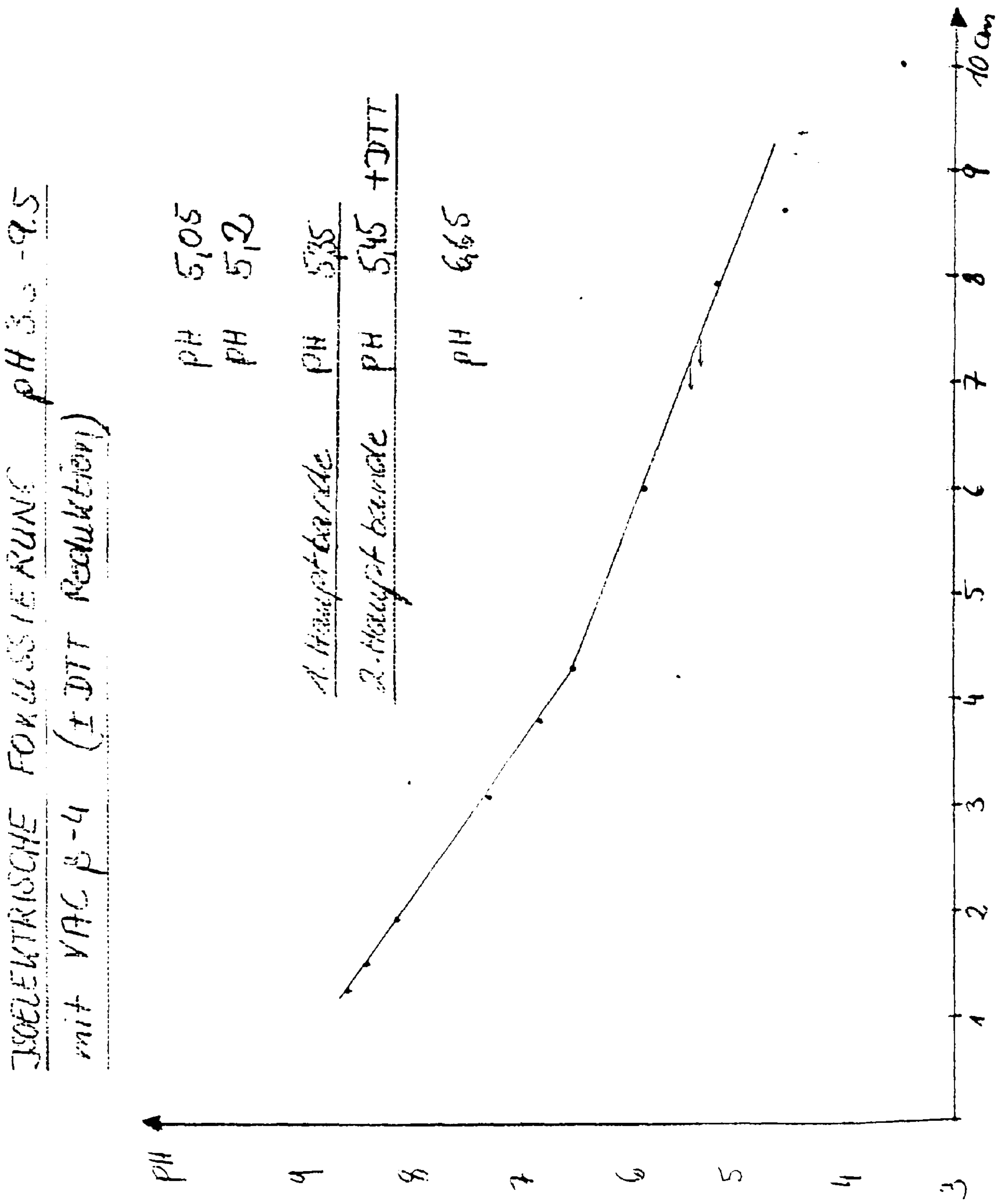
ISOELEKTRISCHE FOKUSSIERUNG
mit VAC β-4 (± DTT Reduktion)
pH 5,05
pH 5,2
1. Hauptbande pH 5,35
2. Hauptbande pH 5,45 +DTT
pH 6,65
pH
10 cm

VACβ-4

F I G. 30

12,5% SDS-PAGE

Coomassiefärbung

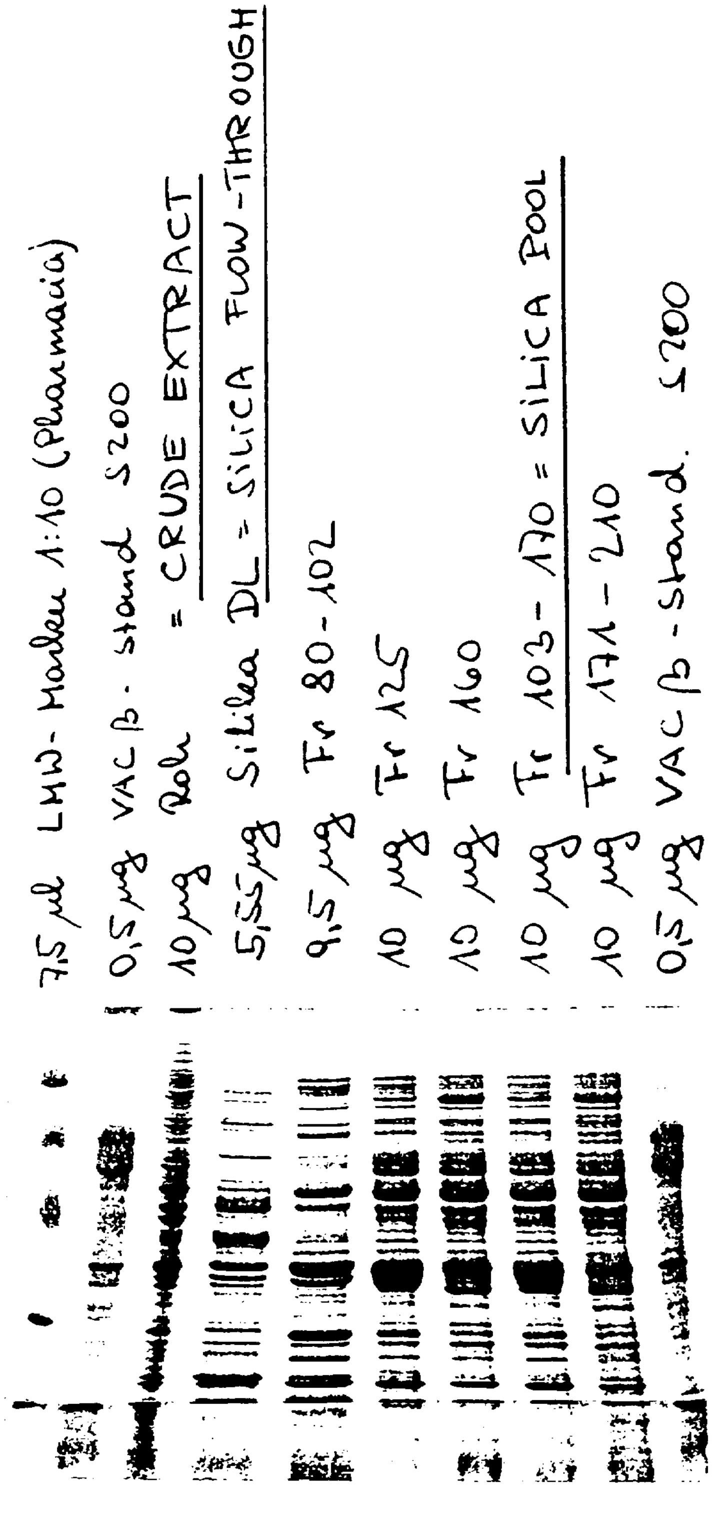

FIG. 31

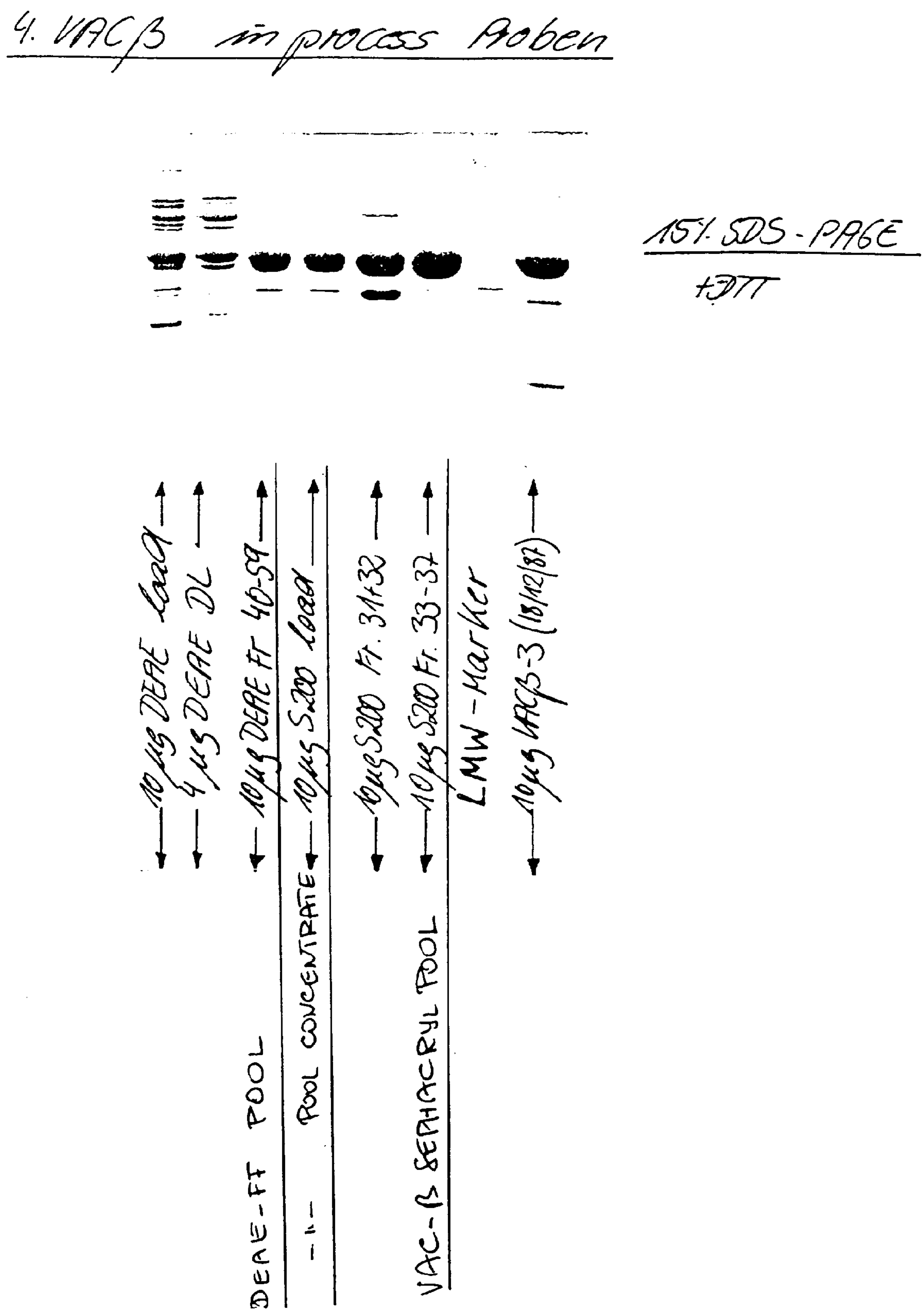
4. VACβ in process Proben
15% SDS-PAGE
+DTT
10 µg DEAE load
4 µg DEAE DL
10 µg DEAE Fr 40-59
10 µg S200 load
10 µg S200 Fr. 31+32
10 µg S200 Fr. 33-37
LMW-Marker
10 µg VACβ-3 (18/12/87)
DEAE-FF POOL
POOL CONCENTRATE
VAC-β SEPHACRYL POOL

Sequenced VAC-peptides:comparison with sequence

```
                                                          BrCN 22
                                                          K G L G T D
           P18                                              P29/I
           G T V T D F P G F D E R                          G L G T D
M A Q V L R G T V T D F P G F D E R A D A E T L R K A M K G L G T D
                10                  20                  30

E E S I L T L L T S X X N A Q
                              P14             P15      -P27
E E S I L T L L T S R         Q E I S A A F K T L F G R D L L
E E S I L T L L T S R S N A Q R Q E I S A A F K T L F G R D L L
        40                  50                  60

                                    BrCN 15
                                    K P S R L Y D A Y E L K H
                                          P16/II
                                          W?L Y D A Y E L K
                          P20/II            P16/I            P5
D D L K S E L T G K F E K L I V A L M K       L Y D A Y E L K H
D D L K S E L T G K F E K L I V A L M K P S R L Y D A Y E L K H
    70`                 80                  90

A L K G A G T N E K V L T E I I
                    P17               P11/II            P20/I
A L K               V L T E I I A S R T P E E L R       Q V Y E
A L K G A G T N E K V L T E I I A S R T P E E L R A I K Q V Y E
100                 110                 120                 130

                                          P29/II
E E Y G S S L E D D V V G D T S G Y Y Q R M L V V L L Q A N R D
E E Y G S S L E D D V V G D T S G Y Y Q R M L V V L L Q A N R D
                140                 150                 160

                                                  P11/I
                                                  W G T D E E K P23/I
                                                  P25           F
P D A G I D E A Q V X Q X A Q A L F Q A           S?G T D E E K F
P D A G I D E A Q V E Q D A Q A L F Q A G E L K W G T D E E K F
            170                 180                 190

I T I F G T R
I T I F G T
I T I F G T R S V S H L R K V F D K Y M T I S G F Q I E E T I D
        200                 210                 220
```

F I G. 32/1

```
  P30/II                              P30/I           -
  E T X G N L E Q L L L A V V K       E I P A Y L A E T L Y Y A
R E T S G N L E Q L L L A V V K S I R S I P A Y L A E T L Y Y A
    230                 240                 250

  BrCN 1
  K G A G T D D H T L I R V
   P12                             P21/I                     P24
M K G A G T D D H T L I R          S E I D L F N I R K         K
M K G A G T D D H T L I R V M V S R S E I D L F N I R K E F R K
260                 270                 280                 290

                BrCN 4
                I K G D T S G D Y K K A
                    P7
N F A T S L Y S M I K G E T S G D Y K
N F A T S L Y S M I K G D T S G D Y K K A L L L L C G E D D *
                300                 310                 320
```

F I G. 32/2

# FIG. 33.

VAC-alpha: Quadruplication of a 67 amino acid long sequence

```
                                                            MAQVLRGTVTDFPGFDERA

DAETLRKAMKG-LGTDEESILTLLTSRSNAQRQEISAAFKTLFGRDLLDDLKSELTGKFEKLIVALMK   PSRLY

DAYELKHALKG-AGTNEKVLTEIIASRTPEELRAIKQVYEEEYGSSLEDDVVGDTSGYYQRMLVVLLQ   ANRDPDAGIDEAQVI

DAQALFQAGELKWGTDEEKFITIFGTRSVSHLRKVFDKYMTISGFQIEETIDRETSGNLEQLLLAVVK   SIRSIPAY

LAETLYYAMKG-AGTDDHTLIRVMVSRSEIDLFNIRKEFRKNFATSLYSMIKGDTSGDYKKALLLLCG   EDD*

      KG-hGTDExxLIpILApR                                        oooooo

DAetL  AmKG aGTDEe lltl  SRS   Lr I   y t fG sLeddlkgeTSG yeklLval k
                                                     d          l
```

"Consensus":  x : 50% of amino acids identical
X : 75% of amino acids identical
X : 100% of amino acids identical

"oooooo" :  Hydrophobic region

Sequence of the alkaline phosphatase gene component in plasmid pRH284T

```
        10        20        30        40        50        60
AATTGGAGATTATCGTCACTGCAATGCTTCGCAATATGGCGCAAAATGACCAACAGCGGT
    CCTCTAATAGCAGTGACGTTACGAAGCGTTATACCGCGTTTTACTGGTTGTCGCCA

        70        80        90       100       110       120
TGATTGATCAGGTAGAGGGGGCGCTGTACGAGGTAAAGCCCGATGCCAGCATTCCTGACG
ACTAACTAGTCCATCTCCCCCGCGACATGCTCCATTTCGGGCTACGGTCGTAAGGACTGC

       130       140       150       160       170       180
ACGATACGGAGCTGCTGCGCGATTACGTAAAGAAGTTATTGAAGCATCCTCGTCAGTAAA
TGCTATGCCTCGACGACGCGCTAATGCATTTCTTCAATAACTTCGTAGGAGCAGTCATTT

       190       200       210       220       230       240
AAGTTAATCTTTTCAACAGCTGTCATAAAGTTGTCACGGCCGAGACTTATAGTCGCTTTG
TTCAATTAGAAAAGTTGTCGACAGTATTTCAACAGTGCCGGCTCTGAATATCAGCGAAAC

       250       260       270       280       290       300
TTTTTATTTTTTAATGTATTTGCTCGAGAGGTTGAGGTGATTTTATGAGCTCGAATTCAT
AAAAATAAAAAATTACATAAACGAGCTCTCCAACTCCACTAAAATACTCGAGCTTAAGTA
                        XhoI       RBS          SacI  EcoRI C

       310       320       330       340       350
CGATAAGCTTGGATCCGTCGACCGCGCCCGGCAGTGAATTTTCGCTGCCGGGTGGTTTTT
GCTATTCGAACCTAGGCAGCTGGCGCGGGCCGTCACTTAAAAGCGACGGCCCACCAAAAA
laI HindIIIBamHI SalI

       360       370
TTGCTGC
AACGACGAGCT
```

FIG. 34.

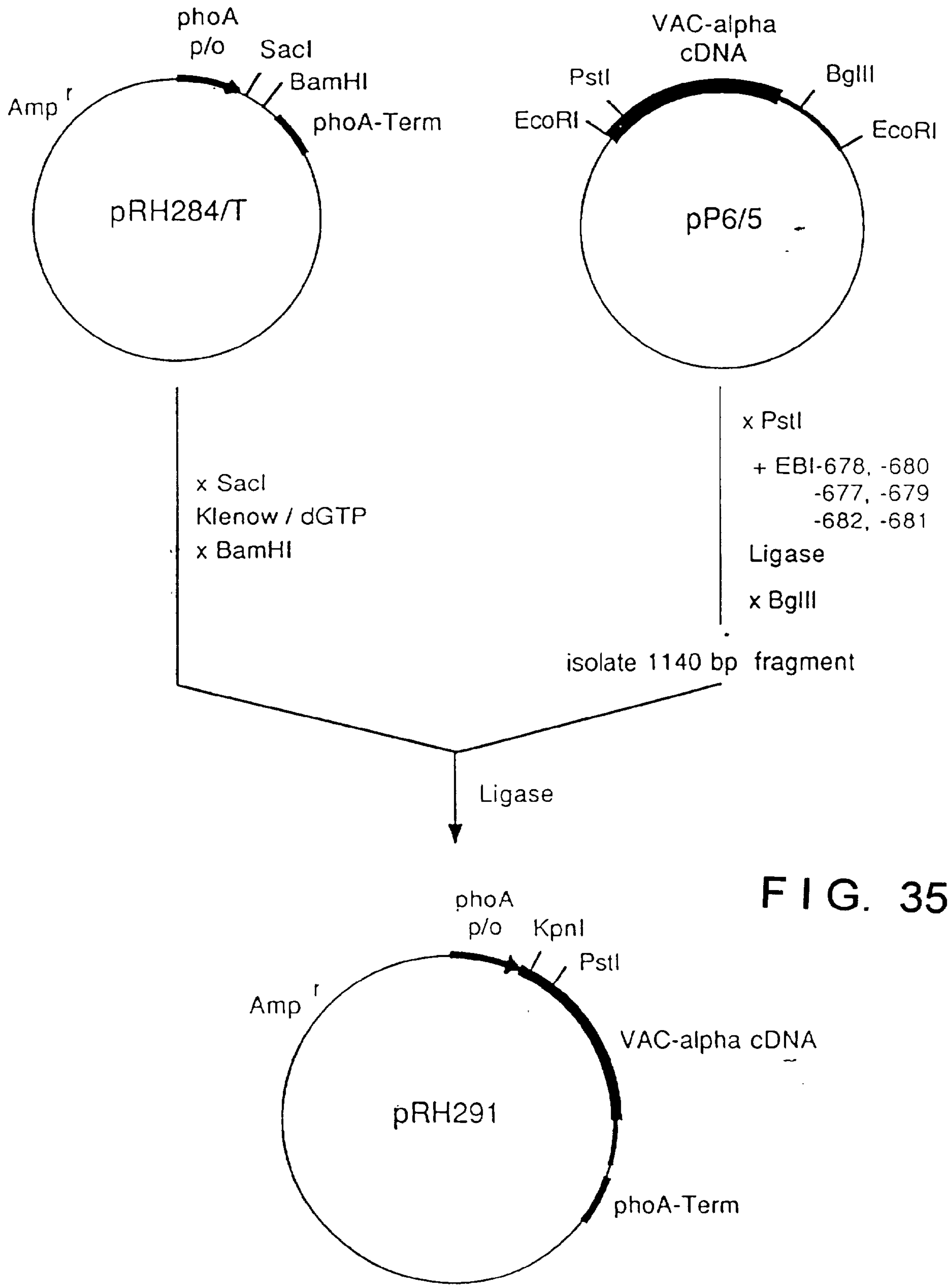
phoA
p/o
SacI
BamHI
Amp r
phoA-Term
pRH284/T
VAC-alpha
cDNA
PstI
BglII
EcoRI
EcoRI
pP6/5
x SacI
Klenow / dGTP
x BamHI
x PstI
+ EBI-678, -680
-677, -679
-682, -681
Ligase
x BglII
isolate 1140 bp fragment
Ligase
phoA
p/o
KpnI
PstI
Amp r
VAC-alpha cDNA
pRH291
phoA-Term

FIG. 35.

FIG. 36.

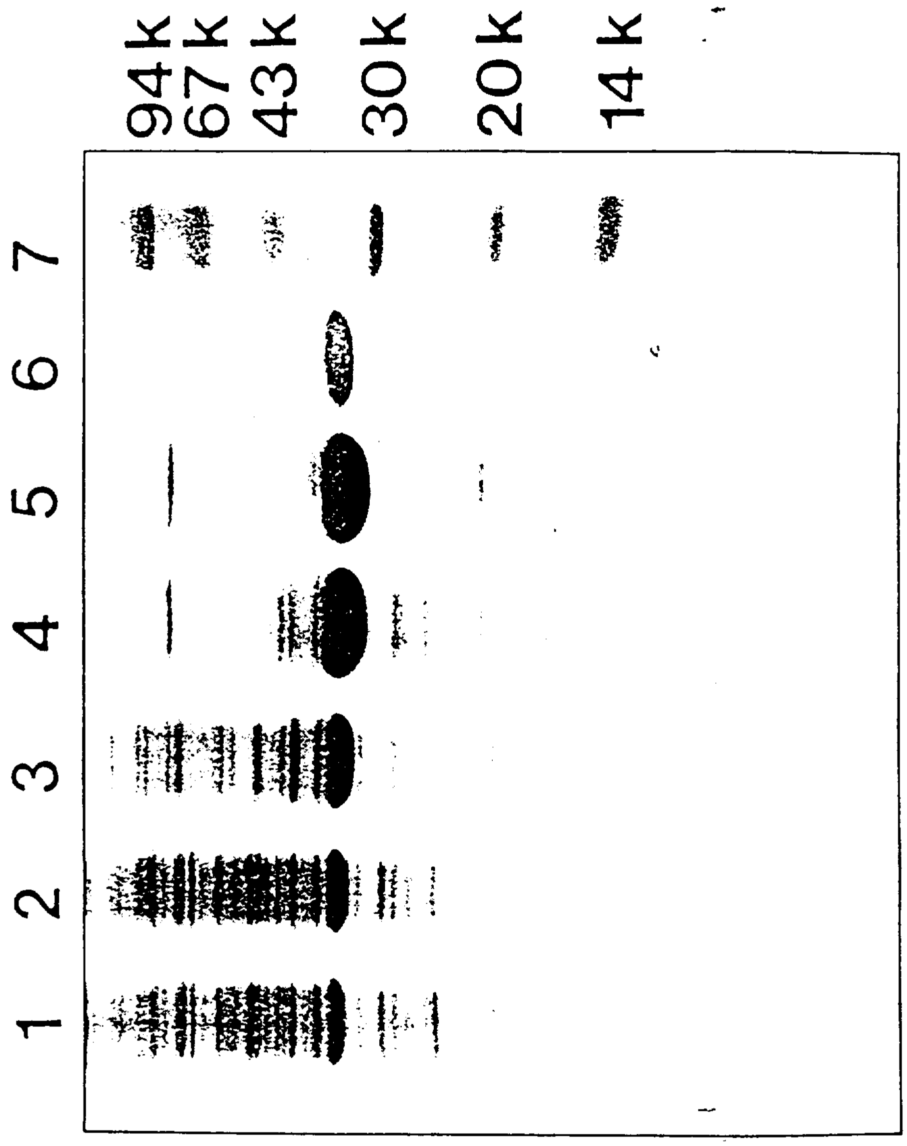

FIG. 37.

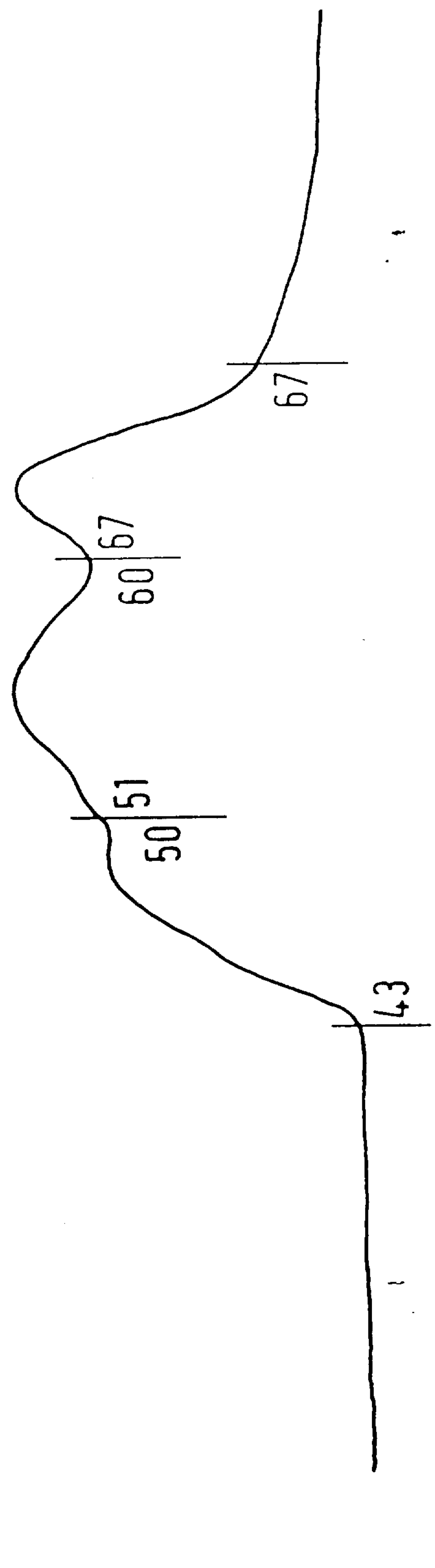

FIG. 38.

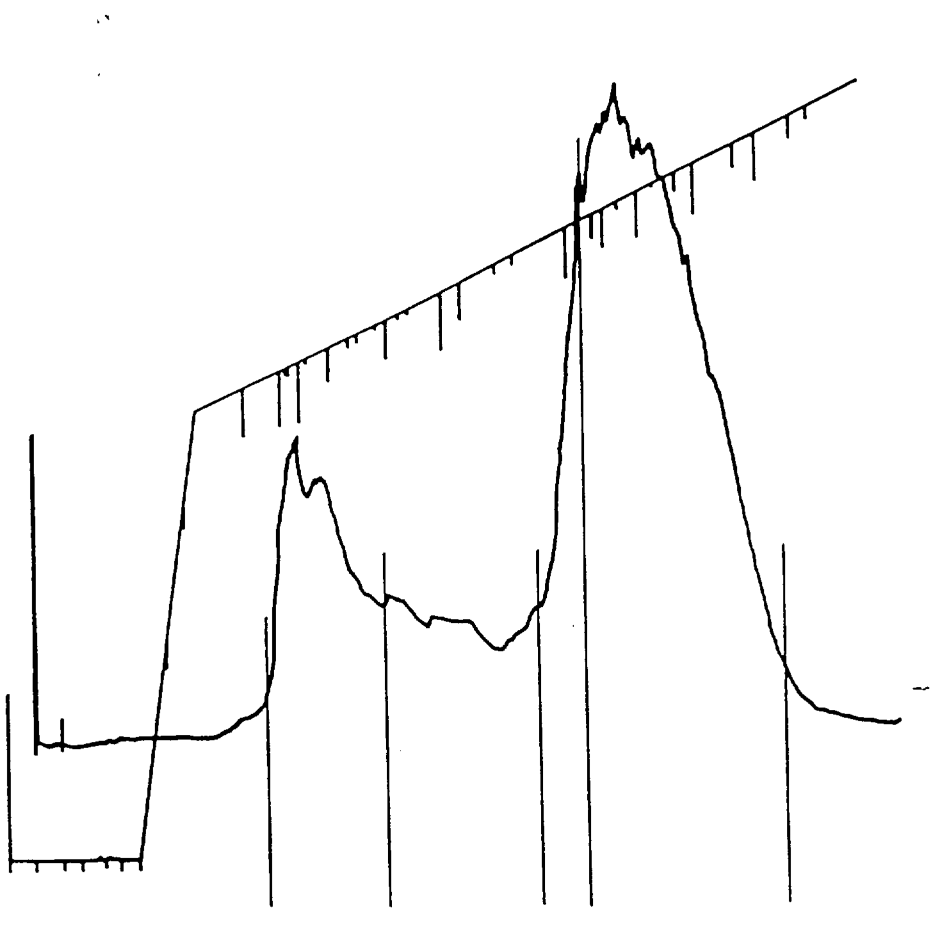

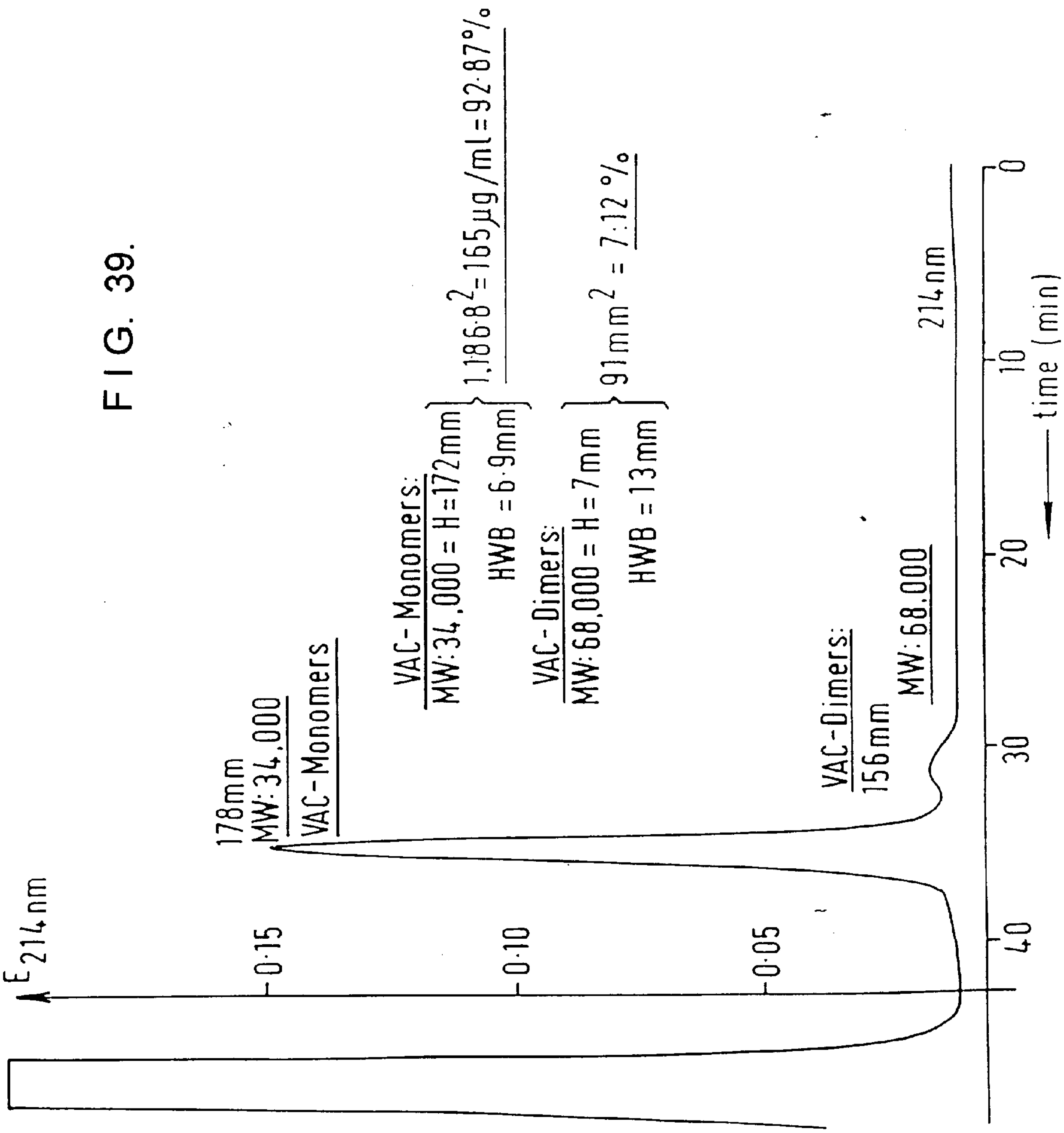
E 214 nm
0·15
0·10
0·05
178mm
MW: 34,000
VAC-Monomers
VAC-Monomers:
MW:34,000 = H = 172mm
HWB = 6·9mm
1,186·8² = 165µg/ml = 92·87%
VAC-Dimers:
MW:68,000 = H = 7mm
HWB = 13mm
91mm² = 7·12%
VAC-Dimers:
156mm
MW: 68,000
214nm
40
30
20
10
0
time (min)

FIG. 39.

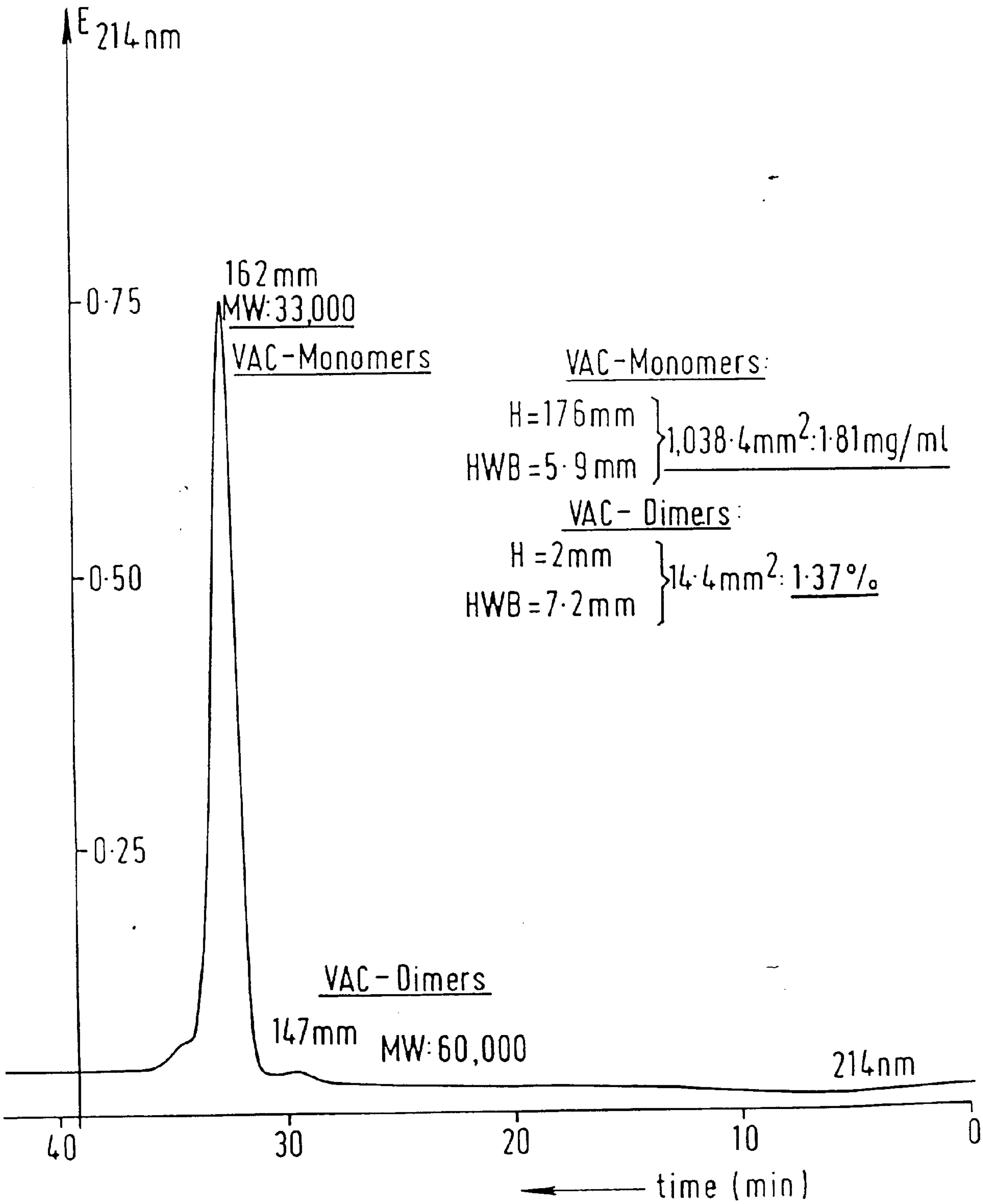
E 214nm
0·75
0·50
0·25
162mm
MW:33,000
VAC-Monomers
VAC-Monomers:
H=176mm
HWB=5·9mm
1,038·4mm2:1·81mg/ml
VAC- Dimers:
H=2mm
HWB=7·2mm
14·4mm2: 1·37%
VAC-Dimers
147mm
MW:60,000
214nm
40
30
20
10
0
time (min)

FIG. 40.

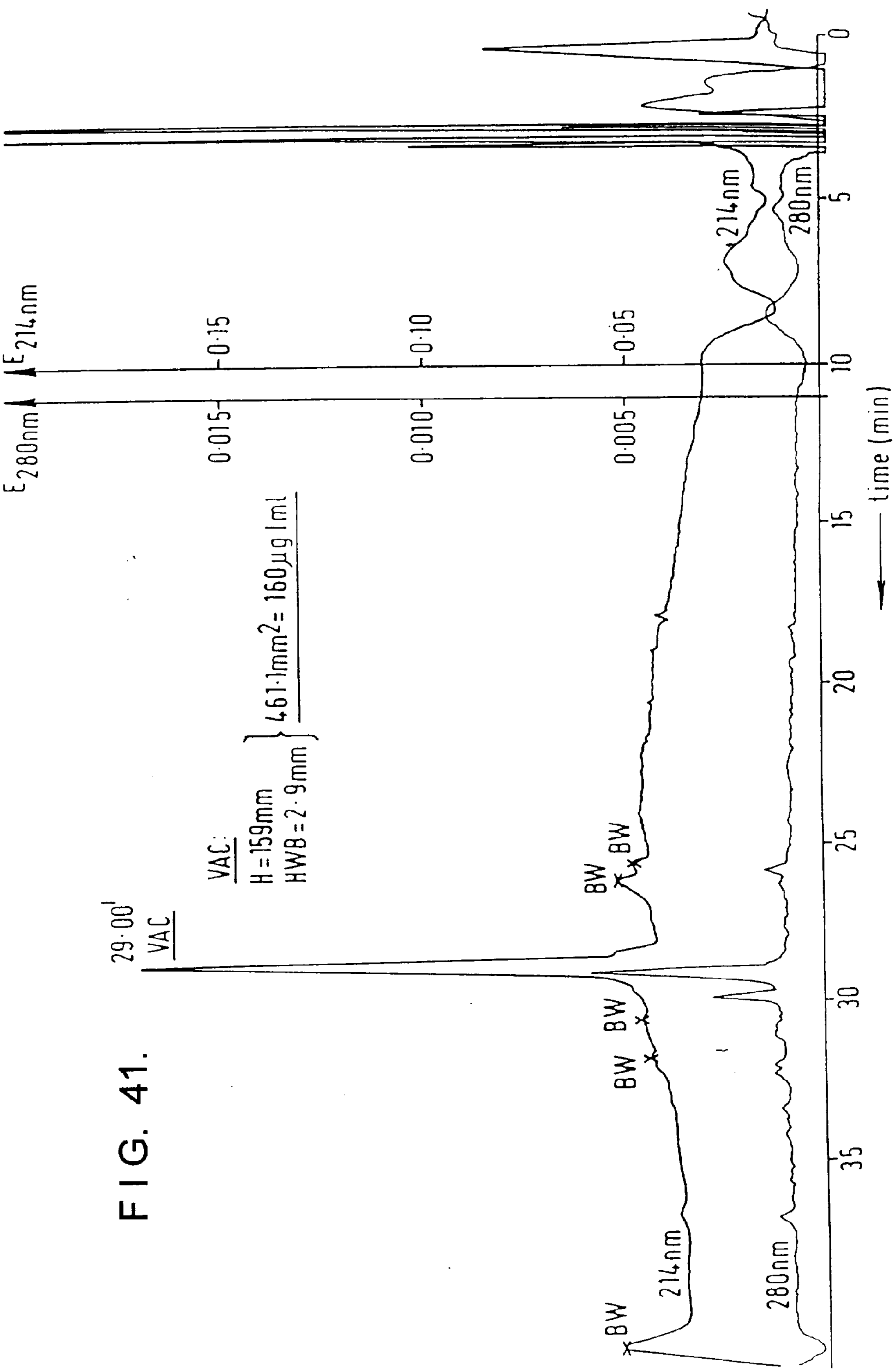
E280nm
E214nm
0·015
0·010
0·005
0·15
0·10
0·05
0
5
10
15
20
25
30
35
time (min)
214nm
280nm
29·00'
VAC
VAC:
H = 159mm
HWB = 2·9mm
461·1mm² = 160μg/ml
BW

FIG. 41.

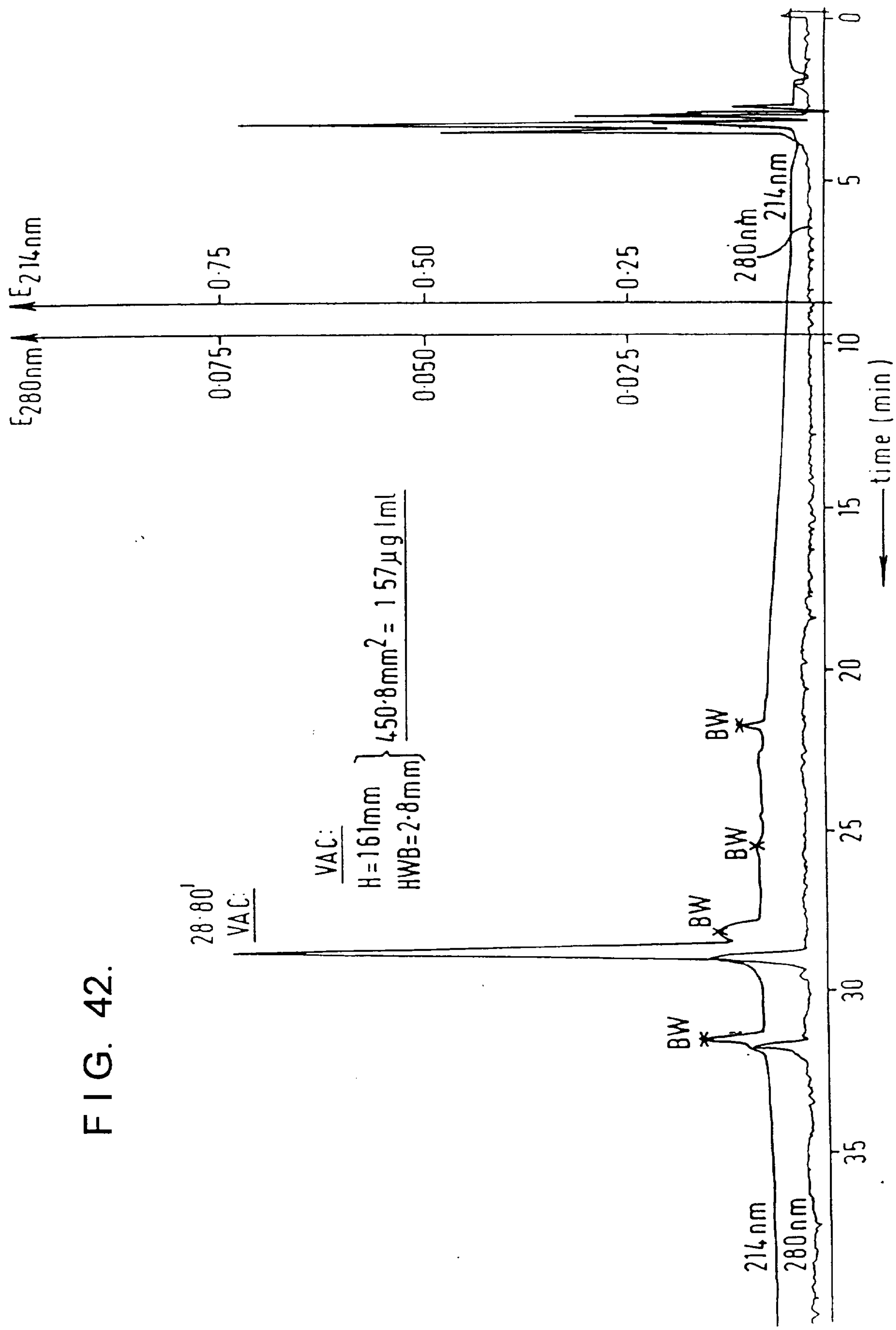
E280nm
E214nm
0·075
0·050
0·025
0·75
0·50
0·25
280nm
214nm
VAC:
H=161mm
HWB=2·8mm
450·8mm2 = 1 57μg/ml
28·80'
VAC
BW
BW
BW
BW
214nm
280nm
0
5
10
15
20
25
30
35
time (min)

FIG. 42.

FIG. 43.

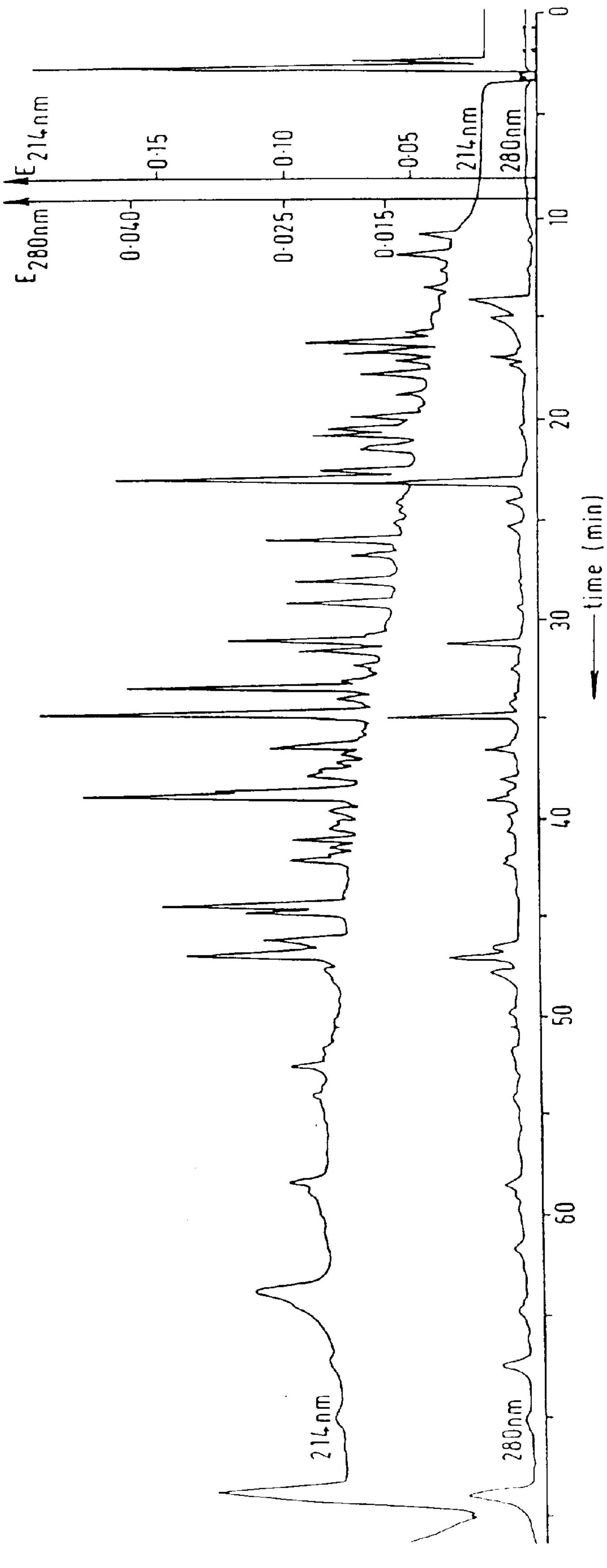
$E_{214nm}$
0·15
0·10
0·05
214nm
280nm
$E_{280nm}$
0·040
0·025
0·015
0
10
20
30
40
50
60
time (min)
214nm
280nm

FIG. 44.

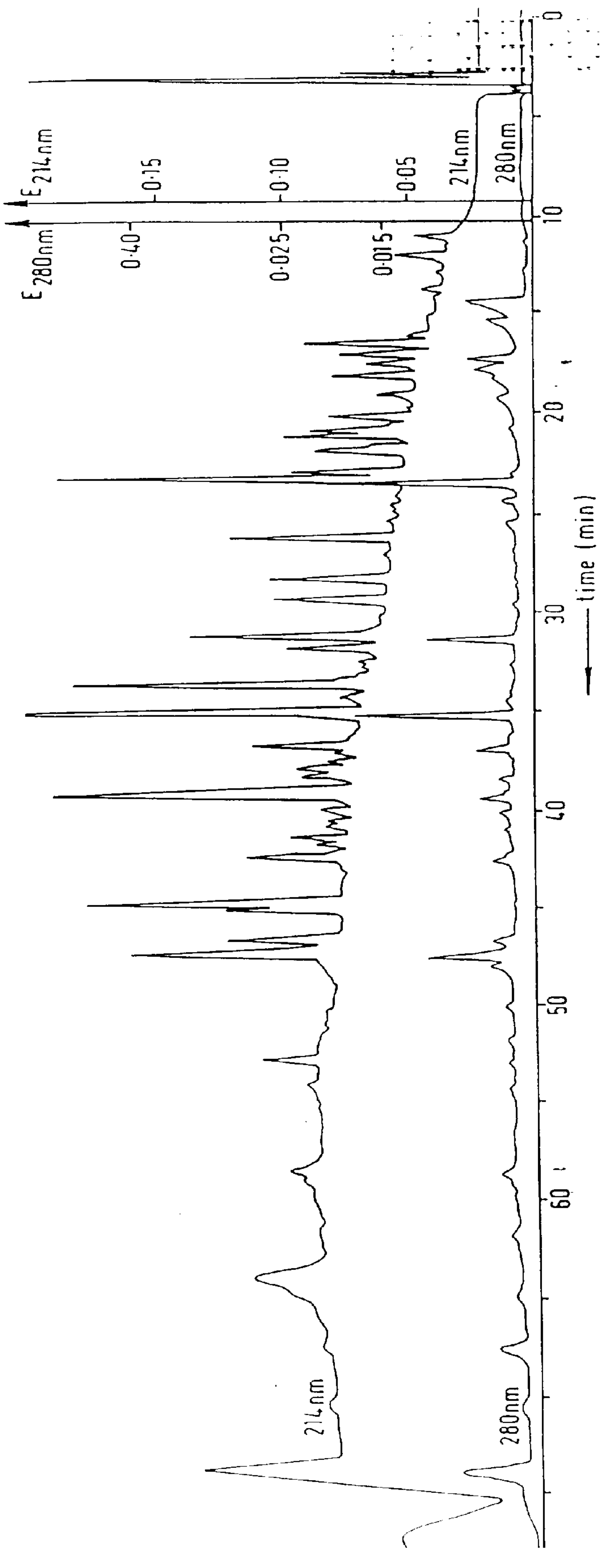
E280nm
E214nm
0·40
0·025
0·015
0·15
0·10
0·05
214nm
280nm
0
10
20
30
40
50
60
time (min)
214nm
280nm

FIG. 45.

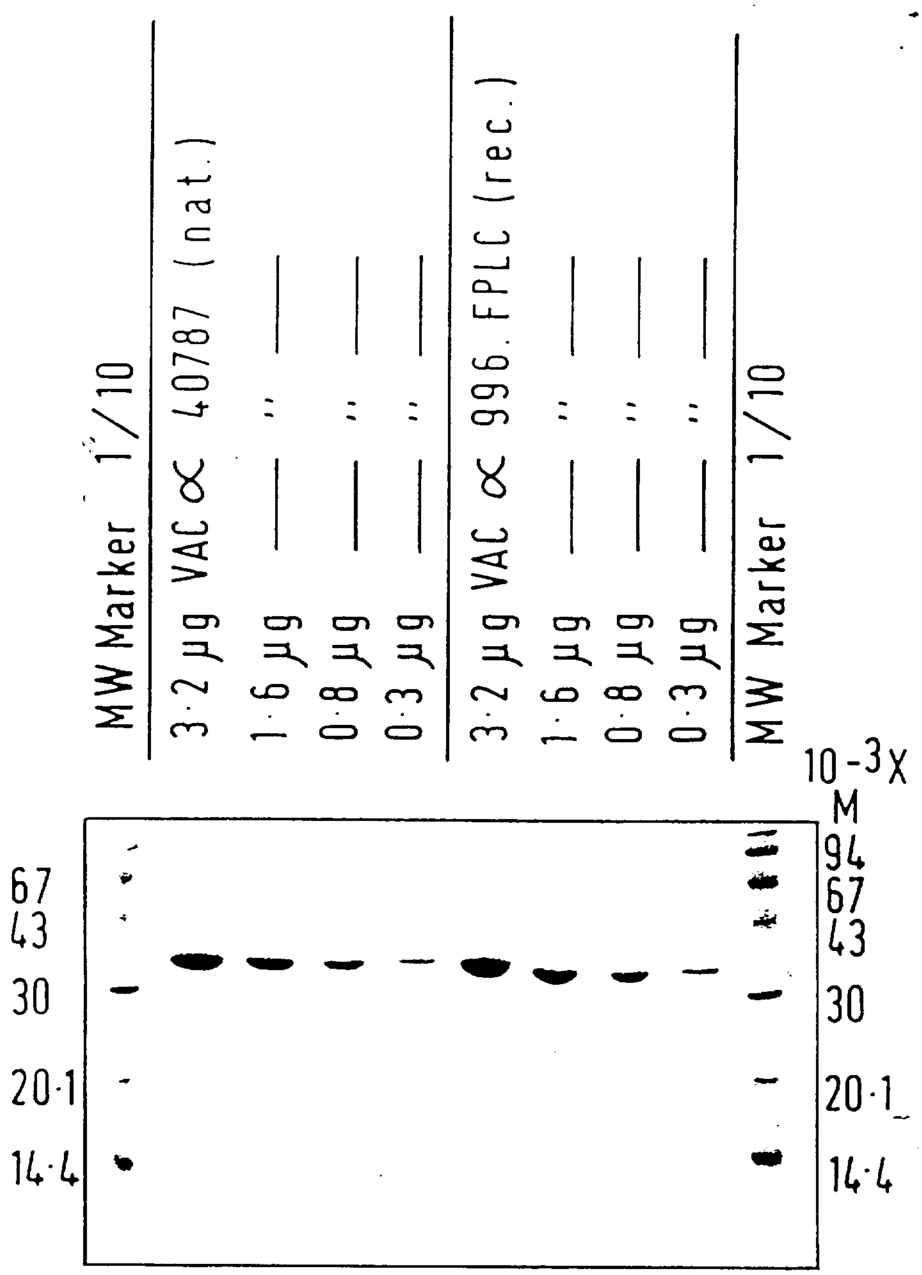


Coomassie – Blue

# FIG. 46.

15% SDS-PAGE

Gel part I: Silver staining
Gel part II: Coomassie staining

STACKING GEL

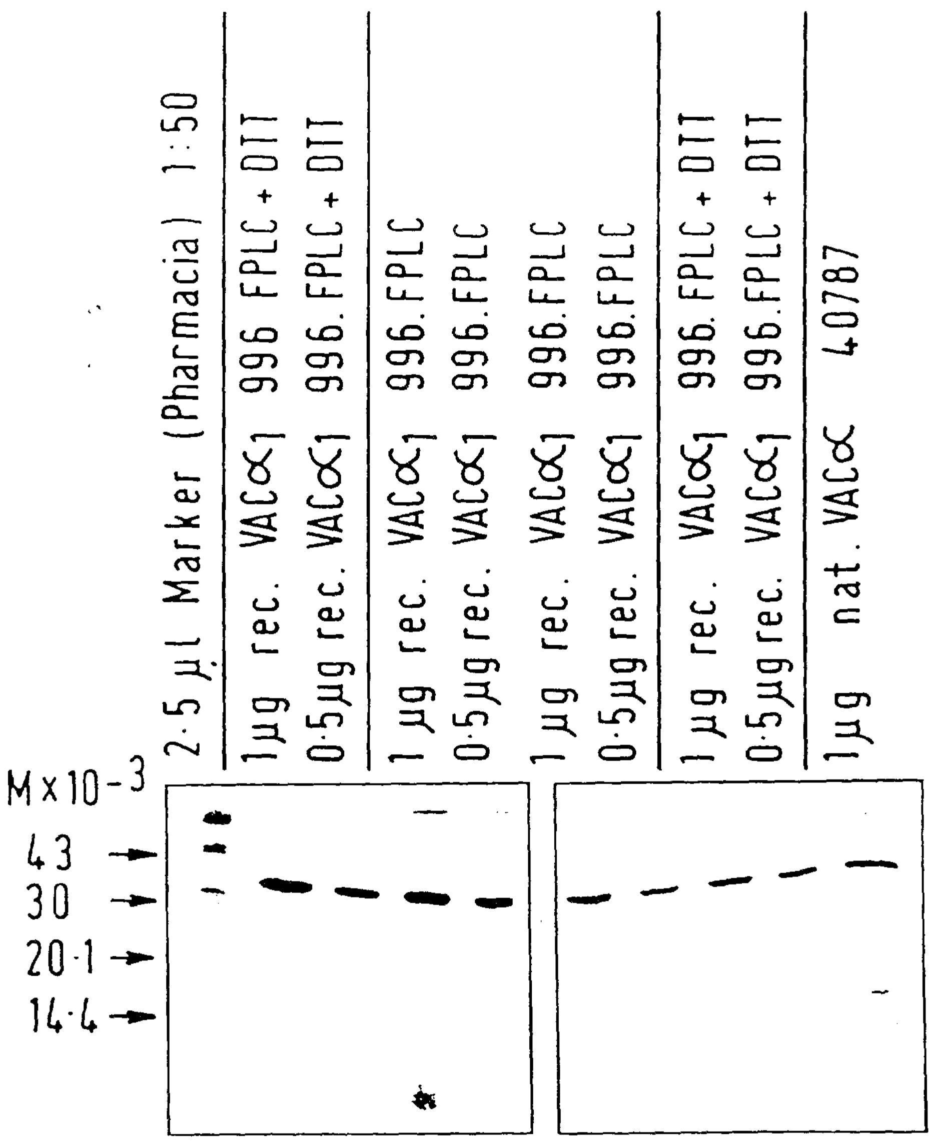


Gel part I
Silver staining

Gel part II
Coomassie staining

FIG. 47.

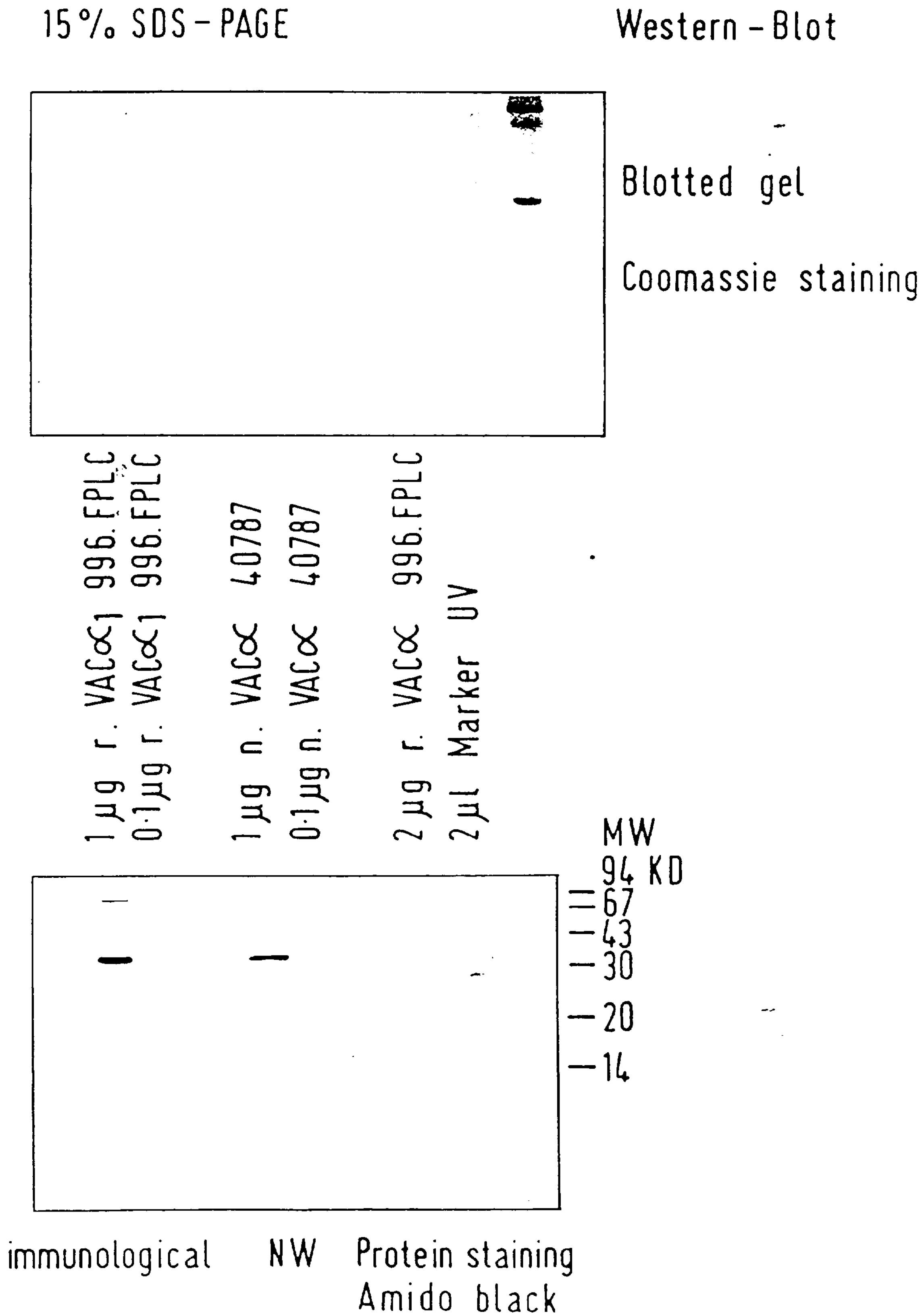
15% SDS-PAGE
Western-Blot
Blotted gel
Coomassie staining
1 µg r. VACα1 996.FPLC
0.1µg r. VACα1 996.FPLC
1 µg n. VACα 40787
0.1µg n. VACα 40787
2 µg r. VACα 996.FPLC
2µl Marker UV
MW
94 KD
67
43
30
20
14
immunological
NW
Protein staining
Amido black

FIG. 48.

LKB AMPHOLINE PAGIF pH 3·5 – 9·5 Coomassie staining

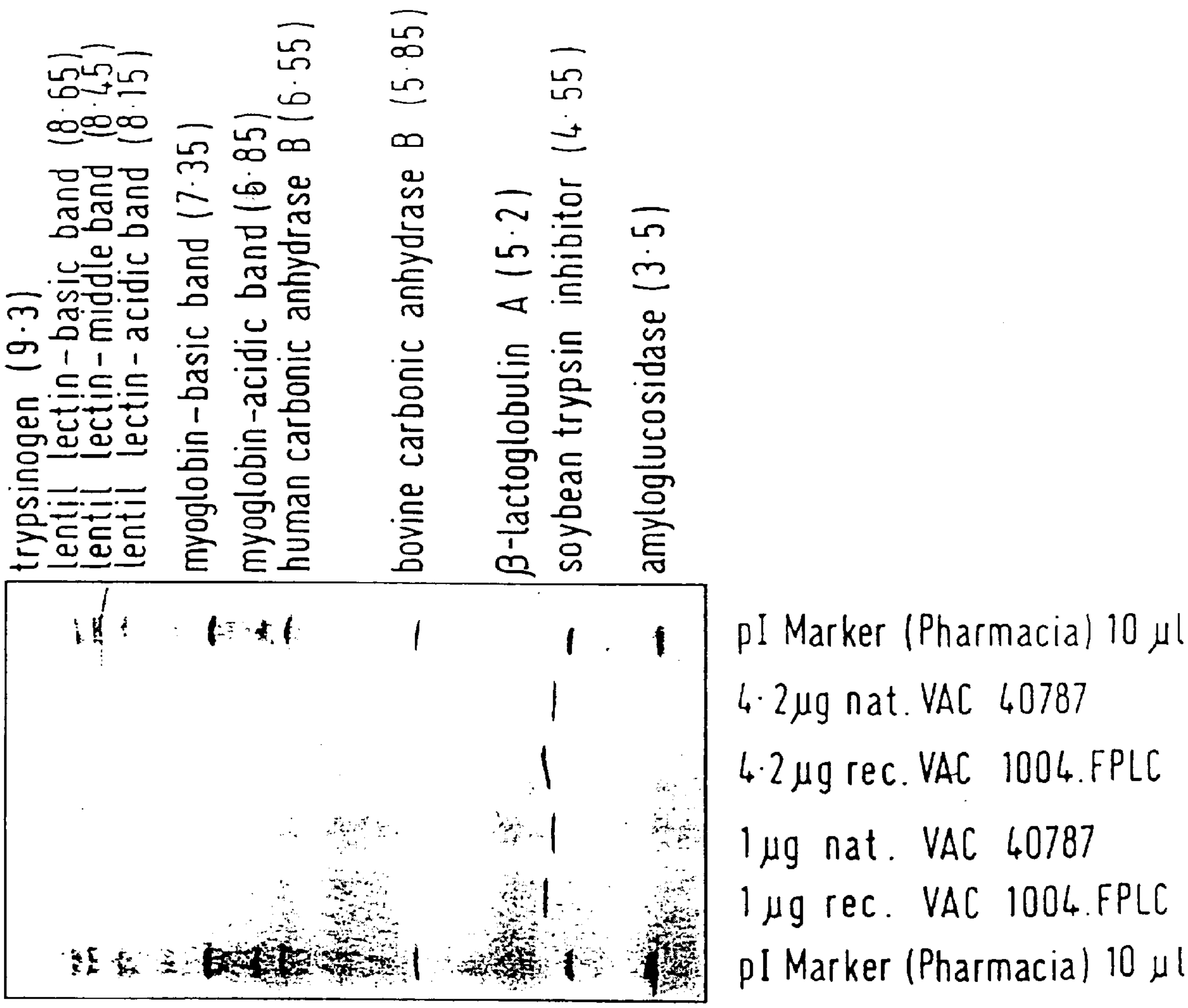

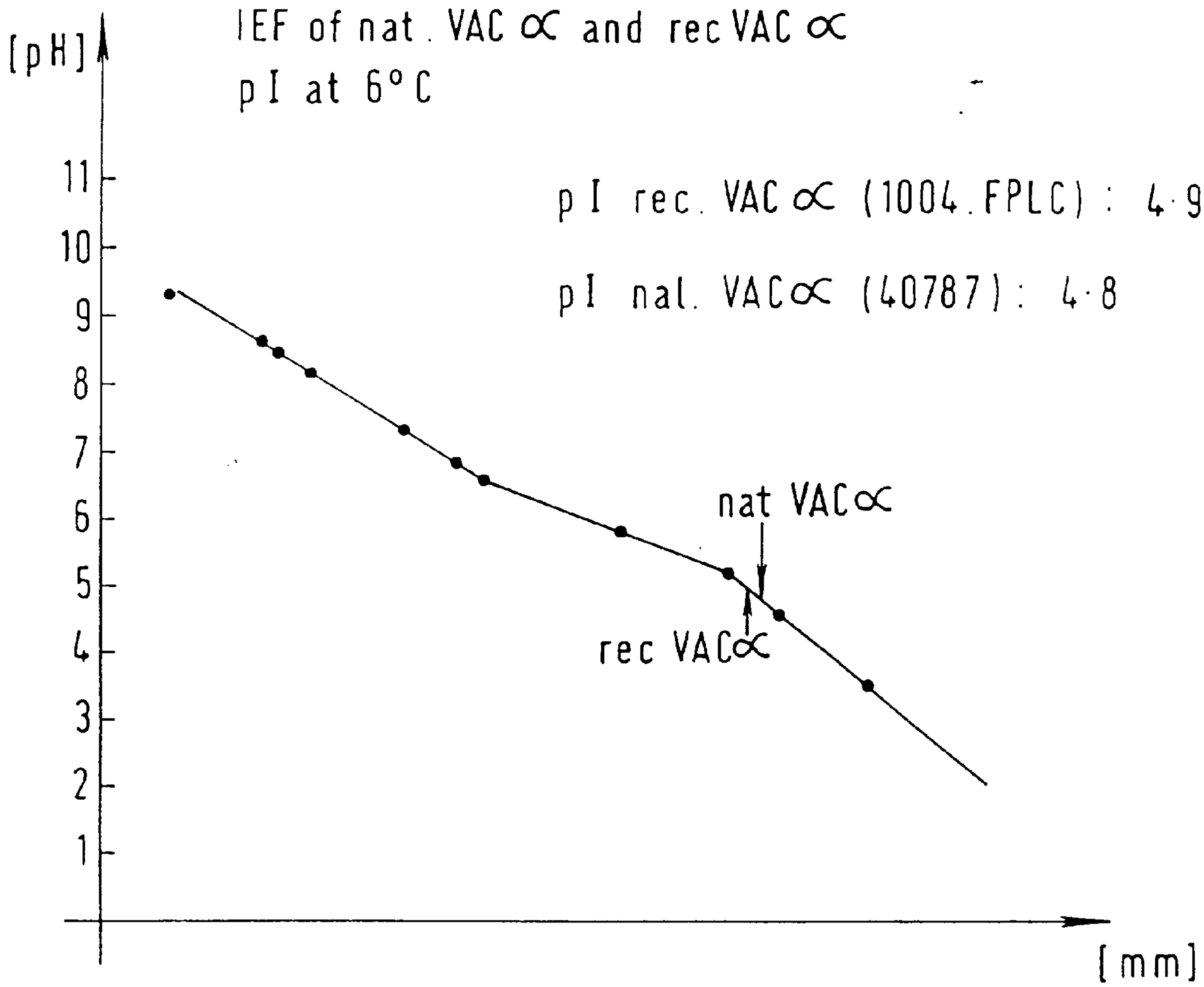

IEF of nat. VAC ∝ and rec VAC ∝
pI at 6°C
pI rec. VAC ∝ (1004.FPLC) : 4·9
pI nal. VAC∝ (40787) : 4·8
[pH]
11
10
9
8
7
6
5
4
3
2
1
nat VAC∝
rec VAC∝
[mm]


FIG. 48 a.

FIG. 49.

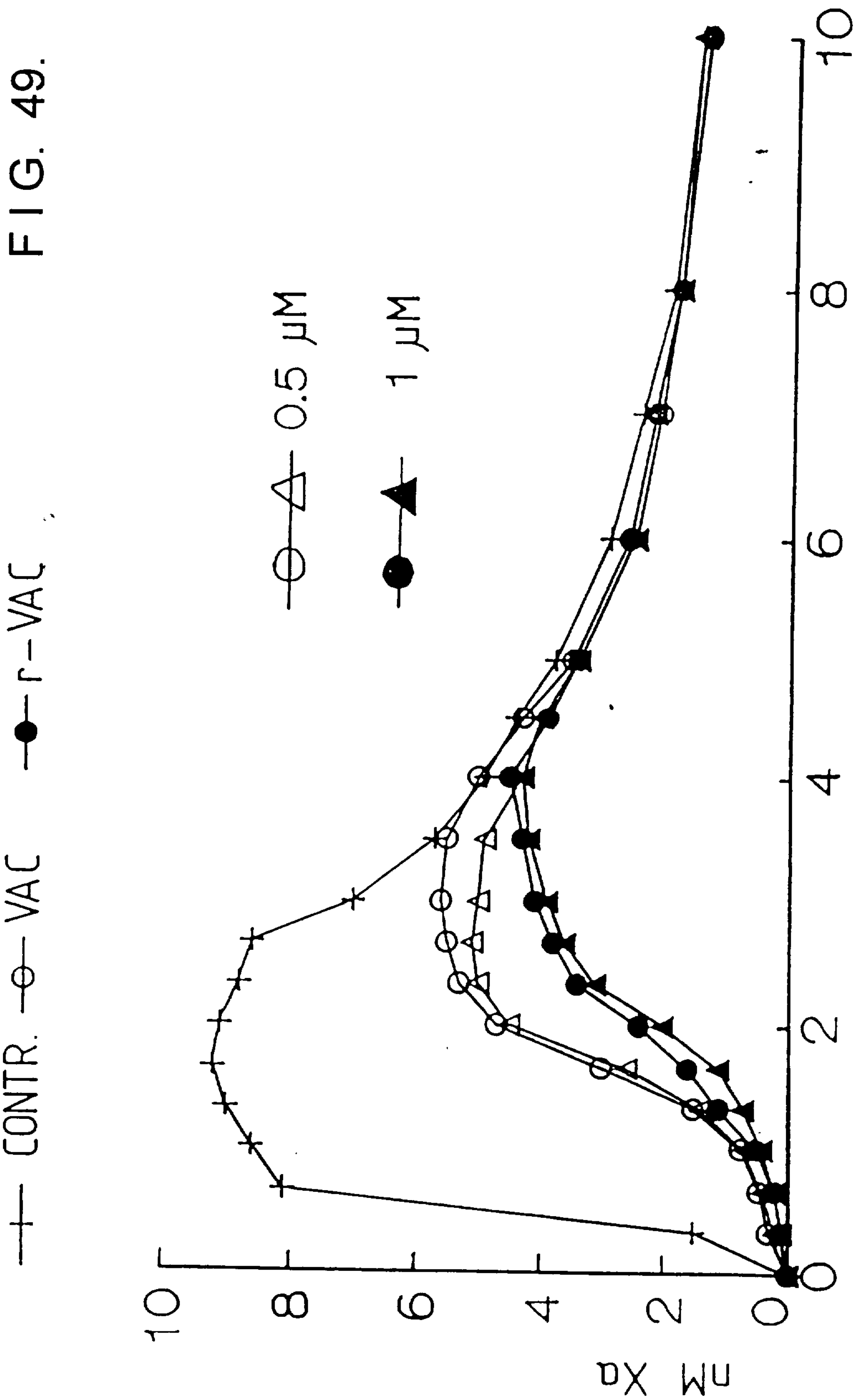

FIG. 50.

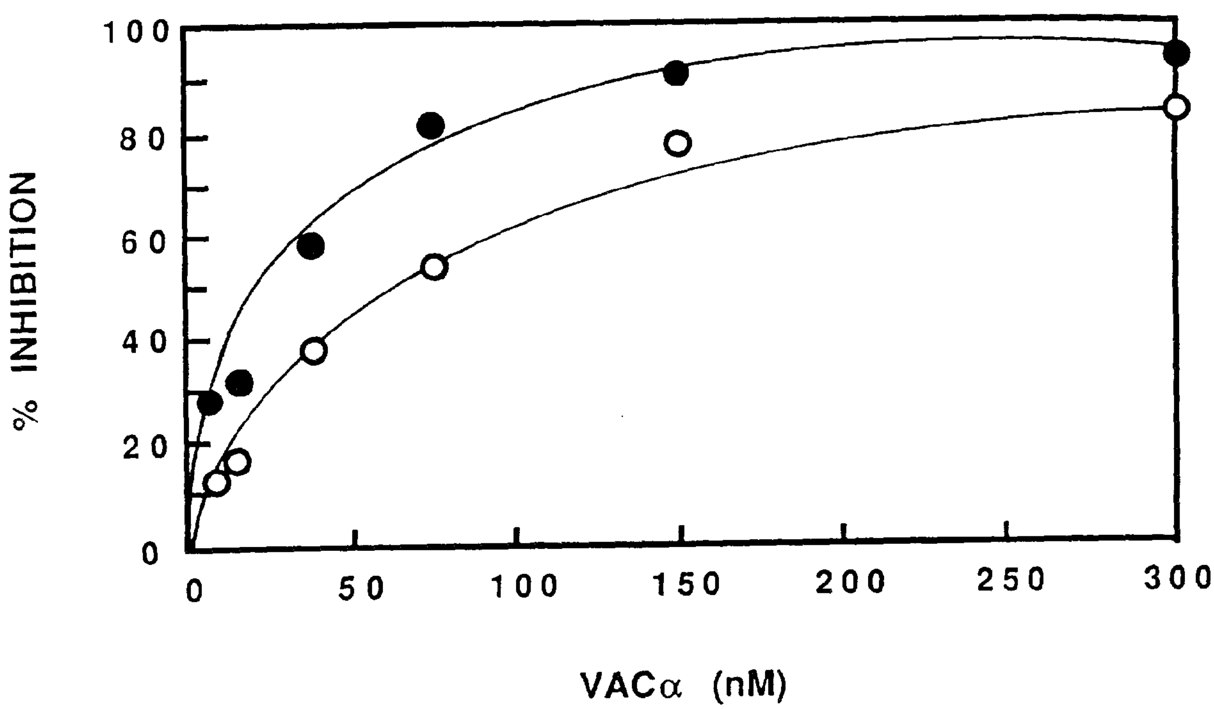

FIG. 51.

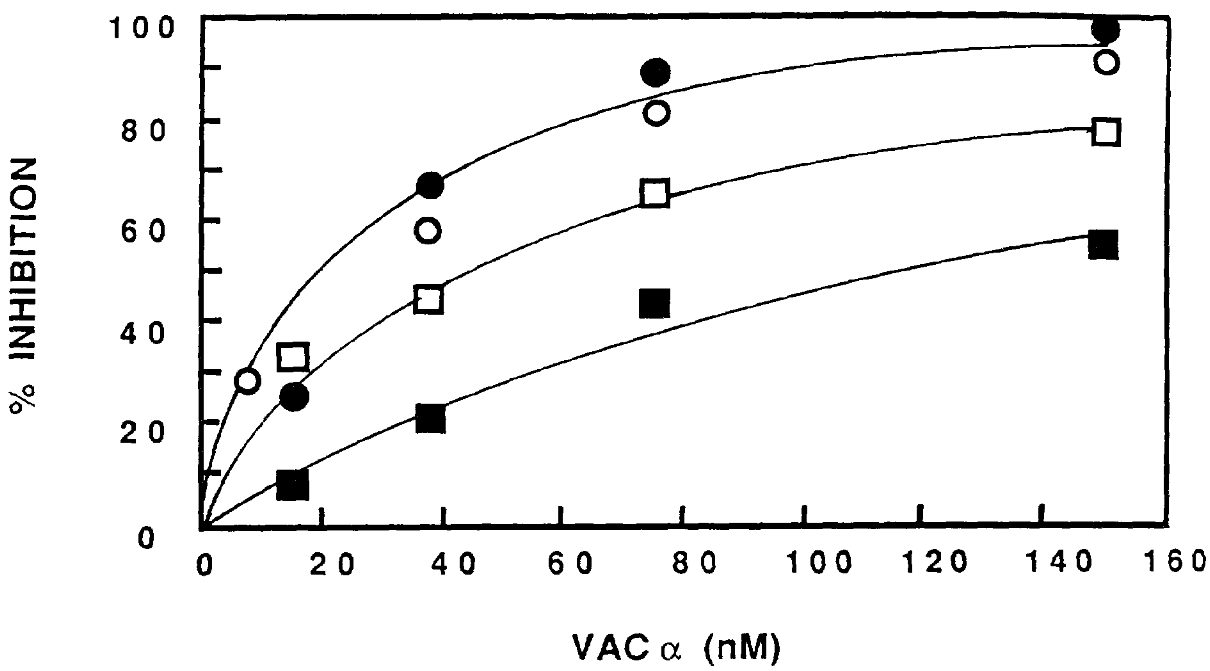

VAC-alpha cDNA

CCTGCTTCACCTTCCCCTGACCTGAGTAGTCGCT

```
1               5                   10                  15
Met Ala Gln Val Leu Arg Gly Thr Val Thr Asp Phe Pro Gly Phe
ATG GCA CAG GTT CTC AGA GGC ACT GTG ACT GAC TTC CCT GGA TTT      45

                20                  25                  30
Asp Glu Arg Ala Asp Ala Glu Thr Leu Arg Lys Ala Met Lys Gly
GAT GAG CGG GCT GAT GCA GAA ACT CTT CGG AAG GCT ATG AAA GGC      90

                35                  40                  45
Leu Gly Thr Asp Glu Glu Ser Ile Leu Thr Leu Leu Thr Ser Arg
TTG GGC ACA GAT GAG GAG AGC ATC CTG ACT CTG TTG ACA TCC CGA     135

                50                  55                  60
Ser Asn Ala Gln Arg Gln Glu Ile Ser Ala Ala Phe Lys Thr Leu
AGT AAT GCT CAG CGC CAG GAA ATC TCT GCA GCT TTT AAG ACT CTG     180

                65                  70                  75
Phe Gly Arg Asp Leu Leu Asp Asp Leu Lys Ser Glu Leu Thr Gly
TTT GGC AGG GAT CTT CTG GAT GAC CTG AAA TCA GAA CTA ACT GGA     225

                80                  85                  90
Lys Phe Glu Lys Leu Ile Val Ala Leu Met Lys Pro Ser Arg Leu
AAA TTT GAA AAA TTA ATT GTG GCT CTG ATG AAA CCC TCT CGG CTT     270

                95                  100                 105
Tyr Asp Ala Tyr Glu Leu Lys His Ala Leu Lys Gly Ala Gly Thr
TAT GAT GCT TAT GAA CTG AAA CAT GCC TTG AAG GGA GCT GGA ACA     315

                110                 115                 120
Asn Glu Lys Val Leu Thr Glu Ile Ile Ala Ser Arg Thr Pro Glu
AAT GAA AAA GTA CTG ACA GAA ATT ATT GCT TCA AGG ACA CCT GAA     360

                125                 130                 135
Glu Leu Arg Ala Ile Lys Gln Val Tyr Glu Glu Glu Tyr Gly Ser
GAA CTG AGA GCC ATC AAA CAA GTT TAT GAA GAA GAA TAT GGC TCA     405

                140                 145                 150
Ser Leu Glu Asp Asp Val Val Gly Asp Thr Ser Gly Tyr Tyr Gln
AGC CTG GAA GAT GAC GTG GTG GGG GAC ACT TCA GGG TAC TAC CAG     450
```

FIG. 52/1.

```
                155                     160                     165
Arg Met Leu Val Val Leu Leu Gln Ala Asn Arg Asp Pro Asp Ala
CGG ATG TTG GTG GTT CTC CTT CAG GCT AAC AGA GAC CCT GAT GCT   495

                170                     175                     180
Gly Ile Asp Glu Ala Gln Val Glu Gln Asp Ala Gln Ala Leu Phe
GGA ATT GAT GAA GCT CAA GTT GAA CAA GAT GCT CAG GCT TTA TTT   540

                185                     190                     195
Gln Ala Gly Glu Leu Lys Trp Gly Thr Asp Glu Glu Lys Phe Ile
CAG GCT GGA GAA CTT AAA TGG GGG ACA GAT GAA GAA AAG TTT ATC   585

                200                     205                     210
Thr Ile Phe Gly Thr Arg Ser Val Ser His Leu Arg Lys Val Phe
ACC ATC TTT GGA ACA CGA AGT GTG TCT CAT TTG AGA AAG GTG TTT   630

                215                     220                     225
Asp Lys Tyr Met Thr Ile Ser Gly Phe Gln Ile Glu Glu Thr Ile
GAC AAG TAC ATG ACT ATA TCA GGA TTT CAA ATT GAG GAA ACC ATT   675

                230                     235                     240
Asp Arg Glu Thr Ser Gly Asn Leu Glu Gln Leu Leu Leu Ala Val
GAC CGC GAG ACT TCT GGC AAT TTA GAG CAA CTA CTC CTT GCT GTT   720

                245                     250                     255
Val Lys Ser Ile Arg Ser Ile Pro Ala Tyr Leu Ala Glu Thr Leu
GTG AAA TCT ATT CGA AGT ATA CCT GCC TAC CTT GCA GAG ACC CTC   765

                260                     265                     270
Tyr Tyr Ala Met Lys Gly Ala Gly Thr Asp Asp His Thr Leu Ile
TAT TAT GCT ATG AAG GGA GCT GGG ACA GAT GAT CAT ACC CTC ATC   810

                275                     280                     285
Arg Val Met Val Ser Arg Ser Glu Ile Asp Leu Phe Asn Ile Arg
AGA GTC ATG GTT TCC AGG AGT GAG ATT GAT CTG TTT AAC ATC AGG   855

                290                     295                     300
Lys Glu Phe Arg Lys Asn Phe Ala Thr Ser Leu Tyr Ser Met Ile
AAG GAG TTT AGG AAG AAT TTT GCC ACC TCT CTT TAT TCC ATG ATT   900

                305                     310                     315
Lys Gly Asp Thr Ser Gly Asp Tyr Lys Lys Ala Leu Leu Leu Leu
AAG GGA GAT ACA TCT GGG GAC TAT AAG AAA GCT CTT CTG CTG CTC   945

                320
Cys Gly Glu Asp Asp  *
TGT GGA GAA GAT GAC TAA CGTGTCACGGGGAAGAGCTCCCTGCTGTGTGCCTG   998
```

F I G. 52/2.

CACCACCCCACTGCCTTCCTTCAGCACCTTTAGCTGCATTTGTATGCCAGTGCTTAACA 1057

CATTGCCTTATTCATACTAGCATGCTCATGACCAACACATACACGTCATAGAAGAAAAT 1116

AGTGGTGCTTCTTTCTGATCTCTAGTGGAGATCTCTTTGACTGCTGTAGTACTAAAGTG 1175

TACTTAATGTTACTAAGTTTAATGCCTGGCCATTTTCCATTTATATATATTTTTTAAGA 1234

GGCTAGAGTGCTTTTAGCCTTTTTTAAAAACTCCATTTATATTACATTTGTAACCATGA 1293

TACTTTAATCAGAAGCTTAGCCTTGAAATTGTGAACTCTTGGAAATGTTATTAGTGAAG 1352

TTCGCAACTAAACTAAACCTGTAAAATTATGATGATTGTATTCAAAAGATTAATGAAAA 1411

ATAAACATTTCTGTCCCCCTG-polyA 1437

FIG. 52/3.

FIG. 53.

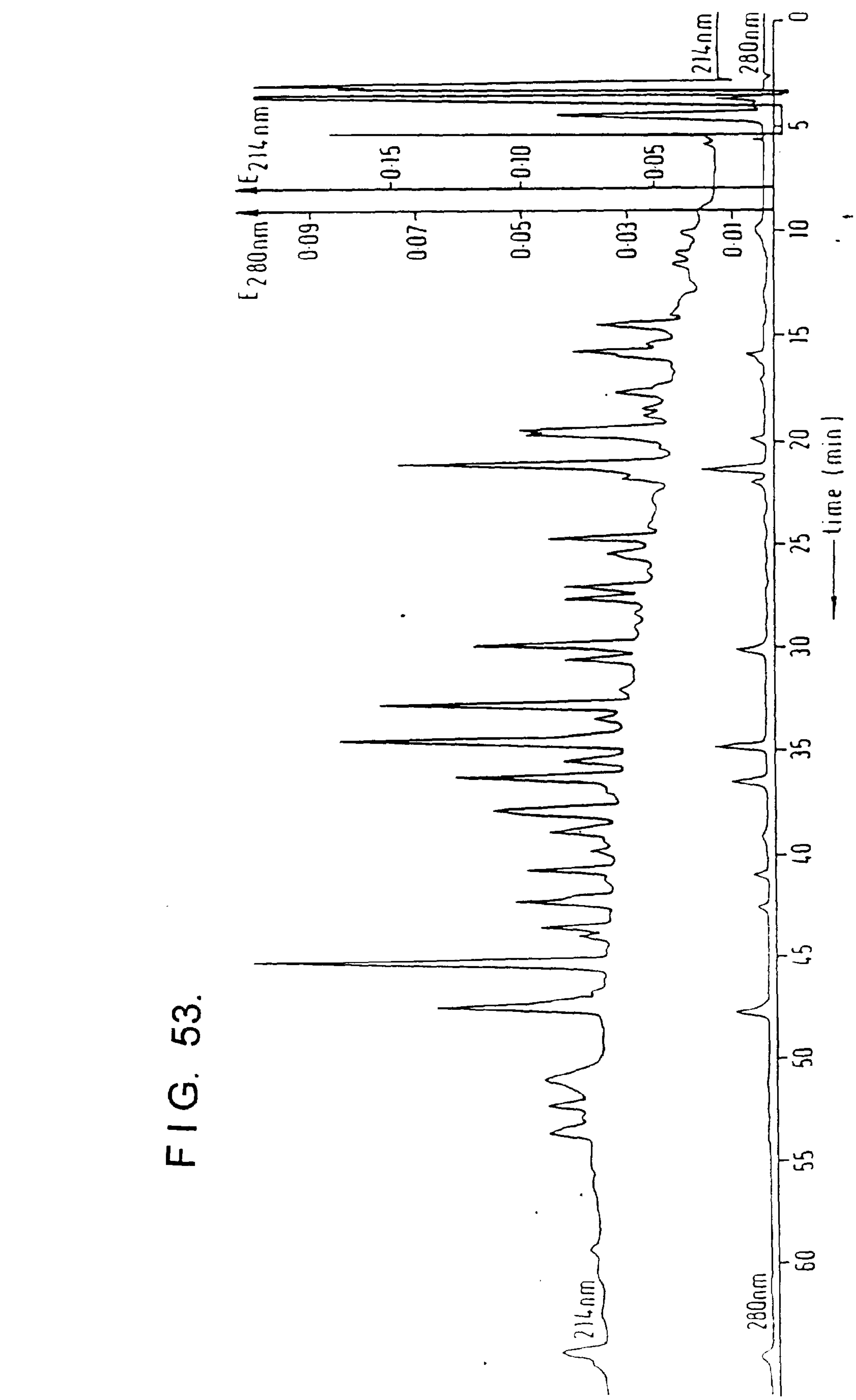
E280nm
E214nm
0·09
0·07
0·05
0·03
0·01
0·15
0·10
0·05
214nm
280nm
0
5
10
15
20
25
30
35
40
45
50
55
60
time (min)
214nm
280nm

FIG. 54.

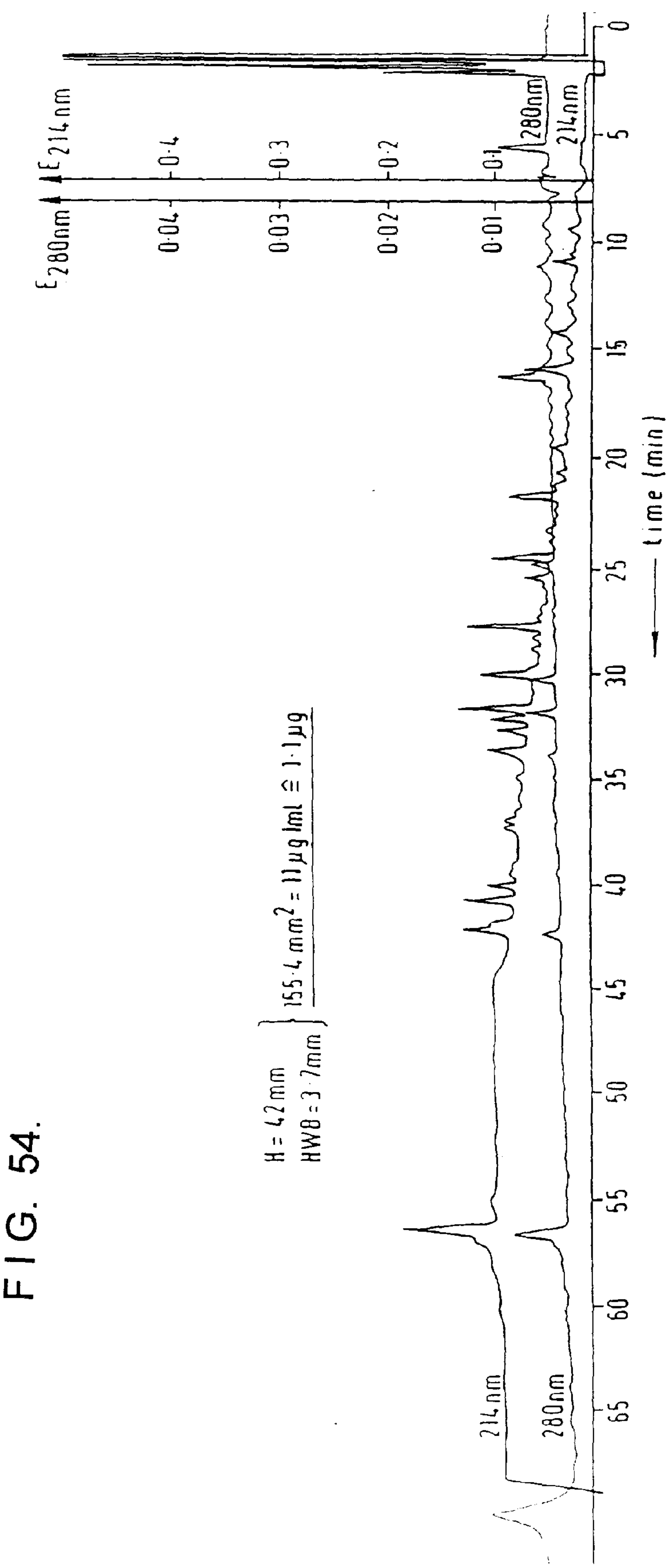
E280nm
E214nm
0·04
0·03
0·02
0·01
0·4
0·3
0·2
0·1
280nm
214nm
H = 42mm
HWB = 3·7mm
155·4 mm² = 11 μg/ml ≙ 1·1 μg
214nm
280nm
0
5
10
15
20
25
30
35
40
45
50
55
60
65
time (min)

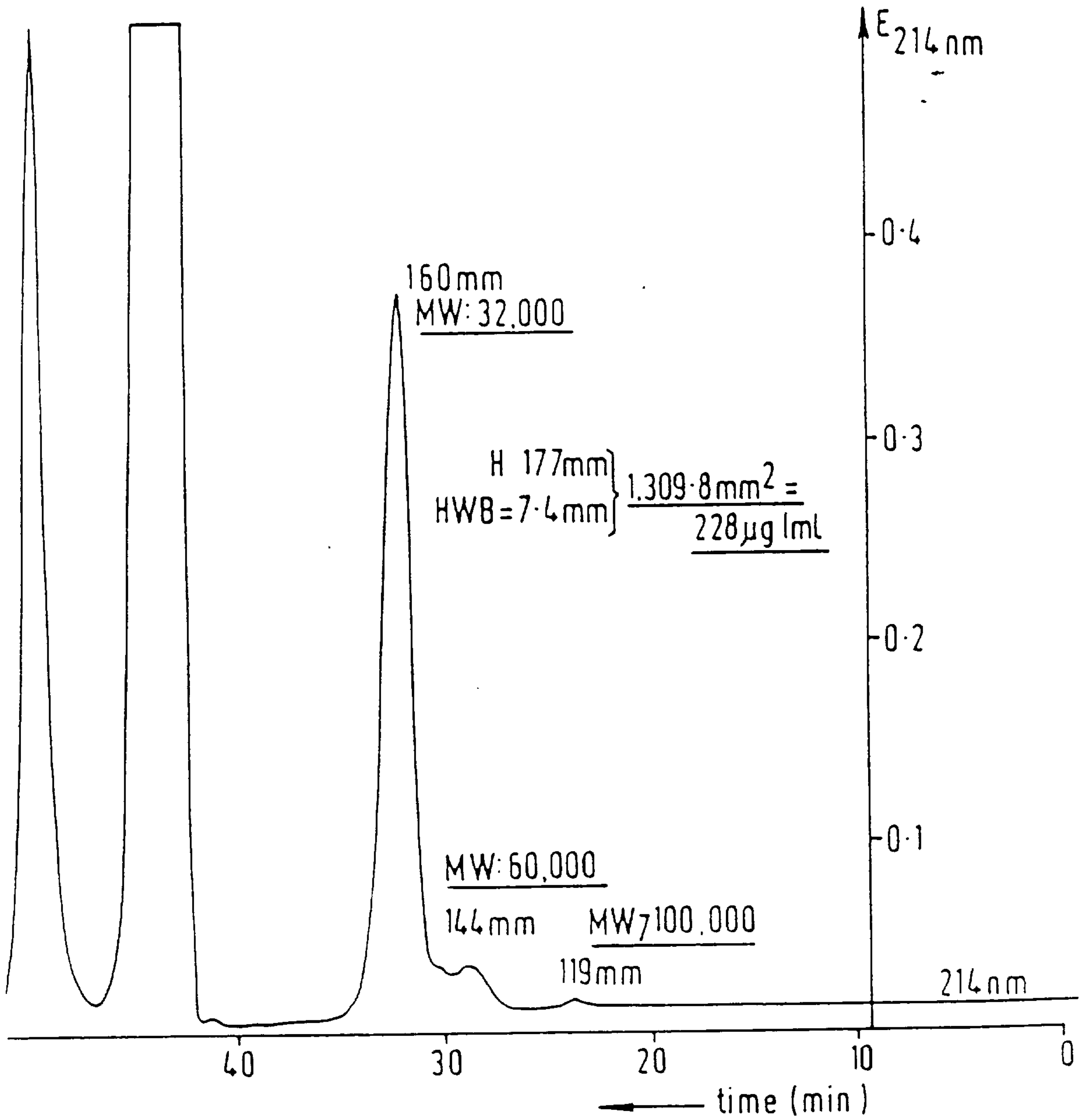
E 214 nm
0·4
0·3
0·2
0·1
160mm
MW: 32,000
H 177mm
HWB = 7·4mm
1,309·8mm2 =
228µg lml
MW: 60,000
144mm
MW7100,000
119mm
214nm
40
30
20
10
0
time (min)

F I G. 55/1.

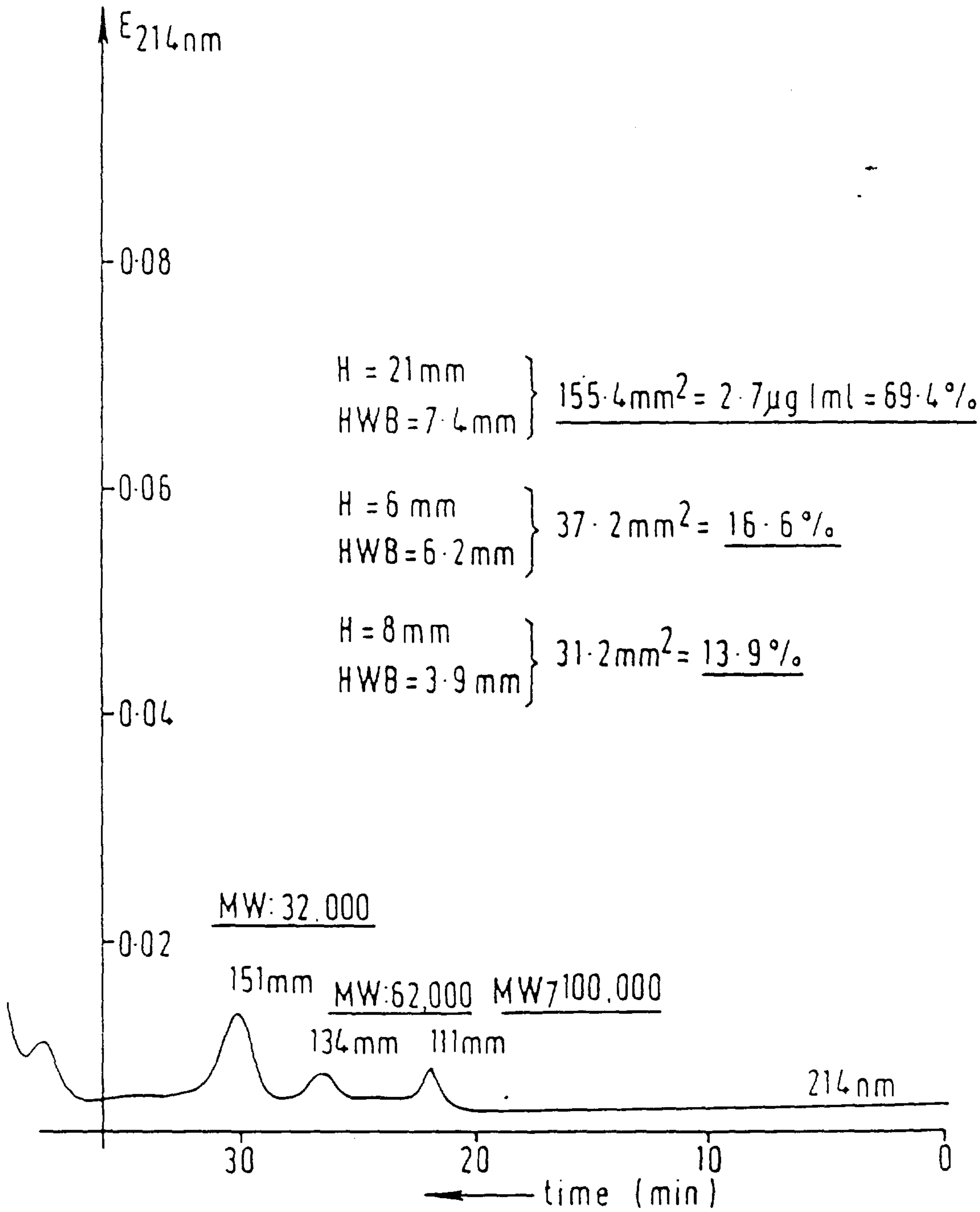
E214nm
0·08
0·06
0·04
0·02
H = 21mm
HWB = 7·4mm
155·4mm² = 2·7µg/ml = 69·4%
H = 6 mm
HWB = 6·2mm
37·2mm² = 16·6%
H = 8mm
HWB = 3·9mm
31·2mm² = 13·9%
MW: 32.000
151mm
MW: 62,000
MW7100,000
134mm
111mm
214nm
30
20
10
0
time (min)

F I G. 55/2.

FIG. 56.

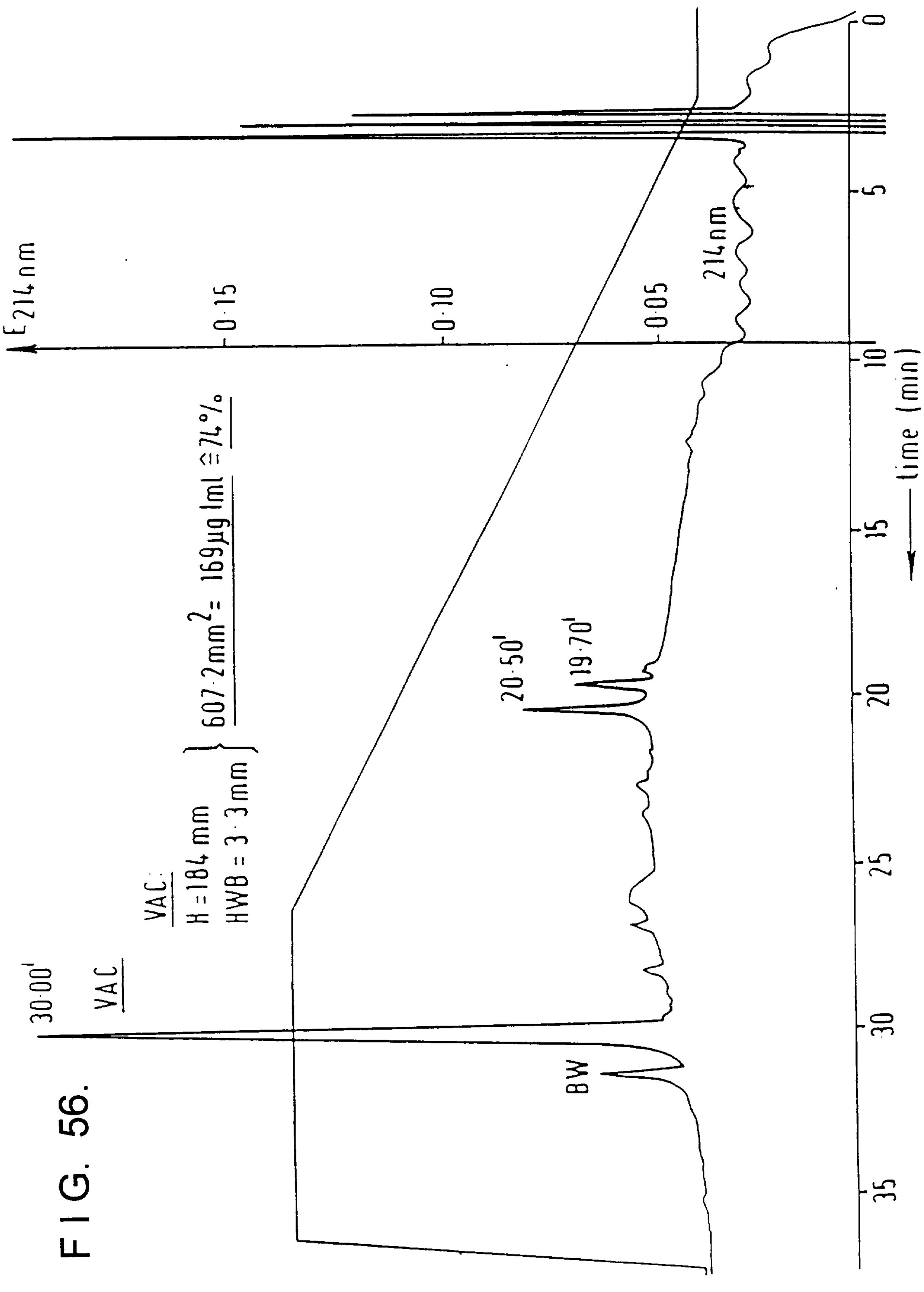

E214 nm
0·15
0·10
0·05
214nm
time (min)
0
5
10
15
20
25
30
35
30·00'
VAC
VAC:
H = 184 mm
HWB = 3·3 mm
607·2mm² = 169µg/ml ≙ 74%
20·50'
19·70'
BW

FIG. 57.

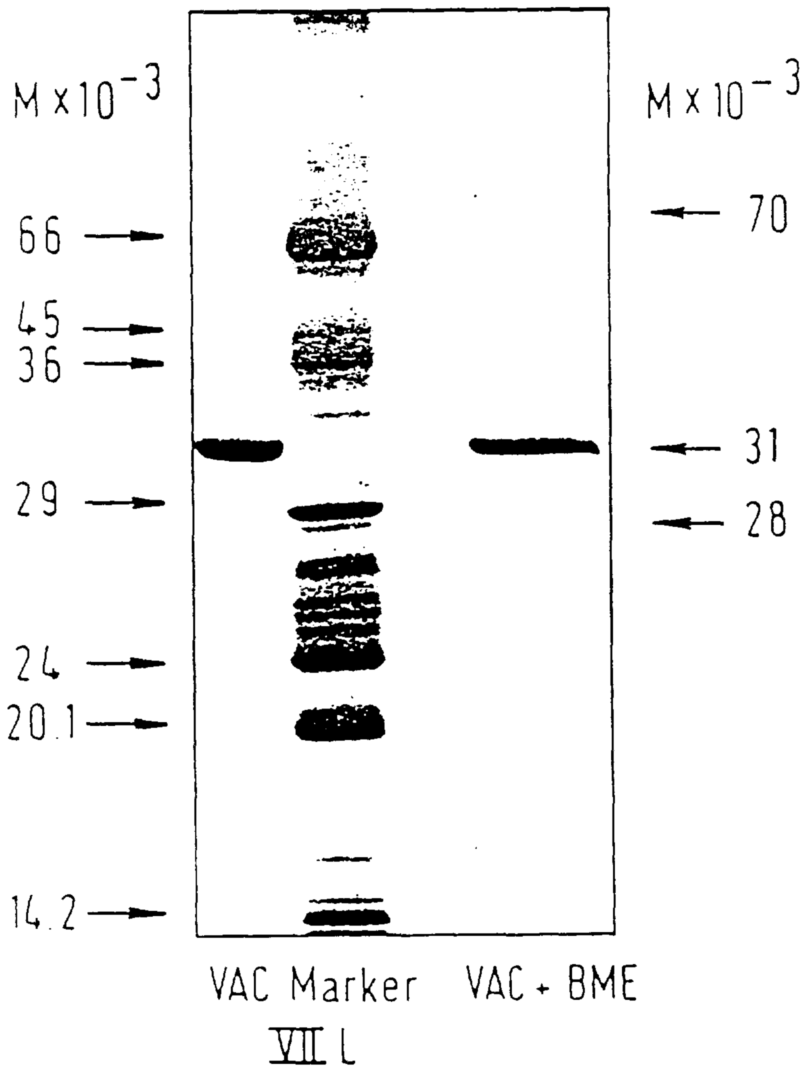
M x 10$^{-3}$
66
45
36
29
24
20.1
14.2
M x 10$^{-3}$
70
31
28
VAC Marker VAC + BME
VII L